# EUROPEAN PATENT APPLICATION

(11) **EP 3 677 693 A1**
(43) Date of publication of application: **08.07.2020**
(21) Application number: 19150151.9
(22) Date of filing: 03.01.2019
(51) Int. Cl.: C12Q 1/6886

(54) **TRANSPOCHIMERIC GENE TRANCRIPTS (TCGTS) AS CANCER BIOMARKERS**

(71) Applicant: Ecole Polytechnique Federale De Lausanne (EPFL) EPFL-TTO, 1015 Lausanne (CH); Aarhus University, 8000 Aarhus C (DK)
(72) Inventor: TRONO, Didier, 1134 Vufflens-Chateau (CH); SIMO RIUDALBAS, Laia, 1004 Lausanne (CH); PLANET LETSCHERT, Evarist, 1113 ST-Saphorin-sur-Morges (CH); DUC, Julien, 1004 Lausanne (CH)
(74) Representative: KATZAROV S.A.

(57) **Abstract**

The invention relates to methods for detecting or predicting the likelihood of a cancer in a subject as well as to compositions, pharmaceutical compositions and methods of treatment of cancer.

## Description

### FIELD OF THE INVENTION

The invention relates to methods for detecting or predicting the likelihood of a cancer in a subject as well as to compositions, pharmaceutical compositions and methods of treatment of cancer.

### BACKGROUND OF THE INVENTION

Overall, current cancer drugs are inefficient in close to 75% of patients, and it is increasingly recognized that the future of cancer management lies in personalized approaches, whereby therapies are selected not just based on a tumor's organ of origin but rather on a precise characterization of its molecular make-up and of the patient's genetic background. New methods are needed to discover novel therapeutic targets as well as biomarkers for staging tumors, predicting their sensitivity to particular treatments, monitoring therapeutic responses and detecting relapses as early as possible.

A newly available signature for an in-depth characterization of healthy and tumoral human cells is the expression pattern of transposable elements (TEs). TEs, some 4.5 millions of which can be readily identified in the human genome, may account for up to 80% of our DNA, hence are the repository of a major part of our genetic heritage. TEs have been linked to cancer through different mechanisms, such as insertional activation of oncogenes and disruption of tumor suppressor genes, chromosomal rearrangements, or TE-gene chimeric transcripts that activate oncogenes. Particular TEs have been found overexpressed in cancer but, due to a lack of proper technologies, a comprehensive characterization of their global expression patterns in human cancer is far from complete. The use of improved DNA and RNA sequencing (RNA-seq) methodology combined with in-house optimized bioinformatics pipelines has allowed us to define and start deciphering the TE-derived transcriptome in healthy and cancerous human tissues.
Colorectal cancer (CRC) has been the focus of our studies because its treatment is an unmet clinical need and its step-wise development has been extremely well documented.
Furthermore, samples of tumor and healthy tissues are systematically obtained during surgery. CRC is the third most common cancer in men, the second in women and it represents almost a 10% of the annual global cancer incidence. 90% of new cases occur in people above 50 years and the risk of developing colon cancer is 3 times greater in cases with a family history. The preventable risk factors of CRC include consumption of processed meat, consumption of alcoholic beverages, tobacco smoking, and excess of body fat, whereas consumption of dietary fiber and diary products and increased levels of physical activity decrease the risk. CRC screening can prevent the disease through the detection and removal of precancerous lesions and detect cancer at an early stage, when treatment is usually more successful. Current CRC therapy is based on surgery often accompanied by radio- and chemotherapy. Although advances in therapy over the last 20 years have led to improvements in survival, 50% of all CRC patients die from metastatic disease. In this application, we aimed to fill this uncovered clinical need by exploiting the information yielded by the TEs in our genomes.

### SUMMARY OF THE INVENTION

This object has been achieved by providing a method for detecting and/or predicting the likelihood of a cancer in a subject, the method comprising detecting, directly or indirectly, in a biological sample of said subject, the presence or absence of at least one nucleic acid sequence comprising
i) at least one transposable element (TE) a fragment or variant thereof, and
ii) a gene sequence, a fragment or variant thereof,
wherein the at least one additional nucleic acid is selected from the group listed in Table 1 and/or Table 2, or a variant or fragment thereof.

A further object of the present invention is to provide a method for classifying a cancer subject, the method comprising
i) detecting, directly or indirectly, in a biological sample of said subject, the presence or absence of at least one nucleic acid sequence comprising at least one transposable element (TE) or a fragment thereof, and a gene sequence, a fragment or variant thereof, wherein the at least one additional nucleic acid is selected from the group listed in Table 1 and/or Table 2 or a variant or fragment thereof and
ii) classifying the cancer subject
   - as having a favourable prognosis based on the absence of the at least one nucleic acid sequence in the biological sample or
   - as having a poor prognosis based on the presence of the at least one nucleic acid sequence in the biological.

A further object of the present invention is to provide a method for classifying a cancer subject, the method comprising
1) detecting, directly or indirectly, in a biological sample of said subject, the presence or absence of
   - at least one nucleic acid sequence correlating with an increase of survival (POSITIVE PROGNOSIS), said nucleic acid sequence comprising at least one transposable element (TE) a fragment or variant thereof, and a gene sequence selected from the group comprising CSNK1G2, LPIN1 and YIPF4, a fragment or variant thereof, and
   - at least one nucleic acid sequence correlating with a decrease of survival (NEGATIVE PROGNOSIS), said nucleic acid sequence comprising at least one transposable element (TE) a fragment or variant thereof, and a gene sequence selected from the group comprising POU5F1B, AHRR, AKAP7, MCUR1, PPP1R16A, SEC24B, SNED1 and ZNF558, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS ),
   wherein the
   - number of protective events > number of harmful events is indicative of increase in survival (POSITIVE PROGNOSIS).
   - number of harmful events > number of protective events is indicative of decrease in survival (NEGATIVE PROGNOSIS).

A further object of the present invention is to provide a method for classifying a cancer subject, the method comprising
1) detecting, directly or indirectly, in a biological sample of said subject, the presence or absence of
   - at least one nucleic acid sequence correlating with an increase of survival (POSITIVE PROGNOSIS), said nucleic acid sequence comprising at least one transposable element (TE) a fragment or variant thereof, and a gene sequence selected from the group comprising CAPN1, IGJ and TMEM131, a fragment or variant thereof, and
   - at least one nucleic acid sequence correlating with a decrease of survival (NEGATIVE PROGNOSIS), said nucleic acid sequence comprising at least one transposable element (TE) a fragment or variant thereof, and a gene sequence selected from the group comprising ADTRP, CAST, CNOT1, DZIP1, PEX13 and TMEM248, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS),
   wherein the
   - number of protective events > number of harmful events is indicative of increase in survival (POSITIVE PROGNOSIS), and
   - number of harmful events > number of protective events is indicative of decrease in survival (NEGATIVE PROGNOSIS).

A further object of the present invention is to provide a method for staging a cancer in a subject, the method comprising measuring, directly or indirectly, in a biological sample of said subject, the level of expression or activity of at least one nucleic acid sequence and comparing said level of expression or activity to a control value or range, wherein said at least one nucleic acid sequence comprises
i) at least one transposable element (TE) a fragment or variant thereof, and
ii) a gene sequence, a fragment or variant thereof,
wherein the at least one additional nucleic acid is selected from the group listed in Table 1 and/or Table 2, or a variant or fragment thereof..

### FIGURES

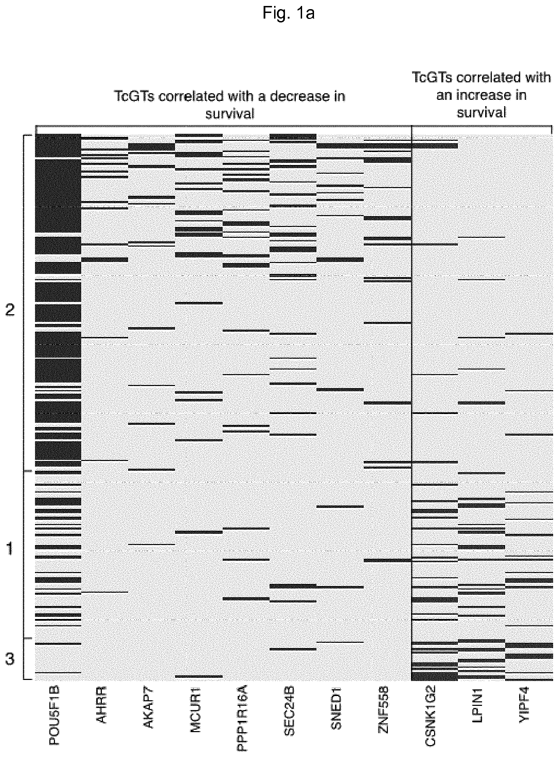

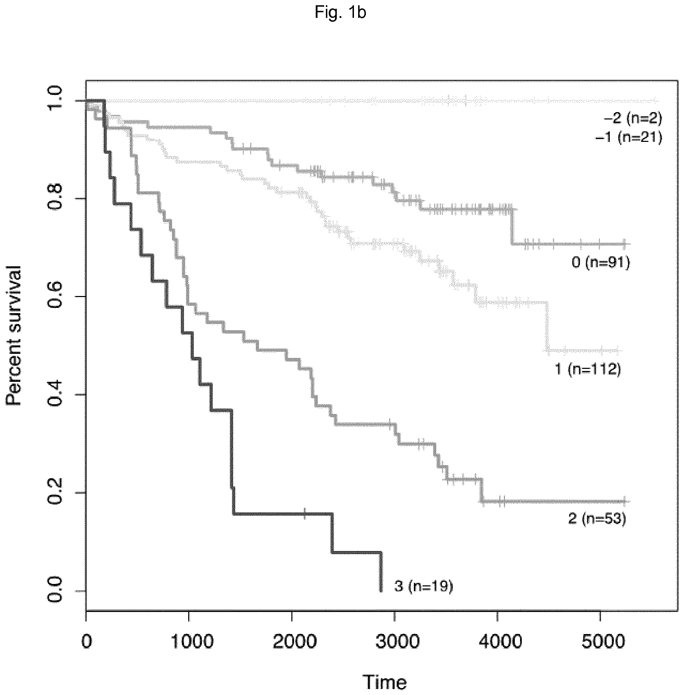

**Figure 1****.** (a) Representation of patients (rows) expressing TcGTs (columns) in the primary tumor (n=11 genes), which are either directly correlated with a decrease or an increase in survival. For every patient, the number of TcGTs correlating with an increase (protective) of survival minus the ones correlated with a decrease (harmful) of survival is computed. This is shown in the bar at the left of the plot. Patients can have either the same number of protective and harmful (1), more harmful tan protective (2) or more protective than harmful (3). (b) The survival rates of these groups of patients are represented here. [GP1]

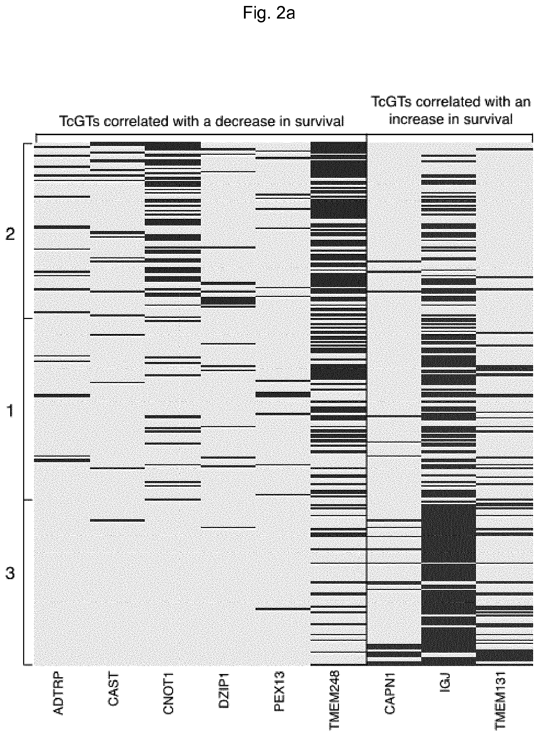

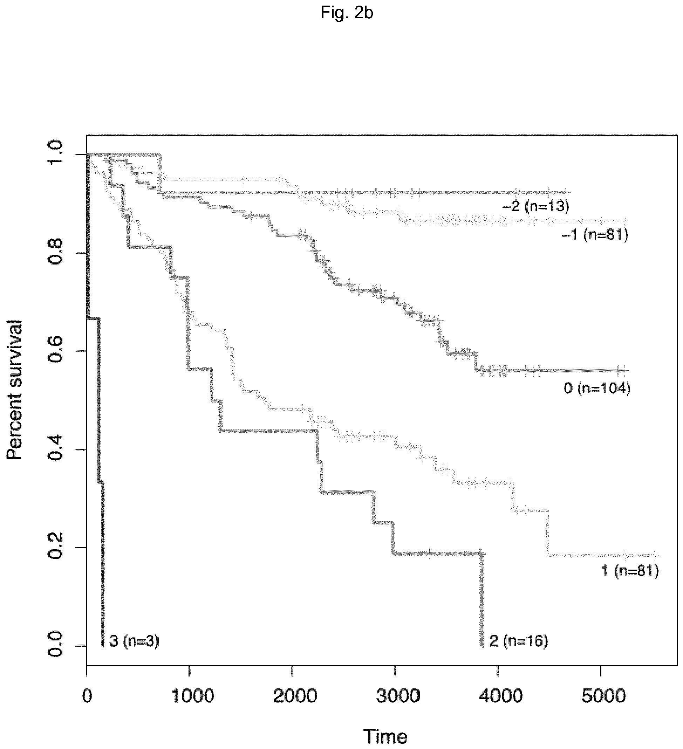

**Figure 2**. (a) Representation of patients (rows) expressing TcGTs (columns) in the normal colon (n=9 genes), which are either directly correlated with a decrease or an increase in survival. For every patient, the number of TcGTs correlating with an increase (protective) of survival minus the ones correlated with a decrease (harmful) of survival is computed. This is shown in the bar at the left of the plot. Patients can have either the same number of protective and harmful (1), more harmful than protective (2) or more protective than harmful (3). (b) The survival rates of these groups of patients are represented here. [GP2]

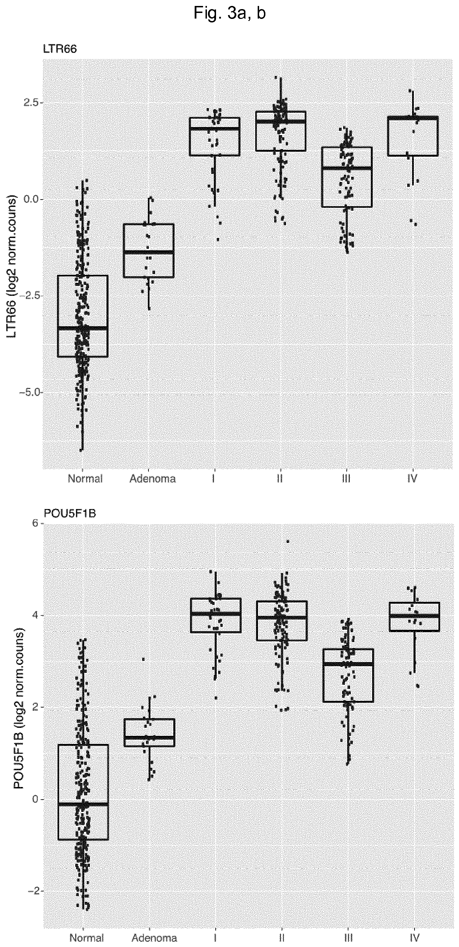

**Figure 3****.** LTR66 (a) and POU5F1B (b) expression increases as disease progresses. Expression of the LTR66-POU5F1B TcGT (documented by RACE-rapid amplification of cDNA ends- in CRC cell lines) tightly correlates with the transition from adenoma to carcinoma. Ad: adenoma; I through IV: stages I to IV carcinoma.

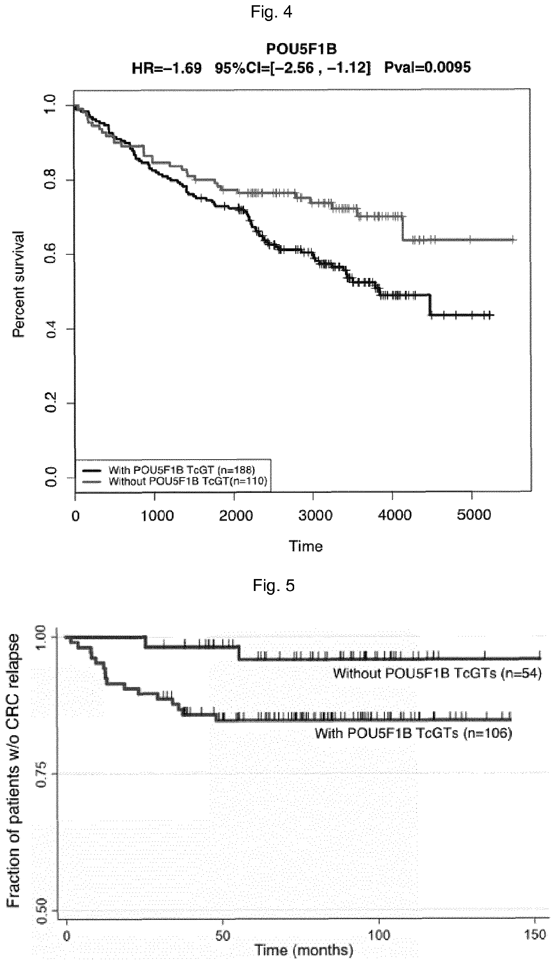

**Figure 4****.** The presence of the 5 TcGTs of POUSF1B in colon primary tumors is linked to a lower survival (HR=-1.69; 95%CI=[-2.56, -1.12]; Pval=0.0095).
**Figure 5****.** The presence of the 5 TcGTs of POUSF1B in CRC primary tumors is associated with a decrease in relapse-free survival in stage II and III patients (HR=4.40±3.30; 95%CI=[1.01,19.14]; Pval=0.048). The relapse-signing event here is distant metastasis, not a local recurrence.

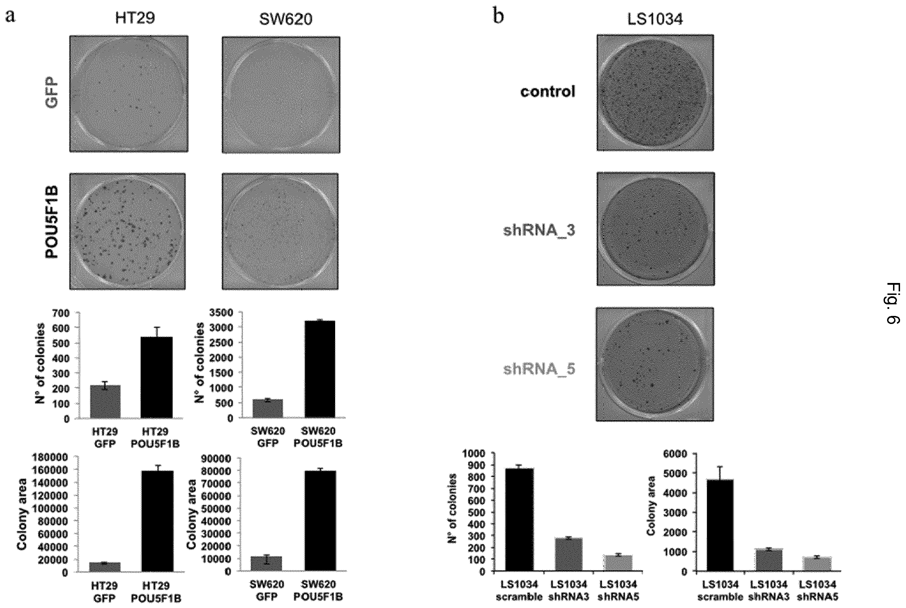

**Figure 6****.** POU5F1B overexpression in HT29 and SW620 CRC cell lines increases their clonogenicity capacity (a) while the downregulation of POUSF1B in LS1034 CRC cell line using two different shRNAs clearly decreases their clonogenicity capacity (b).

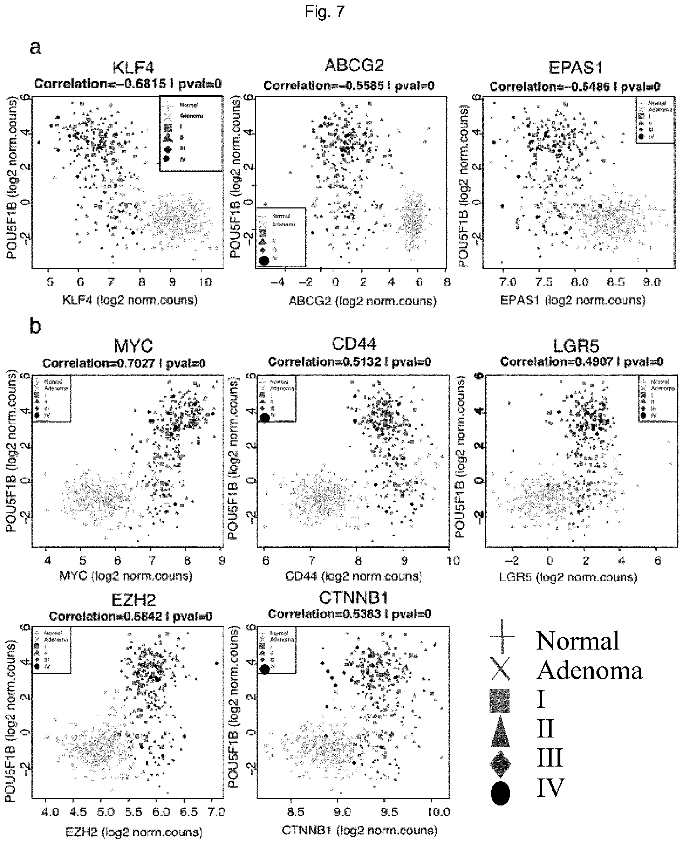

**Figure 7****.** POU5F1B as a stem cell marker, (a) POU5F1B expression anticorrelates with KLF4, ABCG2 and EPAS1 stem cell genes in CRC, 3 genes whose expression also anticorrelates with the mRNA stemness indices defined by Malta et al ^{[10]}. (b) POU5F1B expression correlates with the expression of MYC, CD44, LGR5, EZH2 and CTNNB1 stem cell genes in CRC, 5 genes whose expression also correlates with the mRNA stemness indices defined by Malta et al^{[10]}. In the X axis, the expression of each gene of interest in normalized counts; in the Y axis, the expression of POU5F1B in normalized counts.

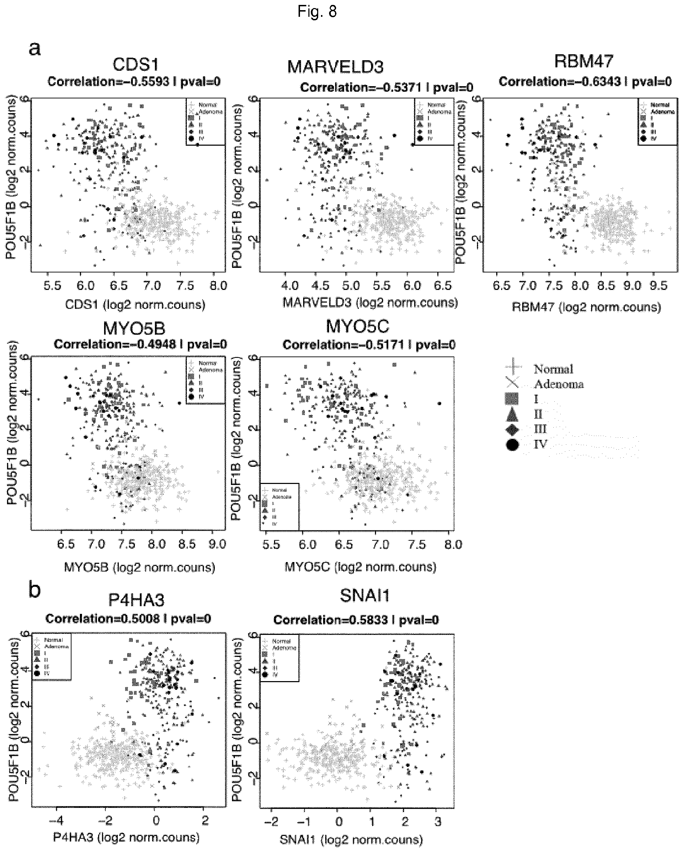

**Figure 8****.** POU5F1B as a mesenchymal marker, (a) POU5F1B expression anticorrelates with the expression of the epithelial markers CDS1, MARVELD3, RBM47, MYO5B and MYO5C. (b) POU5F1B expression correlates with the expression of P4HA3 and SNAI1 mesenchymal genes. In the X axis, the expression of each gene of interest in normalized counts; in the Y axis, the expression of POU5F1B in normalized counts.

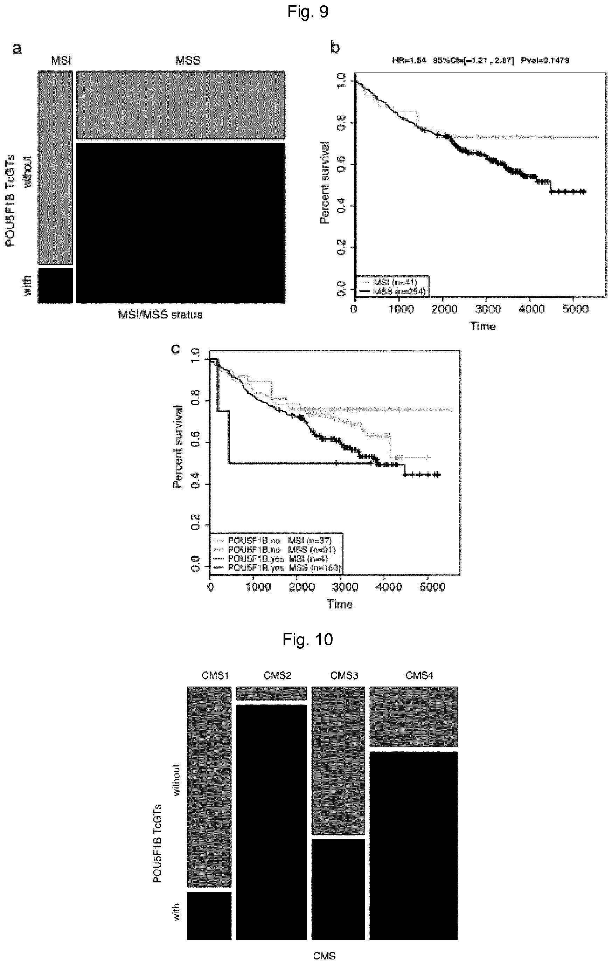

**Figure 9****.** POU5F1B transpochimeric transcripts (in black) are more commonly expressed within the MSS CRC patients, which represent the majority of CRC cases (a). The presence of POUSF1B transpochimeric transcripts has a better predictive power (Fig 3) than MSI or MSS phenotype (b). POU5F1B transpochimeric transcripts are more informative than MSS/MSI to predict survival and they can therefore also be used as an independent predictor (c).
**Figure 10****.** POU5F1B transpochimeric transcripts (in black) are expressed in all CMS groups.

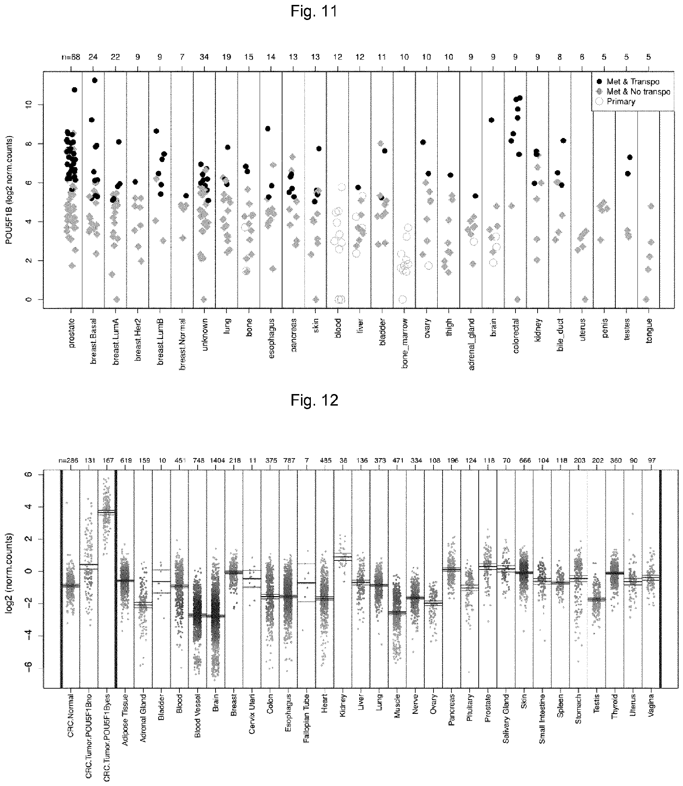

**Figure 11****.** POU5F1B expression in metastatic lesions coming from different primary tumor sites. Several metastases originated from diverse tumor types express POU5F1B transpochimeric transcripts and have high expression of the gene (black dots). The metastatic samples with lower expression of POUSF1B do not present POU5F1B transpochimeric transcripts (grey diamonds). None of the primary tumors (empty circles) present POU5F1B transpochimeric transcripts.
**Figure 12****.** POU5F1B expression in the normal colon samples coming from the 300 CRC patients cohort, in the CRC primary tumors not expressing POU5F1B transpochimeric transcripts, in the CRC primary tumors expressing them, and in all the rest of human normal tissues. The expression of POUSF1B is clearly higher in the CRC primary tumors expressing POU5F1B transpochimeric transcripts compared to the rest of samples.

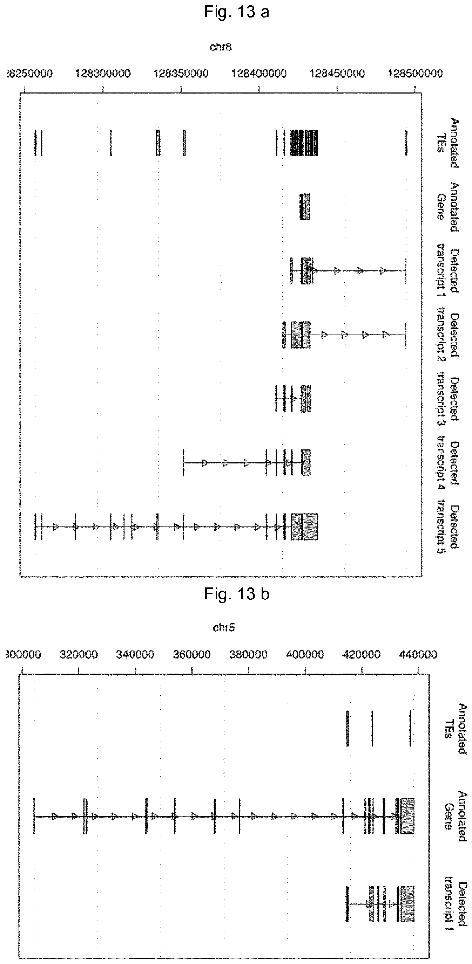

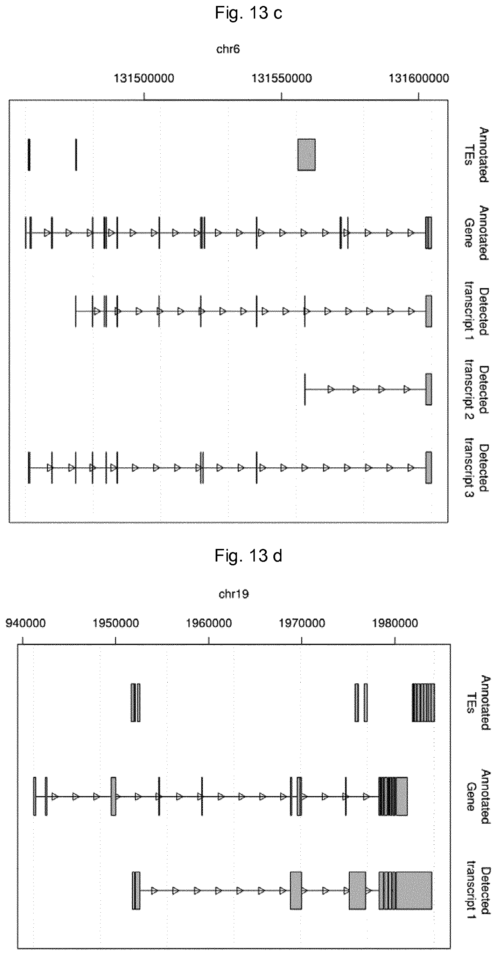

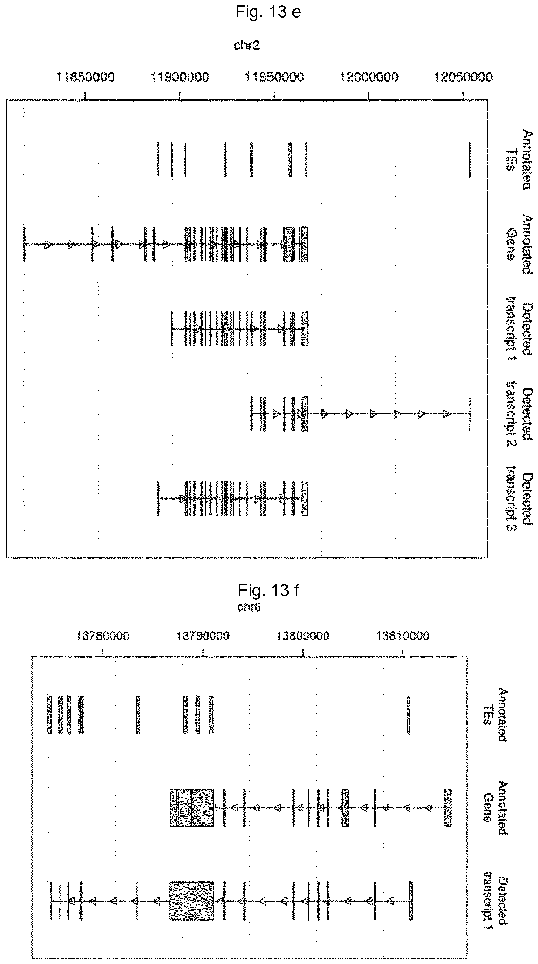

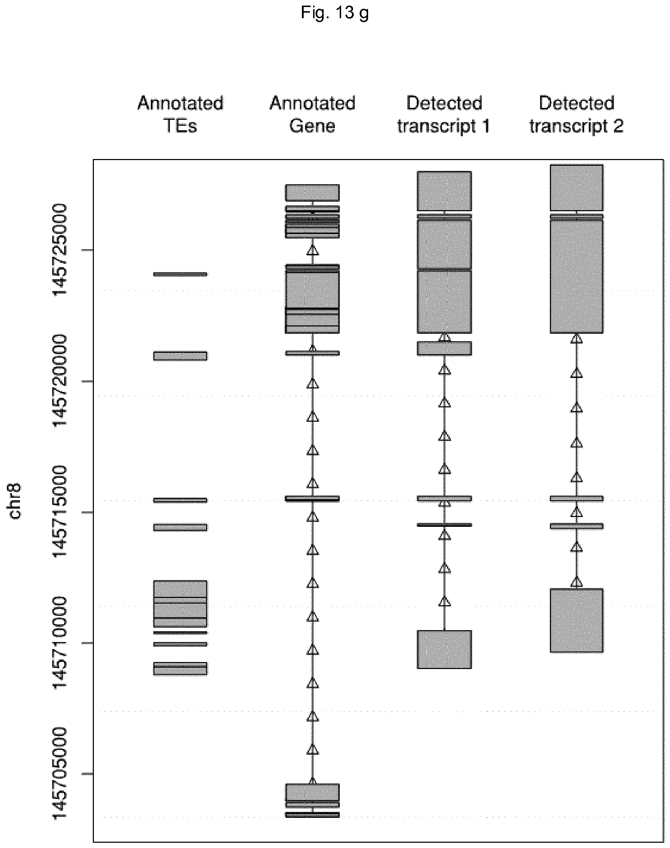

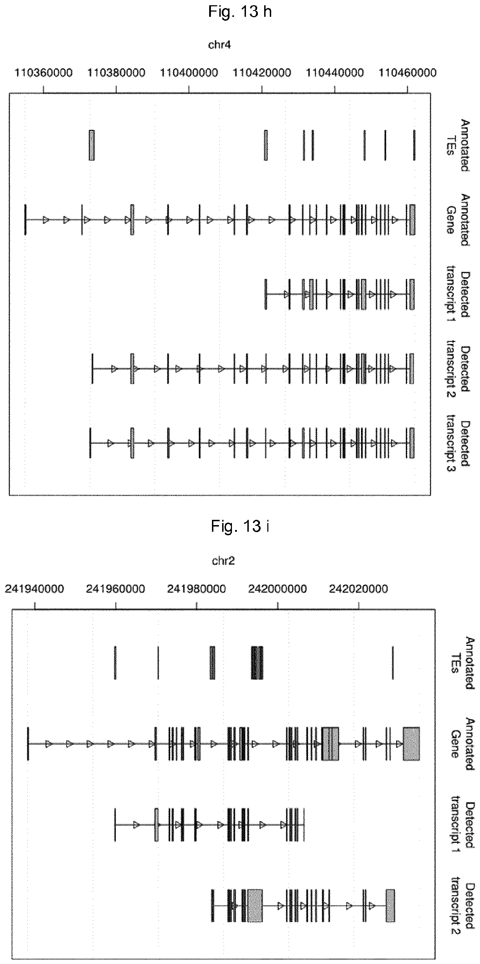

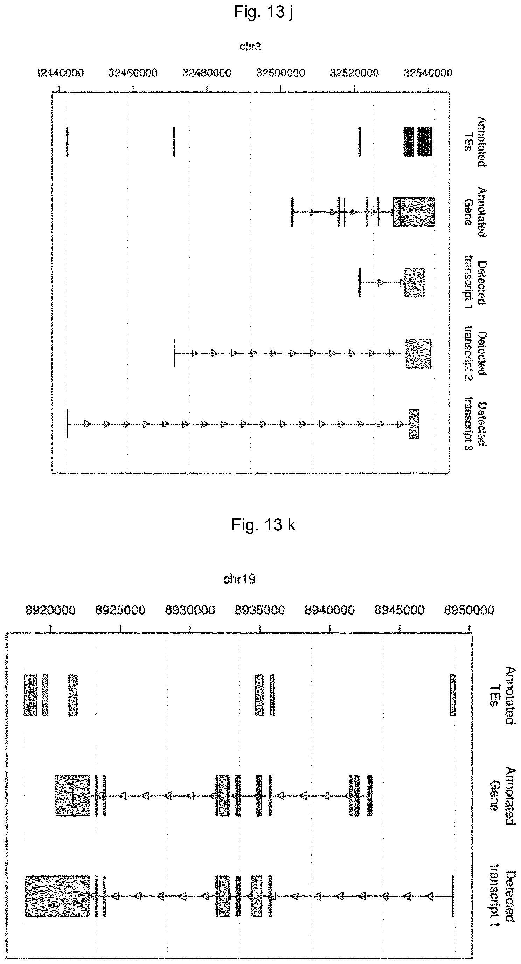

**Figure 13**. Schematic representation of the TcGTs a) POU5F1B, b) AHRR, c) AKAP7, d) CSNK1G2, e) LPIN1, f) MCUR1, g) PPP1R16A, h) SEC24B, i) SNED1, j) YIPF4, and k) ZNF558.

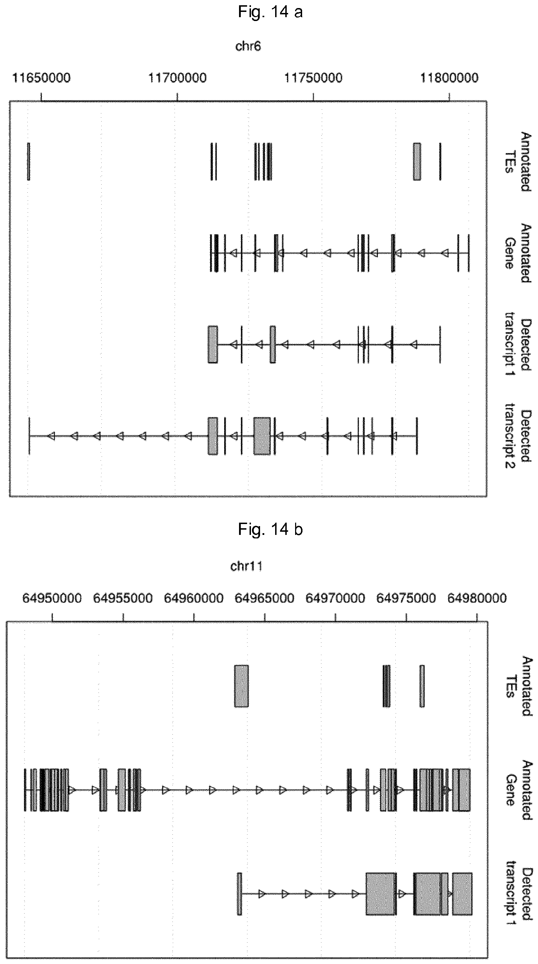

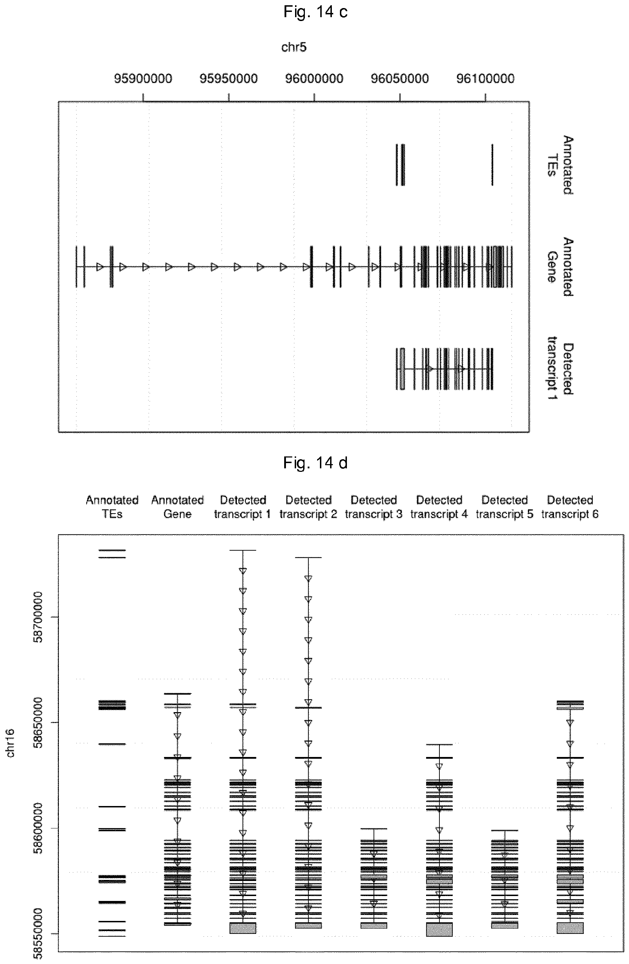

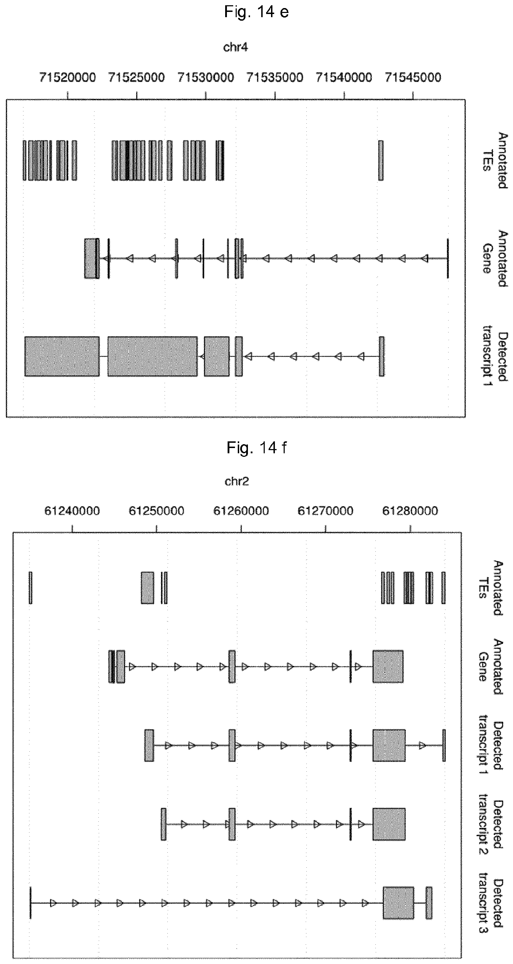

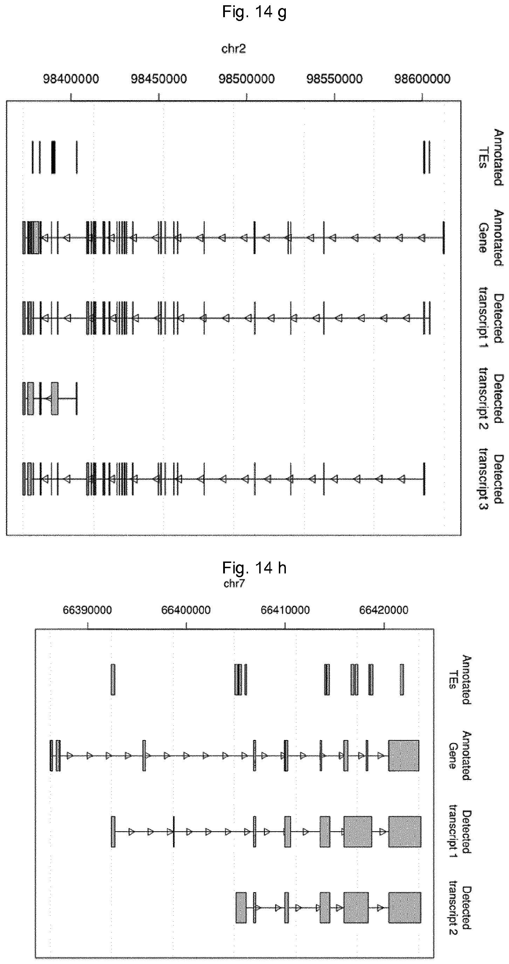

**Figure 14**. Schematic representation of the TcGTs a) ADTRP, b) CAPN1, c) CAST, d) CNOT1, e) DZIP1, f) IGJ, g) PEX13, h) TMEM131, and i) TMEM248.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise" and "comprising" also encompass the more restricted ones "consist" and "consisting", respectively.

As used herein, the singular form "a", "an" and "the" include plural references unless the context clearly dictates otherwise.

As used herein, "at least one" means "one or more", "two or more", "three or more", etc. For example, at least one nucleic acid means "one or more nucleic acid ", "two or more nucleic acids", "three or more nucleic acids ", etc.

As used herein the terms " subject", or " patient" are well-recognized in the art, and, are used interchangeably herein to refer to a mammal, including dog, cat, rat, mouse, monkey, cow, horse, goat, sheep, pig, camel, and, most preferably, a human. In some aspects, the subject is a subject in need of treatment or a subject suffering from cancer or a subject that might be at risk of suffering from cancer. However, in other aspects, the subject can be a normal subject. The term does not denote a particular age or sex. Thus, adult and newborn subjects, whether male or female, are intended to be covered.

According to the present invention, the cancer is selected from the non-limiting group comprising carcinoma, sarcoma, melanoma, lymphoma, and leukemia. Preferably, the cancer is selected from the group comprising Acute Lymphoblastic Leukemia (ALL), Acute Myeloid Leukemia (AML), Adrenocortical Carcinoma, Adult, Childhood Adrenocortical Carcinoma, AIDS-Related Cancers, Kaposi Sarcoma (Soft Tissue Sarcoma), AIDS-Related Lymphoma (Lymphoma), Primary CNS Lymphoma (Lymphoma), Anal Cancer, Astrocytomas, Childhood (Brain Cancer), Atypical Teratoid/Rhabdoid Tumor, Childhood, Central Nervous System (Brain Cancer), Basal Cell Carcinoma of the Skin, Bile Duct Cancer, Bladder Cancer, Bone Cancer (includes Ewing Sarcoma and Osteosarcoma and Malignant Fibrous Histiocytoma), Brain Tumors, Breast Cancer, Childhood Breast Cancer, Childhood Bronchial Tumors, Burkitt Lymphoma, Carcinoid Tumor (Gastrointestinal), Childhood Carcinoid Tumors, Carcinoma of Unknown Primary, Cardiac (Heart) Tumors, Central Nervous System, Childhood Atypical Teratoid/Rhabdoid Tumor, Childhood Embryonal Tumors, Childhood Germ Cell Tumor, Primary CNS Lymphoma, Cervical Cancer, Childhood Cervical Cancer,Cholangiocarcinoma, Chordoma, Childhood, Chronic Lymphocytic Leukemia (CLL), Chronic Myelogenous Leukemia (CML), Chronic Myeloproliferative Neoplasms, Colorectal Cancer, Childhood Colorectal Cancer, Childhood Craniopharyngioma, Cutaneous T-Cell Lymphoma, Ductal Carcinoma In Situ (DCIS), Embryonal Tumors, Endometrial Cancer (Uterine Cancer), Childhood Ependymoma, Esophageal Cancer, Childhood Esophageal Cancer, Esthesioneuroblastoma, Ewing Sarcoma (Bone Cancer), Childhood Extracranial Germ Cell Tumor, Extragonadal Germ Cell Tumor, Eye Cancer, Childhood Intraocular Melanoma, Intraocular Melanoma, Retinoblastoma, Fallopian Tube Cancer, Fibrous Histiocytoma of Bone (Malignant, and Osteosarcoma), Gallbladder Cancer, Gastric,(Stomach) Cancer, Childhood Gastric (Stomach) Cancer, Gastrointestinal Carcinoid Tumor, Gastrointestinal Stromal Tumors (GIST) (Soft Tissue Sarcoma), Childhood Gastrointestinal Stromal Tumors, Germ Cell Tumors, Childhood Central Nervous System Germ Cell Tumors (Brain Cancer), Childhood Extracranial Germ Cell Tumors, Extragonadal Germ Cell Tumors, Ovarian Germ Cell Tumors, Testicular Cancer, Gestational Trophoblastic Disease, Hairy Cell Leukemia, Head and Neck Cancer, Childhood Head and Neck Cancers, Childhood Heart Tumors, Hepatocellular (Liver) Cancer, Langerhans Cell Histiocytosis, Hodgkin Lymphoma, Hypopharyngeal Cancer (Head and Neck Cancer), Intraocular Melanoma, Childhood Intraocular Melanoma, Islet Cell Tumors, Pancreatic Neuroendocrine Tumors, Kaposi Sarcoma (Soft Tissue Sarcoma), Kidney (Renal Cell) Cancer, Langerhans Cell Histiocytosis, Laryngeal Cancer (Head and Neck Cancer), Childhood Laryngeal Cancer, Papillomatosis, Leukemia, Lip and Oral Cavity Cancer (Head and Neck Cancer), Liver Cancer, Lung Cancer (Non-Small Cell and Small Cell), Childhood Lung Cancer, Lymphoma, Male Breast Cancer, Malignant Fibrous Histiocytoma of Bone and Osteosarcoma, Melanoma, Childhood Melanoma, Intraocular (Eye)Melanoma, Childhood Intraocular Melanoma, Merkel Cell Carcinoma (Skin Cancer), Malignant Mesothelioma, Childhood Mesothelioma , Metastatic Squamous Neck Cancer with Occult Primary (Head and Neck Cancer), Midline Tract Carcinoma Involving NUT Gene, Mouth Cancer (Head and Neck Cancer), Multiple Endocrine Neoplasia Syndromes, Multiple Myeloma/Plasma Cell Neoplasms, Mycosis Fungoides (Lymphoma), Myelodysplasia Syndromes, Myelodysplastic/Myeloproliferative Neoplasms, Myelogenous Leukemia, Chronic (CML), Myeloid Leukemia, Acute (AML), Chronic Myeloproliferative Neoplasms, Nasal Cavity and Paranasal Sinus Cancer (Head and Neck Cancer), Nasopharyngeal Cancer (Head and Neck Cancer), Childhood Nasopharyngeal Cancer, Neuroblastoma, Non-Hodgkin Lymphoma, Non-Small Cell Lung Cancer, Oral Cancer, Lip and Oral Cavity Cancer and Oropharyngeal Cancer (Head and Neck Cancer), Childhood Oral Cavity Cancer, Osteosarcoma and Malignant Fibrous Histiocytoma of Bone, Ovarian Cancer, Childhood Ovarian Cancer, Pancreatic Cancer, Childhood Pancreatic Cancer, Pancreatic Neuroendocrine Tumors (Islet Cell Tumors), Papillomatosis, Paraganglioma, Childhood Paraganglioma, Paranasal Sinus and Nasal Cavity Cancer (Head and Neck Cancer), Parathyroid Cancer, Penile Cancer, Pharyngeal Cancer (Head and Neck Cancer), Pheochromocytoma, Childhood Pheochromocytoma, Pituitary Tumor, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Childhood Rhabdomyosarcoma (Soft Tissue Sarcoma), Salivary Gland Cancer (Head and Neck Cancer), Childhood Salivary Gland Tumors, Sarcoma, Childhood Rhabdomyosarcoma (Soft Tissue Sarcoma), Childhood Vascular Tumors (Soft Tissue Sarcoma), Ewing Sarcoma (Bone Cancer), Kaposi Sarcoma (Soft Tissue Sarcoma), Osteosarcoma (Bone Cancer), Uterine Sarcoma, Sezary Syndrome (Lymphoma), Skin Cancer, Childhood Skin Cancer, Small Cell Lung Cancer, Small Intestine Cancer, Soft Tissue Sarcoma, Squamous Cell Carcinoma of the Skin, Squamous Neck Cancer with Occult Primary, Metastatic (Head and Neck Cancer), Stomach (Gastric) Cancer, Childhood Stomach (Gastric) Cancer, T-Cell Lymphoma, Cutaneous, Testicular Cancer, Childhood Testicular Cancer, Throat Cancer (Head and Neck Cancer), Nasopharyngeal Cancer, Oropharyngeal Cancer, Hypopharyngeal Cancer, Thymoma and Thymic Carcinoma, Thyroid Cancer, Childhood Thyroid Tumors, Transitional Cell Cancer of the Renal Pelvis and Ureter (Kidney (Renal Cell) Cancer), Carcinoma of Unknown Primary, Childhood Cancer of Unknown Primary, Unusual Cancers of Childhood, Ureter and Renal Pelvis, Transitional Cell Cancer (Kidney (Renal Cell) Cancer, Urethral Cancer, Endometrial Uterine Cancer, Uterine Sarcoma, Vaginal Cancer, Childhood Vaginal Cancer, Vascular Tumors (Soft Tissue Sarcoma), Vulvar Cancer, and Wilms Tumor or a combination of one of more of these cancers

More preferably, the cancer is a solid tumor cancer. Most preferably, the solid tumor cancer is selected from the group comprising Colorectal Cancer, Gastric Cancer, Prostate Cancer, Liver Cancer, Testis Cancer, Ovary Cancer and Breast Cancer, or a combination of one of more of these cancers. Even more preferably, the cancer is Colorectal Cancer.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

In one aspect the acid nucleic of the invention is an acid nucleic comprising i) at least one transposable element (TE), a fragment or variant thereof, and a ii) a gene sequence, a fragment or variant thereof. The acid nucleic of the invention is preferably selected from the group comprising an RNA transcript, a cDNA of said RNA transcript or a DNA sequence, preferably an amplified DNA sequence. In some aspects the acid nucleic of the invention comprising i) at least one transposable element (TE), a fragment or variant thereof, and ii) a gene sequence, a fragment or variant thereof, is an RNA transcript called "transpochimera" or "Transpochimeric gene transcripts (TcGTs)".

As used herein, a "biological sample" refers to a sample of tissue or fluid isolated from a subject, including but not limited to, for example, urine, blood, plasma, serum, fecal matter, bone marrow, bile, spinal fluid, lymph fluid, samples of the skin, external secretions of the skin, respiratory, intestinal, and genitourinary tracts, tears, saliva, milk, blood cells, organs, biopsies, and also samples containing cells or tissues derived from the subject and grown in culture, and in vitro cell culture constituents, including but not limited to, conditioned media resulting from the growth of cells and tissues in culture, recombinant cells, stem cells, and cell components.

Surprisingly, the inventors of the present invention have shown that transposable element (TE) transcripts fused to complete or partial gene transcripts can serve as cancer biomarkers and source of therapeutic targets for cancer.

In one aspect, the present invention thus provides a method for detecting and/or predicting the likelihood of a cancer in a subject, the method comprising detecting, directly or indirectly, in a biological sample of said subject, the presence or absence of at least one nucleic acid sequence comprising i) at least one transposable element (TE) a fragment or variant thereof, and ii) a gene sequence, a fragment or variant thereof. Preferably, the at least one additional nucleic acid is selected from the group listed in Table 1 and/or Table 2, or a variant or fragment thereof. The presence of at least one nucleic acid sequence predictor of poor survival (harmful predictor, identified by -), and/or the absence of at least one predictor of good survival (protective predictor, identified by +) being indicative of the presence of a cancer and/or of a poor prognosis of said cancer.

It is understood that the presence of the at least one nucleic acid sequence comprising i) at least one transposable element (TE) a fragment or variant thereof, and ii) a gene sequence, a fragment or variant thereof, in a biological sample can be determined by any suitable method known in the art. Detection of said at least one nucleic acid sequence can be direct or indirect.

For example, the presence of the at least one nucleic acid sequence comprising i) at least one transposable element (TE) a fragment or variant thereof, and ii) a gene sequence, a fragment or variant thereof, can be directly detected using, e.g. Northern blot analysis or RNA FISH technique.

Alternatively, the presence of said at least one nucleic acid sequence can be determined indirectly by detecting the presence of cDNAs, amplified RNAs or DNAs, or by measuring activities of RNAs, or other molecules that are indicative of the expression level of said at least one nucleic acid sequence. Preferably, the presence or absence of said at least one nucleic acid sequence is determined indirectly by detecting the presence of the corresponding cDNAs.

The one or more acid nucleic of the invention can be detected and, alternatively, quantitated by a variety of methods including, but not limited to, microarray analysis, polymerase chain reaction (PCR), reverse transcriptase polymerase chain reaction (RT-PCR), Northern blot, serial analysis of gene expression (SAGE), immunoassay, mass spectrometry, and any sequencing-based methods known in the art (such as e.g. RNA-seq).

In a particular aspect, microarrays are used to measure the presence and/or levels of the at least one or more acid nucleic of the invention. An advantage of microarray analysis is that the expression of each of the one or more acid nucleic of the invention can be measured simultaneously, and microarrays can be specifically designed to provide a diagnostic expression profile for a particular disease or condition (e.g., a type of cancer, stage of cancer, ...).
Microarrays are prepared by selecting probes which comprise a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically in vivo, enzymatically in vitro (e.g., by PCR), or non-enzymatically in vitro.

Probes that selectively hybridize the at least one or more acid nucleic of the invention and used in the methods of the invention are preferably immobilized to a solid support which may be either porous or non-porous. For example, the probes may be polynucleotide sequences which are attached to a nitrocellulose or nylon membrane or filter covalently at either the 3' or the 5' end of the polynucleotide. Such hybridization probes are well known in the art (see, e.g., Sambrook, et al., Molecular Cloning: A Laboratory Manual (3rd Edition, 2001). Alternatively, the solid support or surface may be a glass or plastic surface. In one embodiment, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel. In one embodiment, the microarray comprises a support or surface with an ordered array of binding (e.g., hybridization) sites or "probes" each representing one of the biomarkers described herein. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). Each probe is preferably covalently attached to the solid support at a single site. Microarrays can be made in a number of ways, of which several are described below. However they are produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. Microarrays are generally small, e.g., between 1 cm2 and 25 cm2; however, larger arrays may also be used, e.g., in screening arrays. Preferably, a given binding site or unique set of binding sites in the microarray will specifically recognize and bind (e.g., hybridize) to the product of a single gene in a cell (e.g., to a specific mRNA or to a specific cDNA derived therefrom). However, in general, other related or similar sequences will cross hybridize to a given binding site.
As noted above, the "probe" to which a particular polynucleotide molecule specifically hybridizes contains a complementary polynucleotide sequence. The probes of the microarray typically consist of nucleotide sequences of no more than 1,000 nucleotides. In some embodiments, the probes of the array consist of nucleotide sequences of 10 to 1,000 nucleotides. In one aspect, the nucleotide sequences of the probes are in the range of 10-200 nucleotides in length and are genomic sequences of one species of organism, such that a plurality of different probes is present, with sequences complementary and thus capable of hybridizing to the genome of such a species of organism, sequentially tiled across all or a portion of the genome. In other embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, or are 60 nucleotides in length. The probes may comprise DNA or DNA "mimics" (e.g., derivatives and analogues) corresponding to a portion of an organism's genome. In another embodiment, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone (e.g., phosphorothioates).
DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). Typically each probe on the microarray will be between 10 bases and 50,000 bases, usually between 300 bases and 1,000 bases in length. PCR methods are well known in the art, and are described, for example, in Innis et al., eds., PCR Protocols: A Guide To Methods And Applications, Academic Press Inc., San Diego, Calif. (1990); herein incorporated by reference in its entirety. It will be apparent to one skilled in the art that controlled robotic systems are useful for isolating and amplifying nucleic acids.

An alternative, preferred means for generating polynucleotide probes is by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some embodiments, synthetic nucleic acids include non-natural bases, such as, but by no means limited to, inosine. As noted above, nucleic acid analogues may be used as binding sites for hybridization. An example of a suitable nucleic acid analogue is peptide nucleic acid (see, e.g., U.S. Pat. No. 5,539,083).

Probes are preferably selected using an algorithm that takes into account binding energies, base composition, sequence complexity, cross-hybridization binding energies, and secondary structure. See e.g. International Patent Publication WO 01/05935.

A skilled artisan will also appreciate that positive control probes, e.g., probes known to be complementary and hybridizable to sequences in the target polynucleotide molecules, and negative control probes, e.g., probes known to not be complementary and hybridizable to sequences in the target polynucleotide molecules, should be included on the array. In one embodiment, positive controls are synthesized along the perimeter of the array. In another embodiment, positive controls are synthesized in diagonal stripes across the array. In still another embodiment, the reverse complement for each probe is synthesized next to the position of the probe to serve as a negative control. In yet another embodiment, sequences from other species of organism are used as negative controls or as "spike-in" controls.

The probes are attached to a solid support or surface, which may be made, e.g., from glass, plastic (e.g., polypropylene, nylon), polyacrylamide, nitrocellulose, gel, or other porous or nonporous material. One method for attaching nucleic acids to a surface is by printing on glass plates, as known in the art. This method is especially useful for preparing microarrays of cDNA. A second method for making microarrays produces high-density oligonucleotide arrays. Techniques are known for producing arrays containing thousands of oligonucleotides complementary to defined sequences, at defined locations on a surface using photolithographic techniques for synthesis in situ (see, U.S. Pat. Nos. 5,578,832; 5,556,752; and 5,510,270; herein incorporated by reference in their entireties) or other methods for rapid synthesis and deposition of defined oligonucleotides. When these methods are used, oligonucleotides (e.g., 60-mers) of known sequence are synthesized directly on a surface such as a derivatized glass slide. Usually, the array produced is redundant, with several oligonucleotide molecules per RNA.
Other methods for making microarrays, e.g., by masking, may also be used. In principle, any type of array known in the art, for example, dot blots on a nylon hybridization membrane could be used. However, as will be recognized by those skilled in the art, very small arrays will frequently be preferred because hybridization volumes will be smaller.

Microarrays can also be manufactured by means of an ink jet printing device for oligonucleotide synthesis, e.g., using the methods and systems described by Blanchard in U.S. Pat. No. 6,028,189. Specifically, the oligonucleotide probes in such microarrays are synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells which define the locations of the array elements (i.e., the different probes). Microarrays manufactured by this ink jet method are typically of high density, preferably having a density of at least about 2,500 different probes per 1 cm2. The polynucleotide probes are attached to the support covalently at either the 3' or the 5' end of the polynucleotide.

The at least one nucleic acid sequence of the invention comprising i) at least one transposable element (TE) a fragment or variant thereof, and a ii) a gene sequence, a fragment or variant thereof which may be measured by microarray analysis can be expressed RNAs or a nucleic acid derived therefrom (e.g., cDNA or amplified RNA derived from cDNA that incorporates an RNA polymerase promoter), including naturally occurring nucleic acid molecules, as well as synthetic nucleic acid molecules. In one aspect, the target polynucleotide molecules comprise RNA, including, but by no means limited to, total cellular RNA, poly(A)+ messenger RNA (mRNA) or a fraction thereof, cytoplasmic mRNA, or RNA transcribed from cDNA (i.e., cRNA; see, e.g., U.S. Pat. No. 5,545,522, 5,891,636, or 5,716,785). Methods for preparing total and poly(A)+ RNA are well known in the art, and are described generally, e.g., in Sambrook, et al., Molecular Cloning: A Laboratory Manual (3rd Edition, 2001). RNA can be extracted from a cell of interest using guanidinium thiocyanate lysis followed by CsCl centrifugation, a silica gel-based column (e.g., RNeasy (Qiagen, Valencia, Calif.) or StrataPrep (Stratagene, La Jolla, Calif.)), or using phenol and chloroform, as known in the art. Poly(A)+ RNA can be selected, e.g., by selection with oligo-dT cellulose or, alternatively, by oligo-dT primed reverse transcription of total cellular RNA. RNA can be fragmented by methods known in the art, e.g., by incubation with ZnCl2, to generate fragments of RNA.

Preferably, the at least one acid nucleic sequence is selected from the group comprising SEQ ID No 1, 2, 3, 4, 5, 6, 7, 8, 9, 10, 11, 12, 13, 14, 15, 16, 17,18, 19 ,20, 21, 22, 23, 24, 25, 26, 27, 28, 29, 30, 31, 32, 33, 34, 35, 36, 37, 38, 39, 40, 41, 42, 43, 44 and 45 a fragment or variant thereof or a combination of one or more thereof.

RNA-Seq is a High-throughput sequencing method recently developed for transcriptome profiling technology that utilizes next-generation sequencing platforms (Metzker, 2010; Mardis, 2008). RNA-Seq transcripts are reverse-transcribed into cDNA, and adapters are ligated to each end of the cDNA. Sequencing can be done either unidirectional (single-end sequencing) or bidirectional (paired-end sequencing) and then aligned to a reference genome database or assembled to obtain *de novo* transcripts, proving a genome-wide expression profile (Wang et al., Nat. Rev. Genet. (2009) 10:57-63, 2009 and Marguerat and Bahler in Cell. Mol. Life. Sci. (2010) 67:569-579).

As described herein, the nucleic acid sequences of the invention can be detectably labeled at one or more nucleotides. Any method known in the art may be used to label the target the nucleic acid sequences of the invention. Preferably, this labeling incorporates the label uniformly along the length of the RNA, and more preferably, the labeling is carried out at a high degree of efficiency. For example, nucleic acid sequences can be labeled by oligo-dT primed reverse transcription. Random primers (e.g., 9-mers) can be used in reverse transcription to uniformly incorporate labeled nucleotides over the full length of the nucleic acid sequences. Alternatively, random primers may be used in conjunction with PCR methods or T7 promoter-based in vitro transcription methods in order to amplify the nucleic acid sequences of the invention.
The detectable label may be a luminescent label. For example, fluorescent labels, bioluminescent labels, chemiluminescent labels, and colorimetric labels may be used in the practice of the invention. Fluorescent labels that can be used include, but are not limited to, fluorescein, a phosphor, a rhodamine, or a polymethine dye derivative. Additionally, commercially available fluorescent labels including, but not limited to, fluorescent phosphoramidites such as FluorePrime (Amersham Pharmacia, Piscataway, N.J.), Fluoredite (Miilipore, Bedford, Mass.), FAM (ABI, Foster City, Calif.), and Cy3 or Cy5 (Amersham Pharmacia, Piscataway, N.J.) can be used. Alternatively, the detectable label can be a radiolabeled nucleotide.
In one aspect, nucleic acid sequences from a patient sample are labeled differentially from the corresponding polynucleotide molecules of a reference sample. The reference can comprise RNAs from a normal biological sample (i.e., control sample, e.g., biopsy from a subject not having a cancer) or from a reference biological sample, (e.g., sample from a subject having a cancer).
Nucleic acid hybridization and wash conditions are chosen so that the target nucleic acid sequences specifically bind or specifically hybridize to the complementary polynucleotide sequences of the array, preferably to a specific array site, wherein its complementary DNA is located. Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self-complementary sequences.
Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook, et al., Molecular Cloning: A Laboratory Manual (3rd Edition, 2001) . Typical hybridization conditions for the cDNA microarrays of Schena et al. are hybridization in 5×SSC plus 0.2% SDS at 65° C. for four hours, followed by washes at 25° C. in low stringency wash buffer (1 ×SSC plus 0.2% SDS), followed by 10 minutes at 25° C. in higher stringency wash buffer (0.1×SSC plus 0.2% SDS). Particularly preferred hybridization conditions include hybridization at a temperature at or near the mean melting temperature of the probes (e.g., within 51° C., more preferably within 21° C.) in 1 M NaCl, 50 mM MES buffer (pH 6.5), 0.5% sodium sarcosine and 30% formamide.
When fluorescently labeled gene products are used, the fluorescence emissions at each site of a microarray may be, preferably, detected by scanning confocal laser microscopy. In one embodiment, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously. Arrays can be scanned with a laser fluorescent scanner with a computer controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are known in the art. Alternatively, a fiber-optic bundle, may be used to monitor mRNA abundance levels at a large number of sites simultaneously.

In one aspect, the invention includes a microarray comprising a plurality of probes that selectively hybridize to one or more nucleic acid of the invention. Preferably, one or more nucleic acid of the invention are selected from the group listed in Table 1 and/or Table 2, fragments thereof, isoforms thereof and variants sharing at least 80% nucleotide sequence identity thereto, most preferably from the group comprising SEQ ID No 1, 2, 3, 4, 5, 6, 7, 8, 9, 10, 11, 12, 13, 14, 15, 16, 17,18, 19,20, 21, 22, 23, 24, 25, 26, 27, 28, 29, 30, 31, 32, 33, 34, 35, 36, 37, 38, 39, 40, 41, 42, 43, 44 and 45.

Serial Analysis Gene Expression (SAGE), can also be used to determine RNA (e.g., transpochimera) the presence and/or abundance in a cell sample.

Quantitative reverse transcriptase PCR (qRT-PCR) can also be used to determine the presence and/or expression profiles of the nucleic acid sequences of the invention (see, e.g., U.S. Patent Application Publication No. 2005/0048542A1; herein incorporated by reference in its entirety). The first step is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, Calif., USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TAQMAN PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TAQMAN RT-PCR can be performed using commercially available equipment, such as, for example, ABI PRISM 7700 sequence detection system. (Perkin-Elmer-Applied Biosystems, Foster City, Calif., USA), or Lightcycler (Roche Molecular Biochemicals, Mannheim, Germany). In a preferred embodiment, the 5' nuclease procedure is run on a real-time quantitative PCR device such as the ABI PRISM 7700 sequence detection system. The system consists of a thermocycler, laser, charge-coupled device (CCD), camera and computer. The system includes software for running the instrument and for analyzing the data. 5'-Nuclease assay data are initially expressed as Ct, or the threshold cycle. Fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and beta-actin.

Another variation of the RT-PCR technique is the real time quantitative PCR, which measures PCR product accumulation through a dual-labeled fluorigenic probe (i.e., TAQMAN probe). Real time PCR is compatible both with quantitative competitive PCR, where internal competitor for each target sequence is used for normalization, and with quantitative comparative PCR using a normalization gene contained within the sample, or a housekeeping gene for RT-PCR..

Mass spectrometry, and particularly SELDI mass spectrometry, is a particularly useful method for detection of one or more nucleic acid (e.g. biomarkers) of this invention. Laser desorption time-of-flight mass spectrometer can be used in embodiments of the invention. In laser desorption mass spectrometry, a substrate or a probe comprising biomarkers is introduced into an inlet system. The biomarkers are desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of markers of specific mass to charge ratio.

Matrix-assisted laser desorption/ionization mass spectrometry (MALDI-MS) can also be used for detecting the one or more nucleic acid sequence of this invention. MALDI-MS is a method of mass spectrometry that involves the use of an energy absorbing molecule, frequently called a matrix, for desorbing proteins intact from a probe surface. MALDI is described, for example, in U.S. Pat. No. 5,118,937 and U.S. Pat. No. 5,045,694. In MALDI-MS, the sample is typically mixed with a matrix material and placed on the surface of an inert probe. Exemplary energy absorbing molecules include cinnamic acid derivatives, sinapinic acid ("SPA"), cyano hydroxy cinnamic acid ("CHCA") and dihydroxybenzoic acid. Other suitable energy absorbing molecules are known to those skilled in this art. The matrix dries, forming crystals that encapsulate the analyte molecules. Then the analyte molecules are detected by laser desorption/ionization mass spectrometry.

Surface-enhanced laser desorption/ionization mass spectrometry, or SELDI-MS represents an improvement over MALDI for the fractionation and detection of biomolecules, such as the nucleic acid sequences of the invention, in complex mixtures. SELDI is a method of mass spectrometry in which biomolecules, such as the nucleic acid sequences of the invention, are captured on the surface of a biochip using capture reagents that are bound there. Typically, non-bound molecules are washed from the probe surface before interrogation. SELDI is described, for example, in: U.S. Pat. No. 5,719,060 and in U.S. Pat. No. 6,225,047.

The nucleic acid sequences of the invention on the substrate surface can be desorbed and ionized using gas phase ion spectrometry. Any suitable gas phase ion spectrometer can be used as long as it allows biomarkers on the substrate to be resolved. Preferably, gas phase ion spectrometers allow quantitation of biomarkers. In one embodiment, a gas phase ion spectrometer is a mass spectrometer. In a typical mass spectrometer, a substrate or a probe comprising biomarkers on its surface is introduced into an inlet system of the mass spectrometer. The biomarkers are then desorbed by a desorption source such as a laser, fast atom bombardment, high energy plasma, electrospray ionization, thermospray ionization, liquid secondary ion MS, field desorption, etc. The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of the nucleic acid sequences of the invention or other substances will typically involve detection of signal intensity. This, in turn, can reflect the quantity and character of the nucleic acid sequences of the invention bound to the substrate. Any of the components of a mass spectrometer (e.g., a desorption source, a mass analyzer, a detector, etc.) can be combined with other suitable components described herein or others known in the art in embodiments of the invention.

The nucleic acid sequences of the invention can also be detected with assays based on the use of antibodies that specifically recognize the nucleic acid sequences of the invention or oligonucleotide fragments of said nucleic acid sequences of the invention. Such assays include, but are not limited to, immunohistochemistry (IHC), enzyme-linked immunosorbent assay (ELISA), radioimmunoassays (RIA), "sandwich" immunoassays, fluorescent immunoassays, immunoprecipitation assays, the procedures of which are well known in the art.

The nucleic acid sequences of the invention can also be detected with any sequencing based technologies know in the art.

As indicated above, the method of the invention for detecting and/or predicting the likelihood of a cancer in a subject, also comprises detecting, directly or indirectly, in a biological sample of said subject, the presence or absence of a fragment or variant of at least one nucleic acid sequence of the invention that comprises i) at least one transposable element (TE), and a ii) a gene sequence. Preferably, the at least one additional nucleic acid is selected from the group listed in Table 1 and/or Table 2.

As used herein, the term "variant" refers to biologically active derivatives of a nucleic acid sequence. In general, the term "variant" refers to molecules having a native sequence and structure with one or more additions, substitutions (generally conservative in nature) and/or deletions (e.g. splice variants), relative to the native molecule, so long as the modifications do not destroy biological activity and which are "substantially homologous" to the reference molecule. In general, the sequences of such variants are functionally, i.e. biologically, active variants and will have a high degree of sequence homology to the reference sequence, e.g., sequence homology of more than 50%, generally more than 60%-70%, even more particularly 80%-85% or more, such as at least 90% or 95% or more, when the two sequences are aligned.

Alternatively, the term "variant" also refers to post-transcriptionally modified nucleic acid sequence of the invention, i.e methylation, phosphorylation, *etc*...

As used herein, a "fragment" of one or more nucleic acid sequence or polypeptide sequence of the invention refers to a sequence containing less nucleotides or amino acids in length than the respective sequences of the invention while retaining the biological activity described herein. Preferably, this fragment contains, at least 10%, 20%, 30%, 40%, 50%, 60%, 70%, 80%, or 90% of the entire length of the reference nucleic acid sequence or polypeptide sequence.

As used herein, "Homology" refers to the percent identity between two polynucleotide or two polypeptide sequences. Two nucleic acid, or two polypeptide sequences are "substantially homologous" to each other when the sequences exhibit at least about 50% sequence identity, preferably at least about 75% sequence identity, more preferably at least about 80%-85% sequence identity, more preferably at least about 90% sequence identity, and most preferably at least about 95%-98% sequence identity over a defined length of the molecules. As used herein, substantially homologous also refers to sequences showing complete identity to the specified sequence.

In general, "identity" refers herein to an exact nucleotide to nucleotide or amino acid to amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Sequence identity is typically measured using sequence analysis software (for example, Sequence Analysis Software Package of the Genetics Computer Group, University of Wisconsin Biotechnology Center, 1710 University Avenue, Madison, Wis.53705, BLAST, BESTFIT, GAP, or PILEUP/PRETTYBOX programs). Such software matches identical or similar sequences by assigning degrees of homology to various substitutions, deletions, and/or other modifications.

Alternatively, homology can be determined by readily available computer programs or by hybridization of polynucleotides under conditions which form stable duplexes between homologous regions, followed by digestion with single stranded specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art.

The at least one TE of the invention is located either upstream and/or downstream, i.e. 5' and/or 3' regions, preferably upstream, of ii) a gene sequence, a fragment or variant thereof and can be, for example, any non-long terminal repeat (LTR) or any LTR of an endogenous retrovirus.

Preferably, the at least one TE is selected from the non-limiting group comprising AluY, AluSc8, THE1D-int, THE1D, MIRb, Charlie15a, AluSg7, AluSx, MIRb, L2c, L2b, LTR33, L2a, LTR66, LIMed, AluSq2, L1PA4, MIR, Charlie18a, AluSx, FRAM, MamTip2b, AluJo, MamTip2b, AluJo, AluSz, L1MD2, AluSz6, L1MD2, Tigger3b, L1MD3, HERVL40-int, L1Mec, L1ME1 and AluSc8, a fragment or variant thereof, or a combination of one or more thereof. Examples of combinations of one or more of the TE can comprise, e.g. one or more of the same TE (LTR66 & LTR66, *etc*..) or one or more of different TEs (LTR33 & L2a; *etc.*.).

In one aspect, the at least one TE is a LTR selected from the non-limiting group comprising LTR66, MIRb, L2a, L2b, L2c, and LTR33, a fragment or variant thereof, or a combination of one or more thereof.
Preferably, the at least one gene sequence, also named associated gene sequence, a fragment or variant thereof of the invention can be any gene sequence.

Preferably, the at least one gene sequence is selected from the group comprising ADTRP, CAST, CNOT1, DZIP1, PEX13, TMEM248, CAPN1, IGJ, TMEM131, POU5F1B, AHRR, AKAP7, MCUR1, PPP1R16A, SEC24B, SNED1, ZNF558, CSNK1G2, LPIN1, YIPF4, or a combination of one more thereof.

More preferably, the gene sequence is a POU5FB1 sequence, a fragment or a variant thereof.

The present invention also envisions methods for classifying a cancer subject as having a favourable prognosis or as having a poor prognosis based on the presence or absence of the at least one nucleic acid sequence of the invention.

As used herein, the term " favourable prognosis" or "good prognosis" refers to a patient or subject afflicted with cancer receiving, or not, a treatment that is likely to not present metastasis, and/or that is likely to not present cancer relapse, and/or that is likely to present a high overall survival (OS), event-free survival (EFS), and/or metastasis- free survival (MFS).

As used herein, the term "poor prognosis" or "bad prognosis" refers to a patient or subject afflicted with cancer receiving or not a treatment that is likely to present metastasis, and/or that is likely to present cancer relapse, and/or that is likely to present short overall survival (OS), progression free survival (PFS) and/or metastasis.

In one aspect, the present invention relates to a method for classifying a cancer subject, the method comprising i) detecting, directly or indirectly, in a biological sample of said subject, the presence or absence of at least one nucleic acid sequence comprising at least one transposable element (TE), a fragment or variant thereof, and a gene sequence, a fragment or variant thereof, and ii) classifying the cancer subject
- as having a favourable prognosis based on the absence of the at least one nucleic acid sequence in the biological sample or
- as having a poor prognosis based on the presence of the at least one nucleic acid sequence in the biological.
Preferably, the at least one nucleic acid is selected from the group listed in Table 1 and/or Table 2, a variant, a fragment or a combination thereof.

Preferably, the at least one nucleic acid sequence is selected from the group comprising:
- at least one transposable element (TE) selected from the group comprising AluY, AluSc8, THE1D-int, THE1D, MIRb, Charlie15a, AluSg7 and AluSx, a variant, a fragment or a combination thereof and
- a gene sequence selected from the group comprising CSNK1G2, LPIN1, and YIPF4 a fragment, a variant or a combination thereof.
Preferably, the at least one nucleic acid sequence is selected from the group comprising:
- at least one transposable element (TE) selected from the group comprising MIRb, L2c, L2b, LTR33, L2a, LTR66, L1Med, AluSq2, L1PA4, MIR, Charlie18a, AluSx, FRAM, MamTip2b, AluJo, MamTip2b, AluJo, AluSz, L1MD2, AluSz6, L1MD2, Tigger3b, L1MD3, HERVL40-int, L1Mec, L1ME1 and AluSc8, a variant, a fragment or a combination thereof, and
- a gene sequence selected from the group POU5F1B, AHRR, AKAP7, MCUR1, PPP1R16A, SEC24B, SNED1 and ZNF558 a fragment, a variant or a combination thereof.

Preferably, the gene sequence is a POU5F1B sequence, a fragment or a variant thereof and the TE is selected from the group comprising LTR66, LTR33, L2a, L2b, L2c and MIRb, a variant, a fragment or a combination thereof.

Usually, the cancer is selected from the group described above. Preferably, the cancer is a solid tumor cancer. More preferably, the solid tumor cancer is selected from the group comprising Colorectal Cancer, Gastric Cancer, Prostate Cancer, Liver Cancer, Testis Cancer, Ovary Cancer and Breast Cancer, or a combination of one of more of these cancers. Even more preferably, the cancer is Colorectal Cancer.

In another aspect, the present invention further envisions a method for classifying a cancer subject, the method comprising
1) detecting, directly or indirectly, in a biological sample of said subject, the presence or absence of
   - at least one nucleic acid sequence correlating with an increase of survival (POSITIVE PROGNOSIS), said nucleic acid sequence comprising at least one transposable element (TE), a fragment or variant thereof, and a gene sequence selected from the group CSNK1G2, LPIN1, and YIPF4, a fragment or variant thereof, and
   - at least one nucleic acid sequence correlating with a decrease of survival (NEGATIVE PROGNOSIS), said nucleic acid sequence comprising at least one transposable element (TE), a fragment or variant thereof, and a gene sequence selected from the group comprising POU5F1B, AHRR, AKAP7, MCUR1, PPP1R16A, SEC24B, SNED1 and ZNF558, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS),
   wherein the
   - number of protective events > number of harmful events is indicative of increase in survival (POSITIVE PROGNOSIS), and
   - number of harmful events > number of protective events is indicative of decrease in survival (NEGATIVE PROGNOSIS).

As shown in the examples and in particular in Fig 1a, TcGTs are directly correlated with a decrease in survival in a cumulative way. This happens, either by accumulating the presence of predictors of poor survival (harmful predictor), or the absence of predictors of good survival (protective predictor). In other words, a subject that suffering from cancer that has only 1 harmful event (e.g. gain of 2 harmful predictor and loss of 1 protective predictor) has much better prognosis than a patient that has 3 harmful events.

Preferably, the at least one TE is selected from the group comprising AluY, AluSc8, THE1Dint, THE1D, MIRb, Charlie15a, AluSg7, AluSx, MIRb, L2c, L2b, LTR33, L2a, LTR66, LIMed, AluSq2, L1PA4, MIR, Charlie18a, AluSx, FRAM, MamTip2b, AluJo, MamTip2b, AluJo, AluSz, L1MD2, AluSz6, L1MD2, Tigger3b, L1MD3, HERVL40-int, L1Mec, L1ME1 and AluSc8, a fragment or variant thereof, or a combination of one or more thereof.

As shown in the examples and in the corresponding Fig 1a, 300 patients have been classified considering their expression of TcGTs in primary colorectal cancer tumor, listed in Table 1. For every patient, the number of TcGTs correlating with an increase of survival (protective) minus the ones correlated with a decrease of survival (harmful) has been computed. Patients can have either the same number of protective and harmful events (1), more harmful than protective (2) or more protective than harmful (3) (Fig 1a).

A combination of these TcGTs (Table 1 and Fig 1a) builds a fingerprint (i.e. a signature) which is a stronger predictor of survival than any of the TcGTs alone (Fig 1b). The accumulation of expressed harmful predictors and the concomitant loss of expressed protective predictors in a patient suffering from colorectal cancer leads to a remarkably lower survival time (Fig 1b).

In a further aspect, the present invention envisions a method for classifying a cancer subject, the method comprising
1) detecting, directly or indirectly, in a biological sample of said subject, the presence or absence of
   - at least one nucleic acid sequence correlating with an increase of survival (POSITIVE PROGNOSIS), said nucleic acid sequence comprising at least one transposable element (TE), a fragment or variant thereof, and a gene sequence selected from the group comprising CAPN1, IGJ and TMEM131, a fragment or variant thereof, and
   - at least one nucleic acid sequence correlating with a decrease of survival (NEGATIVE PROGNOSIS), said nucleic acid sequence comprising at least one transposable element (TE), a fragment or variant thereof, and a gene sequence selected from the group comprising ADTRP, CAST, CNOT1, DZIP1, PEX13 and TMEM248,, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS),
   wherein the
   - number of protective events > number of harmful events is indicative of increase in survival (POSITIVE PROGNOSIS), and
   - number of harmful events > number of protective events is indicative of decrease in survival (NEGATIVE PROGNOSIS).

As shown in the examples and in particular in Fig 2 a, TcGTs are directly correlated with a decrease in survival in a cumulative way. This happens, either by accumulating the presence of predictors of poor survival (harmful predictor), or the absence of predictors of good survival (protective predictor). In other words, a subject suffering from cancer that has only 1 harmful event (e.g. gain of 2 harmful predictor and loss of 1 protective predictor) has much better prognosis than a patient that has 2 harmful events.

Preferably, the at least one TE is selected from the group comprisingAluSx1, HERVFH21-int, L1MD3, MIR, AluJr, AluSxl, AluY, AluSz6, AluJb, AluSq2, AluSxl, L2c, AluJb, AluSz, L1P2, LIMei, AluJb, AluY, AluSc, L4_C_Mam, MSTB1, AluJo, AluSz, L2a, L1M4, AluJr, a fragment or variant thereof, or a combination of one or more thereof.

The present invention further contemplates, a method for staging a cancer in a subject, the method comprising measuring, directly or indirectly, in a biological sample of said subject, the level of expression or activity of at least one nucleic acid sequence and comparing said level of expression or activity to a control value or range, wherein said at least one nucleic acid sequence comprises
i) at least one transposable element (TE), a fragment or variant thereof, and
ii) at least one gene sequence, a fragment or variant thereof.
Preferably, the at least one nucleic acid is selected from the group listed in Table 1 and/or Table 2, or a variant or fragment thereof.

Preferably, the gene sequence is a POU5F1B sequence, fragment or variant thereof and the cancer is a solid tumor cancer, preferably colorectal cancer. Also preferably, the TE is selected from the group comprising LTR66, LTR33, L2a, L2b, L2c and MIRb, a variant, a fragment or a combination thereof. Most preferably, the TE is LTR66, a variant, a fragment or a combination of one or more thereof.

As shown in the examples, the expression of the LTR66-POU5F1B TcGT tightly correlates with the transition from adenoma to carcinoma (stages I to IV of colorectal cancer).

The present invention also concerns a method for treating and/or preventing cancer and/or cancer metastasis in a subject in need thereof, said method comprising administering an inhibitor of at least one nucleic acid sequence of the invention.

As used herein, the term "treatment" or "treating" means any administration of a composition, pharmaceutical composition, therapeutic agent, compound, *etc*... of the disclosure to a subject for the purpose of:
(i) inhibiting the disease, that is, arresting the development of clinical symptoms; and/or
(ii) relieving the disease, that is, causing the regression of clinical symptoms. As used herein, the term "prevention" or "preventing" means any administration of a composition, pharmaceutical composition, therapeutic agent, compound, *etc*... of the disclosure to a subject for the purpose of preventing the disease, that is, causing the clinical symptoms of the disease not to develop;

In the context of the present invention, the disease is cancer, preferably a solid tumor cancer. More preferably, the solid tumor cancer is selected from the group comprising Colorectal Cancer, Gastric Cancer, Prostate Cancer, Liver Cancer, Testis Cancer, Ovary Cancer and Breast Cancer, or a combination of one of more of these cancers. Even more preferably, the cancer is Colorectal Cancer.

The present invention also contemplates an inhibitor of at least one nucleic acid sequence of the invention for use in the treatment and/or prevention of cancer and/or cancer metastasis.

In one aspect of the present invention, the inhibitor according to the present invention is typically a nucleic acid molecule comprising and/or encoding an oligonucleotide with the reverse complement sequence of the nucleic acid sequence (e.g. RNA) it inhibits. The inhibitor hybridizes to the nucleic acid sequence thereby negating or impairing its activity. The inhibitor typically comprises one or more modifications to enhance the hybridization of the inhibitor to the target nucleic acid sequence. A nucleic acid chemical analog preferably comprises an RNA backbone, preferably with one or more modification such as a 2-0'-methyl modification, a locked nucleic acid (LNA) modification, a morpholino modification and/or a peptide nucleic acid (PNA) modification. The modifications can be at one or more sugar moieties of the backbone. Next to these chemical modifications, it has been shown that adding flanking sequences to the antisense-based RNA inhibitors strongly enhances the inhibitory potency of these molecules. Preferably, these flanking sequences form stem-loop hairpin structures with a total of between 8 and 16 bases on both 5' and 3' end of the antisense oligomer. Most preferred are flanking sequences of 12 bases, with a 4 base pair (2x4 bases) stem region and a loop region of 4 bases. These added structural elements are thought to enhance and stabilize the interaction between the RNA-RISC complex and the inhibitor, thus prolonging the effect of the inhibitor.

A preferred inhibitor of the invention is an inhibitor selected from the group comprising an siRNA, an shRNA, an snRNA, a siRNA capable of interfering the expression of short hairpin (sh), apiRNA, a nucleic acid including an antisense oligonucleotide (e.g. ASOs, modified ASOs such as GapmeRs, ...) or nucleic acid chemical analog capable of interfering with the activity or expression of one or more nucleic acid sequence (e.g. RNA), a small RNA zipper and a morpholino antisense oligonucleotide capable of interfering with the expression of one or more nucleic acid sequence. Preferably, the inhibitor of the invention is an oligonucleotide that interferes with the activity or expression of the nucleic acid sequence (e.g. RNA) of the invention by hybridizing to the nucleic acid sequence of the corresponding nucleic acid sequence or to a variant or fragment thereof.

Alternatively, the inhibitor is a single-stranded RNA molecule complementary to the specific nucleic acid sequence (e.g. RNA) of the invention, or to a variant or fragment thereof and that is conjugated to cholesterol.

The terms "siRNA" and "short interfering RNA" as used herein are interchangeable and refer to single-stranded or double-stranded RNA molecules that are capable of inducing RNA interference. SiRNA molecules typically have a duplex region that is between 18 and 30 base pairs in length.

The terms "piRNA" and "Piwi-interacting RNA" are interchangeable and refer to a class of small RNAs involved in gene silencing. PiRNA molecules typically are between 26 and 31 nucleotides in length.

The terms "shRNA" as used herein refers to a nucleic acid molecule comprising at least two complementary portions hybridized or capable of specifically hybridizing to form a duplex structure sufficiently long to mediate RNAi (typically between 15-29 nucleotides in length), and at least one single-stranded portion, typically between approximately 1 and 10 nucleotides in length that forms a loop connecting the ends of the two sequences that form the duplex. Exemplary shRNA of the invention may, e.g. be selected from the group comprising the shRNA3 (5'-CACAGGTGATTATGATTTAAA-3', SEQ ID No. 46) and the shRNA5 (5'-CACATTCAGTCAACATTTA-3', SEQ ID No. 47) that were designed to be uniquely and selectively recognizing the 5'UTR of POUSF1B gene.

The terms "snRNA" and "small nuclear RNA" are interchangeable and refer to a class of small RNAs involved in a variety of processes including RNA splicing and regulation of transcription factors. The subclass of small nucleolar RNAs (snoRNAs) is also included. The term is also intended to include artificial snRNAs, such as antisense derivatives of snRNAs comprising antisense sequences directed against one or more acid nucleic sequence of the invention.

Examples of modified ASOs include the GapmeRs. As used herein, a GapmeR is a chimeric antisense oligonucleotide that contains a central block of deoxynucleotide monomers sufficiently long to induce RNase H cleavage. Usually, the GapmeRs of the invention are directed against one or more acid nucleic sequence of the invention.

An inhibitor of the invention as well as a variant or fragment thereof may be prepared by recombinant techniques using either prokaryotic or eukaryotic host cells. Alternatively, an inhibitor may be synthesized using commercially available synthesizers in known ways.

Additional considerations can be taken into account when designing antisense oligonucleotides, including: (i) sufficient specificity in binding to the target sequence; (ii) solubility; (iii) stability against intra- and extracellular nucleases; (iv) ability to penetrate the cell membrane; and (v) when used to treat an organism, low toxicity.

Preferably, the inhibitor of the invention is capable of interfering with the activity or expression of a nucleic acid sequence of the invention, preferably a nucleic acid sequence selected from the group comprising SEQ ID No. 1, SEQ ID No. 2, SEQ ID No. 3, SEQ ID No. 4 and SEQ ID No. 5, a variant, a fragment or a combination thereof.

In another aspect of the invention, the inhibitor is a chemical agent, oligonucleotide, antibody or an antigen-binding fragment of said antibody that interferes i) with the expression of a target gene of the nucleic acid sequence of the invention or ii) by specifically binding to a nucleic acid sequence of the invention or to a polypeptide encoded by a nucleic acid sequence of the invention.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to at least one peptide of the invention. Preferably the antibody is a monoclonal antibody. Preferably also the monoclonal antibody will be selected from the group comprising the IgG1, IgG2, IgG2a, IgG2b, IgG3 and IgG4 or a combination thereof. Most preferably, the monoclonal antibody is selected from the group comprising the IgG1, IgG2, IgG2a, and IgG2b, or a combination thereof.

An "antigen binding fragment" comprises a portion of a full-length antibody. Examples of antigen binding fragments include Fab, Fab', F(ab')2, and Fv fragments; diabodies; linear antibodies; single-chain antibody molecules; and multispecific antibodies formed from antibody fragments.

Also contemplated in the present invention is a pharmaceutical composition comprising a therapeutically effective amount of an inhibitor of the invention and a pharmaceutically acceptable carrier or diluent.

Usually, the pharmaceutical composition of the invention is for use in the treatment and/or prevention of cancer and/or cancer metastasis.

The term "therapeutically effective amount" as used herein means an amount of inhibitor of one or more nucleic acid sequence associated with cancer high enough to significantly positively modify the symptoms and/or condition to be treated, but low enough to avoid serious side effects (at a reasonable risk/benefit ratio), within the scope of sound medical judgment. The therapeutically effective amount of the inhibitor of one or more nucleic acid sequence associated with cancer is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the drug required to prevent, counter or arrest the progress of the cancer.

"Pharmaceutically acceptable carrier or diluent" means a carrier or diluent that is useful in preparing a pharmaceutical composition that is generally safe, non-toxic, and desirable, and includes carriers or diluents that are acceptable for human pharmaceutical use. Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

Further contemplated is a pharmaceutical composition comprising a therapeutically effective amount of a host cell (e.g. single cell or population of cells) and a pharmaceutically acceptable carrier or diluent. Preferably, the host cell has been modified by the introduction of i) a gene delivery vector comprising one or more nucleic acid(s) encoding the inhibitor of the invention or ii) one or more nucleic acid(s) encoding the inhibitor of the invention. The pharmaceutical composition comprising a therapeutically effective amount of a host cell and a pharmaceutically acceptable carrier or diluent can be administered to the subject in need thereof by any method and route known in the art and described herein.

The present invention further contemplates a method of treatment of cancer, and/or cancer metastasis comprising:
(a) providing an inhibitor of the invention and
(b) administering said inhibitor to a subject suffering from cancer.

Preferably, said inhibitor is in the form of a pharmaceutical composition comprising a therapeutically effective amount of said inhibitor as described above.

Alternatively, the method of treatment can be associated with a treatment selected from the group comprising radiotherapy, immunotherapy or hormone therapy.

"Administering", as it applies in the present invention, refers to contact of a therapeutically effective amount of an inhibitor of the invention, to the subject.
In general, methods of administering a composition comprising an inhibitor in the form of nucleic acids are well known in the art. In particular, the routes of administration already in use for nucleic acid therapeutics, along with formulations in current use, provide preferred routes of administration and formulation for the nucleic acids described herein.

Nucleic acid compositions can be administered by a number of routes including, but not limited to: oral, intravenous, intraperitoneal, intramuscular, transdermal, subcutaneous, topical, sublingual, or rectal means. Nucleic acids can also be administered via liposomes or nanoparticules. Such administration routes and appropriate formulations are generally known to those of skill in the art.

Administration of the formulations (e.g. pharmaceutical compositions) described herein may be accomplished by any acceptable method which allows the inhibitor or nucleic acid encoding the inhibitor to reach its target. The particular mode selected will depend of course, upon factors such as the particular formulation, the severity of the state of the subject being treated, and the dosage required for therapeutic efficacy. The actual effective amounts of drug can vary according to the specific drug or combination thereof being utilized, the particular composition formulated, the mode of administration, and the age, weight, condition of the patient, and severity of the symptoms or condition being treated.

Any acceptable method known to one of ordinary skill in the art may be used to administer a formulation to the subject. The administration may be localized (i.e., to a particular region, physiological system, tissue, organ, or cell type) or systemic, depending on the condition being treated.

Injections can be e.g., intravenous, intradermal, subcutaneous, intramuscular, or intraperitoneal. The composition can be injected intradermally for treating cancer, for example.

The injections can be given at multiple locations. Implantation includes inserting implantable drug delivery systems, e.g., microspheres, hydrogels, polymeric reservoirs, cholesterol matrixes, polymeric systems, e.g., matrix erosion and/or diffusion systems and non-polymeric systems, e.g., compressed, fused, or partially-fused pellets. Inhalation includes administering the composition with an aerosol in an inhaler, either alone or attached to a carrier that can be absorbed. For systemic administration, it may be preferred that the composition is encapsulated in liposomes.

Preferably, the agent and/or nucleic acid delivery system are provided in a manner which enables tissue-specific uptake of the agent and/or nucleic acid delivery system. Techniques include using tissue or organ localizing devices, such as wound dressings or transdermal delivery systems, using invasive devices such as vascular or urinary catheters, and using interventional devices such as stents having drug delivery capability and configured as expansive devices or stent grafts.
The formulations may be delivered using a bio-erodible implant by way of diffusion or by degradation of the polymeric matrix.

The administration of the formulation may be designed so as to result in sequential exposures to the inhibitor over a certain time period, for example, hours, days, weeks, months or years. This may be accomplished, for example, by repeated administrations of a formulation or by a sustained or controlled release delivery system in which the inhibitor is delivered over a prolonged period without repeated administrations. Administration of the formulations using such a delivery system may be, for example, by oral dosage forms, bolus injections, transdermal patches or subcutaneous implants. Maintaining a substantially constant concentration of the composition may be preferred in some cases.

Other delivery systems suitable include, but are not limited to, time-release, delayed release, sustained release, or controlled release delivery systems. Such systems may avoid repeated administrations in many cases, increasing convenience to the subject and the physician. Many types of release delivery systems are available and known to those of ordinary skill in the art. They include, for example, polymer-based systems such as polylactic and/or polyglycolic acids, polyanhydrides, polycaprolactones, copolyoxalates, polyesteramides, polyorthoesters, polyhydroxybutyric acid, and/or combinations of these. Microcapsules of the foregoing polymers containing nucleic acids are described in, for example, U.S. Patent No. 5,075,109. Other examples include nonpolymer systems that are lipid-based including sterols such as cholesterol, cholesterol esters, and fatty acids or neutral fats such as mono-, di- and triglycerides; hydrogel release systems; liposome-based systems; phospholipid based-systems; silastic systems; peptide based systems; wax coatings; compressed tablets using conventional binders and excipients; or partially fused implants. Specific examples include, but are not limited to, erosional systems in which the miRNA inhibitor is contained in a formulation within a matrix (for example, as described in U.S. Patent Nos. 4,452,775, 4,675,189, 5,736,152, 4,667,013, 4,748,034 and 5,239,660), or diffusional systems in which an active component controls the release rate (for example, as described in U.S. Patent Nos. 3,832,253, 3,854,480, 5,133,974 and 5,407,686). The formulation may be as, for example, microspheres, hydrogels, polymeric reservoirs, cholesterol matrices, or polymeric systems. The system may allow sustained or controlled release of the composition to occur, for example, through control of the diffusion or erosion/degradation rate of the formulation containing the miRNA inhibitor. In addition, a pump-based hardware delivery system may be used for delivery.
Also envisioned is a tumour-specific delivery of an inhibitor-coupled super-paramagnetic iron oxide nanoparticles as known in the art.

The present invention further contemplates a method of treatment of cancer, and/or cancer metastasis, comprising modifying a host cell, and reintroducing the host cell (e.g. single cell or population of cells) into the patient in need thereof, i.e. suffering from cancer and/or cancer metastasis.

Preferably, a biopsy or other tissue or biological fluid sample comprising the single cell or the population of cells may be necessary. Cells such as fibroblast cells or stem cells that can be generated directly from adult cells, such as iPSCs, are particularly preferred in this regard.
A gene delivery vector (e.g. plasmid or vector) or one or more nucleic acid(s) encoding an inhibitor of the invention can be introduced to host cell via one or more methods known in the art. These one or more methods include, without limitation, microinjection, electroporation, calcium phosphate-mediated transfection, cationic transfection, liposome transfection, dendrimer transfection, heat shock transfection, nucleofection transfection, magnetofection, lipofection, optical transfection, proprietary agent-enhanced uptake of nucleic acids, and delivery via liposomes, immunoliposomes, virosomes, or artificial virions.
It will be appreciated that in the present method the modification following the introduction of the gene delivery vector (plasmid or vector), or the one or more nucleic acid(s) encoding the inhibitor of the invention, to the host cell may occur ex vivo or in vitro, for instance in a cell culture and in some instances not in vivo. In other aspects, it may occur in vivo.
The host cell(s) (e.g. single cell or population of cells) of the invention is then reintroduced into the patient in need thereof by any route of administration and/or delivery methods known in the art, as described herein.
The invention also contemplates kits for the treatment and/or prevention of cancer and/or cancer metastasis. In one aspect of the invention, the kit comprises a pharmaceutical composition comprising an inhibitor of the invention.

In a further aspect, the invention contemplates a kit for the treatment and/or prevention of cancer and/or cancer metastasis comprising a host cell of the invention.

The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.
The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

Further contemplated in the present invention are nucleic acids encoding an siRNA, an shRNA, an snRNA, a siRNA capable of interfering the expression of short hairpin (sh), a piRNA, a nucleic acid including an antisense oligonucleotide (e.g. ASOs, modified ASOs such as GapmeRs, ...) of the invention.

Also contemplated is a nucleic acid sequence comprising at least one acid nucleic sequence of the invention. Preferably, the nucleic acid sequence is an RNA transcript, a cDNA of said RNA transcript or a DNA sequence. Most preferably, the at least one acid nucleic sequence of the invention is selected from the group comprising SEQ ID No 1, 2, 3, 4, 5, 6, 7, 8, 9, 10, 11, 12, 13, 14, 15, 16, 17 ,18, 19 ,20, 21, 22, 23, 24, 25, 26, 27, 28, 29, 30, 31, 32, 33, 34, 35, 36, 37, 38, 39, 40, 41, 42, 43, 44 and 45 a fragment or variant thereof or a combination of one or more thereof.

It is also understood that the methods of the invention also comprise detecting the presence or absence of at least one polypeptide encoded by a nucleic acid sequence of the invention, a fragment or variant thereof, in a biological sample. Detection of said at least one one polypeptide can be direct or indirect and determined by any suitable method known in the art.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### EXAMPLES

### Example 1

### Material and Methods

### Primary tumors data

The first dataset analyzed here is a publically available RNA-seq under the GEO accession number GSE50760. It includes RNA-seq data from 54 samples (normal colon, primary CRC, and liver metastasis) from 18 CRC patients.

The second RNA-seq and related clinical data was developed by Professor Claus Lindbjerg Andersen at the Department of Molecular Medicine at Aarhus University Hospital-Skejby, in Denmark. It contains RNA-seq and clinical data from paired tissue biopsies (tumor and normal mucosa) obtained from 301 patients, including 33 adenoma and 281 carcinoma samples, and 301 normal mucosa samples.

### RNA-seq analysis

RNA-Seq reads were aligned to the hg19 genome assembly using HISAT2 ^{[1]} (with parameters --rna-strandness R). Reads that were not uniquely mapped were discarded from the analysis using bamtools filter v2.4.1 with parameters -tag "NH:1". Read summarization on genes and TEs were generated using featureCounts ^{[2]} with parameters -s 2 -t exon -g gene_id -Q 10. Sequencing depth normalization was performed using the TMM method as implemented in the voom function from the R package LIMMA from Bioconductor ^{[3]}. The gene library sizes as given by voom were used to normalize the TEs counts.

### Transpochimeric transcripts analysis

For each sample, transcripts with reads spanning between genomic positions annotated as genes and TE loci were extracted from the aligned RNA-seq file. Genomic positions for genes were obtained from the ENSEMBL database for hg19 (GRCh37). TE loci were obtained from RepBase. Only transcripts, starting at a TE which was not overlapping an annotated gene exon, were considered.

### Cell culture

The cell lines used in this study were purchased from the American Type Culture Collection (HT29, SW620 and LS1034). All cell lines were cultured with their corresponding medium supplemented with 10% fetal calf serum at 37°C and 5% CO₂. HT29 cell line was cultured in McCoy's 5A medium; SW620 in Leibovitz's L-15 medium; and LS1034 in RPMI medium. Cells were transduced, selected with 2ug/mL puromycin and collected after 5 days for the colony formation assays experiments.

### Plasmids and lentiviral vectors

pLKO.puro shRNA vectors were used for POU5F1B knock down. The shRNA3 (5'-CACAGGTGATTATGATTTAAA-3') and the shRNA5 (5'-CACATTCAGTCAACATTTA-3') were designed to be uniquely matching the 5'UTR of POU5F1B gene. pTRE.GFP.puro and pTRE.POU5F1B.puro vectors were used for overexpression of GFP and POU5F1B proteins respectively. Lentiviral vectors production protocols are detailed at http://tronolab.epfl.ch and backbones are available at Addgene (http://www.addgene.org).

### Soft agar colony formation assays

A soft agar colony formation assay of suspension cells was performed in 6-well culture plates. In short, 20,000 cells were mixed in 0.37% low-gelling agar (Sigma)/medium and layered on top of a base layer of 0.7% low-gelling agar (Sigma)/medium. A layer of RPMI 10% FBS was settled on top to avoid agar drying and to allow cell feeding for 3 weeks. Cells were MTT-stained and colonies were quantified using Fiji software.

### Results

Colorectal cancer (CRC) has been the focus of our studies because its treatment is an unmet clinical need and its step-wise development has been extremely well documented. Furthermore, samples of tumor and healthy tissues are systematically obtained during surgery. We first analyzed a publicly available RNA-Seq dataset (GSE50760) comprising normal colon, primary tumor and liver metastasis from 18 CRC patients, searching for CRC-specific TcGTs. We found a very interesting candidate which was expressed in 0 out of 18 normal samples; 10 out of 18 primary CRC samples; and 14 out of 18 liver metastases. This transcript is encoded on chromosome 8 and initiates within an LTR66 to terminate at the 3'end of the POU5F1B gene, 170kb downstream.

In order to validate our findings and link TcGTs expression to survival, we analyzed another RNA-seq dataset comprising paired normal-primary tumor samples from 300 CRC patients and their survival data. We examined the non-annotated TcGTs that met the following criteria: they should start on a TE; this TE should not overlap any annotated coding exon; and they had to be expressed in more than 1% of the total number of samples. Then we fitted an additive Cox proportional hazards regression model and applied a backwards variable selection to determine which TcGTs were better at predicting survival of CRC patients. We ended up with 2 lists of TcGTs that predict survival in CRC patients if expressed in primary tumors (Table 1) or in normal tissue (Table 2). Notably, some TcGTs are predictors of better survival (protective, indicated as (+) in Tables 1 and 2), while some others are predictors of poorer survival (harmful, indicated as (-) in Tables 1 and 2).

**Table 1**

| The list of 25 TcGTs expressed in primary colorectal cancer (CRC) that implicate the coding sequence of 11 different genes and that predict survival in CRC patients. All the TcGTs indicated as (+) are predictors of better survival, while the ones indicated as (-) are predictors of poorer survival. | | | | | | | |
|---|---|---|---|---|---|---|---|
| **Chr** | **Start** | **End** | **Strand** | **Gene** | **TE in first exon** | **SEQ ID No. (cDNA)** | **Survival Predictor** |
| chr8 | 128420979 | 128429391 | + | POU5F1B | MIRb | 1 | - |
| chr8 | 128415837 | 128429403 | + | POU5F1B | L2c | 2 | - |
| chr8 | 128411137 | 128429310.5 | + | POU5F1B | L2b | 3 | - |
| chr8 | 128351525 | 128429402 | + | POU5F1B | LTR33 L2a | 4 | - |
| chr8 | 128256705 | 128429509 | + | POU5F1B | LTR66\|LTR66 | 5 | - |
| chr5 | 414781 | 427195 | + | AHRR | L1MEd | 6 | - |
| chr6 | 131475122 | 131604646 | + | AKAP7 | AluSq2 | 7 | - |
| chr6 | 131558665 | 131604652.5 | + | AKAP7 | L1PA4 | 8 | - |
| chr6 | 131457947 | 131540949 | + | AKAP7 | MIR | 9 | - |
| chr19 | 1951939 | 1981971 | + | CSNK1G2 | AluY | 10 | + |
| chr2 | 11895907 | 11967521 | + | LPIN1 | AluSc8 | 11 | + |
| chr2 | 11938218 | 11967520 | + | LPIN1 | THE1D-int\|THE1D | 12 | + |
| chr2 | 11888826 | 11967529 | + | LPIN1 | MIRb | 13 | + |
| chr6 | 13786823 | 13810832 | - | MCUR1 | Charlie18a | 14 | - |
| chr8 | 145709108 | 145727504 | + | PPP1R16A | AluSx \| FRAM \| MamTip2b \| AluJo | 15 | - |
| chr8 | 145710173 | 145727555 | + | PPP1R16A | MamTip2b \| AluJo \| AluSz \| L1MD2 \| AluSz6 \| L1MD2 | 16 | - |
| chr4 | 110421124 | 110461612 | + | SEC24B | Tigger3b | 17 | - |
| chr4 | 110373565 | 110461610 | + | SEC24B | L1MD3 | 18 | - |
| chr4 | 110372893 | 110426306.5 | + | SEC24B | L1MD3 | 19 | - |
| chr2 | 241959856 | 241973293 | + | SNED1 | HERVL40-int | 20 | - |
| chr2 | 241983886.5 | 241988918.5 | + | SNED1 | L1MEc \| L1ME1 | 21 | - |
| chr2 | 32521449 | 32537385 | + | YIPF4 | Charlie 15a \| AluSg7 | 22 | + |
| chr2 | 32471200 | 32537258 | + | YIPF4 | AluSx | 23 | + |
| chr2 | 32442175 | 32537286 | + | YIPF4 | AluSx | 24 | + |
| chrl9 | 8920352 | 8948808 | - | ZNF558 | AluSc8 | 25 | - |

**Table 2**

| The list of 16 TcGTs expressed in normal colon that implicate the coding sequence of 9 different genes and that predict survival in CRC patients. All the TcGTs indicated as (+) are predictors of better survival, while the ones indicated as (-) are predictors of poorer survival. | | | | | | | |
|---|---|---|---|---|---|---|---|
| **Chr** | **Start** | **End** | **Strand** | **Gene** | **TE** | **SEQ ID No. (cDNA)** | **Survival Predictor** |
| chr6 | 11711860 | 11796592 | - | ADTRP | AluSx1 | 26 | - |
| chr6 | 11712793 | 11788091 | - | ADTRP | HERVFH21-intIHERVFH21-int | 27 | - |
| chr11 | 64963277.5 | 64979477 | + | CAPN1 | L1MD3 | 28 | + |
| chr5 | 96048114 | 96051183 | + | CAST | MIR \| AluJr | 29 | - |
| chr16 | 58552633 | 58731620 | - | CNOT1 | AluSx1 | 30 | - |
| chr16 | 58552629.5 | 58728226 | - | CNOT1 | AluY | 31 | - |
| chr16 | 58573671 | 58599653 | - | CNOT1 | AluSz6 | 32 | - |
| chr16 | 58552630 | 58639605 | - | CNOT1 | AluJb | 33 | - |
| chr16 | 58578154 | 58598757.5 | - | CNOT1 | AluSq2 | 34 | - |
| chr16 | 58552630 | 58659539 | - | CNOT1 | AluSx1 | 35 | - |
| chr13 | 96241734.5 | 96254727.5 | - | DZIP1 | L2c \| AluJb | 36 | - |
| chr4 | 71520817.5 | 71542675 | - | IGJ | AluSz | 37 | + |
| chr2 | 61249240 | 61279298 | + | PEX13 | L1P2 | 38 | - |
| chr2 | 61250966 | 61279273 | + | PEX13 | LIMEi \| AluJb | 39 | - |
| chr2 | 61235089 | 61279343 | + | PEX13 | AluY | 40 | - |
| chr2 | 98372799 | 98603992 | - | TMEM131 | AluSc | 41 | + |
| chr2 | 98372799 | 98403262.5 | - | TMEM131 | L4_C_Mam | 42 | + |
| chr2 | 98435076 | 98600987 | - | TMEM131 | MSTB1 | 43 | + |
| chr7 | 66392653 | 66423538 | + | TMEM248 | AluJo | 44 | - |
| chr7 | 66406002 | 66423538 | + | TMEM248 | AluSz \| L2a \| L1M4 \| AluJr | 45 | - |

The 300 patients can be classified considering their expression of TcGTs in the primary tumor, listed in Table 1 (Fig 1a). For every patient, the number of TcGTs correlating with an increased survival (protective) minus the ones correlated with a decreased survival (harmful) is computed. This is depicted in the bar at the left of the plot (Fig 1a). Patients can have either the same number of protective and harmful events (1), more harmful than protective (2) or more protective than harmful (3) (Fig 1a).

A combination of these TcGTs (Table 1 and Fig 1a) builds a fingerprint which is a stronger predictor of survival than any of the TcGTs alone (Fig 1b). The accumulation of expressed harmful predictors and the concomitant loss of expressed protective predictors in a CRC patient leads to a remarkably lower survival time (Fig 1b).

The 300 patients can be as well classified considering the expression of TcGTs in their normal colon, listed in Table 2 (Fig 2a). As happens with the TcGTs fingerprint from the primary tumor, the accumulation of expressed harmful predictors and the concomitant loss of expressed protective predictors in the normal colon of a CRC patient leads to a lower survival time (Fig 2b).

We deeply studied the primary tumor TcGT signature and we found that the 5 different TcGTs encoding for POU5F1B displayed very striking numbers, being expressed in 2 out of 301 normal vs. 196 out of 313 primary CRC samples. The LTR66-POU5F1B TcGT, previously found in the GSE50760 RNA-seq dataset, is the longest of these 5 transcripts and it is the more frequent within CRC samples. Expression of the LTR66-POU5F1B transcript (SEQ ID No. 5) tightly correlates with the transition from adenoma (a benign tumor) to carcinoma (its malignant counterpart), which points out its potential application as a biomarker for CRC staging (Fig 3). Moreover, the presence of the 5 TcGTs of POUSF1B in CRC is linked to a lower survival time (Fig 4) and to a decrease in relapse-free survival (Fig 5).

The POU5F1B gene has been linked to an aggressive phenotype in gastric cancer^{[5]} and to prostate cancer susceptibility^{[6]}, has been proposed to promote hepatocellular carcinoma cells proliferation by activating AKT^{[7]} and is overexpressed in some human breast cancer^{[8]}. However, the POU5F1B gene has never been linked to colorectal cancer and, to our knowledge, no POU5F1B-related TcGT has been previously described. Our first approach for testing the impact of POU5F1B protein in CRC was to perform colony formation assays in the POU5F1B-transduced HT29 and SW620 CRC cell lines (Fig 6a); and in the POU5F1B-knocked down LS1034 CRC cell line (Fig 6b). This experiment confirmed an increase of the clonogenicity capacity in the CRC cell lines overexpressing POU5F1B protein, and the inverse phenotype in the POU5F1B-knock down conditions (Fig 6).

POU5F1B encodes for a transcription factor that is highly similar to POU5F1, also known as OCT4, a major determinant of embryonic stem cell pluripotency. The colony formation assay experiment (Fig 5), based on the ability of a single cell to transform into a single colony, prompted us to explore the possible participation of POUSF1B in CRC stemness^{[9]}. Taking advantage of recently published mRNA stemness indices^{[10]}, we examined the correlation of POU5F1B expression with stemnessmarkers in CRC. It was recently revealed that expression of KLF4, ABCG2 and EPAS2 anticorrelate with CRC mRNA stemness indices, which conversely correlate with the expression of MYC, CD44, LGR5, EZH2 and CTNNB1^{[10]}. We found POU5F1B transcripts levels to mimic those of cancer stemness indicators in our cohort of 300 CRC patients (Fig 7a and 7b). Therefore, POU5F1B displays the features of a colon cancer stem cell marker.

It has been widely reported that cancer stem cells (CSCs) have a high potential to metastasize and, owing to their resistance to chemo- and radiotherapy, they often cause relapse after treatment^{[11]}. Considering the potential role of POU5F1B in CRC stemness and the strong association between the POU5F1B TcGTs and a decrease in relapse-free survival in CRC patients (Fig 5), we aimed to study a possible link between POU5F1B and metastasis. In cancer progression, the initiation of metastasis occurs through the so-called epithelial-mesenchymal transition (EMT). EMT is a process by which epithelial cells lose their cell polarity and cell-cell adhesion, and gain migratory and invasive properties to become mesenchymal stem cells, which are multipotent stromal cells that can differentiate into a variety of cell types. We looked at the correlation of expression of POUSF1B with several epithelial and mesenchymal markers (Fig 8) and we found that POU5F1B expression anticorrelates with the expression of epithelial genes and correlates with the expression of mesenchymal genes, suggesting a role for POU5F1B in the mesenchymal transformation and, thus, in the metastatic process.

Colorectal cancers (CRCs) can be classified as cancers with microsatellite instability (MSI), which account for 10-20% of all CRC cases and are associated with a better prognosis; and cancers with chromosomal instability but not MSI (MSS), which are associated with worse prognosis^{[12]} We found the POU5F1B TcGTs more frequently expressed amongst MSS than MSI patients (Fig 9a). However, the presence of POU5F1B TcGTs had a better predictive power (Fig 4) than MSI or MSS phenotype (Fig 9b) (HR=1.69 and pval=0.0095 against HR=1.54 and Pval=0.148 for MSI/MSS).

We compared the predictive power of both markers (POU5F1B or MSI/MSS) by making them compete. To do so, we fit an additive Cox proportional hazards model with both POU5F1B transpochimera and MSI/MSS as predictive variables. The results showed that POU5F1B TcGT keeps a good predictive power (HR=1.45, pval=0.074) when used together with MSS, whereas MSS/MSI loses its predictive power (HR=1.23, pval=0.54) when used together with the POU5F1B transpochimera (Fig 9c). Therefore, the POU5F1B transpochimera is more informative than MSS/MSI in predicting survival and can be used as an independent predictor.

More recently, the CRC Subtyping Consortium (CRCSC) integrated and confronted gene expression-based CRC subtyping together with other data sources (mutation, copy number, methylation, microRNA and proteomics) in order to create four consensus molecular subtypes (CMSs) of CRC: CMS1 (microsatellite instability immune, 14%), hypermutated, microsatellite unstable and strong immune activation; CMS2 (canonical, 37%), epithelial, marked WNT and MYC signaling activation; CMS3 (metabolic, 13%), epithelial and evident metabolic dysregulation; and CMS4 (mesenchymal, 23%), prominent transforming growth factor-beta activation, stromal invasion and angiogenesis^{[13]}. POU5F1B transpochimeric transcripts are expressed in all CMS groups (Fig 10), suggesting that they are biomarkers of a newly defined subgroup of patients independent from the CMS classification.

Although the POU5F1B gene has been linked to gastric, prostate, liver and breast cancer^{[5-8]}, its implication in human cancer has not been formally studied. By examining data obtained from a cohort of 500 metastatic lesions originating from different primary tumor sites^{[14],}, we detected POU5F1B-related transpochimeric transcripts not only in CRC, but also in metastatic tissue of other primary tumors including prostate, breast, ovary and testis (Fig 11).

Since POU5F1B seemed to be a potential target for the treatment of CRC and other primary tumors, targeting the mRNAs or the corresponding translated protein is a promising therapeutic approach. In order to avoid as much as possible drug related side effects, it is very important to know which is the expression of this gene throughout human normal tissues. In figure 11 we plotted the expression of POUSF1B gene in the normal colon samples coming from the 300 CRC patients cohort, in the CRC primary tumors not expressing POU5F1B transpochimeric transcripts, in the CRC primary tumors expressing them, and in all the rest of human normal tissues. The expression of POUSF1B is clearly higher in the CRC primary tumors expressing POU5F1B transpochimeric transcripts compared to the rest of samples (Fig 12), which points out that anti-POU5F1B therapy could be a secure option.

### REFERENCES

1. Lamprecht B, Walter K, Kreher S, Kumar R, Hummel M, Lenze D, Kochert K, Bouhlel MA, Richter J, Soler E, Stadhouders R, Jöhrens K, Wurster KD, Callen DF, Harte MF, Giefing M, Barlow R, Stein H, Anagnostopoulos I, Janz M, Cockerill PN, Siebert R, Dörken B, Bonifer C, Mathas S. Derepression of an endogenous long terminal repeat activates the CSF1R proto-oncogene in human lymphoma. Nat Med. 2010;16(5):571-9.
2. Lock FE, Rebollo R, Miceli-Royer K, Gagnier L, Kuah S, Babaian A, Sistiaga-Poveda M, Lai CB, Nemirovsky O, Serrano I, Steidl C, Karimi MM, Mager DL. Distinct isoform of FABP7 revealed by screening for retroelement-activated genes in diffuse large B-cell lymphoma. Proc Natl Acad Sci USA. 2014;111(34):E3534-43.
3. Babaian A, Romanish MT, Gagnier L, Kuo LY, Karimi MM, Steidl C, Mager DL. Onco-exaptation of an endogenous retroviral LTR drives IRF5 expression in Hodgkin lymphoma. Oncogene. 2016;35(19):2542-6.
4. Lock FE, Babaian A, Zhang Y, Gagnier L, Kuah S, Weberling A, Karimi MM, Mager DL. A novel isoform of IL-33 revealed by screening for transposable element promoted genes in human colorectal cancer. PLoS One. 2017;12(7):e0180659.
5. Hayashi H, Arao T, Togashi Y, Kato H, Fujita Y, De Velasco MA, Kimura H, Matsumoto K, Tanaka K, Okamoto I, Ito A, Yamada Y, Nakagawa K, Nishio K. The OCT4 pseudogene POU5F1B is amplified and promotes an aggressive phenotype in gastric cancer. Oncogene. 2015;34(2):199-208.
6. Breyer JP, Dorset DC, Clark TA, Bradley KM, Wahlfors TA, McReynolds KM, Maynard WH, Chang SS, Cookson MS, Smith JA, Schleutker J, Dupont WD, Smith JR. An expressed retrogene of the master embryonic stem cell gene POU5F1 is associated with prostate cancer susceptibility. Am J Hum Genet. 2014;94(3):395-404.
7. Pan Y, Zhan L, Chen L, Zhang H, Sun C, Xing C. POU5F1B promotes hepatocellular carcinoma proliferation by activating AKT. Biomed Pharmacother. 2018;100:374-380.
8. Zhao FQ, Misra Y, Li DB, Wadsworth MP, Krag D, Weaver D, Tessitore J, Li DW, Zhang G, Tian Q, Buss K. Differential expression of Oct3/4 in human breast cancer and normal tissues. Int J Oncol. 2018 doi: 10.3892/ijo.2018.4341.
9. Manandhar S, Kim CG, Lee SH, Kang SH, Basnet N, Lee YM. Exostosin 1 regulates cancer cell stemness in doxorubicin-resistant breast cancer cells. Oncotarget. 2017;8(41):70521-70537.
10. Malta TM, Sokolov A, Gentles AJ, Burzykowski T, Poisson L, Weinstein JN, Kaminska B, Huelsken J, Omberg L, Gevaert O, Colaprico A, Czerwinska P, Mazurek S, Mishra L, Heyn H, Krasnitz A, Godwin AK, Lazar AJ; Cancer Genome Atlas Research Network, Stuart JM, Hoadley KA, Laird PW, Noushmehr H, Wiznerowicz M. Machine Learning Identifies Stemness Features Associated with Oncogenic Dedifferentiation. Cell. 2018;173(2):338-354.e15.
11. Nguyen LV, Vanner R, Dirks P, Eaves CJ. Cancer stem cells: an evolving concept. Nat Rev Cancer. 2012;12(2):133-43.
12. Kim CG, Ahn JB, Jung M, Beom SH, Kim C, Kim JH, Heo SJ, Park HS, Kim JH, Kim NK, Min BS, Kim H, Koom WS, Shin SJ. Effects of microsatellite instability on recurrence patterns and outcomes in colorectal cancers. Br J Cancer. 2016;115(1):25-33.
13. Guinney J, Dienstmann R, Wang X, de Reyniès A, Schlicker A, Soneson C, Marisa L, Roepman P, Nyamundanda G, Angelino P, Bot BM, Morris JS, Simon IM, Gerster S, Fessler E, De Sousa E Melo F, Missiaglia E, Ramay H, Barras D, Homicsko K, Maru D, Manyam GC, Broom B, Boige V, Perez-Villamil B, Laderas T, Salazar R, Gray JW, Hanahan D, Tabernero J, Bernards R, Friend SH, Laurent-Puig P, Medema JP, Sadanandam A, Wessels L, Delorenzi M, Kopetz S, Vermeulen L, Tejpar S. The consensus molecular subtypes of colorectal cancer. Nat Med. 2015;21(11):1350-6.
14. Robinson DR, Wu YM, Lonigro RJ, Vats P, Cobain E, Everett J, Cao X, Rabban E, Kumar-Sinha C, Raymond V, Schuetze S, Alva A, Siddiqui J, Chugh R, Worden F, Zalupski MM, Innis J, Mody RJ Tomlins SA, Lucas D, Baker LH, Ramnath N, Schott AF, Hayes DF, Vijai J, Offit K, Stoffel EM, Roberts JS, Smith DC, Kunju LP, Talpaz M, Cieślik M, Chinnaiyan AM. Integrative clinical genomics of metastatic cancer. Nature. 2017;548(7667):297-303.

## Claims

1. A method for detecting and/or predicting the likelihood of a cancer in a subject, the method comprising detecting, directly or indirectly, in a biological sample of said subject, the presence or absence of at least one nucleic acid sequence comprising
i) at least one transposable element (TE) a fragment or variant thereof, and
ii) a gene sequence, a fragment or variant thereof,
wherein the at least one additional nucleic acid is selected from the group listed in Table 1 and/or Table 2, or a variant or fragment thereof.

2. The method of claim 1, wherein the gene sequence is a POU5F1B sequence, fragment or variant thereof.

3. The method of claim 1 or 2, wherein the nucleic acid sequence is an RNA transcript, a cDNA of said RNA transcript or a DNA sequence, preferably an amplified DNA sequence.

4. The method of anyone of claims 1 to 3, wherein the at least one TE is a long terminal repeat (LTR) of an endogenous retrovirus.

5. The method of anyone of claims 1 to 3, wherein the at least one TE is selected from the group comprising LTR66, MIRb, L2b, LTR33 or a combination of one or more thereof.

6. The method of any one of claims 1 to 4, wherein the at least one acid nucleic sequence is selected from the group comprising SEQ ID No 1, 2, 3, 4, 5, 6, 7, 8, 9, 10, 11, 12, 13, 14, 15, 16, 17 ,18, 19 ,20, 21, 22, 23, 24, 25, 26, 27, 28, 29, 30, 31, 32, 33, 34, 35, 36, 37, 38, 39, 40, 41, 42, 43, 44 and 45, or a combination of one or more thereof.

7. A method for classifying a cancer subject, the method comprising
i) detecting, directly or indirectly, in a biological sample of said subject, the presence or absence of at least one nucleic acid sequence comprising at least one transposable element (TE) or a fragment or variant thereof, and a gene sequence, a fragment or variant thereof, wherein the at least one additional nucleic acid is selected from the group listed in Table 1 and/or Table 2 or a variant or fragment thereof and
ii) classifying the cancer subject
- as having a favourable prognosis based on the absence of the at least one nucleic acid sequence in the biological sample or
- as having a poor prognosis based on the presence of the at least one nucleic acid sequence in the biological.

8. The method of claim 7, wherein the gene sequence is a POU5F1B sequence, a fragment or a variant thereof.

9. The method of claim 7 or 8, wherein the at least one TE is a long terminal repeat (LTR) of an endogenous retrovirus.

10. The method of claim 7 or 8, wherein the at least one TE is selected from the group comprising LTR66, MIRb, L2b, LTR33, a fragment or variant thereof or a combination of one or more thereof.

11. The method for of any one of claims 7-10 wherein the cancer is colorectal cancer.

12. A method for classifying a cancer subject, the method comprising
1) detecting, directly or indirectly, in a biological sample of said subject, the presence or absence of
- at least one nucleic acid sequence correlating with an increase of survival (POSITIVE PROGNOSIS), said nucleic acid sequence comprising at least one transposable element (TE) a fragment or variant thereof, and a gene sequence selected from the group comprising CSNK1G2, LPIN1 and YIPF4, a fragment or variant thereof, and
- at least one nucleic acid sequence correlating with a decrease of survival (NEGATIVE PROGNOSIS), said nucleic acid sequence comprising at least one transposable element (TE) a fragment or variant thereof, and a gene sequence selected from the group comprising POU5F1B, AHRR, AKAP7, MCUR1, PPP1R16A, SEC24B, SNED1 and ZNF558, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS),
wherein the
- number of protective events > number of harmful events is indicative of increase in survival (POSITIVE PROGNOSIS).
- number of harmful events > number of protective events is indicative of decrease in survival (NEGATIVE PROGNOSIS).

13. A method for classifying a cancer subject, the method comprising
1) detecting, directly or indirectly, in a biological sample of said subject, the presence or absence of
- at least one nucleic acid sequence correlating with an increase of survival (POSITIVE PROGNOSIS), said nucleic acid sequence comprising at least one transposable element (TE) a fragment or variant thereof, and a gene sequence selected from the group comprising CAPN1, IGJ and TMEM131, a fragment or variant thereof, and
- at least one nucleic acid sequence correlating with a decrease of survival (NEGATIVE PROGNOSIS), said nucleic acid sequence comprising at least one transposable element (TE) a fragment or variant thereof, and a gene sequence selected from the group comprising ADTRP, CAST, CNOT1, DZIP1, PEX13 and TMEM248, a fragment or variant thereof,
2) scoring the number of nucleic acid sequences correlating with an increase of survival (POSITIVE PROGNOSIS) and the number of nucleic acid sequences correlating with a decrease of survival (NEGATIVE PROGNOSIS),
wherein the
- number of protective events > number of harmful events is indicative of increase in survival (POSITIVE PROGNOSIS), and
- number of harmful events > number of protective events is indicative of decrease in survival (NEGATIVE PROGNOSIS).

14. A method for staging a cancer in a subject, the method comprising measuring, directly or indirectly, in a biological sample of said subject, the level of expression or activity of at least one nucleic acid sequence and comparing said level of expression or activity to a control value or range, wherein said at least one nucleic acid sequence comprises
i) at least one transposable element (TE) a fragment or variant thereof, and
ii) a gene sequence, a fragment or variant thereof,
wherein the at least one additional nucleic acid is selected from the group listed in Table 1 and/or Table 2, or a variant or fragment thereof.

15. The method of claim 14, wherein the gene sequence is a POU5F1B sequence, fragment or variant thereof.

16. The method for staging of claim anyone of claims 14 to 15, wherein the level of expression or activity of said at least one nucleic acid sequence indicates that the subject has or is determined to have Stage I, II, III, or IV colorectal cancer.


SEQUENCE LISTING

<110>  Ecole Polytechnique Federale de Lausanne (E.P.F.L.)
       Aarhus University

<120>  Transpochimeric gene trancripts as cancer biomarkers

<130>  51395/EP

<160>  47

<170>  PatentIn version 3.5

<210>  1
<211>  1725
<212>  DNA
<213>  Homo sapiens

<400>  1
ggcctgtggg gctctgctgg atctcctcac ccagccacct gactggaatt cttcgcagag      60

ccagggcacc taaaggcaaa aggtacccgg tggtgaggac actcaagcag ctttatggaa     120

aagcccacat ggtgagcaac tgaggcctcc taccaacagc caacaagaaa ccggggcctt     180

tgccaacagc caagaaataa gaatcagaaa gaaggacaca ccaagaaaga aggacacacc     240

attcatagca cagaacacaa gtagatggct cccatgaaga atctagcatc atggtatggc     300

agaagcagaa tggactcagg aatcatggtg cctacactgg aatttcagat cggccactga     360

cacaatggac acattattca acatttccaa atcttggcat tcttatccac aaagtgaaga     420

taataattgt caattcacag gtgattatga tttaaagaga ttacttttga agagttccta     480

acacattcag tcaacattta atgatgcttc aggcactgtg ttcattgcta gtgagcgtat     540

gacacacaca gccatacggt cacagagctt tcaatgaaaa gtaatttctt tccggccccc     600

ggcttggggc gccttccttc cccatggcgg gacacctggc ttcggatttc gccttctcgc     660

cccctccagg cggtgggggt gatgggccat gggggcggga gccggctggg ttgatcctct      720

gacctggcta agcttccaag gccctcctgg agggccagat cgggccgggg gttgggccag     780

gctctgaggt gtgggggatt cccccttgcc ccccgccgta tgagttatgt ggggggatgg      840

cgtactgtgg gcctcaggtt ggagtggggc tagtgcccca aggcggcttg gagacctctc     900

agcctgagag cgaagcagga gtcggggtgg agagcaactc caatggggcc tccccggaac     960

cctgcaccgt cccccctggt gccgtgaagc tggagaagga gaagctagag caaaacccgg    1020

agaagtccca ggacatcaaa gctctgcaga aagaactcga gcaatttgcc aagctcctga    1080

agcagaagag gatcaccctg ggatatacac aggccgatgt ggggctcatc ctgggggttc    1140

tatttggaag gtgttcagcc aaagaccatc tgccgcttgg ctagcttcaa gaacatgtgt    1200

aagctgcggc ccttgctgca gaagtgggtg gaggaagctg acaacatgaa aatcttcagg    1260

agatatgcaa agcagaaacc ctcatgcagc ccgaaagaga aagcgaacca gtatcaaacg    1320

ggaggacctg gagaatttgt tcctgcagtg cccgaaaccc acactgcaga tcagccacat 1380

cgcccagcag cttgggctcg agaaggatgt ggtccgagtg tggttctgta accggcgcca 1440

gaagggcaag cgatcaagca gcgactatgc acaacgagag gattttgagg ctgctgggtc 1500

tcctttctca gggggaccag tgtcctttcc tccggcccca gggccccatt ttggtacccc 1560

aggctatggg agccctcact tcactgcact gtactcctca gtccctttcc ctgaggggga 1620

agtctttccc ccagtctccg tcatcactct gggctctccc atgcattcaa actgaggtgc 1680

ctgcccttct aggaatgggg aacaggggag gggaggagct aggga 1725

<210> 2
<211> 2201
<212> DNA
<213> Homo sapiens

<400> 2
ctttgacttc aaggcttaga ctcgtgcttc ctggaatgtc cttcattcct ccaggctggt 60

tccaagcttg tgaagaacat gatcgcagcc ctccagcaag gccgggtgaa aagaaccact 120

gccttagaac cctgcagatg caactttggg gacctgtcct atgatgcctg gcctgcgggc 180

tcttacgtgt ccccgccgca gacttgtcac agtggcactg tggggctctg ctggactcat 240

cagtggctcc ctcacccaca gccttcacaa ctctgactgg aattcttcgc agagccaggg 300

cacctaaagg caaaaggtca atgacgtaag aagactaatg tttccccata gtcaacgagt 360

agacgttagc acaaaatcta cctgctccct cttattctaa cttttcaccc aaatgaacaa 420

tattggcggc aaaacagtga aggctaatta ccctccctgc tctctctttc tatctctctg 480

ggtgaatcca gctgacatgt tgtgaggaca ctcaagcagc tttatggaaa agcccacatg 540

gtgagcaact gaggcctcct accaacagcc aacaagaaac cggggccttt gccaacagcc 600

aagaaataag aatcagaaag aaggacacac cattcatagc ataagaatca gaaagaagga 660

cacaccattc atagcacaga acacaagtag atggctccca tgaagaatct agcatcatgg 720

tatggcagaa gcagaatgga ctcaggaatc atggtgccta cactggaatt tcagatcggc 780

cactgacaca atggacacat tattcaacat ttccaaatct tggcattctt atccacaaag 840

tgaagataat aattgtcaat tcacaggtga ttatgattta aagagattac ttttgaagag 900

ttcctaacac attcagtcaa catttaatga tgcttcaggc actgtgttca ttgctagtga 960

gcgtatgaca cacacagcca tacggtcaca gagctttcaa tgaaaagtac ataattgctc 1020

atttcaccag gcccccggct tggggcgcct tccttcccca tggcgggaca cctggcttcg 1080

gatttcgcct tctcgccccc tccaggcggt gggggtgatg ggccatgggg ggcggagccg 1140

ggctgggttg atcctctgac ctggctaagc ttccaaggcc ctcctggagg gccaggaatc 1200

gggccggggg ttgggccagg ctctgaggtg tgggggattc ccccttgccc cccgccgtat 1260

gagttatgtg gggggatggc gtactgtggg cctcaggttg gagtggggct agtgccccaa 1320

ggcggcttgg agacctctca gcctgagagc gaagcaggag tcggggtgga gagcaactcc 1380

aatggggcct ccccggaacc ctgcaccgtc ccccctggtg ccgtgaagct ggagaaggag 1440

aagctagagc aaaacccgga gaagtcccag gacatcaaag ctctgcagaa agaactcgag 1500

caatttgcca agctcctgaa gcagaagagg atcaccctgg gatatacaca ggccgatgtg 1560

gggctcatcc tgggggttct atttggaagg tgttcagcca aaagaccatc tgccgctttg 1620

aggctctgca gcttagcttc aagaacatgt gtaagctgcg gcccttgctg cagaagtggg 1680

tggaggaagc tgacaacaat gaaaatcttc aggagatatg caaagcagaa accctcatgc 1740

agcccgaaag agaaagcgaa ccagtatcag aacgggaggc aacctggaga atttgttcct 1800

gcagtgcccg aaacccacac tgcagatcag ccacatcgcc cagcagcttg ggctcgagaa 1860

ggatgtggtc cgagtgtggt tctgtaaccg gcgccagaag ggcaagcgat caagcagcga 1920

ctatgcacaa cgagaggatt ttgaggctgc tgggtctcct ttctcagggg gaccagtgtc 1980

ctttcctccg gccccagggc cccattttgg taccccaggc tatgggagcc ctcacttcac 2040

tgcactgtac tcctcagtcc ctttccctga gggggaagtc tttcccccag tctccgtcat 2100

cactctgggc tctcccatgc attcaaactg aggtgcctgc ccttctagga atggggaaca 2160

ggggagggga ggagctaggg aaagagaacc tggagtttgt g 2201

<210> 3
<211> 1621
<212> DNA
<213> Homo sapiens

<400> 3
ccaggctaag tcacaagctt ttgttataag gtctcatcac accatatgcc tctctttcaa 60

aaccagcacc tgcagaaatt gaggacactc aagcagcttt atggaaaagc ccacatggtg 120

agcaactgag gcctcctacc aacagccaac aagaaaccgg ggcctttgcc aacagccaag 180

aaataagaat cagaaagaag gaggacacac cattcatagc acagaacaca agtagatggc 240

tcccatgaag aatctagcat catggtatgg cagaagcaga atggactcag gaatcatggt 300

gcctacactg gaatttcaga tcggccactg acacaatgga cacattattc aacatttcca 360

aatcttggca ttcttatcca caaagtgaag ataataattg tcaattcaca ggtgattatg 420

atttaaagag attacttttg aagagttcct aacacattca gtcaacattt aatgatgctt 480

caggcactgt gttcattgct agtagcggac acacacagcc atacggtcac agagctttca 540

atgaaaagta attcttccag gcggcccccc atggcgggac acctggcttc ggatttcgcc 600

ttctcgcccc ctccaggcgg tgggggtgat gggccatggg gggcggagcc gggctgggtt 660

gatcctcgct aagcttccaa ggccctcctg gagggccagg aatcgggccg ggggttgggc 720

caggctgggg gattccccct tgccccccgc cgtatgagtt atgtgggggg atggcgtact 780

gtgggcctca ggttggagtg gggctagtgc cccaaggcgg cttggagacc tctcagcctg 840

agagcgaagc aggagtcggg gtggagagca actccaatgg ggcctccccg gaaccctgca 900

ccgtcccccc tggtgccgtg aagctggaga aggagaagct agagcaaaac ccggagaagt 960

cccaggacat caaagctctg cagaaagaac tcgagcaatt tgccaagctc ctgaagcaga 1020

agaggatcac cctgggatat acacaggccg atgtggggct catcctgggg gttctatttg 1080

gaaggtgttc agccaaaaga ccatctgccg ctttgaggct gcagcttttc aagaacatgt 1140

gtaagctgcg gctgcgaagt gggtggagga agctgacaac atgaaaatct tcaggagata 1200

tgcaaagcag aaaccctcat gcagcccgaa agagaaagcg aaccatgcat ttcgccgaaa 1260

cccacactgc agatcagcca catcgcccag cagcttgggc tcgagaagga tgtggtccga 1320

gtgtggttct gtaaccggcg ccagaagggc aagcgatcaa gcagcgacta tgcacaacga 1380

gaggattttg aggctgctgg gtctcctttc tcagggggac cagtgtcctt tcctccggcc 1440

ccagggcccc attttggtac cccaggctat gggagcccca cttccactgt actcctcagt 1500

ccctttccct gagggggaag tctttccccc agtctccgtc atcactctgg gctctcccat 1560

gcattcaaac tgaggtgcct gccctctagg aatggggaac aggggagggg aggagctagg 1620

g 1621

<210> 4
<211> 2166
<212> DNA
<213> Homo sapiens

<400> 4
tgatggaaac tattgatgca gtgtccaagg tggagaaagg tcagagtgga tccagaggag 60

ccaagagaag acgtccagca tggtgacctg ggctcaagtc aaggtcctta tttctctggg 120

acagatggct gagttctact agcaaatgcc ggtgtggtcc tgttggccta ggaaaatccc 180

cctgactcac ccaggctaag tcacaagctt ttgttataag gtctcatcac accatatgcc 240

tctctttcaa aaccagcacc tgcagaaatc ccttcagcac agccacatgt gaagaacatg 300

atcgcagccc tccagcaagg ccgggtcccc cgcacgcgac ttgtcacagt ggcactgtgg 360

ggctctgctg gactcatcag tggctcctca cccacagcct tcacaactct gactggaatt 420

cttcgcagag ccagggcacc taaaggcaaa aggtctggtg aatccagctg acatgttgtg 480

aggacactca agcagcttta tggaaagccc cacatggtga gcaactgagg cctcctacca 540

acagccaaca agaaaccggg gcctttgcca acagccaaga aataagaatc agaaagaagg 600

acacaccatt caaaatcaga aagaaggaca caccattcta acagaacaca agtagatggc 660

tcccatgaag aatctagcat catggtatgg cagaagcaga atggactcag gaatcatggt 720

gcctacactg gaatttcaga tcggccactg acacaatgga cacattattc aacatttcca 780

aatcttggca ttcttatcca caaagtgaag ataataattg tcaattcaca ggtgattatg 840

atttaaagag attacttttg aagagttcct aacacattca gtcaacattt aatgatgctt 900

caggcactgt gttcattgct agtgagcgta tgacacacac agccatacgg tcacagagct 960

ttcaatgaaa agtacataat tgctcatttc accaggcccc cggcttgggg cgccttcctt 1020

ccccatggcg ggacacctgg cttcggattt cgccttctcg ccccctccag gcggtggggg 1080

tgatgggcca tggggggcgg agccgggctg ggttgatcct ctgacctggc taagcttcca 1140

aggccctcct ggagggccag gaatcgggcc gggggttggg ccaggctctg aggtgtgggg 1200

gattccccct tgccccccgc cgtatgagtt atgtgggggg atggcgtact gtgggcctca 1260

ggttggagtg gggctagtgc cccaaggcgg cttggagacc tctcagcctg agagcgaagc 1320

aggagtcggg gtggagagca actccaatgg ggcctccccg gaaccctgca ccgtcccccc 1380

tggtgccgtg aagctggaga aggagaagct agagcaaaac ccggagaagt cccaggacat 1440

caaagctctg cagaaagaac tcgagcaatt tgccaagctc ctgaagcaga agaggatcac 1500

cctgggatat acacaggccg atgtggggct catcctgggg gttctatttg gaaggtgttc 1560

agccaaaaga ccatctgccg ctttgaggct ctgcgctagc ttcaagaaca tgtgtaagct 1620

gcggcccttg ctgcagaagt gggtggagga agctgacaac aatgaaaatc ttcaggagat 1680

atgcaaagca gaaaccctca tgcagcccga aagagaaagc gaaccagtat cagaaccgag 1740

gagaggcaac ctggagaatt tgttcctgca gtgcccgaaa cccacactgc agatcagcca 1800

catcgcccag cagcttgggc tcgagaagga tgtggtccga gtgtggttct gtaaccggcg 1860

ccagaagggc aagcgatcaa gcagcgacta tgcacaacga gaggattttg aggctgctgg 1920

gtctcctttc tcagggggac cagtgtcctt tcctccggcc ccagggcccc attttggtac 1980

cccaggctat gggagccctc acttcactgc actgtactcc tcagtccctt tccctgaggg 2040

ggaagtcttt cccccagtct ccgtcatcac tctgggctct cccatgcatt caaactgagg 2100

tgcctgccct tctaggaatg gggaacaggg gaggggagga gctagggaaa gagaacctgg 2160

agtttg 2166

<210> 5
<211> 4374
<212> DNA
<213> Homo sapiens

<400> 5
gtcccagaac aggttgtgtg tgccccttga ggcgttcatc cagcactgtt tcagagaaat 60

ccctatttca atctattcct atacgttagt tattgaaaag caatagacaa tcacaaaaaa 120

caagttgacc tttttgtgtt ccttgagcca aagggccctc atgactgggc ctcacaccga 180

ataactcgtt acaaaaagag ctagggtccc agactgcgcc aaagcttcag gagactgctc 240

ctcgtctgtg cacagatgag tggccaactc tggagcccag gttgttgctt cctagtctgg 300

tggtgaatcc ttcatagtct gaggagaaga agaaaagaaa aagaaagaaa gagagaggaa 360

gaggaagatg atcagagtgg aaatgtggcc aggcgggggt acgatctcga ctcactgaaa 420

cctctgcttc ctggcttcaa gcatttctcc tgcctcagcc tcctgagtag ctgagattac 480

aggcgcccac caccaaaaaa aaatttagca aattcaacct taaacctgag tgcatggaaa 540

ctattgatgc agtgtccaag gtggagaaag gtcagagtgg atccagagga gccaagagaa 600

gacgtccagc atggtgacct gggctcaagt caaggtcctt atttctctga gtgggacagc 660

attggctgag ttctactagc aaatgccgga gatgtggttc ctgttggcct tttaactgat 720

gtgatgaaaa agaaaacaca tcttcaggaa aatcacccct gactcaccca ggctaagtca 780

caagcttttg ttataaggtc tcatcacacc atatgcctct ctttcaaaac cagcacctgc 840

agaaatgatc gtgcttccgg aatgtccttc attcccggtc acaagaacat gatcgcagcc 900

ctccagcacc ggggaaaaga accactgcct tagaaccctg cagatgcaac tttgaattac 960

tggacatcat agggactggg catcctgtcc ctatgatgca ccatgccgcc tgcgggcctt 1020

cttaatatgc agggctcccc cccgacttac agggcactgt ggggctctgc tggactcatc 1080

agtggctccc tcacccacag ccttcacaac tctgactgga attcttcgca gagccagggc 1140

acctaaaggc aaaaggtacc cggtgaacaa acatctcagc tataatttag atccatgctt 1200

tgtccgcatt tgcttccgcc ccccagttgt ccgcagtaag ctcacccagc ttctctatgt 1260

taattttcaa ccatatatcc agctgactct aagactccca ctcctaaatg tcctgcagcc 1320

cctcacctta aaatttccct tttgtgtatt atttctcgtg acagccccca aaacccaaga 1380

gtcttctttg atttttttct ctgtctcaac atctccaaac ctccccccta gccacttcca 1440

gtcttatctt ttatggtgag aaccagctct cagttgaggt ccaacctcag agtagcactt 1500

aagtgatacg ggaagagcaa agaaaccagt ctttatgcca cttggaatgg atttttccat 1560

tgcccaaaaa atgttcagaa aataatgacc aatgacgtaa gaaggactaa tgtttccccg 1620

catttaatag caagagattc cacggaaaat aaattgatgt taatactgac aacaaagaca 1680

aacaatggtg ataaatgaaa tggcaggttt caaactcaat ttcatcagga atttcatcaa 1740

catcagagcc cctgggccac agtcttcaat gtttgacaaa aaggattaaa gggcttatga 1800

ccttctgtcc agcattcatc ctgccccctt ccctagctgt acctcattat ctatcccagt 1860

gcctggcctg tgatatccct tattcagctt agcggattta cgagagacgt tcaggtcaca 1920

aaatctagcc tgtctcccca tgaaaccaca atgccattgt ctgaacagat taaataaagc 1980

agagcatcac ctcaaacgat tttcatggtt caaatctagt aaccttctat ttcactttca 2040

ttttaaaaaa atctaagtaa caatgaccag aaatatttta gtcagtctaa agacagatga 2100

taaaatgata actgaaaatc attaacagaa ttagcagaag ttattaccct gagaaagggg 2160

acttaaggtt ggaaggtaaa cactcaagac aaatactaca gaagtaaaga tggccccatg 2220

gagaaactgg cagcatggca tgatggaaat ggaatagaat caggtttttg tccggtgtga 2280

ctttggtgtt ctgactcaga cacatatttg agaggagaag gaggtaaaca cttaccttgt 2340

gccagacatt gcactaggac tttattacct gcaacccact gcaaaactgt gaagatttga 2400

tatgatcatt ctcattttat ggttgagaaa caagatctta ttctacctct tttcacaatc 2460

cctttaagaa atggacatcc aatatttgag ggcgaggcaa aaacatggcc taccaactat 2520

agctttacaa tacccaaata gtattccctt ctaatgaata aaataaaggt cttcctcttt 2580

cttaatctgg tgaatccagc tgacatgttg tgaggacact caagcagctt tatggaaaag 2640

cccacatggt gagcaactga ggcctcctac caacagccaa caagaaaccg gggcctttgc 2700

caacagccaa gaaataagaa tcagaaagaa ggacacacca ttcatagcaa agaatcagaa 2760

agaaggacac accattcata gcacagaaca caagtagatg gctcccatga agaatctagc 2820

atcatggtat ggcagaagca gaatggactc aggaatcatg gtgcctacac tggaatttca 2880

gatcggccac tgacacaatg gacacattat tcaacatttc caaatcttgg cattcttatc 2940

cacaaagtga agataataat tgtcaattca caggtgatta tgatttaaag agattacttt 3000

tgaagagttc ctaacacatt cagtcaacat ttaatgatgc ttcaggcact gtgttcattg 3060

ctagtgagcg tatgacacac acagccatac ggtcacagag ctttcaatga aaagtacata 3120

attgctcatt tcaccaggcc cccggcttgg ggcgccttcc ttccccatgg cgggacacct 3180

ggcttcggat ttcgccttct cgccccctcc aggcggtggg ggtgatgggc catggggggc 3240

ggagccgggc tgggttgatc ctctgacctg gctaagcttc caaggccctc ctggagggcc 3300

aggaatcggg ccgggggttg ggccaggctc tgaggtgtgg gggattcccc cttgcccccc 3360

gccgtatgag ttatgtgggg ggatggcgta ctgtgggcct caggttggag tggggctagt 3420

gccccaaggc ggcttggaga cctctcagcc tgagagcgaa gcaggagtcg gggtggagag 3480

caactccaat ggggcctccc cggaaccctg caccgtcccc cctggtgccg tgaagctgga 3540

gaaggagaag ctagagcaaa acccggagaa gtcccaggac atcaaagctc tgcagaaaga 3600

actcgagcaa tttgccaagc tcctgaagca gaagaggatc accctgggat atacacaggc 3660

cgatgtgggg ctcatcctgg gggttctatt tggaaggtgt tcagccaaaa gaccatctgc 3720

cgctttgagg ctctgcagct tagcttcaag aacatgtgta agctgcggcc cttgctgcag 3780

aagtgggtgg aggaagctga caacaatgaa aatcttcagg agatatgcaa agcagaaacc 3840

ctcatgcagc ccgaaagaga aagcgaacca gtatcaacgg gaggaacctg gagaatttgt 3900

tcctgcagtg cccgaaaccc acactgcaga tcagccacat cgcccagcag cttgggctcg 3960

agaaggatgt ggtccgagtg tggttctgta accggcgcca gaagggcaag cgatcaagca 4020

gcgactatgc acaacgagag gattttgagg ctgctgggtc tcctttctca gggggaccag 4080

tgtcctttcc tccggcccca gggccccatt ttggtacccc aggctatggg agccctcact 4140

tcactgcact gtactcctca gtccctttcc ctgaggggga agtctttccc ccagtctccg 4200

tcatcactct gggctctccc atgcattcaa actgaggtgc ctgcccttct aggaatgggg 4260

aacaggggag gggaggagct agggaaagag aacctggagt ttgtgaggac tctgaaacta 4320

gatattttct caagccctat ggacaaacac aaatcatgga gaaataaaga tgcc 4374

<210> 6
<211> 3161
<212> DNA
<213> Homo sapiens

<400> 6
ggcatggacc ctccccaggt gggtttggga gcccgccctt ggagacagga gatgatgcta 60

tcctgggggc tgccagctca gggcccagga gtggggccag gcagcccacc gagtactcgg 120

ccttcctgac ccgctgcttc atctgccgtg tgcgctgcct gctggacagc acctcgggct 180

tcctaaaccc agagcagaca ccgcagccac cgcggatgca aattggtgct cagggaacag 240

actgacgctg gccgatgggc acaggttccc gccagggccc catgcctgtg cctccggggt 300

tccttgaccc caaggggggc tcaggggaca gggaggagga gcagcaaggc acggatctga 360

gcagggcctc tggagtgaca gggcggaggg agactccagg acccacaaag cccctgccct 420

ggacagcggg aaagcacagt gaggatgggc ccgaggctgc agcccagcaa gaatgacccg 480

ccctcccccc ccgccccgcc cccccgggtt tcggactccc atctcccccc cccccccagc 540

accatgcggg atgtcggtga ggaccaggtg cccctcccct ctgccacttt ccccagagga 600

gcctgcagca ccagctccct cagcctggag ccagcgtttt gccacgaggg ccgaagccga 660

tgcctccggg ggacctgtgt ggtccgacag ccaccttccc accgcaaagc cagtctcgcc 720

agatcccggg ggctcagccc catgggaggg ccctgctggg cgcagcaggg agctgacccc 780

tttccaccct gcacactgtg cctgcctgga gcccacaacg gccttcccag tcggagcctc 840

ctctgtgccg gggccgaggc agtccttgca gagctgctga ggccgcccct gtggtcaagc 900

gggagccctg gacgcccggg atgtccaaaa ggcttgccac gctcgcccaa gctgggggcg 960

catagcgcag tgaccaccca gatctgtctt ctgtgtgggc ctaagacaca cctttagtgc 1020

atgcgagtct gctagtgtgt gtgtgcagca tagcaaaaat atcccaacag ttcttaaatg 1080

aaaactggcc ttaagtctat tcaagcatga cagcatttct ctttgaggat aaattggaaa 1140

gtgagagccc ggctgccaag tccacagtcg gggatgaagc agtcgggtga tgctcccaag 1200

tccgcagtcg gggatgaagc ggcgggtgat gctcccaagt ccgcagtcgg ggatgaagcg 1260

gtcgggtgac acacctagct cagccctccc aggcccctgc agctcccgcc tgtgcttgca 1320

ggcagggcag cccatcgcca tgactgtaga ggccagcaca cggcaaatta gaaatacaac 1380

agcgagaaag gggtccgtga gcccactcat agaggagttc caggcagcag aggctggggg 1440

gtcccacact gccccacccg tgcgcaggtg ctccggccat gacagagctg ggccagacct 1500

ggactggaat tgacagcata acccctgttc cttctggaca tctcccgagt tctcagtggg 1560

tctctgcgga cggttcttcc taatctgcct cttggtacat cacgtaatac agagttcaca 1620

gactccgggt ttggaagtac agagaaacac acaacgtaga gagacacagg aaactgcgct 1680

gcctgtgggg gttttctctg gctcctagga gaataattag ggacaagaca gacaagtatt 1740

aattgagagt aggaaattgg atacaaaagc ataagtcaga aagtcaccaa tccaccaacc 1800

cgagaaccta cagctgatgg tgcatttcag gctctgaacc ccacccggct ggtgcaggca 1860

tcagatcagg gtgtagaagt cccccaagca gaggagccag gcgtgaggcc ctggggtgtg 1920

gctgcagctg ggcccacctg tgcgggggga gggcatcggc gccctgacca ggagtgcgga 1980

gctgggggcc catgggtgag ggcccccgtg cattgtgccc ccgtgtggcg ccctcctgtt 2040

gttggcccgc gggtgaggga cccaccaccc cgcccccccg ttttcagtgg ctttgggtgt 2100

atgctggacc catttgttaa acgcaccttc actttgtcaa aacccaggtt ttggtccgtg 2160

cctgtgcgga tagggataag gatctcctct cagtactgga gagaacagcc aaccatctga 2220

gcccagagtc acagatccat cgtggccccc tatgaccccc aagccctacc gagggggcct 2280

cactctctgc ttagccaggg ggcgtctttc aaaaggtgcc tccatgctgt gctgtcgtgg 2340

gtgtgagacg tgctcatggc cttccactgc catctctccc ttatctgatg cctaaagttc 2400

acctgtggag agcaggcacc tgtgaagacc aggcacctga ggactggcgc ctcccactct 2460

ggcctctggg catttatgga tttaagacca ggatggtatt tcagaagctt cccacttcct 2520

tcctattcta accgagtgcc cagctccttg ctgatcatgg agacccttat aattaggcct 2580

gcaggccagg cgcagtggct catgcctata atcccagcct ttaggaggtc aaggtaggag 2640

gatcgcttaa gcccaggagt tcaagaccag cctgggcaac acaggaagaa tgtgtctcta 2700

caaaaaataa taaaaatcca acttggtcct ctgccagcca cgtcctgtgt cctggagctt 2760

agccctcagc agctcccttc agcctgggcg ccgcctgggt cccaaacgtg gcagctgctc 2820

tttacgtcta gtgtcctgga gccaacggct gtcactggga aaaacaccag ttggttgcac 2880

aagcgccctg cccaggaggc ttcttcccac gtcctgcccc gtgccctcca gcaggccacg 2940

ctgtgttgac gctgcactca gcacgggggt tggctctgcc gttttgtggt gtggggaccc 3000

tacaggaggc aaaaactccc caagtcagca ctttggttat tttatagcca caaccctctt 3060

ggaaaacagg gaagactatg gaacatagaa agtgtggatg tatcacttct ctctaaaatg 3120

tcattgtttt ctctcttttc gcttgaagtt caaatttttt t 3161

<210>  7
<211>  2795
<212>  DNA
<213>  Homo sapiens

<400>  7
   cgtggatcac ctgagtcact gatgaacctc aaataaattt gaagagaaga aagaatgggt      60
   caagagtgat caagtaaaga agaggaaaaa aaagagaaaa gattatcaac ccaactattt     120
   cctgtccatt ccaatcacca acaaagagat tataaaagga aaaaattaag atcctgcaga     180
   atgcaataat acaacaagat gagcgactgg ccaaagtgat ggttcctttc atattaccct     240
   gctggtgatg caattattaa atgaagatga agtaaacatt ggtattgatg ctcttttgga     300
   attgaaacca ttttaaaacc gaaaacattt gactcctttc aagggattgg tacttttgga     360
   aatcaggttg gatttgtgaa gctggcagaa ggagatcatg taaactcact tttggagata     420
   gcagtgcaaa taggacattt caagaaaaag gcatcctggt aggagagagc agaattttaa     480
   acctcatttg accttcatga agttgtcaaa atcaccgtgg ctccgtaaga atggagtgaa     540
   aaaaataatc ctatttataa agaaaaaaaa catttatatg aaaattatca gtcacagatt     600
   tggagaagaa atattatatc gcatagatct ttgctccatg ctgaagaaaa aacaaagtaa     660
   tggttattat cactgtgaat cttccattgt gattggtttg ctgctttcct ttctcaagag     720
   atgaaggaaa aatcagtgaa aagaacggag gggagcccga tgacgcgaac agcagcaagg     780
   atatacccag ttggtcaagt ggtgaaaaga acggagggga gggcaaaaga acggagggga     840
   gcccgatgac gctgaactag taaggctcag taagaggctg gtggagaacg cggtgctcaa     900
   ggctgtccag cagtatctgg aggaaacaca gaataaaaac aagccggggg aggggagctc     960
   tgtgaaaacc gaagcagctg atcagaatgg caatgacaat gagaacaaca ggaaatgagc    1020
   ccggaacgca ggcccccatg tctctgtgca aagcctccct gcttccctct gctgagtcta    1080
   gggactgact tgcagcgtgc tgtttaagtt aagtttctct ggtgcaatct gtgaagattg    1140
   cctaatactt ttcatgatcg atgtgttcgc attgctgaaa cacaacagaa gaaaaatgga    1200
   gtgctgggac tggcagagga aattaattga tgaaagaaga atggcccaag tttcattcgc    1260
   cctcagccac gcacaaggga aagggaactt tgggttatgc ctcctggacg caaattaaag    1320
   gccgagaaag aggccttgcc atcaatggaa tactgccatt tatattgctt agcagggcat    1380
   ttgactactt tatctgaggc cagaactctc acacacagct atcaagtgct aagtttaaaa    1440
   taatcactgt tggaattgtc atctgtacaa ttagtccata atgtttcatg tttgtcctaa    1500
   gtgtgctgtt gctatgcagt gtgatcttta tttatagtaa attatgtttc atgtaaatga    1560
   tatatttttg gtgaaatgca acctttctta taaaatgtgg gcaacatttt aaagtttttt    1620
   taaaatccta ttttgataag tcagatgcca tatttaatga aatgttatta tataattttt    1680
   ttttcttagg caagaaacct attggaattc gagacttaat taatgaagct ttgcatcgag 1740
   aaacgatggg tctgaagtcc aaagtgaaac agataaagga acttttatta aagcctgaga 1800
   ctcaggccag aattaggagg gagctttttg aaggaagact tattaacaac agtaattcag 1860
   caaatgacgt tgatttcagc acaactttga cataagctct acattgcgat tgtgacaaca 1920
   tagcttatga aatcttttca gcttattaag tagctctttg gtaaacacca aagaagtttc 1980
   tgatagtgtc tgcacaacag caaaccaaca tttggtgagg aattagcaat ttcttgccaa 2040
   agaaaattga ttctgcccaa ttattttttg agctacactt gtgttttaga atatctgttt 2100
   ctgtaatatt gagagttatt ttatagaaat gatttcttaa ttagctgttg tgagatattt 2160
   ctcgggtcct tgcagaaaaa aacatacaga ctgtgaacaa atcattcaca aacagaataa 2220
   aacagagcca acaacagtat tttaagggtc acttgcctcc tgttgacaca attgttgcta 2280
   aatcaaaaga agcgttgtcc aggtgtgtct acatctagtg ttacttttaa tgagaatttg 2340
   aatgtttatt gaacaatagt acttgaatga acatttataa atgtaattat tgcgatcact 2400
   ggttaagaat gttttatata tccttataat atttttcact gatcaaaatg ttgttctgct 2460
   ttttcatttc ttaaggaata catgtttggg atttttattt tttacgtgtc cgaagataag 2520
   ctccaggtct tatcgtatcc cttgccatct gaacttgttt gcactgcttc tgtttgaaag 2580
   agcatcttga aaaacttccc cggtatgatg attgttggta acaacttttt ctatagtcat 2640
   tgatggagta gatcatgatg gaggggaaat cactggagat caaatatgta aatcatttca 2700
   aatataaaat ccagtttact catggatttt agctattttt tcactgggaa attatactac 2760
   atttatttac aaatgagttt atgcattttc atggc 2795

<210> 8
<211> 2077
<212> DNA
<213> Homo sapiens

<400> 8
gtcctggact tttttgtgaa agagggagtc ctttctcaag agatgaagga aaaatcagtg 60

aaaagaacag gatataccag ttggtcaagt ggtgaaaaga acggagggag gaaggagggg 120

agcccgatga cgctgaacta gtaaggctca gtaagaggct ggtggagaac gcggtgctca 180

aggctgtcca gcagtatctg gaggaaacac agaataaaaa caagccgggg gaggggagct 240

ctgtgaaaac cgaagcagct gatcagaatg gcaatgacaa tgagaacaac aggaaatgag 300

cccggaacgc aggcccccat gtctctgtgc aaagcctccc tgcttccctc tgctgagtct 360

agggactgac ttgcagcgtg ctgtttaagt taagtttctc tggtgcaatc tgtgaagatt 420

gcctaatact tttcatgatc gatgtgttcg cattgctgaa acacaacaga agaaaaatgg 480

agtgctggga ctggcagagg aaattaattg atgaaagaag aatggcccaa gtttcattcg 540

ccctcagcca cgcacaaggg aaagggaact ttgggttatg cctcctggac gcaaattaaa 600

ggccgagaaa gaggccttgc catcaatgga atactgccat ttatattgct tagcagggca 660

tttgactact ttatctgagg ccagaactct cacacacagc tatcaagtgc taagtttaaa 720

ataatcactg ttggaattgt catctgtaca attagtccat aatgtttcat gtttgtccta 780

agtgtgctgt tgctatgcag tgtgatcttt atttatagta aattatgttt catgtaaatg 840

atatattttt ggtgaaatgc aaccttttct ataaaatgtg ggcaacattt taaagttttt 900

ttaaaatcct attttgataa gtcagtatgc catatttaat gaaatgttat tatataattt 960

ttttttctta ggcaagaaac ctattggaat tcgagactta attaatgaag ctttgcatcg 1020

agaaacgatg ggtctgaagt ccaaagtgaa acagataaag gaacttttat taaagcctga 1080

gactcaggcc agaattagga gggagctttt tgaaggaaga cttattaaca acagtaattc 1140

agcaaatgac gttgatttca gcacaacttt gacataagct ctacattgcg attgtgacaa 1200

catagcttat gaaatctttt cagcttatta agtagctctt tggtaaacac caaagaagtt 1260

tctgatagtg tctgcacaac agcaaaccaa catttggtga ggaattagca atttcttgcc 1320

aaagaaaatt gattctgccc aattattttt tgagctacac ttgtgtttta gaatatctgt 1380

ttctgtaata ttgagagtta ttttatagaa atgatttctt aattagctgt tgtgagatat 1440

ttctcgggtc cttgcagaaa aaaacataca gactgtgaac aaatcattca caaacagaat 1500

aaaacagagc caacaacagt attttaaggg tcacttgcct cctgttgaca caattgttgc 1560

taaatcaaaa gaagcgttgt ccaggtgtgt ctacatctag tgttactttt aatgagaatt 1620

tgaatgttta ttgaacaata gtacttgaat gaacatttat aaatgtaatt attgcgatca 1680

ctggttaaga atgttttata tatccttata atatttttca ctgatcaaaa tgttgttctg 1740

ctttttcatt tcttaaggaa tacatgtttg ggatttttat tttttacgtg tccgaagata 1800

agctccaggt cttatcgtat cccttgccat ctgaacttgt ttgcactgct tctgtttgaa 1860

agagcatctt gaaaaacttc cccggtatga tgattgttgg taacaacttt ttctatagtc 1920

attgatggag tagatcatga tggaggggaa atcactggag atcaaatatg taaaatcatt 1980

tcaaatataa aatccagttt actcatggat tttagctatt ttttcactgg gtaaattata 2040

ctacatttat ttacaaatga gtttatgcat tttcatg 2077

<210> 9
<211> 2608
<212> DNA
<213> Homo sapiens

<400> 9
cgcagctgtc aacatttact aacgtgagga attaatgtat caagaaaaaa gaaaatgtca 60

gaggaatttg aagccaatac tatggattct ctggtagaca tgccatttgc tactgtagat 120

attcaggatg actgtggaat cactgatgaa cctcaaataa atttgaagag aagcaagcaa 180

gaaaatgaat gggtcaagag tgatcaagta aagaagagga aaaaaaaaaa aattaccatt 240

tctctcatca ccaacaaaga gattataaaa ggaattaaga tcctgcagaa tgcaaagcag 300

aatgcaataa tacaacaaga tgagcgactg gccaaagcaa tggtcagtga tggttccttt 360

catattaccc tgctggtgat gcaattatta aatgaagatg aagtaaacat tggtattgat 420

gctcttttgg aattgaaaca gggaaaacat ttgactttgc cctttcaagg gattggtact 480

tttggaaatc aggttggatt tgtgaagctg gcagaaggag tcatgtaaac tcacttttgg 540

agatagcagg atgcaaatag ttaagaaaaa ggcatcctgg taggagagag cagaattttt 600

tatatgaaaa gtttacagca cagatttgga gaagaaatat tatatcgcat agatctttgc 660

tccatgctga agaaaaaaaa agttcactgg aatcttccat tgtgattggc ccgatgacgc 720

tgaactagta aggctcagta agaggctggt ggagaacgcg gtgctcaagg ctgtccagca 780

gtatctggag gaaacacaga ataaaaacaa gccggggggg agctctgtga aaaccgaagc 840

agctgatcag aatggcaatg acaatgagaa caacaggaaa tgagcccgga acgcaggccc 900

ccatgtctct gtgcaaagcc tccctgcttc cctctgctga gtctagggac tgacttgcag 960

cgtgctgttt aagttaagtt tctctggtgc aatctgtgaa gattgcctaa tactttggtt 1020

cgcattgctg aaacacaaca gaagaaaaat ggagtgctgg gactggcaga ggaaattaat 1080

tgatgaaaga agaatggccc aagtttcatt cgccctcagc cacgcacaag ggaaagggaa 1140

ctttgggtta tgcctcctgg acgcaaatta aaggccgaga aagaggcctt gccatcaatg 1200

gaatactgcc atttatattg cttagcaggg catttgacta ctttatctga ggccagaact 1260

ctcacacaca gctatcaagt gctaagttta aaataatcac tgttggaatt gtcatctgta 1320

caattagtcc ataatgtttc atgtttgcct ttatttatag taaattatgt ttcatgtaaa 1380

tgatatattt ttggtgaaat gcaacctttt ctataaaatg tgggcaacat tttaaagttt 1440

ttttaaaatc ctattttgat aagtcagtat gccatattta atgaatgtta ttatataatt 1500

tttttttctt gaaaacattg gaattcgaga cttaattaat gaagctttgc atcgagaaac 1560

gatgggtctg aagtccaaag tgaaacagat aaaggaactt ttattaaagc ctgagactca 1620

ggccagaatt aggagggagc tttttgaagg aagacttatt aacaacagta attcagcaaa 1680

tgacgttgat ttcagcacaa ctttgacata agctctacat tgcgattgtg acaacatagc 1740

ttatgaaatc ttttcagctt attaagtagc tctttggtaa acaccaaaga agtttctgat 1800

agtgtctgca caacagcaaa ccaacatttg gtgaggaatt agcaatttct tgccaaagaa 1860

aattgattct gcccaattat tttttgagct acacttgtgt tttagaatat ctgtttctgt 1920

aatattgaga gttattttat agaaatgatt tcttaattag ctgttgtgag atatttctcg 1980

ggtccttgca gaaaaaaaca tacagactgt gaacaaatca ttcacaaaca gaataaaaca 2040

gagccaacaa cagtatttta agggtcactt gcctcctgtt gacacaattg ttgctaaatc 2100

aaaagaagcg ttgtccaggt gtgtctacat ctagtgttac ttttaatgag aatttgaatg 2160

tttattgaac aatagtactt gaatgaacat ttataaatgt aattattgcg atcactggtt 2220

aagaatgttt tatatatcct tataatattt ttcactgatc aaaatgttgt tctgcttttt 2280

catttcttaa ggaatacatg tttgggattt ttatttttta cgtgtccgaa gataagctcc 2340

aggtcttatc gtatcccttg ccatctgaac ttgtttgcac tgcttctgtt tgaaagagca 2400

tcttgaaaaa cttccccggt atgatgattg ttggtaacaa ctttttctat agtcattgat 2460

ggagtagatc atgatggagg ggaaatcact ggagatcaaa tatgtaaaat catttcaaat 2520

ataaaatcca gtttactcat ggattttagc tattttttca ctgggaaatt atactacatt 2580

tatttacaaa tgagtttatg cattttca 2608

<210> 10
<211> 3650
<212> DNA
<213> Homo sapiens

<400> 10
gccggccgcc cagacgctgc ccgcgggccc ggccacggcg gagccaagct gtgagccgtg 60

agctttgagg cggtgggatg tgtcaggccg tgagctttga ggcggtggga tgtgtcagca 120

gaatgtctcc tgcccccgag agcgaccccg aggccactga gaagagcagc gcggcctggc 180

cggcccgaac gcctgcgtct cagtagctgg gagccacggg cccacgcccg cccaccggcc 240

gcagtgatgt tctagccaca gaggagccaa gacctcaggt ttccagagac ttgggatttg 300

cacggcagca gagtcaccgt ggagaggcca gggtatcaca aacttatgga ttttgacaag 360

aaaggaggga aaggggagac ggaggagggc cggagaatgt ccaaggccgg cgggggccgg 420

agcagccacg gcatccggag ctcggggacc agctcggggg tcctgatggt gggccccaac 480

ttccgcgtcg gcaagaagat cggctgcggc aacttcgggg agctccgcct aggaaagaat 540

ctctatacaa atgaatacgt ggctatcaaa ttggaaaggc ccgcaagacg ctgccaagag 600

catgaggcca gccaggctgg gggtggcacc gcccgtcccg ggatacatcg acagacagca 660

gaggacgtag cacacgcacg tctgaccttt gccggagaag gggacggctg ccgcaggaag 720

agctacacag ccgtgttcgc cggaactcaa aacttcaaac cggggcagtg tcctgagctc 780

taaaacttcg aaactgccag gcgaatcgct tgaacccggg tccgggcaac agaacgagac 840

tctgctccaa aaagaaaaaa actttgaaat ccctctaaaa ataaaatgga ccggggatgg 900

tagctgtgag gatggaccgt gtggcaggga aagaatctct atacaaatga atacgtggct 960

atcaaattgg agccgatcaa gtcccgggcc ccgcagctgc acctggagta ccggtgccga 1020

tcaagtcccg ggccccgcag ctgcacctgg agtaccggtt ctacaagcag ctcagcgcca 1080

cagagggcgt ccctcaggtc tactacttcg gtccgtgcgg ggagcgcggc gtctgtcctc 1140

cgccgcagag ggcgtccctc aggtctacta cttcggtccg tgcgggaagt acaacgccat 1200

ggtgctggag ctgctggggc ccagcctgga ggacctgttc gacctgtgcg accggacctt 1260

cacgctcaag acggtgctga tgatcgccat ccagctgatc acgcgcatgg agtatgtgca 1320

caccaagagc ctaatctacc gggtcacgcg catggagtat gtgcacacca agagcctaat 1380

ctaccgggac gtgaagcccg agaacttcct ggtgggccgc ccggggacca agcggcagca 1440

tgccatccac atcatcgact tcgggctggc caaggagtac atcgaccccg agaccaagaa 1500

gcacatcccg taccgcgagc acaagagcct gacgggcacg gcgcgctaca tgagcatcaa 1560

cacgcacctg ggcaaggagc agacgccgcg ggggccctcc gtccccgcag agcagagccg 1620

ccgcgacgac ctggaggcgc tgggccacat gttcatgtac ttcctgcgcg gcagcctccc 1680

ctggcagggg ctcaaggccg acacgctcaa ggagcggtac cagaagatcg gggacaccaa 1740

acgccgacac gctcaaggag cggtaccaga agatcgggga caccaaacgc gccacgccca 1800

tcgaggtgct tgcgagaact tcccagagga gatggccacg tacctgcgct atgtgcggcg 1860

cctggacttc ttcgagatgg ccacgtacct gcgctatgtg cggcgcctgg acttcttcga 1920

gaagcccgac tatgactacc tgcggaagct cttcaccgac ctcttcgacc gcagtggctt 1980

cgtgttcgac tatgagtacg actgggccgg gaagcccctg ccgaccccca tcggcaccgt 2040

ccacaccgac ctgccctccc agcctcaccc catcggcacc gtccacaccg acctgccctc 2100

ccagcctcag ctccgggaca aaacccagcc gcacagcaaa aaccaggcgt tgaactccac 2160

caacggggag ctgaatgcgg acgacccccg gccgcgttga actccaccaa cggggagctg 2220

aatgcggacg accccacggc cggccactcc aacgccccga tcacagcgcc tgcagaggtg 2280

gaggtggccg atgaaaccaa atgctgctgt ttcttcaaga ggagaaagag aaaatcgctg 2340

cagcgacctg ctgtttcttc aagaggagaa agagaaaatc gctgcagcga cacaagtgac 2400

cctgggcgcg tgcagccccc tgaatcttct ccgtgcagcc ccttggggcg cgaccttgtg 2460

cgaggccctc ggggcccacc cacagcggcc cagggccaga ccctggctgg aagccagaac 2520

gcagactgca ggggccgcgc ctggctcagg cggccccacc cccgggacgt ggggtcactt 2580

ccttcatgta agactttggc cgaaatttct acacctgtgt ctagtcctcc cctccaagag 2640

cattaactat ttaaaacaag gaaaagagga aaaaaaaaag ccccccccca ctcctgcccc 2700

tccgtttctt tgctgaagtg agtagtgtga tcctggaggc cccccggcct ggccccgccc 2760

cgccagccgc ccccgttagc gtcataaagt ccagcttgtc tccctcgatc caaaggccgt 2820

tttctcgagg ggagggcagg cccggcctgg aggggtgctg tggagctgtc ttgcccaggc 2880

cctcctggga gggggacagg cattgttgcc aggggtgagg ccgtgcccca ggcctccccg 2940

aaaccaaagg ggaaggcagg ggtggggccg tggctgaagc cggctcccca accaaaatgc 3000

tgcaccaaag ctcgggcgcc gcgggcacgg ctgctgcagt ctcttcccag cctggccctg 3060

gcaaggggcg ggtgggcgct gccaggcggg tgcttctcga cgcacttgct cccggaggct 3120

gcgccccggc gcctggaacc cgaggtggga ggaccggttg gtgtcaccct gctcggccct 3180

cagccctgcc gcgtggggcg cgtgggcacg gagcttcctg cctctctgct ccgacacccg 3240

gcaagcagcc ggagacaaaa cgccttaaag cccccggccc agccctgcag gtatattgca 3300

ggggcctggg ggcggccctg gactggcggg cggttcccca gtggggtgcc ctggaggctg 3360

ccgggcagag tggagcagct tgggccgtgc ccagggcggt ggctgtgagt ctagtttttg 3420

ctttaccaag tgtacagaaa tggcatttac gtttctctga tgctcccttg aagccataga 3480

atttaggggc tttttaaaaa aaaaaaaaag ggaatcagca gtagctccca gaagaacagc 3540

ggaaactgca ggcaggctgc cccgtccgcc ccgtgccttc tccccagaca acaccccata 3600

cccgcagcaa gggtggaaca gaaataatcc caagtcggca aggtggaatg 3650

<210> 11
<211> 6670
<212> DNA
<213> Homo sapiens

<400> 11
cggccacgcg cggcgccgct cggcgctgat tctggggcag ttaattacgt ggggcagtta 60

gccggccagg tgtttgtcac cgtgaaggag ctctacaagg ggctgaatcc cgccacactc 120

tcagggtgca ttgacatcat tgtcatccgc cagcccaatg gaaacctcca atgctcccct 180

ttccacgtcc gctttgggaa gatgggggtc ctgcgctccc gagagaaagt ggttgacata 240

gaaatcaatg gggaatctgt ggatttgcaa tagaaatcaa tggggaatct gtggatttgc 300

atatgaaatt gggagataat ggagaagcat tttttgttca agaaacagat aatgatcagg 360

aagttatccc tatgcacctg gccacctccc ccatcctgtc agaagctggc cacctccccc 420

atcctgtcag aaggagcttc gagaatggaa tgccagctga aaaggggctc tgtggacagg 480

atgagaggcc tggaccccag cacgccagcc caagtgatcg ctcccagcga gacgccgtca 540

agcagctctg tagtaaagaa gagaagaaaa aggaggagaa agtcacagct ggacagcctg 600

aagagagatg acaacatgaa cacatctgag gatgaggaca tgttccccat cgagatgagc 660

tcggatgagg ccatggagct gctggagagc agcagaactc ttcctaatga tatacctcca 720

ttccaagatg aagatatacc tccattccaa gatgatattc ctgaggaaaa cctctccctg 780

gctgtgattt accctcagtc agcctcatac cctaattcgg atagagagtg gtcacccact 840

cccagtcctc atcttgcagt tgcggccgag ggaggtctgt ctagttcttg ccctccacag 900

tcttccctgt tccatccttc ggaaagtcct tccggttccc gacctcaacg ttcccgacct 960

tcaacaccta aaagtgattc agaattggtc agcaagtcca cggaaaggac agggcagaag 1020

aacccagaaa tgctttggct gtggggagag ctgccgcagg ctgctaagtc ttcttctcca 1080

cacaagatga aagagtccag cccttgaaag agtccagccc attgagcagt agaaaaattt 1140

gtgataaaag tcactttcag gccattcaca gcgaatcttc agacactttt agtgaccaat 1200

cgccaactct ggtcggtggg gcacttttgg accagaacaa gcctcagaca gaaatgcagt 1260

ttgtgaatga agaagacctg gagaccttag gagcagcagc gccactcttg cccatgatcg 1320

aggagctcaa acccccctct gccagtgtag tccagacagc aaacaagacg gattctcctt 1380

ccaggaaaag agataaacga agccgacatc ttggtgctga cggcgaacga agccgacatc 1440

ttggtgctga cggcgtctac ttggatgacc tcacagacat ggatcctgaa gtggcggccc 1500

tgtattttcc caaaaaaatc tatcaaacga tccggattag aaagtgtgca agaagctggg 1560

cgcggtggct cacgcctgta atcccagcac tttgggaggc tgggggcgga tccttgaggt 1620

caggagttca agaccaccct ggccaacatg gtgaatcctc gttctactga aaataccaaa 1680

attggctgca cgtctggcat gcacctgtaa tcccagctac tcgggaggct gaggcaggag 1740

aattgcttga atccaggagg cggaggttgc attgagctga gatcatgcca ctgcacacca 1800

gcctgggcca agagtgcctt aaacctgcct gaataagggg gctgggcggc gccccacctc 1860

tggacagggg cgcagcaccg ggctgccctc cctgcaaagc cagtcatgtg tccacagtgg 1920

tcgtggtcca cagcccagta agtggcagct gtgcagccct tttttccccg gggtccctga 1980

tcctggggcc caccctcagt ccccctgcga tctagggcag ggctggcgcc tggaggggaa 2040

gggcagggaa cagggaggac agcttgcttc ttcttcagta tattttaggg ggatacaggg 2100

agatgcagcc agctgagaag aatggttttc tctcctaagc taaggcgttt aatgtctctt 2160

tccttgtcgg gtgctttaat aaacatctgt gctgagatgc agccgtctgc gctctggggt 2220

ggccgcgtgc cagagcatca ctggatgggt agaccggact ccgcttttcc tccttccagc 2280

ggagatcctt ccggactcgc aaaacatgca agcgacaacg gagcccggtc agccaaccag 2340

tccccgcagt cggtgggcag ctcgggcgtg gacagtggcg tggagagcac ctcggacggg 2400

ctgagggacc tcccttccat cgccatctcc ctctgcgggg gcctcagcga ccaccgggag 2460

atcacgaaag atgcattcct ggagcaagct gtgtcatatc aacagtttgt ggacattcct 2520

ggagcaagct gtgtcatatc aacagtttgt ggacaacccc gctattatcg atgaccccaa 2580

tctcgtggta aagattggga gtaaatatta taactggaca acagcagcac ccctcctcct 2640

ggcaatgcgc aacagcagca cccctcctcc tggcaatgca ggccttccag aaacctttgc 2700

caaaggccac tgtggaatct atcatgaggg ataaaatgcc caaaggaatc tatcatgagg 2760

gataaaatgc ccaaaaaggg aggaagatgg tggttttcat ggaggggaag aaacaccaca 2820

atcaaggagg aaagtaagcc agagcagtgc ttggctggca aggcccatag cccaagccag 2880

agcagtgctt ggctggcaag gcccatagca ccggagagca accgccgcag ctcagcttgg 2940

ccaccagggt aaagcatgaa tcatcctcca gtgatgagga gcgcggcatg aatcatcctc 3000

cagtgatgag gagcgcgcag ctgccaagcc atcaaacgca ggccacctcc ctcttctgcc 3060

taatgtcagc tacaagaaga ctctccggct gacttccgag cagcttaaaa gcttgaagtt 3120

gaagaatggc cccaacgact ggcttagttg aagaatggcc ccaacgacgt ggttttcagt 3180

gtcaccacgc agtaccaagg cacgtgccgc tgtgagggca ccatctatct gtggaactgg 3240

gatgataaag tcatcatttc tgatattgat gggacaatta ccagatcaga tactcttggc 3300

cacattttgc ccacccttgg gaaggatact cttggccaca ttttgcccac ccttgggaag 3360

gattggaccc atcagggcat cgctaagctg taccataaag tgagccagaa tggatataaa 3420

tttctctact gttctgcccg tgataaattt ctctactgtt ctgcccgtgc catcgggatg 3480

gcggacatga cgcggggcta cctgcactgg gtcaacgaga ggggcacggt gctgccccag 3540

gggcccctgc tgctgagtcc cagcagcctc ttctctgccc tgcacagaga agtgattgaa 3600

aagaagccag aaaagtttaa agtccagtgt gattgaaaag aagccagaaa agtttaaagt 3660

ccagtgtttg acagacatca aaaacctgtt tttccccaac acagaaccct tttatgctgc 3720

ttttggaaac cgaccagctg atgtgtattc atacaagcaa gtaggagtgt ctttgaatag 3780

aatattattc atacaagcaa gtaggagtgt ctttgaatag aatatttacc gtcaacccta 3840

aaggagagct ggtacaggaa catgcaaaga ccaacatctc ttcgtatgtg agactctgtg 3900

aagtagtcga ccacgttttc ccgttgctgg tgagactctg tgaagtagtc gaccacgttt 3960

tcccgttgct gaaaagaagc cattcttcag actttccctg ttcggatacc ttcagtaact 4020

tcaccttttg gagagagcca ctgccacctt ttgaaaacca ggacattcat tctgcctcag 4080

cgtaaaatgt cccaagcagc ctcttgccag cagtgcagag cctggttgtc acccattaaa 4140

ggataggtct ccccggagtg cacagctcca cctgggagcc tggcgcgtca tcattggcct 4200

gacagcagag agaattgaga agcatttctc ccctgcccca ccccggggct gacatttcta 4260

agcaagatag gaagggagca ctttctaggc taggagttgg gtgcatttgt accgtgaaaa 4320

gcattcctca gttgtggctt aatgccagtt acgacgctgc ctttccggcc tgctccagca 4380

agtagctact ggttcacgtg cagtttgggg ctgtgaaacc taggcagaag gcggctgtct 4440

gagggctgtc cccgcctagg acagggtcaa tcgaggaatg ccagatgtgc acggtttttg 4500

gcaaagtagg gggcacattt ccattatagc aatgttagtg ccaccacctt ctgaacacag 4560

tggggagggc tgtgaaggct catgtgacct ggatctgagg tctctgatag aaatctggac 4620

gccaccgggt ccaggcctgg cctcagactt ggccttgtgg atgggcccct tacagtattt 4680

gctgactagt ctcattttta ggtgataatt tttctttaat tcctttggtt aaagatagtc 4740

tatttcattg gcatatctcc ccccagtttt tgtggctcaa ggctggaata tttatgcctt 4800

aatatatcta tggcagacat ttaagaatgc gctttatcta gctcatggta actttgcaac 4860

gccttagatt aaaatgacag taaatattac taaggcagta ttttgaatga gtttgacact 4920

gccggcttcc ttccatccag cgaggtggtg ctgacagtgt ggacttgagc acacttatgc 4980

aaatgataat gatactgact tctgttggga gctctccaaa gaaactggtt ggttttaaga 5040

aaatagtttc aagaagttca actatattct tttagatatt atgtattgtt ttactctgat 5100

taggttactg tgataggcat ttattcatat tctttctata ccactgtcat taatatatta 5160

aaaagatgta tgtgttagac tatcgaaagg gccttattct ctctttctca tagactgacc 5220

ttcttttgga atttctgagt catttatttt ccttagcttt ttccactcaa attaagggca 5280

agcgaaaaag taataatttg gcattcttta agcctacaga atgtgattct ttcacttgtt 5340

tattacactg gctcgtggac agaacaattt gaaaagtgaa agaattattt tggtaaaaga 5400

ttttgcttta cttttcgaag cattattttt ttaaagagtg ttttactcca acgattgaaa 5460

cattttccta tttaaatttc attgttagaa tcacaggagg caaaaaatgg aacggtgaat 5520

gaaattttac tctttctgtg aaagaaaatc cacagagttg ttgcctccgt tgtagttggt 5580

gggccccgtt agcattggat gcctttgcca aatggttcat gtggacacac aaaggcaaac 5640

agatctgcca tcgatcgcag atttctgtag aaacacggat gtgcatgtgc agattccctt 5700

ttgcaggtat taaaaataat taaaaatagt cctgcctgag gttgcagtga gccgagcttg 5760

cactactgca ctccagcctg ggtgacagag taagactcca tgtcaaaaaa aaaaaaaaaa 5820

aaaaaaaacc tcaactcctc tgcgcttgtt cactagtaac ctaaagaggc tatattcatt 5880

ctttatgcaa tgagggtatt tttgagtgaa ttttaactgc tctgaactaa gtataagctc 5940

atgggcctgc aaaggttcag acggtttctc ctttgcaccc aggaggaact ttggctgcga 6000

gaatgggggg atgtatccct catgcagttg gcatccaggc agccctctgc agcagcacac 6060

cctgcaggcg gagttttcag aggatgcaat tttggatccc gaattttgat gtaccttaaa 6120

cttccacatc actgcaccct gaaacagagc atgctttcca gaaagtcaca ctctcagatc 6180

tgtgtcaagt tcaatgtgag ccctggcaag gctggcatat taacacctgc cttctggctt 6240

ctgaaagtga gatttgtata tgggctgcac tcacgcatat acgagttggt ttatctttgt 6300

gtacatgact ataacccagt gatgctgagg tcatgtgctg gaatgctgta tttggaccac 6360

acatttcaaa gttgccctat ggaaatgaat cctacttagt gacaagtcat caaatgtttg 6420

tcacatgtga tgaagacaaa tatgtatacc tggcatagag aaaaatatat acctggtaca 6480

ttggagaaaa ataattacac tttcaaagag aattcccttt gcaattttat gtttggatca 6540

ccactgtaag cacactttat ttgcatttga tctgtatttg tatatgctga tgcaatgata 6600

aaaatcactg taatacttca ttgtgttgta ctggatgcaa agctagaaaa tattgcaata 6660

aatgagaccg 6670

<210> 12
<211> 3750
<212> DNA
<213> Homo sapiens

<400> 12
agcttggcca ccagggtaaa gcatgaatca tcctccagtg atgaggagcg cgctgctttt 60

cctttgcctc agccatgatt ggtaaagcat gaatcaccag catgaatcat cctccagtga 120

tgaggagcgc gcagctgcca agccatcaaa cgcaggccac ctccctcttc tgcctaatgt 180

cagctacaag aagactctcc ggctgacttc cgagcagctt aaaagcttga agttgaagaa 240

tggccccaac gacggtagct tgaagttgaa gaatggcccc aacgacgtgg ttttcagtgt 300

caccacgcag taccaaggca cgtgccgctg tgagggcacc atctatctgt ggaactggga 360

tgataaagtc atcatttctg atattgatgg gacaattacc agatcagata ctcttggcca 420

cattttgccc acccttggga atttggccac attttgcccc cttgggaagg attggaccca 480

tcagggcatc gctaagctgt accataaagt gagccagaat ggatataaat ttctctactg 540

ttctgcccga atttctctac tgttctgccc gtgccatcgg gatggcggac atgacgcggg 600

gctacctgca ctgggtcaac gagaggggca cggtgctgcc ccaggggccc ctctgctgag 660

tcccagcagc ctcttctctg ccctgcacag agaagtgatt gaaaagaagc cagattgaaa 720

agaagccaga aaagtttaaa gtccagtgtt tgacagacat caaaaacctg tttttcccca 780

acacagaacc cttttatgct gcttttggaa accgaccagc tgatgtgtat tcatacaagc 840

aagtaggagt gtctttgaat agtgtgtttt aaagcaagta ggagtgtctt tgaatagaat 900

atttaccgtc aaccctaaag gagagctggt acaggaacat gcaaagacca acatctcttc 960

gtatgtgaga ctctgtgaag tagtcgacca cgttttcccg ttgtgtgaga ctctgtgaag 1020

tagtcgacca cgttttcccg ttgctgaaaa gaagccattc ttcagacttt ccctgttcgg 1080

ataccttcag taacttcacc ttttggagag agccactgcc accttttgaa aaccaggaca 1140

ttcattctgc ctcagcgtaa aatgtcccaa gcagcctctt gccagcagtg cagagcctgg 1200

ttgtcaccca ttaaaggata ggtctccccg gagtgcacag ctccacctgg gagcctggcg 1260

cgtcatcatt ggcctgacag cagagagaat tgagaagcat ttctcccctg ccccaccccg 1320

gggctgacat ttctaagcaa gataggaagg gagcactttc taggctagga gttgggtgca 1380

tttgtaccgt gaaaagcatt cctcagttgt ggcttaatgc cagttacgac gctgcctttc 1440

cggcctgctc cagcaagtag ctactggttc acgtgcagtt tggggctgtg aaacctaggc 1500

agaaggcggc tgtctgaggg ctgtccccgc ctaggacagg gtcaatcgag gaatgccaga 1560

tgtgcacggt ttttggcaaa gtagggggca catttccatt atagcaatgt tagtgccacc 1620

accttctgaa cacagtgggg agggctgtga aggctcatgt gacctggatc tgaggtctct 1680

gatagaaatc tggacgccac cgggtccagg cctggcctca gacttggcct tgtggatggg 1740

ccccttacag tatttgctga ctagtctcat ttttaggtga taatttttct ttaattcctt 1800

tggttaaaga tagtctattt cattggcata tctcccccca gtttttgtgg ctcaaggctg 1860

gaatatttat gccttaatat atctatggca gacatttaag aatgcgcttt atctagctca 1920

tggtaacttt gcaacgcctt agattaaaat gacagtaaat attactaagg cagtattttg 1980

aatgagtttg acactgccgg cttccttcca tccagcgagg tggtgctgac agtgtggact 2040

tgagcacact tatgcaaatg ataatgatac tgacttctgt tgggagctct ccaaagaaac 2100

tggttggttt taagaaaata gtttcaagaa gttcaactat attcttttag atattatgta 2160

ttgttttact ctgattaggt tactgtgata ggcatttatt catattcttt ctataccact 2220

gtcattaata tattaaaaag atgtatgtgt tagactatcg aaagggcctt attctctctt 2280

tctcatagac tgaccttctt ttggaatttc tgagtcattt attttcctta gctttttcca 2340

ctcaaattaa gggcaagcga aaaagtaata atttggcatt ctttaagcct acagaatgtg 2400

attctttcac ttgtttatta cactggctcg tggacagaac aatttgaaaa gtgaaagaat 2460

tattttggta aaagattttg ctttactttt cgaagcatta tttttttaaa gagtgtttta 2520

ctccaacgat tgaaacattt tcctatttaa atttcattgt tagaatcaca ggaggcaaaa 2580

aatggaacgg ttgaatgaaa ttttactctt tctgtgaaag aaaatccaca gagttgttgc 2640

ctccgttgta gttggtgggc cccgttagca ttggatgcct ttgccaaatg gttcatgtgg 2700

acacacaaag gcaaacagat ctgccatcga tcgcagattt ctgtagaaac acggatgtgc 2760

atgtgcagat tcccttttgc aggtattaaa aataattaaa aatagtcctg cctgaggttg 2820

cagtgagccg agcttgcact actgcactcc agcctgggtg acagagtaag actccatgtc 2880

aaaaaaaaaa aaaaaaaaaa aaaaccttaa ctaactcctc tgcgcttgtt cactagtaac 2940

ctaaagaggc tatattcatt ctttatgcaa tgagggtatt tttgagtgaa ttttaactgc 3000

tctgaactaa gtataagctc atgggcctgc aaaggttcag acggtttctc ctttgcaccc 3060

aggaggaact ttggctgcga gaatgggggg atgtatccct catgcagttg gcatccaggc 3120

agccctctgc agcagcacac cctgcaggcg gagttttcag aggatgcaat tttggatccc 3180

gaattttgat gtaccttaaa cttccacatc actgcaccct gaaacagagc atgctttcca 3240

gaaagtcaca ctctcagatc tgtgtcaagt tcaatgtgag ccctggcaag gctggcatat 3300

taacacctgc cttctggctt ctgaaagtga gatttgtata tgggctgcac tcacgcatat 3360

acgagttggt ttatctttgt gtacatgact ataacccagt gatgctgagg tcatgtgctg 3420

gaatgctgta tttggaccac acatttcaaa gttgccctat ggaaatgaat cctacttagt 3480

gacaagtcat caaatgtttg tcacatgtga tgaagacaaa tatgtatacc tggcatagag 3540

aaaaatatat acctggtaca ttggagaaaa ataattacac tttcaaagag aattcccttt 3600

gcaattttat gtttggatca ccactgtaag cacactttat ttgcatttga tctgtatttg 3660

tatatgctga tgcaatgata aaaatcactg taatacttca ttgtgttgta ctggatgcaa 3720

agctagaaaa tattgcaata aatgagaccg 3750

<210> 13
<211> 6462
<212> DNA
<213> Homo sapiens

<400> 13
ggcggccacg cgcggcgccg ctcggtgcag accatgaatt acgtggggca gttagccgag 60

gtgcagacca tgaattacgt ggggcagttc atgaagccca gcaaggagtg gagagaggag 120

ggctggaggc cccgcctgcc atgacccatc ccgtggggca gttagccggc caggtgtttg 180

tcaccgtgaa ggagctctac aaggggctga atcccgccac actctcaggg tgcattgaca 240

tcattgtcat ccgccagccc aatggaaacc tccaatgctc ccctttccac gtccgctttg 300

ggaagatggg ggtcctgcgc tcccgagaga aagtggttga catagaaatc aatggggatc 360

tgggatttgc atatgacata gaaatcaatg gggaatctgt ggatttgcat atgaaattgg 420

gagataatgg agaagcattt tttgttcaag aaacagataa tgatcaggaa gttatcccta 480

tgcacctggc cacctccccc atcctgtcag aaggatccta tgcacctggc cacctccccc 540

atcctgtcag aaggagcttc gagaatggaa tgccagctga aaaggggctc tgtggacagg 600

atgagaggcc tggaccccag cacgccagcc caagtgatcg ctcccagcga gacgccgtca 660

agcagctctg tagtaaagaa gagaagaaaa aggaggagaa agtcacagct ggacagcctg 720

aagagagatg acaacatgaa cacatctgag gatgaggaca tgttccccat cgagatgagc 780

tcggatgagg ccatggagct gctggagagc agcagaactc ttcctaatga tatacctcca 840

ttccaagatg atattcctga gctcctaatg atatacctcc attccaagat gatattcctg 900

aggaaaacct ctccctggct gtgatttacc ctcagtcagc ctcataccct aattcggata 960

gagagtggtc acccactccc agtggacctt caggagttaa gccccttgca gttgcggccg 1020

agggaggctg tctagttctt gccctccaca gtcttccctg ttccatcctt cggaaagtcc 1080

ttccggttcc cgacctcatc cccgaccttc aacacctaaa agtgattcag aattggtcag 1140

caagtccacg gaaaggacag ggcagaagaa cccagaaatg ctttggctgt ggggagagct 1200

gccgcaggct gctaagtctt cttctccaca caagatgaaa gagtccagcc cattgagcaa 1260

ctccacacaa gatgaaagag tccagcccat tgagcagtag aaaaatttgt gataaaagtc 1320

actttcaggc cattcacagc gaatcttcag acacttttag tgaccaatcg ccaactctgg 1380

tcggtggggc acttttggac cagaacaagc ctcagacaga aatgcagttt gtgaatgaag 1440

aagacctgga gaccttagga gcagcagcgc cactcttgcc catgatcgag gagctcaaac 1500

ccccctctgc cagtgtagtc cagacagcaa acaagacgga ttctccttcc aggaaaagag 1560

ataaacgaag ccgacatctt ggtgctgacg gcgtctactt taaacgaagc cgacatcttg 1620

gtgctgacgg cgtctacttg gatgacctca cagacatgga tcctgaagtg gcggccctgt 1680

attttcccaa aaacggagat ccttccggac tccaaaacga tcgtttcccc ttatgtttga 1740

ttagaaagtg tgcaagacag ctgggcgcgg tggctcacgc ctgtaatccc agcactttgg 1800

gaggctgagg tgggcggatc acttgaggtc aggagttcaa gacaccctgc caacatggtg 1860

aatcctcgtt ctactgaaaa taccaaaatt ggctgcacgt gctggcatgc acctgtaatc 1920

ccagctactc gggaggctga ggcaggagaa tgcttgaatc caggaggcgg aggttgcatt 1980

gagctgagat catgccactg cacaccagcc tgggccacgg agatccttcc ggactcgcaa 2040

aacatgcaag cgacaacgga gcccggtcag ccaaccagtc cccgcagtcg gtgggcagct 2100

cgggcgtgga cagtggcgtg gagagcacct cggacgggct gagggacctc ccttccatcg 2160

ccatctccct ctgcgggggc ctcagcgacc accgggagat cacgaaagat gcattcctgg 2220

agcaagctgt gtcatatcaa cagtttgtgg acaagtgcaa gctgtgtcat atcaacagtt 2280

tgtggacaac cccgctatta tcgatgaccc caatctcgtg gtaaagattg ggagtaaata 2340

ttataactgg acaacagcag cacccctcct cctggcaatg ataactggac aacagcagca 2400

cccctcctcc tggcaatgca ggccttccag aaacctttgc caaaggccac tgtggaatct 2460

atcatgaggg ataaaatgcc caaaaagggg gaatctatca tgagggataa aatgcccaaa 2520

aagggaggaa gatggtggtt ttcatggagg ggaagaaaca ccacaatcaa ggaggaaagt 2580

aagccagagc agtgcttggc tggcaaggcc catagcaccg gaaccagagc agtgcttggc 2640

tggcaaggcc catagcaccg gagagcaacc gccgcagctc agcttggcca ccagggtaaa 2700

gcatgaatca tcctccagtg atgaggagcg cgcagtgaac atgaatcatc ctccagtgat 2760

gaggagcgcg cagctgccaa gccatcaaac gcaggccacc tccctcttct gcctaatgtc 2820

agctacaaga agactctccg gctgacttcc gagcagctta aaagcttgaa gttgaagaat 2880

ggccccaacg acgtggtttt agctgagttg aagaatggcc ccaacgacgt ggttttcagt 2940

gtcaccacgc agtaccaagg cacgtgccgc tgtgagggca ccatctatct gtggaactgg 3000

gatgataaag tcatcatttc tgatattgat gggacaatta ccagatcaga tactcttggc 3060

cacattttgc ccacccttgg gaaggattgg atactcttgg ccacattttg cccacccttg 3120

ggaaggattg gacccatcag ggcatcgcta agctgtacca taaagtgagc cagaatggat 3180

ataaatttct ctactgttct gcccgtgcca tcgggatgaa tggatataaa tttctctact 3240

gttctgcccg tgccatcggg atggcggaca tgacgcgggg ctacctgcac tgggtcaacg 3300

agaggggcac ggtgctgccc caggggcccc tgctgctgag tcccagcagc ctcttctctg 3360

ccctgcacag agaagtgatt gaaaagaagc cagaaaagtt taaagtccag gtaaggattg 3420

aaaagaagcc agaaaagttt aaagtccagt gtttgacaga catcaaaaac ctgtttttcc 3480

ccaacacaga acccttttat gctgcttttg gaaaccgacc agctgatgtg tattcataca 3540

agcaagtagg agtgtcttta tagaaagtgt gtattcatac aagcaagtag gagtgtcttt 3600

gaatagaata tttaccgtca accctaaagg agagctggta caggaacatg caaagaccaa 3660

catctcttcg tatgtgagac tctgtgaagt agtcgaccac gttttcccgt tgctgtttgt 3720

gagactctgt gaagtagtcg accacgtttt cccgttgctg aaaagaagcc attcttcaga 3780

ctttccctgt tcggatacct tcagtaactt caccttttgg agagagccac tgccaccttt 3840

tgaaaaccag gacattcatt ctgcctcagc gtaaaatgtc ccaagcagcc tcttgccagc 3900

agtgcagagc ctggttgtca cccattaaag gataggtctc cccggagtgc acagctccac 3960

ctgggagcct ggcgcgtcat cattggcctg acagcagaga gaattgagaa gcatttctcc 4020

cctgccccac cccggggctg acatttctaa gcaagatagg aagggagcac tttctaggct 4080

aggagttggg tgcatttgta ccgtgaaaag cattcctcag ttgtggctta atgccagtta 4140

cgacgctgcc tttccggcct gctccagcaa gtagctactg gttcacgtgc agtttggggc 4200

tgtgaaacct aggcagaagg cggctgtctg agggctgtcc ccgcctagga cagggtcaat 4260

cgaggaatgc cagatgtgca cggtttttgg caaagtaggg ggcacatttc cattatagca 4320

atgttagtgc caccaccttc tgaacacagt ggggagggct gtgaaggctc atgtgacctg 4380

gatctgaggt ctctgataga aatctggacg ccaccgggtc caggcctggc ctcagacttg 4440

gccttgtgga tgggcccctt acagtatttg ctgactagtc tcatttttag gtgataagtt 4500

ttttttatct ttggttaaag atagtctatt tcattggcat atctcccccc agtttttgtg 4560

gctcaaggct ggaatattta tgccttaata tatctatggc agacatttaa gaatgcgctt 4620

tatctagctc atggtaactt tgcaacgcct tagattaaaa tgacagtaaa tattactaag 4680

gcagtatttt gaatgagttt gacactgccg gcttccttcc atccagcgag gtggtgctga 4740

cagtgtggac ttgagcacac ttatgccaaa tgataatgat actgacttct gttgggagct 4800

ctccaaagaa actggttggt tttaagaaaa tagtttcaag aagttcaact atattctttt 4860

agatattatg tattgtttta ctctgattag gttactgtga taggcattta ttcatattct 4920

ttctatacca ctgtcattaa tatattaaaa agatgtatgt gttagactat cgaaagggcc 4980

ttattctctc tttctcatag actgaccttc ttttggaatt tctgagtcat ttattttcct 5040

tagctttttc cactcaaatt aagggcaagc gaaaaagtaa taatttggca ttctttaagc 5100

ctacagaatg tgattctttc acttgtttat tacactggct cgtggacaga acaatttgaa 5160

aagtgaaaga attattttgg taaaagattt tgctttactt ttcgaagcat tattttttta 5220

aagagtgttt tactccaacg attgaaacat tttcctattt aaatttcatt gttagaatca 5280

caggaggcaa aaaatggaac ggttgaatga aattttactc tttctgtgaa agaaaatcca 5340

cagagttgtt gcctccgttg tagttggtgg gccccgttag cattggatgc ctttgccaaa 5400

tggttcatgt ggacacacaa aggcaaacag atctgccatg atcgcagatt tctgtagaaa 5460

cacggatgtg catgtgcaga ttcccttttg caggtattaa aaataattaa aaatagtcct 5520

gcctgaggtt gcagtgagcc gagcttgcac tactgcactc cagcctgggt gacagagtaa 5580

gactccatgt caaaaaaaaa aaaaaaaaaa aaaacttata actcctctgc gcttgttcac 5640

tagtaaccta aagaggctat attcattctt tatgcaatga gggtattttt gagtgaattt 5700

taactgctct gaactaagta taagctcatg ggcctgcaaa ggttcagacg gtttctcctt 5760

tgcacccagg aggaactttg gctgcgagaa tggggggatg tatccctcat gcagttggca 5820

tccaggcagc cctctgcagc agcacaccct gcaggcggag ttttcagagg atgcaatttt 5880

ggatcccgaa ttttgatgta ccttaaactt ccacatcact gcaccctgaa acagagcatg 5940

ctttccagaa agtcacactc tcagatctgt gtcaagttca atgtgagccc tggcaaggct 6000

ggcatattaa cacctgcctt ctggcttctg aaagtgagat ttgtatatgg gctgcactca 6060

cgcatatacg agttggttta tctttgtgta catgactata acccagtgat gctgaggtca 6120

tgtgctggaa tgctgtattt ggaccacaca tttcaaagtt gccctatgga aatgaatcct 6180

acttagtgac aagtcatcaa atgtttgtca catgtgatga agacaaatat gtatacctgg 6240

catagagaaa aatatatacc tggtacattg gagaaaaata attacacttt caaagagaat 6300

tccctttgca attttatgtt tggatcacca ctgtaagcac actttatttg catttgatct 6360

gtatttgtat atgctgatgc aatgataaaa atcactgtaa tacttcattg tgttgtactg 6420

gatgcaaagc tagaaaatat tgcaataaat gagaccgata aa 6462

<210> 14
<211> 4894
<212> DNA
<213> Homo sapiens

<400> 14
aagtaacaaa aaagttaaga aattggtagc aaaggaacat aagtaaaaga aagataaata 60

ttttccaaat aaaattctaa taaaagctaa acattctcaa atattttaca tacatgaaga 120

ataaattatt tctggttaac atgtttttct agtgccaaaa aacaaaaatt tgataagctc 180

acattaaaat caaagttttc tagatatgaa ttctatatac catctctata tttgtttaga 240

attttgcaaa attcttagaa gattgggggg gagaaaattg ccaaactgac atggttaatt 300

aacagacagg cttgaaagga gaaggctaga agagggaaga ttatgtcact aagcatagct 360

acaaaaggaa aagccacatg gaagcagaga caaaggcaat cccgacacac agaggcatgt 420

ttgttgcggc tcagggtttc tgtttttcaa gtgttcagag acgaattcca gcaagacttc 480

tggtttgagt caagcctttc aaccgaggtc ttggcatact taacggaaaa agcgagtaca 540

ttcagattta gttttgctag atattaaact agaaaactgg atgacagtaa aaccaataca 600

gaaacaaaaa gtcatgtttc cagtaaagaa ccgtacacac aagcaggttt tgcttattaa 660

tgtagcagga gagcatggaa atcggagttc agactcttta gaaaagacaa aaggcagggg 720

aggggagaga agaaaaggag gaatgcagag aagccctcca agaatgatga gtgattcgtg 780

caggagaggg aaatacagac gttccgtcca tgtgagattc tgcttcaggt attggacatc 840

gttccctcca cctgcaccta gaggatatgt gttcaggatc tcgctatggt tagttaccca 900

tgtgctagac ataggttgct agctagaagt tttgccagcc atcttccccc ctcctagtgc 960

taatgcctaa tttcctaaag taatctgaat ccttataagc cagggtagct caggaggttc 1020

tatttactta tttatttatt ttgagacaga gtctcactgt gtcgcccagg cagatctcgt 1080

ctgggcccga gtctcgttgc agtggtgtaa tctctgctca ctgcaacctc tgcctcccag 1140

gttcatgcaa ttttcctgtc tcagcctccc gagtagctag gatacagggg tgtgccacca 1200

cgcctggcta atttttctat ttttagtagc gagagcattt cgtcatgttg gccagtctgg 1260

tctccaactc ctgacctcag gtgatctgcc tgccttggcc tcccaaagtg ctgggattat 1320

aggcatgagc caccgcgccc agcatcagta agttttaata ttacgtctag tcggctcacg 1380

ggtgcttgag ccagaagtgg gaagggtaca cttgtatttt gggctcaaat ggttttcaaa 1440

taaaatacat ctgaccaaat aaagtgaaca cagtcctgag ccctcctctc tccagatatg 1500

catgcaatcc acaccatcaa aagaagagtt cactgttccc aaggccaaga agaggctatg 1560

ctgtactgca aagaactcta tagcaaaatc gaacacccaa attaagtgcc agaatgatgc 1620

agcccgctct caccaggaag aacgacatgt gagaaaactt atagcagatg caaaaatgtc 1680

aacccaccat gttatttaaa aaaaaatttt atcaggatac aaggacactt cactgttttt 1740

aacccacaca aggttaggta atgtttacct tgaaacataa tcgcatatgg ccctttatgc 1800

cactcaccta ggcctttaat aatgagggca attgctaagt gtgctgtctc ttctggggac 1860

ttctctctga agggggtata tattgataaa atattttatt aatcagttaa gaatttttga 1920

tctacttgaa aaagattaaa ttataatatt tcactttgaa actgctattg ctcatgcaaa 1980

agtacttgat tttcatacta tatggagctc acaatctttg gactacacaa acattctcta 2040

aaagggcaat gacttatatt ttccaaagtt tagacatgac atccaaagga caaaaggtaa 2100

ctattttttt taccaatgtg gttggaacag tgacaacgag caatgtattc accaaactga 2160

taaaaaatct gcctgtggtt ccttcactgt atgtgtgtgc agagaaaggc agaaaagctt 2220

ctatccatgt cattgtctct cttgcccatg tgccagtaat ttgaataaga tgagaatggg 2280

ccaggcgcag cggctcacgc ctgtaatccc acactttggg agaccaaggc gggtgaatca 2340

cgaggtcagg agttagagac cagcctggcc aacatggtga aacctcatct ctactaaaaa 2400

tacaaaaatt agctgggcgt ggtggtgggc acctgtaatc ccagctactc aggagactga 2460

ggcaggagaa tcgcttgaac ccaggaggca gaggttgcag tgagcagaga tcacgacaat 2520

gcactccagc cctgcgacag tgcgagactc cgtctcaaaa aaaaaaaaaa agttttccag 2580

aataacccca gttgtatcta gaatttgttt tcaatataaa ggcaggtatg agaatgtttt 2640

gaacaacaga attaatccat ctagattaga atgctgtgtg gcatgaccat tctcgaattt 2700

tgaaagcctg agctggttgt gggcaagggg atattcatta atgacataaa tttcatttat 2760

gaaatatgta tccatcatct aggtatctta tacatggaga gctgactgca tcccttgtat 2820

aaaaagtaaa tgaagaccgg aaggaagcag actttgtcct gcaaactgaa gcaaagttcc 2880

atgctaagaa tcagcaggtt ttctaggtag gtaatgccac cagtggagga ttattacagt 2940

tctcaaatca tgagggaaag agatggagaa gagggaggga ggagggaaaa tcttgcattc 3000

taagaggtaa gagtccccat tacaattgca atgcttcttt agacagctga tttttctggc 3060

tggaaggcac attaaaaata accctaacaa taacttacca agacaatgga aatcaaaaga 3120

aaagaaggta gacgtgcagc tgtcttatta acatgacaac caactacagc tgaaacaata 3180

ctatggtaca gggactagct catgttaaag actggtattt aggtaaagag gatctggtgc 3240

tcttggttta gcttcagtga ttttccagca cttattaaag ccattgtgcc ctgaacatgg 3300

agaccccaaa tcctctatgt gccaaagatt aagttgtaga gaattacatc aatgtttgct 3360

gttggtgtgc accacagatg agaccaagct tcctggtccg gctaagtgga atcttccatg 3420

cactgtgcac cactcagcta gaaagtcaac taagcagaaa ggagataagg aatggctatg 3480

gaatacctga cttctttatc ttggttatgt gtccacaatt ttcataaaaa taaataatac 3540

acattaagtc aaaatctggg ctcaaatgtg taacaagaaa acaaaaggac aacaatctgg 3600

tattccggtt tttttttttt tttgcccggc tggagtgcag tgacgtgatc tggctcacta 3660

caagctccgc ctcccaggtt cacaccttag cctcccgagt agctgggaca caggcgcccg 3720

ccaccacgcc cgcctaatgt tttaattttt tagtagagac ggggtttcac cgtgttggcc 3780

aggctggtct cgaactcctg acctcaggta atccacctgc ctcagcctcc caaagtgctg 3840

gaattacagg tgtgagccac cgcacccagc caacaatctg gtattcttaa ggcaaaacag 3900

tagaaatcac tttaaataga cactttatta gatccacagg cgataaaatc ccagagctac 3960

tgttaggcac gtaaatatag accctgctaa atatttaata ttatcaagct ttgacgtaaa 4020

tatttaatat tatcaagctt gtgtgactca agcatggttt tgaggccagc aacctcagtt 4080

tcgatcttcc tgtctgtctg ggtaagggcc cgatcttgct gggcatgcaa tgccactatt 4140

tctgttctca attccagcag cttcttttcg ctttctgttc tcaattccag cagcttcttt 4200

tcgttcaatg aatacaattc ttttactctg ctcttttcta ggttgaagtc taatttggct 4260

tttactctgc tcttttctag gttgaagtct aatttggtat ctgttcggac tttgatcact 4320

tcatccatta cttgttgttt taactgatgt agttcgagtt ttattttatt acttgttgtt 4380

ttaactgatg tagttcgagt tttattttct cattttctgc tctgagggct gaaaattcac 4440

tcttctccaa aattttctgc tctgagggct gaaaattcac tcttctccaa aataatcata 4500

tcctttttca cattcgcaat ctgagacatt acttgctgaa aaggatttcc tgctgcatct 4560

tggtgaccat atctttgtag acgtctgcct gcatcttggt gaccatatct ttgtagacga 4620

tgtccatgtt ggcctccagg atcttgacca atgcagacac aatgatttct gcttgttgag 4680

tagcaaaccc attgtcttcc agtaagcaca ctaaggcatg agtgtcgaag tagattgtct 4740

tccagtaagc acactaaggc atgagtgtcg aagtagagtt tcctgctccc agaagaggtg 4800

aaatctctcc ttttgcgctc cagctgcagg gatccagcag acaagcttag ctctctcggc 4860

ctctaataga aagatgctcc agctgatcag cttc 4894

<210> 15
<211> 6604
<212> DNA
<213> Homo sapiens

<400> 15
gcggaagcca gcggacccac ttgtgcggcg gtcggcgcgg ggcgcaggta ctggcacacg 60

gatgaggagt cgtagaagag gctgccgaag gctgccgggg gcgcttcgcg ttttgttcca 120

aggcgaaggt caaactcctg gtcaagcaat cctcctgctt tggcctccca tatgttggga 180

ttataggcgt gagccaccat gcccagccct tccctgtttt ctttttcctt gccacctccc 240

ctctctgctc tgacagctcc taccatcttt ccttccaact tctaggaatg tgctggctga 300

tgggctctct ttcttgaaac ttccagctcc tgtcccaggc tcacaggcat ctcagtggct 360

tactgtgtca cctgcgccct aggttggttg tctagacccc accctctgtt gtccaaggct 420

ggggaccctc acggtccgct gcctccttcc ctccaggccc tgtgccactg gcacgtctgt 480

ccgcaggtcc ccttcccaca gccccaagac cgaaaggctg tgtctctgtg aggaagtgaa 540

tccgagaacg gtgccgaccc ctctgaagct cacttttgtg attttggttt cctcaccatg 600

tacttgacag accttagttg ccacatctac tccgtggggt agcctgaagc gcttgtggcc 660

ctggcttcta aaggtgtatt tgcaggggtg ctagggccct cagggctgtt tttcgttctt 720

tacaggcctt tgacaccgct catttatttt gaagccaggt ggcaagggga gagaggcttc 780

cccaattctc cagccctggt taggggcagt ccctggtgtg gctcctgtcc cgggctggaa 840

gcaatgtgtg caaatgtcca aatcacgggg tcttccctcc ctcacagggc tttagccagg 900

cagctgttac gaggtaaatt gtgttaaaat tgggtgttat ggggaactgg gagacgacga 960

aggggggggc ccctgtatcc cagcacttcg gggcgaggca gaatgaagaa tgccaggggc 1020

cagaaaggag ctgagaccag agtgttttga cccggagctg gaggttgcag tatttcatgc 1080

ctcgcaggtc tagaggtaga gtgcacacgg atgaggagtc gtagaagagg ggcctgcaga 1140

ttgtgctctc agcaagcctg tcgtctagtg gaagacactg gccgggacac agagcccacg 1200

tgtggacacc gctgtgggtt ggtggcaggg gtcgagcagg tgccatcagg cacatgaacg 1260

gtggagtctt caaaggtgtg agcagctgct ggaagaggag gtgctcctga agacactgcg 1320

gccctcccaa agtgctggat taaggctgag ccaccgcgcc tgcaagcaca gagtgccttc 1380

tgaggtgtga gagctgctgg aagaggaggt gctcctgaag acactgcggc cggcccgcct 1440

gtgcccttgg tgccctggct gcctagagag cctcacccct gggccctggg gccaggactc 1500

caggactctg actacctgcc ctcccccagc ctcagcggct gcacctcctc gttagtactg 1560

atgcactgac ctcggcacac agctgggagg ggttgggggc tgggtcatgg ctgctcccag 1620

gccccaccca ggctcctgag cctagaaggt gaaaagagga ctctcagggg ctcacagggg 1680

ctctcactgc tggttggccc tgccctccct tccccctcag cagggtgccc ggaagctgga 1740

accttgttat ctgggtaatt agtttcagac cctgcactga ggccggccag gtctcggggc 1800

tgcctcccat aggttgtgca ccctgacccc gagagggagg cgaggcgctg cttgtcgaca 1860

gctagaggct ggcctgggga gcaggtttgg ggtgccctcc cacactgccc tccctgcccc 1920

ggcccatgcc ccccagggct gcctgggcct ggttattgtg tggggcctcc tgacccagcc 1980

aagggcacga agctctggga aggggatgcc cccgagggtg ccagtccagc tagctgcccc 2040

acccctcagg cccagcctgg cccccaagct ccccactctg gtgccccgag cagccctgtg 2100

ggcaagcagc cgccgccatg gccgagcacc tggagctgct ggcagagatg cccatggtgg 2160

gcaggatgag cacacaggag cggctgaagc atgcccagaa gcggcgcgcc cagcaggtga 2220

agatgtgggc ccaggctgag aaggaggccc agggcaagaa gggtcctggg gagcgtcccc 2280

ggaaggaggc agccagccaa gggctcctga agcaggtcct cttccctccc agtgttgtcc 2340

ttctggaggc cgctgcccga aatgacctgg aagaagcccg gcccaggcca ccgcccaggg 2400

ggtggctcca cagctcctgg gctccagccc ccagatcttg cctggtctct tcaggctggg 2460

tggcccaggg tgcccccatc aggcaagccc caagcagtgg gcagccctga ggtgagcctg 2520

tgcgggacag ccgtccttca ggcgggggct gcatgcagag ggctcctgct gtgaggtgag 2580

ctgaggccat gagtggaccc ccaggagcct gcagaaaaac ctagtgcggg gccccacggg 2640

agcaggggtg gccggtagtc attgctgctt cttgttaggc tgctcaggag agccctggtc 2700

tccacctccg tgaaataggg tgggaccagc cacctctgtg ggtgagagag ccaggccctg 2760

ggcaggcaca gagctatgca ggtggcccct ctcagtgcct gccctaggct ctgcttgtga 2820

ctctcttctc ctcacctgtg ccccacgctt gcagccccag ctgtgtgggt tccacttctg 2880

gtgctctgga gtctggggga agaggcactg tggtggccct tgagactcct tcctgcctcc 2940

ctccggagca gagagtaaag aggagccggc agtgtctggc ctgcatcttt ccatgtggac 3000

aggagccctc ccgggaggcc tggaggcaga gggaagaggc ggagggtgcc atggtccaca 3060

gggagctgtg ggaaggtggg gccctcctgc tgcaccccag gcaccgtcag ggagaaccct 3120

ggtgacctgg gcgagagctg ggcccgagtt gggatctttg atggggaggg gggagagcag 3180

ggatgtgctc tggtggtggg gagcctcagg tcaggtccca gaggacccag gagagctcat 3240

gaacacaggg ctggcaggtt gtcagggaag agccgaagga gctgtggtag cctccaggca 3300

gggagctctt cctgggctgg agggaggtgt gggattgttg gagtgctgct ctcacacagg 3360

agcacggcct gttctggggg tcggagggct ggagcaccac catgaagggc ctgcaggtgg 3420

gtggaggcag agggcttctg ggagtgtgac cggcctgggc cagacagatg cctggtgctt 3480

ctgcccccac ccctgggctc tggtgtgcct cctgccatcg gcaccactgt cttccttgtc 3540

cttccctggg tcctgggccg aagcacagtt gacaggacct gaccaggggc aggaccctgt 3600

cctcacctcg ccaccttttt gcagtccgcc agttccttgg gagtggggtc agccctgact 3660

tggccaacga ggacggcctg acggccctgc accagtgctg cattgatgat ttccgagaga 3720

tggggcgcct gctgcattga tgatttccga gagatggtgc agcagctcct ggaggctggg 3780

gccaacatca atgcctgtga cagtgagtgc tggacgcctc tgcatgctgc ggccacctgc 3840

ggccacctgc acctggtgga gctgctcatc gccagtggcg ccaatctccg gcggtcaaca 3900

gaggcccgcg gtatctgtgg ctctcggcct ccgtgttccc gccttccctt tgctgccctg 3960

cctgtgtcct ggcatggaga gcagggcctg gggctccagg ggaatgggca tgtgcccagg 4020

gcagcgcggt ccctgcctcc ccagcattcg tcctcctgcc gagagggcgg cctcgggccc 4080

ccccccagag tgtgcacgag aaatgtcttg gtcagtgagg agggcagaac tgctaaggag 4140

ggaagcagag gaggaggcca ggtggcttgg gaggagaggg tacctgagca caggtagggc 4200

ctttaccttt aacacagagg gacgtagagc ggggggaact ctgagcaggg agggtccatg 4260

agcctcctcc cttccccttt gctccaggca tctgtagtgg gagagaggtc agggctgcgg 4320

tcattctcga gtgcccctgt gaggccgggt ggggtgtgat ggcggtcgcg tcacacacag 4380

gcgctgcggg gcaggcgggg atgtgggggt ccacaggtag gcctgctcct ccactgcaca 4440

cagacacccc cttctgtgtt ctgggctccc cttcaccgcc ctcttccctg gggccatgct 4500

ccccacagct cagcagcccc tcagcgtggc tgcctggtcc tgcacccccg ccaccctgtg 4560

ctccctgctc cccagcaggc aggcccatcc aatgcatgtt tatgggggca ccgctgcccc 4620

tggaggtgcc aggaacctgg atccctggat ggatagagac tgcaggaggc ctgtctggct 4680

taatgacagg atctaggaca gccgatgggc cccaaggctg cctctcccag cagcaggtgg 4740

acagggtgcc tcctgagggg cagccctggg ctgaggggcc ctggcgggga cttctccaag 4800

tgaggagcct ggcaagcggg gaagggcctt gtgcccagca ccccgtccgt cttccctgca 4860

gtggcgccaa tctcctggcg gtcaacaccg acgggaacat gccctatgac ctgtgtgatg 4920

atgagcagac gctggactgc ctggagactg ccatggccga ccgtggacgg gggcgcgtcc 4980

cactgcggca tcacccagga cagcatcgag gccgcccggg ccgtgccaga actgcgcatg 5040

ctggacgaca tccggagccg gctgcaggcc ggggcagacc tccatgcccc cctggaccac 5100

ggggccacgc tgctgcacgt cgcagccgcc aacgggttca gcgaggcggc tgcgggcgac 5160

aggccggggc ttgcctgcag ctgcacgtcg cagccgccaa cgggttcagc gaggcggctg 5220

ccctgctgct ggaacaccga gccagcctga gcgctaagga ccaagacggc tgggagccgc 5280

tgcacgccgc ggcctactgg ggccaggtgc ccctggtgga gctgctcgtg gcggcccggt 5340

gcccctggtg gagctgctcg tggcgcacgg ggccgacctg aacgcaaagt ccctgatgga 5400

cgagacgccc cttgatgtgt gcggggacga ggaggtgcgg gccaagcgcg gagggcccgc 5460

cgcggggacg aggaggtgcg ggccaagctg ctggagctga agcacaagca cgacgccctc 5520

ctgcgcgccc agagccgcca gcgctccttg ctgcgccgcc gcacctccag cgccggcagc 5580

cgcgggaagg tggtgaggcg gggagcctaa cccagcgcac cgacctgtac ctctcctctc 5640

tcctccccgg ggtgaggcgg gtgagcctaa cccagcgcac cgacctgtac cgcaagcagc 5700

acgcccagga ggccatcgtg tggcaacagc cgccgcccac cagcccggag ccgcccgagg 5760

acaacgatga ccgccagaca ggcgcagagc tcaggccgcc gcccccggag gaggacaacc 5820

ccgaagtggt caggccgcac aaggcggggc tcccgctacc ctgcctgtgt caggaatggt 5880

gccctgcagg ggccagcttt tgccccatct ctcctgtctg tcccttcatg accatacagc 5940

ctgaacccaa ggccagggag gggggaggag cctcctgatg gctctgggac cttcactgcc 6000

cgcaggagga caaccccgaa gtggtcaggc cgcacaatgg ccgagtaggg ggctccccag 6060

tgcggcatct atactccaag cgactagacc ggagtgtctc ctaccagctg agccccctgg 6120

acagcaccac cccccacacc ctggtccacg acaaggccca ccacaccctg gctgacctga 6180

agcgccagcg agctgctgcc aagctgcagc gacccccacc tgagggcccc gagagccctg 6240

agacagctga gcctggcctg cctggtgaca cggtgacccc ccagcctgac tgtggcttca 6300

gggcaggcgg ggacccaccc ctgctcaagc tcacagcccc ggcggtggag gctcccgtgg 6360

agaggaggcc gtgctgcctg ctcatgtgag gctgttgctc agcatgcagg ggccctgtcg 6420

cgggcacagc ccaaggctgc ctccccacgg tgcgtgccct ggtgctgcgg gtgcagcacg 6480

gaaaccccgg cttctactgt acaggacact ggcccctctc aggtcagaag acatgcctgg 6540

agggatgtct ggctgcaaag actattttta tcctgcaact cttgataaag ggcgttttgc 6600

catg 6604

<210> 16
<211> 5714
<212> DNA
<213> Homo sapiens

<400> 16
   gcggacccac ttgtgcggcg gtcggcgcgg ggcgcaggta ctggcacacg gatgaggagt 60
   cgtagaagag ggctgccggt gggcgcttcg cgttttgttc caaggcgaag gtagccatag 120
   tgaggaggac atgtgggaca ggctcaagga ttgcaggtgc agtatttcat gcctacgcag 180
   gtctacggat gaggagtgtc acacggatga ggagtcgtag aagaggggcc tgcagattgt 240
   gctctcagca agcctgtcgt ctagtggaag acactggccg ggacacagag cccacgtgtg 300
   gacaccgctg tgggttggtg gcaggggtcg agcaggtgcc atcaggcaca tgaacggtgg 360
   agtcttcaaa ggtgtgagca gctgctggaa gaggaggtgc tcctgaagac actgcgtgag 420
   cagctgctgg aagaggaggt gctcctgaag acactgcggc cggcccgcct gtgcccttgg 480
   tgccctggct gcctagagag cctcacccct gggccctggg gccaggactc caggactctg 540
   actacctgcc ctcccccagc ctcagcggct gcacctcctc gttagtactg atgcactgac 600
   ctcggcacac agctgggagg ggttgggggc tgggtcatgg ctgctcccag gccccaccca 660
   ggctcctgag cctagaaggt gaaaagagga ctctcagggg ctcacagggg ctctcactgc 720
   tggttggccc tgccctccct tccccctcag cagggtgccc ggaagctgga accttgttat 780
   ctgggtaatt agtttcagac cctgcactga ggccggccag gtctcggggc tgcctcccat 840
   aggttgtgca ccctgacccc gagagggagg cgaggcgctg cttgtcgaca gctagaggct 900
   ggcctgggga gcaggtttgg ggtgccctcc cacactgccc tccctgcccc ggcccatgcc 960
   ccccagggct gcctgggcct ggttattgtg tggggcctcc tgacccagcc aagggcacga 1020
   agctctggga aggggatgcc cccgagggtg ccagtccagc tagctgcccc acccctcagg 1080
   cccagcctgg cccccaagct ccccactctg gtgccccgag cagccctgtg ggcaagcagc 1140
   cgccgccatg gccgagcacc tggagctgct ggcagagatg cccatggtgg gcaggatgag 1200
   cacacaggag cggctgaagc atgcccagaa gcggcgcgcc cagcaggtga agatgtgggc 1260
   ccaggctgag aaggaggccc agggcaagaa gggtcctggg gagcgtcccc ggaaggaggc 1320
   agccagccaa gggctcctga agcaggtcct cttccctccc agtgttgtcc ttctggaggc 1380
   cgctgcccga aatgacctgg aagaagccgg cccaggccac ccgggccctc atgccaaggc 1440
   tgggggctgg gcctgtccac agctcctggg ctccagcccc cagatcttgc ctggtctctt 1500
   caggctgggt ggcccagggt gcccccatca ggcaagcccc aagcagtggg cagccctgag 1560
   gtgagcctgt gcgggacagc cgtccttcag gcgggggctg catgcagagg gctcctgctg 1620
   tgaggtgagc tgaggccatg agtggacccc caggagcctg cagaaaaacc tagtgcgggg 1680
   ccccacggga gcaggggtgg ccggtagtca ttgctgcttc ttgttaggct gctcaggaga 1740
   gccctggtct ccacctccgt gaaatagggt gggaccagcc acctctgtgg gtgagagagc 1800
   caggccctgg gcaggcacag agctatgcag gtggccccct ctcagtgcct gccctaggct 1860
   ctgcttgtga ctctcttctc ctcacctgtg ccccacgctt gcagccccag ctgtgtgggt 1920
   tccacttctg gtgctctgga gtctggggga agaggcactg tggtggccct tgagactcct 1980
   tcctgcctcc ctccggagca gagagtaaag aggagccggc agtgtctggc ctgcatcttt 2040
   ccatgtggac aggagccctc ccgggaggcc tggaggcaga gggaagaggc ggagggtgcc 2100
   atggtccaca gggagctgtg ggaagggggg cctcctgctg caccccaggc accgtcaggg 2160
   agaaccctgg tgacctgggc gagagctggg cccgagttgg gatctttgat ggggagggga 2220
   gtgagagcag ggatgtgctc tggtggtggg gagcctcagg tcaggtccca gaggacccag 2280
   gagagctcat gaacacaggg ctggcaggtt gtcagggaag agccgaagga gctgtggtag 2340
   cctccaggca gggagctctt cctgggctgg agggaggtgt gggattgttg gagtgctgct 2400
   ctcacacagg agcacggcct gttctggggg tcggaggctt ggagcaccac catgaagggc 2460
   ctgcaggtgg gtggaggcag agggcttctg ggagtgtgac cggcctgggc cagacagatg 2520
   cctggtgctt ctgcccccac ccctgggctc tggtgtgcct cctgccatcg gcaccactgt 2580
   cttccttgtc cttccctggg tcctgggccg aagcacagtt gacaggacct gaccaggggc 2640
   aggaccctgt cctcacctcg ccaccttttt gcagtccgcc agttccttgg gagtggggtc 2700
   agccctgact tggccaacga ggacggcctg acggccctgc accagtgctg cattgatgat 2760
   ttccgagaga tggtgcagcg ccggggcacg tgtgctgcat gatgatttcc gagagatggt 2820
   gcagcagctc ctggaggctg gggccaacat caatgcctgt gacagtgagt gctggacgcc 2880
   tctgcatgct gcggccacct gcggccacct gcacctggtg gagctgctca tcgccagtgg 2940
   cgccaatctc ctggcggtca cagaggccgc cggggctcct cttgggcagt atctgtggct 3000
   ctcggcctcc tgtgttcccg ccttcacctt tgctgaccct gcctgtgtcc tggcagagag 3060
   cagggcctgg ggctccaggg gaatgggcat gtgcccaggg cagcgcggtc cctgcctccc 3120
   cagcattcgt cctcctgccg agagggcagg cctcgggccc ccccccagag tgtgcgagaa 3180
   atgtcttggt cagtgaggag ggcagaactg ctaaggaggg aagcagagtg aggaggccag 3240
   gtggcttggg aggagagggt acctgagcac aggtagggcc tttaccttta acacagaggg 3300
   acgtagagcg gggggaactc tgagcaggga gggtccatga gcctcctccc ttcccctttg 3360
   ctccaggcat ctgtagtggg agagaggtca gggctgcggt cattctcgag tgcccctgtg 3420
   aggccgggtg gggtgtgatg gcggtcgcgt cacacacagg cgctgcgggg caggcgggga 3480
   tgtgggggtc cacaggtagg cctgctcctc cactgcacac agacaccccc ttctgtgttc 3540
   tgggctcccc ttcaccgccc ttcttccctg gggccatgct ccccacagct cagcagcccc 3600
   tcagcgtggc tgcctggtcc tgcacccccg ccaccctgtg ctccctgctc cccagcaggc 3660
   aggcccatcc aatgcatgtt tatgggggca ccgctgcccc tggaggtgcc aggaacctgg 3720
   atccctggat ggatagagac tgcaggaggc ctgtctggct taatgacagg atctaggaca 3780
   gccgatgggc cccaaggctg cctctcccag cagcaggtgg acaggggcct cctgaggggc 3840
   agccctgggc tgagggccct ggcggggact tctccaagtg aggagcctgg caagcgggga 3900
   agggccttgt gcccagcacc ccgtccgtct tccctgcgtg gcgccaatct cctggcggtc 3960
   aacaccgacg ggaacatgcc ctatgacctg tgtgatgatg agcagacgct ggactgcctg 4020
   gagactgcca tggccgaccg tggacggggg cgcgtccctg agcctgccgc ctcgcaggca 4080
   tcacccagga cagcatcgag gccgcccggg ccgtgccaga actgcgcatg ctggacgaca 4140
   tccggagccg gctgcaggcc ggggcagacc tccatgcccc cctggaccac ggggccacgc 4200
   tgctgcacgt cgcagccgcc aacgggttca gcgaggcggc tgccggcgat tcaggccggg 4260
   cgcttgcctg cagctgcacg tcgcagccgc caacgggttc agcgaggcgg ctgccctgct 4320
   gctggaacac cgagccagcc tgagcgctaa ggaccaagac ggctgggagc cgctgcacgc 4380
   cgcggcctac tggggccagg tgcccctggt ggagctgctc gtggcgccgc ccgtgcagct 4440
   ccggcgcgcc cctggtggag ctgctcgtgg cgcacggggc cgacctgaac gcaaagtccc 4500
   tgatggacga gacgcccctt gatgtgtgcg gggacgagga ggtgcgggcc aagctgctgg 4560
   aggtgtgcgg ggacgaggag gtgcgggcca agctgctgga gctgaagcac aagcacgacg 4620
   ccctcctgcg cgcccagagc cgccagcgct ccttgctgcg ccgccgcacc tccagcgccg 4680
   gcagccgcgg gaaggtggtg aggcggggag cctaacccag cgcaccgacc tgtacagggg 4740
   tgaggcgggt gagcctaacc cagcgcaccg acctgtaccg caagcagcac gcccaggagg 4800
   ccatcgtgtg gcaacagccg ccgcccacca gcccggagcc gcccgaggac aacgatgacc 4860
   gccagacagg cgcagagctc aggccgccgc ccccggagga ggacaacccc gaagtggtca 4920
   ggccgcacaa gggggctccc ctggttctct ctccgcttgg accccctcct gcctgtgtca 4980
   ggaatggtgc cctgcagggg ccagcttttg ccccatctct cctgtctgtc ccttcatgac 5040
   catacagcct gaacccaagg ccagggaggg gggaggagcc tcctgatggc tctgggacct 5100
   tcactgcccg caggaggaca accccgaagt ggtcaggccg cacaatggcc gagtaggggg 5160
   ctccccagtg cggcatctat actccaagcg actagaccgg agtgtctcct accagctgag 5220
   ccccctggac agcaccaccc cccacaccct ggtccacgac aaggcccacc acaccctggc 5280
   tgacctgaag cgccagcgag ctgctgccaa gctgcagcga cccccacctg aggggcccga 5340
   gagccctgag acagctgagc ctggcctgcc tggtgacacg gtgacccccc agcctgactg 5400
   tggcttcagg gcaggcgggg acccacccct gctcaagctc acagccccgg cggtggaggc 5460
   tcccgtggag aggaggccgt gctgcctgct catgtgaggc tgttgctcag catgcagggg 5520
   ccctgtcgcg ggcacagccc aaggctgcct ccccacggtg cgtgccctgg tgctgcgggt 5580
   gcagcacgga aaccccggct tctactgtac aggacactgg cccctctcag gtcagaagac 5640
   atgcctggag ggatgtctgg ctgcaaagac tatttttatc ctgcaactct tgataaaggg 5700
   ctgttttgcc atgg 5714

<210> 17
<211> 3949
<212> DNA
<213> Homo sapiens

<400> 17
cagtaacccg gtatattctg gattccagca gtatcctcaa tcctgtgtag gactaagcaa 60

attatcctgg tgtggtgtga accagctatc ctccagtata ggaggattga gtcttcagag 120

ttctccacaa ccagaaagcc tgagacctgt aaaccttact caggagagga atattttacc 180

tatgactcct gtttgggctc ctgtacctaa cttgaatgca gacctcaaaa aattaaactg 240

tagcccagat tcatttcggt gtactttgac aaatattcca cagacacagg cttcggtgta 300

ctttgacaaa tattccacag acacaggctt tactgaataa agctaagctt cctttaggat 360

tgttgttaca tcccttcaga gacctaacgc aattaccagt gataacatca aataccattg 420

tgaggtgccg atcctggata acatcaaata ccattgtgag gtgccgatcc tgtcgaacgt 480

atattaaccc ctttgtatcc ttcattgatc aacgtagatg gaaatgcaat ttgtgctata 540

gagtaaacga tgttcctgaa gaatttatgt ataaccccct tacccgatct tatggagaat 600

ttatgtataa cccccttacc cgatcttatg gagagcctca taaacgacca gaagttcaga 660

attcaactgt ggagttcatt gcttcttcag attacatgct gcgtcctcct caacctgcag 720

tttacttgtt tgttttagac ctcctcaacc tgcagtttac ttgtttgttt tagatgtgtc 780

tcataatgca gtggaagctg gatatttgac aattttgtgc cagtcactcc tagaaaatct 840

agacaagctt cctggagatt cacgaacaag aataggattc atgacctttg attggagatt 900

cacgaacaag aataggattc atgacctttg atagcactat tcatttctac aatttacaag 960

aaggattatc acagcctcaa atgttgattg tgtctgatat agatgatgtt tttctaccta 1020

caccggaagt ttacttgtga ttttctacct acaccggata gtttacttgt gaatctatat 1080

gaaagtaaag agcttataaa agacttactg aatgcattac caaacatgtt cacataaaag 1140

acttactgaa tgcattacca aacatgttca ccaatacaag agaaacacac agtgcccttg 1200

gtcctgcact tcaggctgcc tttaaattaa tgtctccaac aggtggccgt gtgtctgtat 1260

ttcagacaca gttaccttcc ttgggtgcag gacttctgca atccagagaa gatcctaatc 1320

agagatcaag tacaaaggtg gtacaacatc ttggccctgc aactgatttt tataagaaac 1380

caacatcttg gccctgcaac tgatttttat aagaaacttg cattagattg ctcgggacag 1440

caaactgcag tggatttgtt ccttttaagt tcacagtatt ctgatcttgc ttctctagct 1500

tgcatgtcca agtattctgc agggtgcatc tattattatc ctgtccaagt attctgcagg 1560

gtgcatctat tattatccat cattccacta tactcacaat ccttcacaag cagaaaagtt 1620

acaaaaagac ctaaaacggt atctcacaag aaaaattggg tttgaagctg ttatgagaat 1680

aaggtgtact aaaggtcttt caatgcacac ttttcacggt aacttctttg tccgttcttt 1740

caatgcacac ttttcacggt aacttctttg tccgttctac tgatttgtta tcccttgcca 1800

acatcaatcc tgatgctgga tttgcggtgc agttgtcaat tgaagaaagt ttaacagata 1860

cttccttagt atgttttcaa acagccctat tatatacatc aagcaaaggt gagcggagaa 1920

ttaatcgttg ggtggggaag aatgaataca cagggtggtt attttctttc tttttttaac 1980

tctcactttt gcatctttaa atagcaattt gttaatttca aaacagttca tgctaaatcc 2040

agctttgtat tattctgccc caaaaaaaaa aaatttagaa tgaggatata ggagaattag 2100

agtacataca ctttgtttgc cagtggtaag ttcactagca gatgtatatg cgggagtgga 2160

tgtacaagct gccatctgcc ttctggcaaa catggctgtg gatcggtccg tttcatcaag 2220

tctgtcagat gcaagagatg cctgtggatc ggtccgtttc atcaagtctg tcagatgcaa 2280

gagatgcctt agtgaatgct gtagtggact cattgtctgc atatggctca actgtctcaa 2340

atttacagca ctctgcattg atggcgccca gctccctcaa gttgtttcct ctctatgttt 2400

tggcccttct caaacagaaa gcatttagaa cgggtacaag cacacggctg gatgatcgtg 2460

gcatttagaa cgggtacaag cacacggctg gatgatcgtg tatatgccat gtgtcagata 2520

aagtctcagc cacttgttca tctaatgaaa atgattcatc ccaacttata caggatagac 2580

agattgacag atgagggtgc agtacatgtt aatgacagga ttgtaccaca gccacctctt 2640

gtacatgtta atgacaggat tgtaccacag ccacctcttc aaaaattgtc tgcagagaag 2700

ctgacaagag aaggtgcttt ccttatggac tgtggctctg ttttttacat ttgggttggg 2760

aaaggctgtg acaataactt cagtttttta catttgggtt gggaaaggct gtgacaataa 2820

cttcatagag gatgtgcttg gatatactaa ttttgcatca ataccacaga aaatgacaca 2880

tcttccagag ctagatacac tttcatcaga aagagccaga tccagagcta gatacacttt 2940

catcagaaag agccagatcc ttcataactt ggcttagaga cagcagacca ttaagtccaa 3000

tccttcacat agtaaaagat gagagtcctg ccaaagcaga attttttcag catttgattg 3060

aagtgagagt cctgccaaag cagaattttt tcagcatttg attgaagacc ggacagaggc 3120

tgcattttct tactatgaat ttttgcttca tgttcagcag cagatttgta agtgaagtag 3180

aataaaattg aataagaaaa agatctataa cctaggtaaa gcataattgt cagagaagcg 3240

cgtgagaaat ttgaaatgaa ggcatttgtt aatacaagat gcaacgcaca gcactctgtc 3300

tgaggctttg gtaaaaagta aaggggaaga aagaacttga cagatctttt tcaactcaaa 3360

ttaatggtaa cgatgatgct gtttcaccaa gtatattttg aattggtttc tacacatttc 3420

cagtagtatg gcagtacagt gctctgttca ttgcaagctg gcaaatttat gtagctatgt 3480

ggaatgatat gtcataatgt aaaattagat aaattctttt ttcttataat taatataaca 3540

tttctggact tgaactctgg caagagatgc caaaaggcat tggtaccgtg ttatttgttt 3600

atatgaatta ctttttaaca aggaatgttt cttattcatt aaatgaattc aacattttct 3660

ctgtaaaaac aatagagttt cagtacatga actatagaaa aaaatctata tataatgtac 3720

ataaatgtta catttgtaaa gaaaatgtaa aaatgtaact atagcatatg aattgcttaa 3780

actgtgctgc attgttggat gacaactctt tttgtcacag ttagagaatg aggttgacta 3840

atgcatatta tgaattgagt ccacaaagga acacaaaact ctttgtaatt ctgttaaatc 3900

ttttatgtag atttatttat gatcagccta ctaattaaaa ctatttcgc 3949

<210> 18
<211> 5140
<212> DNA
<213> Homo sapiens

<400> 18
tgcgggcccg gcgccgcacc agcagaacgg tccagcccag aatcaaatgg ctcacttagg 60

tccagcccag aaccatctgg atatggattg catcatcaaa actatattgc tccctcagga 120

cattactctc aaggacctgg gaaaatgacc tcattgccat tggataccca gtgtggtgat 180

tactactctg ctctctatac agtaccaaca caaaatgtga ctcctaacac agtgaaccag 240

caaccaggag cacagcagtt gtacagcagg ggtcctcctg cccctcatat tgtgggatcc 300

actctaggat ctttccaagg tgctgcatcg tcagcatccc atttgcatac gagtgcctcc 360

caaccatact cctcttttgt gaatcactac aatagtccag ccatgtactc tgccagctct 420

tctgttgcgt ctcagggatt tccctctact tgtggtcatt atgctatgtc aactgtttct 480

aatgccgcgt atcctagtgt ttcatatccc tctctgcctg ctggtgatac atatgggcaa 540

atgtttacct cacagaatgc tccgactgtt aggccagtta aagataattc attctctggt 600

caaaatacag ctatcagcca tccatcgcca cttccacctc taccatcaca acagcaccac 660

cagcagcaaa gtctttcagg atacagtact ctaacgtggt catctccagg ccttccatcg 720

actcaagaca atctcatccg aaaccacaca ggatccctgg ctgtagcgaa caacaaccca 780

ccattactgt tgcagattct ttatcctgcc tgtataatgt cagcctcccg attctttatc 840

ctgtcctgtt atgcaaaatg ttcagcctcc caagtccagc ccagtggtat ccactgtttt 900

atcaggatcc tcaggatcct catcaacaag aacacctccc actgcaaatc acccagttga 960

gcctgtgacc tcagttacac agccatcaga gctattacaa caaaaagggt actccctgat 1020

caacttccga tgatgagagg ggagctcttc taccacaagc agtgcttctc caatgcccaa 1080

cagttatgat gccctggaag gaggcagtta cccagatatg ctttcttcat cagcaagcag 1140

tcctgctctg cccttaagca gtcctgctcc tgatcccgcc cctgaacctg atcctgcttc 1200

tgctccagct ccagcttcag ctccagctcc tgtcgtccct cagccttcaa aaatggctaa 1260

gccttttggc tatggctatc caacacttca gcctggttat cagaatgcta cagcaccact 1320

tatttctgga gtacagccca gtaacccggt atattctgga ttccagcagt atcctcaaca 1380

gctatcctcc agtataggag gattgagtct tcagagttct ccacaaccag aaagcctgag 1440

acctgtaaac cttactcagg agaggaatat tttacctatg actcctgttt gggctcctgt 1500

acctaacttg aatgcagacc tcaaaaaatt aaactgtagc ccagattcat ttcggtgtac 1560

tttgacaaat attccacaga cacaggcttt ttcggtgtac tttgacaaat attccacaga 1620

cacaggcttt actgaataaa gctaagcttc ctttaggatt gttgttacat cccttcagag 1680

acctaacgca attaccagtg ataacatcaa ataccattgt gaggtgccgc gataccaaca 1740

tcaaatacca ttgtgaggtg ccgatcctgt cgaacgtata ttaacccctt tgtatccttc 1800

attgatcaac gtagatggaa atgcaatttg tgctatagag taaacgatgt tcctgaagaa 1860

tttatgtata acccccttac ccgatcttat gggaatttat gtataacccc cttacccgat 1920

cttatggaga gcctcataaa cgaccagaag ttcagaattc aactgtggag ttcattgctt 1980

cttcagatta catgctgcgt cctcctcaac ctgcagttta cttgtttgtt ttcctcaacc 2040

tgcagtttac ttgtttgttt tagatgtgtc tcataatgca gtggaagctg gatatttgac 2100

aattttgtgc cagtcactcc tagaaaatct agacaagctt cctggagatt cacgaacaag 2160

aataggattc atgacctttg atcctggtaa acaagaaaga ttcatgacct ttgatagcac 2220

tattcatttc tacaatttac aagaaggatt atcacagcct caaatgttga ttgtgtctga 2280

tatagatgat gtttttctac ctacaccgga agtttacttg tgagtttttc tacctacacc 2340

ggatagttta cttgtgaatc tatatgaaag taaagagctt ataaaagact tactgaatgc 2400

attacaaatt aaacttactg aatgcattac caaacatgtt caccaataca agagaaacac 2460

acagtgccct tggtcctgca cttcaggctg cctttaaatt aatgtctcca acaggtggcc 2520

gtgtgtctgt atttcagaca cagttacctt ccttgggtgc aggacttctg caatccagag 2580

aagatcctaa tcagagatca agtacaaagg tggtacaaca tcttggccct gcaactgatt 2640

tttataagaa ataacatctt ggccctgcaa ctgattttta taagaaactt gcattagatt 2700

gctcgggaca gcaaactgca gtggatttgt tccttttaag ttcacagtat tctgatcttg 2760

cttctctagc ttgcatgtcc aagtattctg cagggtgcat ctatccaagt attctgcagg 2820

gtgcatctat tattatccat cattccacta tactcacaat ccttcacaag cagaaaagtt 2880

acaaaaagac ctaaaacggt atctcacaag aaaaattggg tttgaagctg ttatgagaat 2940

aaggtgtact aaaggtcttt caatgcacac ttttcacggt aacttctttg tacttttcac 3000

ggtaacttct ttgtccgttc tactgatttg ttatcccttg ccaacatcaa tcctgatgct 3060

ggatttgcgg tgcagttgtc aattgaagaa agtttaacag atacttcctt agtatgtttt 3120

caaacagccc tattatatac atcaagcaaa ggtgagcgga gaattatgtt tggagagaat 3180

gaatacacac tctcactttt gcatctttaa atagcacact tttttaattt atgctaaatc 3240

cagctatgcc tttgtattat ttcttgcccc aaaaagtaaa aaactgctta gcaattaaga 3300

tacactttgt ttgccagtgg taagttcact agcagatgta tatgcgggag tggatgtaca 3360

agctgccatc tgccttctgg caaacatggc tgtggatcgg tccgtttcat caagtctgtc 3420

agatgcaaga gatgccactt tggttgcttt ttagctgtgg atcggtccgt ttcatcaagt 3480

ctgtcagatg caagagatgc cttagtgaat gctgtagtgg actcattgtc tgcatatggc 3540

tcaactgtct caaatttaca gcactctgca ttgatggcgc ccagctccct caagttgttt 3600

cctctctatg ttttggccct tctcaaacag aaagcattta gaacggacaa agcacacggt 3660

ggatgtcgtg ttagaacggg tacaagcaca cggctggatg atcgtgtata tgccatgtgt 3720

cagataaagt ctcagccact tgttcatcta atgaaaatga ttcatcccaa cttatacagg 3780

atagacagat tgacagatga gggtgcagta catgttaatg acaggattgt accacagcca 3840

cctcttagag tacatgttaa tgacaggatt gtaccacagc cacctcttca aaaattgtct 3900

gcagagaagc tgacaagaga aggtgctttc cttatggact gtggctctgt tttttacatt 3960

tgggttggga aaggctgtga caataacttc gtttttacat ttgggttggg aaaggctgtg 4020

acaataactt catagaggat gtgcttggat atactaattt tgcatcaata ccacagaaaa 4080

tgacacatct tccagagcta gatacacttt catcagaaag agccaggata cactttcatc 4140

agaaagagcc agatccttca taacttggct tagagacagc agaccattaa gtccaatcct 4200

tcacatagta aaagatgaga gtcctgccaa agcagaattt tttcagcatt tgattgaaga 4260

gagcctgcca aagcagaatt ttttcagcat ttgattgaag accggacaga ggctgcattt 4320

tcttactatg aatttttgct tcatgttcag cagcagattt gtaagtgaag tagaataaaa 4380

ttgaataaga aaaagatcta taacctaggt aaagcataat tgtcagagaa gcgcgtgaga 4440

aatttgaaat gaaggcattt gttaatacaa gatgcaacgc acagcactct gtctgaggct 4500

ttggtaaaaa gtaaagggga agaaagaact tgacagatct ttttcaactc aaattaatgg 4560

taacgatgat gctgtttcac caagtatatt ttgaattggt ttctacacat ttccagtagt 4620

atggcagtac agtgctctgt tcattgcaag ctggcaaatt tatgtagcta tgtggaatga 4680

tatgtcataa tgtaaaatta gataaattct tttttcttat aattaatata acatttctgg 4740

acttgaactc tggcaagaga tgccaaaagg cattggtacc gtgttatttg tttatatgaa 4800

ttacttttta acaaggaatg tttcttattc attaaatgaa ttcaacattt tctctgtaaa 4860

aacaatagag tttcagtaca tgaactatag aaaaaaatct atatataatg tacataaatg 4920

ttacatttgt aaagaaaatg taaaaatgta actatagcat atgaattgct taaactgtgc 4980

tgcattgttg gatgacaact ctttttgtca cagttagaga atgaggttga ctaatgcata 5040

ttatgaattg agtccacaaa ggaacaactc tttgtaattc tgttaaatct tttatgtaga 5100

tttatttatg atcagcctac taattaaaac tatttcgctt 5140

<210> 19
<211> 5225
<212> DNA
<213> Homo sapiens

<400> 19
cccgggtgcg ggcccggcgc cgcaccagca gaacggtcca gcccagaatc aaatgcagtc 60

tctgaggctc tgaaagtcga cagagctatg aaatgtccag cccagaatca aatgcaggtt 120

ccatctggat atggattgca tcatcaaaac tatattgctc cctcaggaca ttactctcaa 180

ggacctggga aaatgacctc attgccattg gatacccagt gtggtgatta ctactctgct 240

ctctatacag taccaacaca aaatgtgact cctaacacag tgaaccagca accaggagca 300

cagcagttgt acagcagggg tcctcctgcc cctcatattg tgggatccac tctaggatct 360

ttccaaggtg ctgcatcgtc agcatcccat ttgcatacga gtgcctccca accatactcc 420

tcttttgtga atcactacaa tagtccagcc atgtactctg ccagctcttc tgttgcgtct 480

cagggatttc cctctacttg tggtcattat gctatgtcaa ctgtttctaa tgccgcgtat 540

cctagtgttt catatccctc tctgcctgct ggtgatacat atgggcaaat gtttacctca 600

cagaatgctc cgactgttag gccagttaaa gataattcat tctctggtca aaatacagct 660

atcagccatc catcgccact tccacctcta ccatcacaac agcaccacca gcagcaaagt 720

ctttcaggat acagtactct aacgtggtca tctccaggcc ttccatcgac tcaagacaat 780

ctcatccgaa accacacagg atccctggct gtagcgaaca acaacccacc attactgttg 840

cagattcttt atcctgtcct gttatgcaaa atgttcagtg cagattcttt atcctgtcct 900

gttatgcaaa atgttcagcc tcccaagtcc agcccagtgg tatccactgt tttatcagga 960

tcctcaggat cctcatcaac aagaacacct cccactgcaa atcacccagt tgagcctgtg 1020

acctcagtta cacagccatc agagctatta caacaaaaag ggttaaccag ctagctccgc 1080

accaactccc ttgtcatcaa cttccgatga tgaggaagag gaggaggagg atgaggaagc 1140

aggctcttct ccacaagcag tgcttctcca atgcccaaca gttatgatgc cctggaagga 1200

ggcagttacc cagatatgct ttcttcatca gcaagcagtc ctgctcctga tcccgccctt 1260

tctcagcaag cagtcctgct cctgatcccg cccctgaacc tgatcctgct tctgctccag 1320

ctccagcttc agctccagct cctgtcgtcc ctcagccttc aaaaatggct aagccttttg 1380

gctatggcta tccaacactt cagcctggtt atcagaatgc tacagcacca cttatttctg 1440

gagtacagcc cagtaacccg gtatattctg gattccagca gtatcctcaa tgaaccagct 1500

atcctccagt ataggaggat tgagtcttca gagttctcca caaccagaaa gcctgagacc 1560

tgtaaacctt actcaggaga ggaatatttt acctatgact cctgtttggg ctcctgtacc 1620

taacttgaat gcagacctca aaaaattaaa ctgtagccca gattcatttc ggtgtacttt 1680

gacaaatatt ccacagacca ggctttcggt gtactttgac aaatattcca cagacacagg 1740

ctttactgaa taaagctaag cttcctttag gattgttgtt acatcccttc agagacctaa 1800

cgcaattacc agtgataaca tcaaatacca ttgtgaggtg cctcctcagt gataacatca 1860

aataccattg tgaggtgccg atcctgtcga acgtatatta acccctttgt atccttcatt 1920

gatcaacgta gatggaaatg caatttgtgc tatagagtaa acgatgttcc tgaagaattt 1980

atgtataacc cccttacccg atcttatgga atttatgtat aaccccctta cccgatctta 2040

tggagagcct cataaacgac cagaagttca gaattcaact gtggagttca ttgcttcttc 2100

agattacatg ctgcgtcctc ctcaacctgc agtttacttg tttgttttag attcctcaac 2160

ctgcagttta cttgtttgtt ttagatgtgt ctcataatgc agtggaagct ggatatttga 2220

caattttgtg ccagtcactc ctagaaaatc tagacaagct tcctggagat tcacgaacaa 2280

gaataggatt catgaccttt gattcctgga gattcacgaa caagaatagg attcatgacc 2340

tttgatagca ctattcattt ctacaattta caagaaggat tatcacagcc tcaaatgttg 2400

attgtgtctg atatagatga tgtttttcta cctacaccgg atagtttact tgtgtttttc 2460

tacctacacc ggatagttta cttgtgaatc tatatgaaag taaagagctt ataaaagact 2520

tactgaatgc attaccaaac atgttcacaa aagacttact gaatgcatta ccaaacatgt 2580

tcaccaatac aagagaaaca cacagtgccc ttggtcctgc acttcaggct gcctttaaat 2640

taatgtctcc aacaggtggc cgtgtgtctg tatttcagac acagttacct tccttgggtg 2700

caggacttct gcaatccaga gaagatccta atcagagatc aagtacaaag gtggtacaac 2760

atcttggccc tgcaactgat ttttataaga aactaacatc ttggccctgc aactgatttt 2820

tataagaaac ttgcattaga ttgctcggga cagcaaactg cagtggattt gttcctttta 2880

agttcacagt attctgatct tgcttctcta gcttgcatgt ccaagtattc tgcagggtgc 2940

atctattatt atccgtccaa gtattctgca gggtgcatct attattatcc atcattccac 3000

tatactcaca atccttcaca agcagaaaag ttacaaaaag acctaaaacg gtatctcaca 3060

agaaaaattg ggtttgaagc tgttatgaga ataaggtgta ctaaaggtct ttcaatgcac 3120
acttttcacg gtaacttctt tgtccgttct ctttcaatgc acacttttca cggtaacttc 3180
tttgtccgtt ctactgattt gttatccctt gccaacatca atcctgatgc tggatttgcg 3240
gtgcagttgt caattgaaga aagtttaaca gatacttcct tagtatgttt tcaaacagcc 3300
ctattatata catcaagcaa aggtgagcgg agaattagag tacatacact ttgtttgcca 3360
gtggggagaa ttagagtaca tacactttgt ttgccagtgg taagttcact agcagatgta 3420
tatgcgggag tggatgtaca agctgccatc tgccttctgg caaacatggc tgtggatcgg 3480
tccgtttcat caagtctgtc agatgcaaga gatgccgctt ggtcggtccg tttcatcaag 3540
tctgtcagat gcaagagatg ccttagtgaa tgctgtagtg gactcattgt ctgcatatgg 3600
ctcaactgtc tcaaatttac agcactctgc attgatggcg cccagctccc tcaagttgtt 3660
tcctctctat gttttggccc ttctcaaaca gaaagcattt agaacgggta caagcacacg 3720
gctggatgat cggagattta aacgggtaca agcacacggc tggatgatcg tgtatatgcc 3780
atgtgtcaga taaagtctca gccacttgtt catctaatga aaatgattca tcccaactta 3840
tacaggatag acagattgac agatgagggt gcagtacatg ttaatgacag gattgtacca 3900
cagccacctc ttaggcagta catgttaatg acaggattgt accacagcca cctcttcaaa 3960
aattgtctgc agagaagctg acaagagaag gtgctttcct tatggactgt ggctctgttt 4020
tttacatttg ggttgggaaa ggctgtgaca ataacttcat agttttttac atttgggttg 4080
ggaaaggctg tgacaataac ttcatagagg atgtgcttgg atatactaat tttgcatcaa 4140
taccacagaa aatgacacat cttccagagc tagatacact ttcatcagaa agagccagtc 4200
cagagctaga tacactttca tcagaaagag ccagatcctt cataacttgg cttagagaca 4260
gcagaccatt aagtccaatc cttcacatag taaaagatga gagtcctgcc aaagcagaat 4320
tttttcagca tttgattgaa tgagagtcct gccaaagcag aattttttca gcatttgatt 4380
gaagaccgga cagaggctgc attttcttac tatgaatttt tgcttcatgt tcagcagcag 4440
atttgtaagt gaagtagaat aaaattgaat aagaaaaaga tctataacct aggtaaagca 4500
taattgtcag agaagcgcgt gagaaatttg aaatgaaggc atttgttaat acaagatgca 4560
acgcacagca ctctgtctga ggctttggta aaaagtaaag gggaagaaag aacttgacag 4620
atctttttca actcaaatta atggtaacga tgatgctgtt tcaccaagta tattttgaat 4680
tggtttctac acatttccag tagtatggca gtacagtgct ctgttcattg caagctggca 4740
aatttatgta gctatgtgga atgatatgtc ataatgtaaa attagataaa ttcttttttc 4800
ttataattaa tataacattt ctggacttga actctggcaa gagatgccaa aaggcattgg 4860
taccgtgtta tttgtttata tgaattactt tttaacaagg aatgtttctt attcattaaa 4920
tgaattcaac attttctctg taaaaacaat agagtttcag tacatgaact atagaaaaaa 4980

atctatatat aatgtacata aatgttacat ttgtaaagaa aatgtaaaaa tgtaactata 5040

gcatatgaat tgcttaaact gtgctgcatt gttggatgac aactcttttt gtcacagtta 5100

gagaatgagg ttgactaatg catattatga attgagtcca caaaggaaca caaaactctt 5160

tgtaattctg ttaaatcttt tatgtagatt tatttatgat cagccactaa ttaaaactat 5220

ttcgc 5225

<210> 20
<211> 2631
<212> DNA
<213> Homo sapiens

<400> 20
gtggctcacg tcaggcactg gaccggaagg tgaacaacaa cgggtccctt cctgaaggag 60

gtttctcagt tcaccccagt ggccttcccc attgccaagg accgctgcgt ggtggcagcc 120

ttctgggcag atgtggacaa ccgcggcggc gacgtgtact accgggaggc caccgaccca 180

gccatgctgc gccgagccac ggaggacgtc aggcactact tccccgagct cctggacttc 240

aatgccacct gggtttttgt tgccacctgg taccgagtga ccttctttgg aggcagttcc 300

tcatcccctg tcggcaagct ctccttcacc atcttcaact atgagtccat cgtgtggacc 360

acaggcacac acgccagcag cgggggcaac gccactggcc tcgggggcat cgcagcccag 420

gctggcttca acgcaggcga tgggcagcgt tacttcagta tccccggctc gcgcacagca 480

gacaggccga ggtggagacc accaccaacg tgggtgtgcc cgggcgctgg gcgttcagaa 540

tcgatgatgc ccagcggggg ctgcggccat acaacgtccg tgtgcctggc cctgcgcccc 600

tccctgcctc aacggcggca agtgcatcga cgactgcgtc acgggcaacc cctcctaccc 660

tgctcctgcc tctcgggctt cacggggcgg aggtgccacc tggacgtgaa cgaatgtgcc 720

cccgcccggg cctcccagcc ctgtcagaat ggtgggacct gtactcacgg catcaacagt 780

ttccgctgcc agtgcccggc tggctttggg ggacccacct gtgagacagc caatcccccc 840

aaagagtgtc aacatggtgg ccagtgccag gtggagaagg ggaggctctt gtgttgacct 900

agtggggaat tacacctgct gtgtgccgag cccttcaagg gacttcgctg tgagacagag 960

accatccagt gccagacgcg cctctcggcc ccttgccaca atgggggcac ctgtgtggat 1020

gcggaccagg gctacgtgtg cgagtgcccc gaaggcttca tgggcctggc tgcagggaga 1080

ccgcaacgga ggcagatgcc tgggcgccaa caccaccctc tgccagtgcc cctgggattc 1140

tttgggcttc tctgtgaatt tgaaatcaca gccatgccct gcaacatgaa cacaatgaac 1200

acacagtgcc cagatggggg ctactgcatg gagcacggcg ggagctacct ctgcgtctgc 1260

cacaccgacc acaatgccag ccactccctg ccatcaccct gcgactcgcc cggacccctg 1320

cttcaacgga ggctcctgcg atgcccatga cgactcctac acctgcgagt gcccgcgcgg 1380

gttccacggc aagcactgcg agaaagcccg gccacacctg tgcagctccc gaacgggggc 1440

acgtgcaagg aggcgggcgg cgagtaccac tgcagctgcc cctaccgctt cactgggagg 1500

cactgtgaga tcgggaagcc acggcggcac ctgcttccac acattggcaa atacaaggtg 1560

actgtccccc aggcttctcc gggcggcact gcgagatagc ccccccccct gcttccggag 1620

cccgtgtgga atggggcctc cccgtgtgaa tggggcctgc ggccgggaca cggatttctt 1680

ctgccactgc caagcagggt acatggacgc ccggtgccag gcagaggtgg actgcggccc 1740

cccgaagcac gccacactgc gcttcaacgg cacgcggctg ggcgcggtgg ccctgtatgc 1800

atgtgaccgt ggctacagcc tgagcgcccc cagccgcacc gggctgccag ccaccgggcg 1860

gagtgagccc cgagtgccgg tctcagccgt gcctgcatgg gggctcttgt caggaccgcg 1920

ttgctgggta cctgtgcctc tgcagcacag gctatgaggg cgcccactgt gagctgggga 1980

gagtgccgag ctcacccgtg cagaaatgga gggtcctgca ggaacctccc aggggcctat 2040

gtctgccggt gccctgcagg cttcgttgga gtccactgtg agacagaggg gacgcctgcg 2100

actcccccag catggaggcc ggtgtgagag cggcgggggg cctacctgtg cgtctgccca 2160

gagagcttct tcggctacca ctgcgagaca gtgagtgacc cctgcttctc cagcccctgt 2220

gggggccgtg gtgcctggcc agcaacggct ccccagctgc accgcaaagg gctacacggg 2280

cgaggactgc gccaaagagc tcttcccacc gacggccctc aagatggaga gagtggacaa 2340

gatggagaga gtggaggaga gtggggtctc tatctcctgg aacccgccca atggtccagc 2400

cgccaggcag atgcttgatg gctacgcggt cacctacgtc tcctccgacg gctcctaccg 2460

ccgcacagac tttgtggaca ggacccgctc ctcgcaccag ctccaggccc tggcggccgg 2520

cagggcacaa catctccgtc ttctcagtga agcgaaacag taacaacaag aatgacatca 2580

gcaggcctgc cgtgctgctg gcccgcacgc gaccccgccc tgtggaaggt t 2631

<210> 21
<211> 4002
<212> DNA
<213> Homo sapiens

<400> 21
cttgccacaa tgggggcacc tgtgtggatg cggaccaggg ctacgtgtgc gagtgccccg 60

aaggcttcat gggcctggac tgcagggaga gcctgggcgc caacaccacc ctctgccagt 120

gccccctggg attctttggg cttctctgtg aatttgaaat cacaggggct actgcatgga 180

gcacggcggg agctacctct gcgtctgcca caccgaccac aatgccagcc actcccgcct 240

cccgcgctcg gcgatgccca tgacgactcc tacacctgcg agtgcccgcg cgggttccac 300

ggcaagcact gcgagaaagc agtcagggcc cgccggaacg gggcacgtgc aaggaggcgg 360

gcggcgagta ccactgcagc tgcccctacc gcttcactgg gaggcactgt gagatcggtg 420

cttccactac attggcaaat acaagtgtga ctgtccccca ggcttctccg ggcggcactg 480

cgagatagcc ccctccccct gctgggggca cctgcgagga ccgggacacg gatttcttct 540

gccactgcca agcagggtac atgggacgct tcaacggcac gcggctgggc gcggggccct 600

gtatgcatgt gaccgtggct acagcctgag cgcccccagc cgcatccggg tctgccagcc 660

acacggtgtc tggaggagcc tccccagtgc cttgggggct gatgcccagc tctgacctgg 720

cagagttcca ggagagatta aaggggccaa gaacgagggc aaaggggaaa caaacacagc 780

cgagctcacc cgtgcagaaa tggagggtcc tgcaggaacc tcccaggggc ctatgtctgc 840

cggtgccctg caggcttcgt tggagtccac tgtgagacag aggccggtgt gagagcggcg 900

gggggcctac ctgtgcgtct gcccagagag cttcttcggc taccactgcg agacagtgag 960

tgacccctgc ttctccggcc gtggctattg cctggccagc aacggctccc acagctgcac 1020

ctgcaaagtg ggctacacgg gcgaggactg cgccaaagag ctcttcccac cgacggccct 1080

caagatggag ggaggagagt ggggtctcta tctcctggaa cccgcccaat ggtccagccg 1140

ccaggcagat gcttgatggc tacgcggtca cctacgtctc ctccgacggc tcctaccgcc 1200

gcacgacttt gtggacagga cccgctcctc gcccgcctgg ggccggcagg cctacaacat 1260

ctccgtcttc tcagtgaagc gaaacagtaa caacaagaat gacatcagca ggcctgccgt 1320

gctgctggcc cgcatctcag tgcagtgggc cctgcacagg atccgccatg ccaccgtcag 1380

tggggtccgt gtgtccatcc gccaccctga ggccctcagg gaccaggcca ccgatgtgga 1440

caggagtgtg gacaggttca cctttagggc cctgctgcct gggaagagga caccatccag 1500

ctgaccaccc tcagtgggct caggggagag gagcacccca cagagagcct ggccaccgcg 1560

ccgacgcacg tgtggacccg gcccctgcct ccagcaaacc ccctgcccac gtggtctggg 1620

atgccccgac tccaggcagc ttgctggagg cttatgtcat caatgtgacc ccagccagag 1680

caccaagagc cgctatgtcc ccaacgggaa gctggcgtcc tacacggtgc gcgacctgct 1740

gccgggacgg cggtaccagc tctctgtgat agcagtgcag agcacggagt cgggccggca 1800

cgcgcccccc gccggagcgc gcctgctcaa tgaccccgcc cccgagaccc ccggtggctc 1860

acccagcaaa cccgtgagat cacggtcagc ctggccctcc agctccctga acacggcagc 1920

aaggacatcg gaaccgggcg cagacgccca cagctgtgac tgcgggccag ggttcaaagg 1980

cagacgctgc gagctccccc caaggctgtt ctccgagaca aagcctttcc agtctgggag 2040

ggaggcgtct gtcaccacgt gtagtctccg agtcaccaac tggttttaca aagctttctt 2100

gtgacaacag gtataaaaga gtctaccgag ttcaccaaga catctgcttc aaagagagct 2160

gtgaaagcac aagcctcaag aagaccccaa acaggcgcaa tgtagcccac agggtgatgg 2220

ggcaagggcg ggggtgggct gcggccgtgg gctgagccag gcggtctact cgcccagata 2280

ggcagcccct gggcagccac gttctgctac gaaactgtat gtccatatat gtgcatgtga 2340

gcatacccag aggggcacag ccgggcatgc tgggtgctgg ggggggtcct ctggttatgc 2400

atattcacac agaacatggt gctacaattg ggaagcacag ggtgttacgg acagccccga 2460

gtatttaatg gccgatatag cgcgtcgctc tttacttttt attcactcac cccaggttct 2520

ttccacaaag ggttcaaggt agttacaaga attactactg tttggcgttt gctgaaagaa 2580

gtacgtgaga atattatatg ctttagaaac cacagagtcg aagcctgtca tcgtcctcat 2640

agaccaatga gtagccacac gtacttgttt ggcattacca agtgtccggg caaggccgca 2700

gcctgcagct caccagccgt gctgctcggc ctgtgcacgt caccggctct tccctagggt 2760

agcttttgct tgctttctcc cacgtcctcc tctctctctc tgactcacag ccagccaggt 2820

ttctagcctt gtcattccta aactactgcc tcaagccagg cgggcgcaca caaactaaaa 2880

tgctaatctc cacagcggtg tctggactaa tggtgtcccc caccgtggaa tgtatgtgag 2940

ctaaagacag gatctcagca ggcagcctgg actgaggtag ctgtagaatt caaggtacac 3000

agtctctttt tcattaaacg acttttctct aatttttcag gaaacaaagt aagagtcaga 3060

cactggagaa atcttaaggg ttccccacct aggggggcga ctgcccagcc ccgggatctc 3120

agagtgccgc tgcctgctta ctcagcactg ctaaaggaag cgcggtcctg tgctgctgtg 3180

ttacagactc ccgggcagga aggttcagaa gtccccaagg ggaaacagac atgaacctgg 3240

tctagccact gacaaataca ccttcaggtg gaaatgaggc tctctgtgga tagggcaaat 3300

cccactctct ctcaagggaa atgtggccaa gaaaccccta gatcagctgc agtgggagga 3360

agaggcaggg gggtgggccc ttggagaggc tggaagacca cctggcacct gggaggaggg 3420

gccacgggca gcgggagggc ctgggctgct gagaaattcc gggggctcca ggagagggct 3480

gagagagtgc ctcccagatc ttgtgaaggc attctgttgg aactaaaagt ggggcagggg 3540

ccttgatcgc ttaatattta gaaagtgttg aagaccgccg ctagaaagtc gtcactgaca 3600

ctgggctcat aggcattcat aaatcctgac gtgaaaggaa cttcctctcg cctttgttta 3660

agccagcatt tcccaaactc ttttggcaac caaaccccat cctgcccccc cccaagccga 3720

atcttaaaca acactggcat tcttcacaat cgtttgaaga gacggacgaa agccatctac 3780

aacctgttac cagtttcata ctgaccacat tttttatcaa catgtcactg catgaaagat 3840

gaatctctgg ttggcctagg acagctttgc tcttcctgcc ccttatagga gccctcacag 3900

ttcatcttcc tggtggcgca gatgagtgag gccccttggg tacaagccac ttcaaaacag 3960

gtctatgttt tgttacgtgc ctgggcaggg cgcttagcgt ag 4002

<210> 22
<211> 3297
<212> DNA
<213> Homo sapiens

<400> 22
gctgttgttc ttttcttttc catgatatca ttatatggac agtttagggt ggtctcatgg 60
attataacca tttggatatt tggttcacta acaatgctga gatcgcgcca ttgcactcca 120
gcctgggcaa caggagtgaa actccatctc aagaaaaaca caaaatacaa aacaaaacaa 180
aacaaaaaaa aggcggggct cgaccccact ttgggaggcc gagcgggggt caggagcaga 240
ccatggtgaa cccgtctcta ctaaaaatac aataaattag ccgggcatgg tggcgggcgc 300
ctgtagtccc agctacttgg gggctgaggc aggagaatcg cttgaacccg ggaggcagag 360
gttgcagtga gccaagatcg tccagcctag gtgacagagt gagactccgt ctcaaaaaaa 420
aaaaaaaaaa aaataatttt ttttaaaata tttaggaaga tttgtaaagc ttatgaacca 480
ttgtaaaacc ttatttctaa ggtctgtaag acagttttat atgtaataat gtgtccattt 540
ccaggaattc cccaaatact catacagcca ttgagcaccc caatccaaaa ttcagaatgc 600
ttcagataag aacatttttg agtgccgatg tgacacttta aaggaaatgt tcattggagc 660
attttgcatt tcagattttt ggattggaaa actcaaccac tgtatttctg cacatattcc 720
aaaatctgaa aaagtctgaa acctgagaaa ctctggtccc aagcattttg gaaagggata 780
ctcaatctgt attaacatag acttctaaat tagtgaaaat ttgcattgaa taaatggaag 840
taaatatttt tgtttaaata ttgttaattt agtcatttaa aaattttgtg atgtaacaaa 900
atgttgaatg aaataatgct tctacaactt aaaattattt ttcatagtga acaaaactag 960
ttgaatactt ctaaatattc atttttgtta gtgccatttt tccagtttta ggctttaggg 1020
aattgtgacc cttttttttt tttcgcggtg tgtggcgcaa tctcgcccat tttttaattt 1080
tatggagaca gggttccctt ggttaactcc tgacctcagg tgatccaccc cgattggctt 1140
aagccccgtg cccggcactt ttgtttttaa aataatatct tactttcata tgattctttc 1200
agactgtggt atggcattct gaagcaagtc ttaacagagc taaaaatcac ccttacccaa 1260
ttctctgcac tgacatatgt acacaaagta atattttatt attctccttc ataaatggtt 1320
ctttaattga acctttgagc cttatactat ttttaaattt ccctttaaaa attgaattac 1380
agccaggcat ggtggctcat gcctgtaatc ccagaacttt gggaggccat ggcaggagga 1440
ttgcttgaac ccaggagttg tagaccagcc tgggcaagat agcgagatcc tgcctctgaa 1500
aaaatcataa taatacttta atttagaaaa taccctgaat tagaagtgct tcattcctca 1560
tttgtgtaca gctgttcctc tatatacgaa ggggattggt accagaacca ctgcatacca 1620
aattccaccc atactcaagt ctcacagtct tccctgcaga atccaagtat acaaaaagta 1680
ggcctatcat ttatatgggt tttgcatcct gacagtactg tattttcaat cctagtttgt 1740
ttgggaaaaa aaaattttta cggtacagta tcatacctgt tcccagcact ttggaaggct 1800
gaggcggagg actgttttgt ggccaggagt tcagcggggc ccaaaaaact gggcatgtgg 1860

ccctgcctgt ggtcccgcta ctcggggctg tgaggcagga gaattgcttg gcctgggagg 1920

ttgaggctgc agtgagccgc gatcacacca ctgcatgcca gcctgggcga cagagtgaga 1980

ccctgtctca gaaaagaaaa aaaaagtaaa aattgcatgt aagttgaccc gcactattca 2040

aatttgtggt gttcaaggtt caactgtaat ttcctagcag cattttgtgt gtttgagaat 2100

ctcttgacac tcttcaagta aatccctaaa ttacaacttt gacatcaaaa aagctagaca 2160

ttcctacatt tttgcactac aatacataaa aactcccatg ctgatcggtt gtgggatcgt 2220

gcctgtgaat aaccactgca ctccagcctg ggcaacatag taagtaagac cttgtctctt 2280

aaaaaaaata cattctgaag aaagttctac ttatgaatac attttattta taacaaactg 2340

gtgaaaattt tagaccaaac catgtctttc tgggttgtag tgattaaaaa atggttaaga 2400

gaatgttccc tatacaaggc atatgttatt aaacatgaaa tttaggatta gttttctctt 2460

tgaaaattct tttgatatgc tgaggttaca gtttggaggt tacattagag aaagaatata 2520

gggaagatgg cagtggtaaa acatttccct gctttacatt tttatattgg cagtatcttg 2580

gtttcaactt ttctattttc agcaagatat ttgcaagttt ttactttgaa gatgtttggt 2640

ttatagtagt ccatgctata ctaatgctgt tgctgagata attaagttag atactaatat 2700

gaaaaagaat atttgtgtaa aatattttga agttgtaatt agtgtatata caagttgtta 2760

atacttacga aaaaaggaag cattcccacc tgtaataatt ttgtaacact agactatcag 2820

taaattatca agaataaaaa ccttggtttt tcttttaaaa caaattcaca agaaaagtga 2880

aattacacaa aatgataaat ttctcatctg gtgataaagg gaaaaagata atgtaaaagc 2940

agattgctgc tatttgcata ataagccttg ttgaagaata agattaagtt caaattttat 3000

actctttggt ggaattagtc accatttaat aatgaaacaa ctcatatgtt ttgtaatgta 3060

aaaatactca cgtttatgat gatgggccta ggtactaaat tcctagggat acagtgataa 3120

tgcaaaaaag ccccctgcac tcccgcctga gagcgagact ctgtctcaaa aaaaaagccc 3180

cccccatata cactgaaatc ttagggaaaa ataaaagtta actaggcaag aaggtaggga 3240

gacctttcag ccagcctgtc agagcttttg agtagaaata cggtatcaga aatgtgc 3297

<210> 23
<211> 4075
<212> DNA
<213> Homo sapiens

<400> 23
actccagcct gggtgacaga gtgagactcc gtgagccaag atcgtgccac tgcactccag 60

cctaggtgac aaaaaaaaaa aatatataat taagcattgt tttttaaaaa gtagtttagg 120

aagatttgta aagcttatga accattgtaa aaccttattt ctaaggtctg taagacagtt 180

ttatatgtaa taatgtgtcc atttccagga attccccaaa tactcataca gccattgagc 240

accccaatcc aaaattcaga atgcttcaga taagaacatt tttgagtgcc gatgtgacac 300
tttaaaggaa atgttcattg gagcattttg catttcagat ttttggattg gaaaactcaa 360
ccactgtatt tctgcacata ttccaaaatc tgaaaaagtc tgaaacctga gaaactctgg 420
tcccaagcat tttggaaagg gtatactcaa tctgtattaa catagacttc taaattagtg 480
aaaatttgca ttgaattaaa atgaagtaaa tatttttgtt taaatattgt ttaaaattta 540
gtcatttaaa aattttgtga tgtaacaaaa tgttgaatga aataatgctt ctacaactta 600
aaattatttt tcatagtgaa caaaactagt tgaatacttc taaatattca tttttgttag 660
tgcacatttt atccagtttt aggctttagg gaattgtgat tttttggtct ctcgcccagg 720
ctggagtgtg tggcgcaatc tcagctcact gcacctctga ttcctgggtt caagcaattc 780
tcatgtctcg cctccctaat agctggtgcc cccccccatt ttttaaattt ttaatggaga 840
cagggtttca ccatgttggc cgtacccacc ttggcctccc aaagtgctgg gattaggctt 900
aagccaccgc ccggcacttt tgtttttaaa ataatatctt actttcatat gattctttca 960
gactgtggta tggcattctg aagcaagtct taacagagct aaaaatcacc cttacccaat 1020
tctctgcact gacatatgta cacaaagtaa tattttatta ttctccttca taaatggttt 1080
ctttaattga acctttgagc cttatactat ttttaaattt ccctttaaaa attgaattac 1140
agccaggcat ggtggctcat gcctgtaatc ccagaacttt gggaggccat ggcaggagga 1200
ttgcttgaac ccaggagttg tagaccagcc tgggcaagat agcgagatcc tgcctctgaa 1260
aaaatcataa taatacttta atttagaaaa taccctgaat tagaagtgct tcattcctca 1320
tttgtgtaca gctgttcctc tatatacgaa ggggattggt accagaacca ctgcatacca 1380
aattccaccc atactcaagt ctcacagtct tccctgcaga atccaagtat acaaaaagta 1440
ggcctatcat ttatatgggt tttgcatcct gacagtactg tattttcaat cctagtttgt 1500
ttgggaaaaa aaaatgtttg tatgcctggg tacagtatca tacctgtaat cccagcactt 1560
tggaaggctg aggcgggagg actgttttgt ggccaggagt tcaaaatcag cctggtcgac 1620
gtagtgagac cccactctac aaagaaaaat tagctgggca tgtggcacct gcctgtggtc 1680
ccagctactc ggaggctgtg aggcaggaga attgcttggc ctgggaggtt gaggctgcag 1740
tgagccgcga tcacaccact gcatgccagc ctgggcgaca gagtgagacc ctgtctcaga 1800
aaagaaaaaa aaagtaaaaa ttgctgtaag ttgacccgca ctattcaaat ttgtggtgtt 1860
caaggttcaa ctgtaatttc ctagcagcat tttgtgtgtt tgagaatctc ttgacactct 1920
tcaagtaaat ccctaaatta caactttgac atcaaaaaag ctagacattc ctacattttt 1980
gcactacaat acataaaaac tcccatgctg atcggttgtg ggatcgtgcc tgtgaataac 2040
cactgcactc cagcctgggc aacatagtaa gtaagacctt gtctcttaaa aaaaatacat 2100

tctgaagaaa gttctactta tgaatacatt ttatttataa caaactggtg aaaattttag 2160

accaaaccat gtctttctgg gttgtagtga ttaaaaaatg gttaagagaa tgttccctat 2220

acaaggcata tgttattaaa catgaaattt aggattagtt ttctctttga aaattctttt 2280

gatatgctga ggttacagtt tggaggttac attagagaaa gaatataggg aagatggcag 2340

tggtaaaaca tttccctgct ttacattttt atattggcag tatcttggtt tcaacttttc 2400

tattttcagc aagatatttg caagttttta ctttgaagat gtttggttta tagtagtcca 2460

tgctatacta atgctgttgc tgagataatt aagttagata ctaatatgaa aaagaatatt 2520

tgtgtaaaat attttgaagt tgtaattagt gtatatacaa gttgttaata cttacgaaaa 2580

aaggaagcat tcccacctgt aataattttg ttaacactag actatcagta aattatcaag 2640

aataaaaacc ttggtttttc attgccacca acagaagtat ttacacaaga acaagtgaaa 2700

ttacacaaaa tgataatggg gagaaatttc tcatctggtg ataaagggaa aaagataatg 2760

taaaagcaga ttgctgctat ttgcactttg ggaatttctc catgaaattt taacccattt 2820

aaaataataa aacattgaga agaaacagcc tttgaatttg gcaccctggg agaagtgagg 2880

ggcggccccc cccaatatac acacaccctg actttaatga gcttattttg ctggggactc 2940

agccaattca caataaaatg agaaaaagag gcctgtggtg gtaagcaagt atataaaaga 3000

agccttcctt gaagcaacga tgcttaagct gaaatcttag ggaaaaaaag gaaagaagag 3060

ggaagactct ttcagataga atcgcagttt gtgagtttgc ttggggaaaa aaatttaaaa 3120

atataaaaaa aaattgttaa tgaaattgaa caggtatgtc agcctacctg acattctagc 3180

ttctttcttt gtcagagtgc taacctcggt gatggattat ggctgaacac cccaattata 3240

ctcttgacat atattcaacc gaagcatgtc aagaatgtcc ttaatattca aatagccggg 3300

aacatcaaga atagacaagg gtaattacac agatacaatg aaaaagaact aaagccgcat 3360

gaaaataaaa agacaaaaaa taagcatagt tagggagcat actgaaatgc agtcttttaa 3420

aacagcttta gaattccttg ccgggcggta aaaatacaaa attgctgggc gtggtgtcgc 3480

gtgccgtgcg caagaagagc aaaacttcgt ctcaaaaaaa aaaaaaaata gattttggca 3540

ttcaacaaac attggctgtt attatattag ctattaaaaa tttatccata ttacagggaa 3600

ggtagcatgt caaattggtt aaaaagaaaa aagggaactg tagataggaa catatttggt 3660

gcaagaaaaa ttcctgtgat tactagcttc actgtgagtt tattttttca cgtaataaat 3720

ccaaggttac ataatccata actgatatgg cagtttaaca ggttattaag aactcaaggc 3780

ttattgctct attctgccat ctttggtgag tggctttcat cctcattgtc tcagaaaagt 3840

tatttcacct ctaagtatta catctacatt tcaagcagga agaagatgaa gcttaagaga 3900

ccaaatggag catgtcagtc aaaggtgcct tatttaaatc tctggaagcc ccactgtccg 3960

gaaaccccct tgctgtaaga ggtggggttt taagcgggac atcccagccc tgagtgaagt 4020

cagggattag aagaatggat gctaggtagg taaatggcaa agacatggta ggcca 4075

<210> 24
<211> 2256
<212> DNA
<213> Homo sapiens

<400> 24
ccaaatgaac atttttggac cctaggtttt ggctcattgg ctggatcagt gcatgtaaga 60

attcgacaga tccgtcttga actcctgacc tcaggtgatc cccccaggct ggtcttaccc 120

caggtgatcc acccaccttg gcctcccaat gggattcagg cttaagccac cggcccggca 180

cttttgtttt taaaataata tcttactttc atatgattct ttcagactgt ggtatggcat 240

tctgaagcaa gtcttaacag agctaaaaat cacccttacc caattctctg cactgacata 300

tgtacacaaa gtaatatttt attattctcc ttcataaatg gtttctttaa ttgaaccttt 360

gagccttata ctatttttaa atttcccttt aaaaattgaa ttacagccag gcatggtggc 420

tcatgcctgt aatcccagaa ctttgggagg ccatggcagg aggattgctt gaacccagga 480

gttgtagacc agcctgggca agatagcgag atcctgcctc tgaaaaaatc ataataatac 540

tttaatttag aaaataccct gaattagaag tgcttcattc ctcatttgtg tacagctgtt 600

cctctatata cgaaggggat tggtaccaga accactgcat accaaattcc acccatactc 660

aagtctcaca gtcttccctg cagaatccaa gtatacaaaa agtaggccta tcatttatat 720

gggttttgca tcctgacagt actgtatttt caatcctagt ttgtttggga aaaaaaaatt 780

gtttgtatgc ctgggtacag tatcatacct gtaatcccag cactttggaa ggctgaggcg 840

ggaggactgt tttgtggcca ggagttcaaa tcagcctggt cgacgtagtg agaccccatc 900

tctacaaaag aaaaattagc tgggcatgtg gcacctgcct gtggtcccag ctactcggag 960

gctgtgaggc aggagaattg cttgagcctg ggaggttgag gctgcagtga gccgcgatca 1020

caccactgca tgccagcctg ggcgacagag tgagaccctg tctcagaaaa gaaaaaaaaa 1080

aaaaattgcg aagttgaccc gcactattca aatttgtggt gttcaaggtt caactgtaat 1140

ttcctagcag cattttgtgt gtttgagaat ctcttgacac tcttcaagta aatccctaaa 1200

ttacaacttt gacatcaaaa aagctagaca ttcctacatt tttgcactac aatacataaa 1260

aactcccatg ctgatcggtt gtgggatcgt gcctgtgaat aaccactgca ctccagcctg 1320

ggcaacatag taagtaagac cttgtctctt aaaaaaaata cattctgaag aaagttctac 1380

ttatgaatac attttattta taacaaactg gtgaaaattt tagaccaaac catgtctttc 1440

tgggttgtag tgattaaaaa atggttaaga gaatgttccc tatacaaggc atatgttatt 1500

aaacatgaaa tttaggatta gttttctctt tgaaaattct tttgatatgc tgaggttaca 1560

gtttggaggt tacattagag aaagaatata gggaagatgg cagtggtaaa acatttccct 1620

gctttacatt tttatattgg cagtatcttg gtttcaactt ttctattttc agcaagatat 1680

ttgcaagttt ttactttgaa gatgtttggt ttatagtagt ccatgctata ctaatgctgt 1740

tgctgagata attaagttag atactaatat gaaaaagaat atttgtgtaa aatattttga 1800

agttgtaatt agtgtatata caagttgtta atacttacga aaaaaggaag cattcccacc 1860

tgtaataatt ttgttaacac tagactatca gtaaattatc aagaataaaa accttggttt 1920

ttctgtaata tttgccactg agcaattttt attaaaaacc aaacagaagt atttacacaa 1980

gaacaagtga aattacacaa aatgataatg gggagaaatt tctcatctgg tgataaaggg 2040

aaaaagataa tgtaaaagca gattgctgct atttgcataa taagcacaag ttcaatcact 2100

ttggtggatt tctccatgta aagtatcttt ttaatagtaa gcaccattta ataatgaaaa 2160

accatatgtt ttgtattctg aaaacagtaa aaatactcac gtttatgatg atgggcctag 2220

gtactaaatt ccggaaaatt tctaaaaaca ttttgg 2256

<210> 25
<211> 3871
<212> DNA
<213> Homo sapiens

<400> 25
caggttcagg cattctcctg tctcagtttc cgtttctttt agtcacataa gaagtataag 60

aaagccttga ggccacttca gaaggaaaca cggagtgttc cacacagtga cttcgaccaa 120

attttattaa aaaaaaaaaa aaaaatatca gcagtccaat ctcagatgag acactgataa 180

aggatatccc cactcaaaac agtgattata ctttttgttc ccagtgaaaa ttgtggccac 240

tcagtgaaga gttcaggctg aggcatccta acactgatta cattcctagg ggtttctcca 300

ttaagttctc tcatatacaa gaaagagaag aatcactgaa aaattttcca atattaatat 360

actcataggt tttgcatcca gggagaagtt ccatattttc cctaacacag aggcatcact 420

gaaggctttc tcttcttctt tatatttcaa gtttctctgt agtgtgaatg ctcacaagtt 480

tcctaacaaa tgtagaaaaa cagaaagctc tccaatattg atgatgttca atggggcatt 540

ctctccagaa ttaattctca agtcccttaa aaaccgatag aacattcaag gctttcccat 600

agtcactgca gaataccaca ctatagtttg ctcagtaatg tgaacacttt tgctgagggt 660

attccgcatt tctgagtgtt gttccattgt gtgatttcca gtggtcttaa gggatgactg 720

tcactgaagc cttctgagac cttcacaggt tttctccctt agtgtgagct gtcacatgaa 780

tccctgctga tagcactgaa ggctttctac agctgttcca ttcttcagga ttctctcagt 840

acataatctt ctgggcccat gaaggtgaga attcgagctg agagctttac catacagatt 900

gtcctcaaaa ggtttctctt cagagtgaat taataggagt tactgaatgc tatttcatat 960

tgattagagt agaaggatga tgtgctgagg gcttttcttc attaattcca tggcaaattt 1020

cccagatgct tctgagttat ctgtttcctg gtattcatac cttctgcagt ctctcacatt 1080

gtatcaggat tggtgtgttg tgagaccaac acaataggaa ataagtgaaa gcttagcagt 1140

cctcctgagg ataggcataa aagccatttt ggcctctgta ttggtcctct cttggatcac 1200

taaccttggc gagaacttat tgccaaattg tcaaggagct gggccttgta aagaggccta 1260

catggtaatg actgaaggtc ccctgacaac aaccaacatc aactttccag gcatgtgaat 1320

aagtcatctt ggaagtggac tgttcatcag taaagatgtt agatgactat agctttggct 1380

gacatcttga ttgtaagtaa aggcatgaga gatcctgcag tgtaactaca gagataagct 1440

gttcttgaat tcctgatctg tagaaattgt tagagaataa acatttcgtg tttgaagcca 1500

caaaatttgc ggggatcatt tgttatgctg caacaaataa cttatatatc cagtgtgagc 1560

tctctcaaac tatcttaggg atgaaagatc aatgagtgct ttcctccaga agttcctgca 1620

gtaattcata tccatctatt atgtattctc ttgtgcacag aaagatagga gttacttgtg 1680

aaggatttcc cacaatgatt acattcatag ggtttttctc cagtgtgagt tcttaagtgt 1740

ttcttcacag ctgagagatt attaaaggct tttccacagt cactgcactc gtagggtttc 1800

tctccggtat gtattctctt gtgcacagta agagaaaagc tactgctgaa ggatttccca 1860

cactcgttac attcataggg tttttctcca gtatgagttc ttacgtgctg tgtcagatag 1920

gagctcttcc tgaaggtttt cccacaatcg tgacattcat aaggtttctc ccctgtatga 1980

atgctattgt gcccagtgag agaggacctt gtgctgaaag cttttccaca ctgattacat 2040

tcatggtgat tctcccccgt gtgaatcctc ttgtggcttt tgaggttaca actggtgcgg 2100

aaaacgtgaa aacactggtt acattcatag ggtttttctc cagtgtgaat tctcaaatgc 2160

agtctaaggg atgagggatc actaaatgct ttcccacagt gattgcattc atagggtttc 2220

tccccattat ggattctctt atgaatagta aggtaagatc tgctactgaa ggacttccca 2280

cattgactac agtcataggg tttttctcca gtatgaattc tcttgtgctg agttaggtta 2340

gatttcgtgc tgaagacttt aaaacactga ttacattcat tgagtttcac tccacgatga 2400

cttctttccg tttttacacc tttagactgt tcttttctga aaactttttt ttaaaacatt 2460

cttcttagga gttaaccatt tggctttaag tagagtctcc aaatctggac aggtgcttgg 2520

tagaattcct ctttcctctt cacccgtggt agaattcctc tttcctctgt caccaccttc 2580

ttgtcttgtt ccaactggga tatcagactg ggtttattaa cacgacaccc tagtgaggcc 2640

aggttcctgc agttctccag ccaggtctca gttctccagc atcacatccc tgtacagtgt 2700

cctttgggca gggtccagca acgcccactc ctcctgggtg aactccacgg ccacatcctc 2760

gaaggttacc aagcccccga ctctctcctg gggttctgag ctcttctccc tggcttctga 2820

gctcagccct caatgcttgt ctccttaggc cattcccagg tccagtgcac agtaccacag 2880

agcagctgcc ttttctcact cagactggga gctcctgtga tcttatttcc ctcaatctcc 2940

aggggatcat gggcccgtgc atggcagaaa gcagagaaat actgagattc ctgcttatta 3000

attgcttggg tttagaacat catcacgtct ttacttggtt cccttaaaac tctcaggagc 3060

acatgatgta ctcggcggag ggagctctga acctgcagta aatgttgaca cacactgaca 3120

tttctgggca gccctcaccc tccatctgat gcctggaaat cctgggatgc ctgaggacct 3180

ggtttggcca tgaggctcca ggctgctgga aggtatcaag tccaaccttc gccaaattgg 3240

gaaaaggaac atctgactca cccgtagcca gcttgtcagg agctcattaa ccagctctcc 3300

gccctgtgtg tgtccttttt gctgagaggc tgggaacagg gaagacggag cagcagtcga 3360

gggcaggatg acagccgcca tcctgggcca tcctgtgact ccgacagcaa cagggcagcc 3420

gggaagcagg acgctcctcc tttatcccgc agcgctctgt gtgagcagaa tatgtgagga 3480

gaagcggggg ggggaggggt gactttcagc gcttccctgc gtgtgtctag ggacaccagt 3540

tgccatggtg ggaagagctc aagtggccct gtgggaggcc tatgtggaga ggaactgagg 3600

cctcccaccg acggccagct ctgactcgca ggccatggag tggcccccgg tacccgttga 3660

gccttcagat gatgcagacc cagctgacag ccttctgcaa gtttccagcc agagccttcc 3720

agccaagctg ctcctgaatt ccagggagac gtggattgca gcaacttgcg ctttcggtca 3780

gcaactctgg cgctatttcc tatctggcag gtcaacagag acacagtggc ccacactctt 3840

cattctcaca cactgctggg ggtaacccct g 3871

<210> 26
<211> 4210
<212> DNA
<213> Homo sapiens

<400> 26
aaggcctggc ctcctcgctc ctcgctcagc ccctcacgct ccccacgtgt gggtgcaatg 60

tgttgggatt acagtatatg aaactggctc ttagggaaat aaagcctggg agggtagttt 120

gaaatccatt aaatgtttca aatgtgagac agcaacactg gaacagaagc atgagtatag 180

gccagtccct ggggtagtgg tggcagcgtc cttcctcctc ctgtaagggg acagtttcca 240

ccaatttggg atctcttgac cctctttttc agctctgcaa gatgtggaca catttctgtt 300

ttgtttttta aataaagttt ttggatgacc cggtactgct ttgcttcttt cccgctctga 360

accgggtgtg ctgtctcgag gacatcagag cctgacatct tttccccaga gagctggttg 420

ttaaaccttt cccacaccac cccgaaacca ctcctactta cgaagtttcc cagaataagg 480

ggagtggtga tgaattactt gacactacac aaatattgct gcgtagttgt cattttctat 540

taagggccag caaatacccc ttttagcaaa tgtttaatgt gttattgttt tgttgtgtat 600

cttttcttaa gctactgcag caccagcatg cccttggtgc ttaggcatgt ctaggaattt 660

ttgaatattg cacaatggcc aggcatgctg caatcaaggt ttaatttggc ctgggccaga 720

gccaaaaaaa ttggctcatc cccaccaaga gcttactgta ctgagagtta cgcagcccga 780

gtcacctctg gggtgtggaa catttgggtg atcccccgag ggcttcacgt ttggggaatt 840

tccatgtggc aactgtttct gagctcctgc tgagaccagg tgcttggagc agtgaagagg 900

cagcagtaga tgtggggaaa gaggtggtat tcccagctcc aactggaaaa gacttgaggc 960

ccatattaac agaacagtag aaaaagaagt gggaattaag caggtttctt ggtgcctgcc 1020

ctggtcttcc cttaggctgc cttcccacct ggatattcct tctgtaacct caacactctt 1080

ctcattttta ccagatttct cctacataat gttaggttgg gaagtccaag tccaatttcc 1140

ttcagtgtcc acaccccaac ccatatgagc ctagtgacat actagtagac tcaaatatgt 1200

ctacccacca atagccattt gccatttgtg ttctttaaaa aatgtgccca caaataaagg 1260

ccaggaaaat aacactagga aaatcacagc agggatcatg aatccttgtt aagcatctgg 1320

aaatcagctt tgttaaacca cacactaatt cagaaaagtg ctttgtagca aagcaatctc 1380

aacagccctt ataattctat cacttcccat ccagattctt ctgcaagcaa aatttatttt 1440

acatttattt ttaaatttca aatggaagag gttttgacaa tgtcactatg tttgatgttt 1500

atacctgccc tgaatgcttg ctcagaagag aaacagattt cccagtattt tttataactt 1560

actttcccat tgtcttcaat taatttgcta ttatcccaag tagacagaca acttcagtag 1620

tagccatctc cctacatttt tagatcactg aaaaaaatgg atgagcaacc catgaaaata 1680

actagcttac tgaaatgctt gtcttttaaa gaaatggttt aaaaaaaagg gggagagaag 1740

cattgccttg gttctgacac taagtaaccc ttttgccttt tgcaggtaac ttctcatcta 1800

ggtcctcagc atccttctct gtaaactgaa gagtttaaat gactctaagt tcctctctct 1860

ctctctagat gttctctaac accaagttta tgtgagtttc tttggccaga ttttaaccag 1920

agaccatcct ccacgaaggt caaagataat taattctgag atagcaggag ttgtttttgg 1980

catgtgcagt caacctttca aaaaaccaag agttctcaag ttaagctacc ttcacgaacc 2040

tcttattaaa tttaagtatc actctctgtc cctcctactt tgctatgttc ctccaccacc 2100

tccctccacc agaaaaaaaa aaaaaaaaaa tttttcttct ttcttggttc ttggaaaatg 2160

gtgtgcaatt acttcctctt cttccgtggc tgcctcatgt caccccattt ccagtggttg 2220

agcttctctc caagaggtag atcttgcaag atctgaacag aaacccattt ccagtggttg 2280

agcttctctc cagagtgccc atttccagtg gttgagcttc tctccaagta ggtagatgct 2340

ggcgatgaag acgtagctga gagagaagaa agctgctaga cccaagaggc tgagtttggc 2400

aaacacagga tacacccagg tacccgtctc aaagtagagc cataggatgc ggctgatgta 2460

agcaatgctg gcagcagcca gcaaggtgag tcctgtcaaa tacagcatac aaattcatgt 2520

gtataatcag ctagatccac tttgcttctc ttcaacacat gcaagaacaa caacaacaaa 2580

atctttatcc cacgcccaga tactctgtct ttagggatat atccttgtgc cagatatgcc 2640

actgtccatt gtctgatcaa ctttttcttg acacttttac agtccgttaa attcctttgt 2700

taaaagaaca atatagagca ggggtttatt taggaagaac agaaaggtgg gaggggtaga 2760

caccacaaaa gggaaggaag ggctagggaa acaggatgag gtctggcacc agtttatttc 2820

tctaagacag aggctggcag tccataaaac aggcactgct agagcccatg ttctttggaa 2880

tgcactctct ccatcggagg gttgtccaga ttgtcacaca tgaatagctt gtgtgagtct 2940

agagaaggaa cactgattag cttccaatct gcgtgttcag caagcctctt tctttcagaa 3000

gaaaagcatg gtgctagatg agaaggttat agcacggagg ccaagcacgt ctggtttaaa 3060

tgagattgcg gtaaacaaac atccaaatgc cttctaggag ctctgatgcc agctgctgat 3120

gcaaataagc ccataatgaa agcaacagga gtggacaaac cactaaattt gaatgtagtt 3180

agatgcctca gatccctcag ccttcaagca gttcattgaa gatagaacca aggagcaaca 3240

ttttcttgaa gatgagcaat gattaattct tcacgagaga gagaggggaa gtggtgacct 3300

gcatgtttct gtgcttgggt cattaaacca agataaggta ttgaggcaag cctaagttga 3360

ctttctccat gtaattcttg ctgatgtaag caatgctggc agcagccagc aaggtgagtc 3420

ctgtcttctt tgatggatag gagtgaggcc tgaggacgac ttcagccaat gtgatgggga 3480

atatgaaagt gtgcattgca tgattcagcc acacggggat gacagtatct aggaccttgg 3540

gcatgattca gccacacggg gatgacagta tctaggacct tggggtaaat gagatctcga 3600

ttgtagagaa agaggatcca gaatgccaaa aatacaaacg tggatacagg aaaagccaga 3660

gtggtgaaaa gcaggtctct gaagacagga aaagccagag tggtgaaaag caggtctctg 3720

aaggcagtta ggaacttaat gtcttttccc cctttggttc ttttcagcac atcatccagg 3780

caggtgaccc cgtagaaaat ggtctgcaag agcagattaa gcagcgtcat atatttccac 3840

cttgcccatt tgcctaagca gcgtcatata tttccacctt gcaccatttg ccaagatttt 3900

gggtttcacc tcgtcttttc cttcctgtga gatgtaataa ttgaggaaag tataccagct 3960

cagaacaagg aagtggtata tgcatgtaga agtcttcgtc atggcgagtg ctgaccgggg 4020

caccgtgaat gtcttgagta ctttctggcg tccttctgtg ggtcacagcc taagtccaac 4080

agcaggccgg agagctaaaa ccacacccct ctgggactga gagttccaac acaggctgaa 4140

tttggttctc ttttaagctc ctttcctgat aaacttcaga ggcaggatta ctgctggttt 4200

tcagatgcct 4210

<210> 27
<211> 2204
<212> DNA
<213> Homo sapiens

<400> 27
tgggtgatcc cgagggcttc tgggatttcc tgtggcaact gtttctgagc tcctgctgag 60

accaggggca gtgaagaggc agtggggaaa ggatcccagc cgaaatcaag tgggaattaa 120

gcaggtttct tggtgcctgc cctggtcttc ccttaggctg ccccccctga gatattctgt 180

cctcttctcc ttcaaacacc gctacataat gttaggttgg gaagtccaag tccaatttcc 240

ttcagtgtcc acaccccaac ccatatgagc ctagtgacta gacccatttc taaccacaaa 300

taaaggcgaa aaatcatctt ggaatcagtt ttaaccacac taattcagaa aagtgctttg 360

tagcaaagca atctcaacac cttaattcta tcacttccca tccagattct tctgcaagca 420

aaatttatta ctatatatta ataatgttca aatgaagttt tgacaatgtc actatgtttg 480

atgtttatac ctgccctgaa tgcttgctca gaagagaaac agatttccca gtatttttta 540

taacttactt tcccattgtc ttcaattaat ttgctattat cccaagtaga cagacaactt 600

cagtagtagc catctcccta catttttaga tcactgaaaa aaatggatga gcaacccatg 660

aaaataacta gcttactgaa atgcttgtct tttaaagaaa tggtttaaaa aaaaaacgcc 720

aggagtggga gagagaagtc attgccttgg ttctgacact aagtaaccct tttgcctttt 780

gcaggtaact tctcatctag gtcctcagca tccttctctg taaactgaag agtttaaatg 840

actctaagtt cctctctctc tctctagatg ttctctaaca ccaagtttat gtgagtttct 900

ttggccagat tttaaccaga gaccatcctc cacgaaggtc aaagataatt aattctgaga 960

tagcaggagt tgtttttggc atgtgcagtc aacctttcaa aaaaccaaga gttctcaagt 1020

taagctacct tcacgaacct cttattaaat ttaagtatca ctctctgtcc ctcctacttt 1080

gctatgttcc tccaccacct ccctccacca gaaaaaaaaa aaaaaaattt tcttctttct 1140

tggttcttgg aaaatggtgt gcaattactt cctcttcttc cgtggctgcc tcatgtcacc 1200

ccatttccag tggttgagct tctctccagt ttccagtggt tgagcttctc tccaagtagg 1260

tagatgctgg cgatgaagac gtagctgaga gagaagaaag ctgctagacc caagaggctg 1320

agtttggcaa acacaggata cacccaggta cccgtctcaa agtagagcca taggatgcgc 1380

tgatgtaagc aatgctggca gcagccagca aggtgagtcc tgggctgatg taagcaatgc 1440

tggcagcagc cagcaaggtg agtcctgtct tctttgatgg ataggagtga ggcctgagga 1500

cgacttcagc caatgtgatg gggaatatga aagtgtgcat tgcatgattc agccacacgg 1560

ggatgacagt atcttgattc agccacacgg ggatgacagt atctaggacc ttggggtaaa 1620

tgagatctcg attgtagaga aagaggatcc agaatgccaa aaatacaaac gtggatacag 1680

gaaaagccag agtggtgaaa agcagtctcg gaaaagccag agtggtgaaa agcaggtctc 1740

tgaaggcagt taggaactta atgtcttttc cccctttggt tcttttcagc acatcatcca 1800

ggcaggtgac cccgtagaaa atggtctgca agagcagatt aagcagcgtc atatatttcc 1860

accttgtcat atatttccac cttgcaccat ttgccaagat tttgggtttc acctcgtctt 1920

ttccttcctg tgagatgtaa taattgagga aagtatacca gctcagaaca aggaagtggt 1980

atatgcatgt agaagtcttc gtcatggcga gtgctgaccg gggcaccgtg aatgtcttga 2040

gtactttctg gcgtccttct gtgggtcaca gcctaagtcc aacagcaggc cggagagcta 2100

aaaccacacc cctctgggac tgagagttcc aacacaggct gaatttggtt ctcttttaag 2160

ctcctttcct gcagaggcag gattactgct ggttttcaga tgcc 2204

<210> 28
<211> 4577
<212> DNA
<213> Homo sapiens

<400> 28
cacctggcgg cgggggagca ccgcgggggg ctgccgaaac tacccagcca ccttctgggt 60

gaaccctcag ttcaagatcc ggctggatga gacggttcga agccatctgg tcctggacat 120

ttctttgccc ttctgccacc ttctgggtga accctcagtt caagatccgg ctggatgaga 180

cggatgaccc ggacgactac ggggaccgcg agtcaggctg cagcttcgtg ctcgccctta 240

tgcagaagca ccgtcgccgc gagcgccgct tcggccgcga catggagact attggcttcg 300

cggtctacga ggtccctccg gagctggtgg gccagccggc cgtacacttg aagtgatggt 360

gccagagggg agcaacccag tggcaaaccc accctggcct ggagagctaa gaagtcacct 420

tgtttcactg cttgaaaaat gcttctcccc acctgttcag tgggcagagc aaacattgct 480

ctcctgaggg tcagcacagg tagtcagtga gggagctgga tttgagccct ggtcctcaga 540

gcccctgtct gacatgtaca catgaggctt ttcctgcctt gccatctaca ccaagtaagt 600

gccaactagg gcggtccagc aatgtcagga gcagggcatt agacaggctg agagttctgt 660

acccttgagc agttgcttaa gctccgagcc tgtgtttgtg tctgtaaaat gggtgtaaca 720

gcagaacagc aaatgaggtg ccgagtaccc tgcagtacct gacctgggga aagctcccag 780

tgataataat gacagtgtta ttgaaacaca tgcctaatct gcaatccggt gctcctctcc 840

tctaccctct gcagtgagta gggttgttac attttacaga tgaggaaact gagcaaaatg 900

tcttgttagg gtcacacagg ctgtgagtga gccatgcccc agctctccag ccccttggcc 960

aggacaagga gcctggagtc tgggtctggg ggtggcccct gagggtgggc tgagtgggca 1020

gctgcagccc tgctccctcc tccctcccac cggccccgct aggcttcttc ctccaatgcg 1080

tctcgggcgc gctcagagca gttcatcaac ctgcgagagg tcagcacccg cttccgcctg 1140

ccacccgggg agtatgtggt ggtgccctcc accttcgagc ccaacaagga gggcgacttc 1200

gtgctgcgct tcttctcaga gaagagtgct gggactgtgg agctggatga ccagatccag 1260

gccaatctcc ccgatgagca agtgctgagc tggatgacca gatccaggcc aatctccccg 1320

atgagcaagt gctctcagaa gaggagattg acgagaactt caaggccctc ttcaagtgct 1380

ctcagaagag gagattgacg agaacttcaa ggccctcttc aggcagctgg caggggagga 1440

catggagatc agcgtgaagg agttgcggac aatcctcaat aggatgacat ggagatcagc 1500

gtgaaggagt tgcggacaat cctcaatagg atcatcagca aacacaaaga cctgcggacc 1560

aagggcttca gcctagagtc gtgccagaat ccaggctcct gctcacacat tgagctgaac 1620

ctcatccctt ggtctgcatg agtcggggaa tcctccagtt tttctgagcc cagttccctg 1680

ccatttccat cccatactcc tcctcctgac ctgccatcct gataatccct gtgctcgggt 1740

gctgtgcctt tgtgggatta gcagcgcagc ctggccaggc acagtggctc acacctgtag 1800

tcccaacact ctgggaggcc gaggcaggag tcgcttcagc ccaggagttc aacaccagcc 1860

tggacaatat ggagagaccc tgtctctgca aaaaagtaca aaaattagct gggcgtggtg 1920

gcatgcacct atagtcccag ctactcagga ggctgagttg ggaggatggc ttgagaccag 1980

ggaggttaag gctgcagtga gctgtgatca catcagtgca ctccagcctg ggcaacagag 2040

caagaccctg tctcaaaaaa aaaagctagg ccaaagaaaa gtctaccttt cctcccagag 2100

cctcctgggc caggctgagt ggcttgggga acaccacgtg actggcttca ctccccaggg 2160

cagacaactg ctggcctctc atcccctccc ctcccctttg ctgccactga cctccatcca 2220

cacggtttcc ttaagcagag cttttctcct cctcttctct gctagtgaag cactgcttac 2280

cctcctagcc tacagcaagc cctgccctgc cccatctttc tacccaattt ctcgctcttc 2340

tgtttcacac tcatttcttt ttactttgga ctaattgtgg ctgttgggac tttggcttga 2400

ttgctaaaat actttacttg tacttcctga tgatacaaac aatcctgccc acttcctctg 2460

ccagatattg cacccactcg tcaggatttg tgcgtccttg actctgcccc acccagtgct 2520

cccagcaggg gcaggtgcag gaagctcact aggtggagat gttggaattg gtttttcttg 2580

cagacaaaga cctgcggacc aagggcttca gcctagagtc gtgccgcagc atggtgaacc 2640

tcatggatcg tgatggcaat gggaagctgg gcctggtgga gttcaacatc gggtatcggt 2700

gctgggagaa ctgcccagga cagcacagag aacaggacag agccatgcgg agtgtggaca 2760

cacagtgccc catgctcaga tgtctccctg gacgtcttcc tgttggttgg gagggagaaa 2820

ctgaggcaca gacctgtgtc aagtgaaagg tggagcaaga cctgggtccc cattgaccct 2880

gaggctggtg ctatttctga cccctcccca gcccactcca ctgcagccca gctcagagaa 2940

taaggcaagc ccggctgtgg gctgactgta catggctttt gctgcttctc ctccccagcc 3000

ccaagtcgac ttgccggctc ggcggccatc tccccttctg cacagttgcc cactctccca 3060

tgactggtct aagtgatgga atttccttct taacggccac ccagcgtgat ggcaatggga 3120

agctgggcct ggtggagttc aacatcctgt ggaaccgcat ccggaattac ctgtccatct 3180

tccggaagtt tgacctggac aagtcgggca gcatgagtgc cccccctcag tccatcttcc 3240

ggaagtttga cctggacaag tcgggcagca tgagtgccta cgagatgcgg atggccattg 3300

agtcggcagg cttcaagctc aacaagaagc tgtacgagct catcatcacc cgcgcttcaa 3360

gctcaacaag aagctgtacg agctcatcat cacccgctac tcggagcccg acctggcggt 3420

cgactttgac aatttcgttt gctgcctggt gcggctagag accatgttcc gatttttcaa 3480

aactctggac acagatctgg atggagttgt gacctttgga tttttcaaaa ctctggacac 3540

agatctggat ggagttgtga cctttgactt gtttaagtgg ttgcagctga ccatgtttgc 3600

atgaggcagg tccaattttc ttatctggct tcttcctggg ctcagagcca accctgaagc 3660

ccgggccacc tgaatcctga aaggctgggg tgggggtgtc tggattctgg gaaggagagg 3720

ggggaggtcc aggcccttgg aggccgaaag acccctccat gaggttcctg aggtggcgag 3780

gagaagccag ggagccattt ctgcggcccc agctgacctg cctgttctcc cgcagtggtt 3840

gcagctgacc atgtttgcat gaggcaggga ctcggtcccc cttgccgtgc tcccctccct 3900

cctcgtctgc caagcctcgc ctcctaccac accacaccag gccaccccag ctgcaagtgc 3960

cttccttgga gcagagaggc agcctcgtcc tcctgtcccc tctcctccca gccaccatcg 4020

ttcatctgct ccgggcagaa ctgtgtggcc cctgcctgtg ccagccatgg gctcgggatg 4080

gactccctgg gccccaccca ttgccaagcc aggaaggcag ctttcgcttg ttcctgcctc 4140

gggacagccc cgggtttccc cagcatcctg atgtgtcccc tctccccact tcagaggcca 4200

cccactcagc accaccggcc tggccttgcc tgcagactat aaactataac cactagctcg 4260

acacagtctg cagtccaggc gtgtggagcc gcctcccggc tcggggaggc cccggggctg 4320

ggaacgcctg tgccttcctg cgccgaagcc aacgccccct ctgtccttcc ctggccctgc 4380

tgccgaccag gagctgccca gcctgtgggc ggtcggcctt ccctccttcg ctcctttttt 4440

atattagtga ttttaaaggg gactcttcag ggacttgtgt actggttatg ggggtgccag 4500

aggcactagg cttggggtgg ggaggtcccg tgttccatat agaggaaccc caaataataa 4560

aaggccccac atctgtc 4577

<210> 29
<211> 4504
<212> DNA
<213> Homo sapiens

<400> 29
agtcaatcct ccagaaccta tgctggtgga acagcctcgg ccaccaaggt gtcagcttcc 60

tctggtgcaa ccagcaagtc ttccagtatg aattgaaaac caagccgagg actgcggggt 120

ccggtgtcca cgcagagtgt cagcttcctc tggtgcaacc agcaagtctt ccagtatgaa 180

tgaattattt catgcatcaa gagaagcaac tggaatccct agaggaaaga aatctgcttt 240

tctattcttt gatgactgac ctaggctgtg tggctagctc ttctccttac ttacaaaatt 300

attatcccat aaaaaagcac tggaaaacta aaagcaatga aaatgaagca gttgcctcga 360

gaactggaag ctcaacttca tgccttaaat gttttttttt gggcggtagc tcataatcag 420

accctcagag aatatggcgt gttctctgct aggcaacgtt tgtatgttta tttcgaggcc 480

tacccacatg ttgcatcaga ggccaaagct catgtgctct gcactcccag aacaagtaga 540

gctctttatt ttaactaact gtttcctaat gtttatgaat gtacaaccca agcctccagt 600

cattctctaa tttttgcatt caacttcatc taacctgttg cattctggac tgaatatctc 660

tgggcaaaaa gggacattat tgtcatatct gtgttcaaat tcaggcttac atagagctca 720

ccgttacata agcaatagtc ccgcttgcta gaagcctttc ctaaccatct tccaaaccag 780

cacctcagaa atagtctccc tcttctgacc gcctgtagca ttgtattgta ccattcaaat 840

ccttacaagt tggcactgta actagatctg ctagcacaac accatattct gcccaaaaga 900

gatggcttag atgatccatt gtttatcttt atcagaacac actgaatatt catttcctga 960

agtgaataat ccatcattac agaaagaaaa attgtgagca gccttgtttt gttgcggttg 1020

tcttacagga agcagctcat gtcagcttcc tctggtgcaa ccagcaagtc ttccagtatg 1080

aatcccacag aaaccaaggc tgtaaaaaca gaacctgaga agaagtcaca gtcaaccaag 1140

cttcctaaca agaaaaaaca caaaaaacag gctgtaaaaa cagaacctga gaagaaactg 1200

taaaaacaga acctgagaag aagtcacagt caaccaagct gtctgtggtt catgagaaaa 1260

aatcccaaga aggaaagcca aagctgtctg tggttcatga gaaaaaatcc caagaaggaa 1320

agccaaaaga acacacagag ccaaaaagcc tacccaagca ggcatcagat acaggaagta 1380

acgacaaaaa gcctacccaa gcaggcatca gatacaggaa gtaacgatgc tcacaataaa 1440

aaagcagttt ccagatcagc tgaacagcag ccatcagaga aatcaacaga accaaagact 1500

aaaccacaag acatgatttc tgctggtgga gagagtgttg ctggactaaa ccacaagaca 1560

tgatttctgc tggtggagag agtgttgctg gtatcactgc aatatctggc aagccgggtg 1620

acaagaaaaa agaaaagaaa tcattaaccc cagctgtgcc agttgaatct aaaaaaaaag 1680

aaaagaaatc attaacccca gctgtgccag ttgaatctaa accggataaa ccatcgggaa 1740

agtcaggcat ggatgctgct ttggatgact taatagatac tttaggaggc aggcatggat 1800

gctgctttgg atgacttaat agatacttta ggaggacctg aagaaactga agaagaaaat 1860

acaacgtata ctggaccaga agtttcagat ccaatgagtt ccacctacat agaggaattg 1920

ggtaaaagag aaggatccaa tgagttccac ctacatagag gaattgggta aaagagaagt 1980

cacaattcct ccaaaatata gggaactatt ggctaaacta aaggaagggg atcacagggc 2040

ctcctgcaga ctcttcgaaa cccatagggc cagatgatgc tatagacgcc ttgtcatctg 2100

acttcaaccc atagggccag atgatgctat agacgccttg tcatctgact tcacctgtgg 2160

gtcgcctaca gctgctggaa agaaaactga aaaagaggaa tctacagaag ttttaaaagc 2220

tcagtcagca gggacagtca gaagtaatct acagaagttt taaaagctca gtcagcaggg 2280

acagtcagaa gtgctgctcc accccaagag aagaaaagaa aggtggagaa ggatacaatg 2340

agtgatcaag cactcgaggc tctgtcggct tcactgggcg atacaatgag tgatcaagca 2400

ctcgaggctc tgtcggcttc actgggcacc cggcaagcag aacctgagct cgacctccgc 2460

tcaattaagg aagtcgatga ggcaaaagct aaagaagaaa aactagagaa gtgtggtgag 2520

gatgatgcaa aagctaaaga agaaaaacta gagaagtgtg gtgaggatga tgaaacaatc 2580

ccatctgagt acagattaaa accagccacg gataaagatg gaaaaccact attgccagag 2640

cctgaagaaa aacccaagta aagatggaaa accactattg ccagagcctg aagaaaaacc 2700

caagcctcgg agtgaatcag aactcattga tgaactttca gaagattttg acctcggagt 2760

gaatcagaac tcattgatga actttcagaa gattttgacc ggtctgaatg taaagagaaa 2820

ccatctaagc caactgaaaa gacagaagaa tctaaggccg ctgctccagc tcctgtgtcg 2880

gaggctgtgt tcggaatcta aggccgctgc tccagctcct gtgtcggagg ctgtgttcgg 2940

acctccatgt gtagtataca gtcagcaccc cctgagccgg ctaccttgaa gggcacagtg 3000

ccagatgatg ctgtagaagc cttggctgat agcctggggc acagtgccag atgatgctgt 3060

agaagccttg gctgatagcc tggggaaaaa ggaagcagat ccagaagatg gaaaacctgt 3120

gatggataaa gtcaaggaga aggccaaaga agaagaccgt gaaaagcttg gtgaaaaaga 3180

agagagaagg ccaaagaaga agaccgtgaa aagcttggtg aaaaagaaga aacaattcct 3240

cctgattata gattagaaga ggtcaaggat aaagatggaa agccactcct gccaaaagag 3300

tctaaggaac agtttccccc ccggatagat aaagatggaa agccactcct gccaaaagag 3360

tctaaggaac agcttccacc catgagtgaa gacttccttc tggatgcttt gtctgaggac 3420

ttctctccca tgagtgaaga cttccttctg gatgctttgt ctgaggactt ctctggtcca 3480

caaaatgctt catctcttaa atttgaagat gctaaacttg ctgctgccat ctctgaagtg 3540

gtttcctttt ttcaaaaaga aatttgaaga tgctaaactt gctgctgcca tctctgaagt 3600

ggtttcccaa accccagctt caacgaccca agctggagcc ccaccccgtg atacctcgca 3660

gaggacaaaa ccgatgagga atgttcaagc ttttagtgac aaagacctcg atgatgcctt 3720

ggataaactc tctgacagtc taggacaaag gcagcctgac ccagatgaga acaaaccaat 3780

ggaagataaa gtaaaggaaa aagctaaagc tgaacataga gacaagcttg gagaaagaga 3840

tgacgaaaaa gctaaagctg aacatagaga caagcttgga gaaagagatg acactatccc 3900

acctgaatac agacatctcc tggatgataa tggacaggac aaaccagtga agccacctac 3960

aaagaaatca gaggattcaa aggacaaacc agtgaagcca cctacaaaga aatcagagga 4020

ttcaaagaaa cctgcagatg accaagaccc cattgatgct ctctcaggag atctgaaacc 4080

tgcagatgac caagacccca ttgatgctct ctcaggagat ctggacagct gtccctccac 4140

tacagaaacc tcacagaaca cagcaaagga taagtgcaag aaggctgctt ccagctccaa 4200

agcacctaag aatggtaaaa gttatattga ttgatctatc tatcggttgt cttgtaacag 4260

ggtcttactc tatagcccag gctggagtgc agtggtgtga tctcagctta ctgcgatctc 4320

tgcttcctgg gctcaagcaa tcctcctacc ttagcttcct gagtagctgg gactacaggc 4380

gtgcaccacc acacccagct aatttttgta tttttttggg gttctcgctg gtctcagctc 4440

ttggaagtga tctgccaacc tctacctccc aaagtactgg gattacaggc ttgagccact 4500

gcgc 4504

<210> 30
<211> 13391
<212> DNA
<213> Homo sapiens

<400> 30
gcagcacacc tgctccatcg gcggcctgct ggagcgagag cgagttgcgc tgcagagcca 60

gtcgggctgg gcgggctggg tgccactgct gctggcgctg ctccacgagc tgcaggcccc 120

ggcctcacgc tggaggccct actttgcgct ctggcccgag ctgggccgct tggagcaccc 180

gatgtttggg tgcgcacgtg aagggaacct gggtgtgggt gtccctgcag gccagaggag 240

gagcgccggt gcctgctcca gggcacaggc gtacctgagg ccgtggagaa ggatttggcc 300

aacatccgca gcgagtacca gtccatcgtg ctgcccttca tggaagccca ccccgatctc 360

ttcagcctca gggttcgctc cctagaactc taccaccagc tggtggccct tgtgatggcc 420

tatagtggga gaggaaaccc cccccaggcc ttagccaggt tctggaacac tggaggaaga 480

agaggatgaa aaggagccca actcccccgt gatggtgcct gctgcagaca tactaaacca 540

cttagccaat cacaacgcca atctagaata ctctgcggtc taattctgct ctctagtgct 600

acaaaatgag tagggacctt ttagtatgca tattactgta gaatcattaa attagtaagt 660

tgtggttaat cctctgataa actatactac catcatttat ttagtgctag acattgagtg 720

gtctgcatag ccatgtttca ttcctttctg gctgatcaca tcagtgagcc cagctctgtg 780

gcagcctgga acagtgatag tctccgtgtt ttggagggat gaggacattc acaggccaaa 840

ggtctaagcg atcctagaac tgatataaag tgtgtttata ccagctctat tctatataaa 900

aagagccaaa ggttgaatat gttttttact ccctttcctt tccactttga tctgcgaaag 960

ttccattttt tcccccccga ggcggagtct cactgtgtcg cccaggctgg agtgcagtgg 1020

caactgccgc ctcctgggtt caagcaattc tccctgcctc agcctcccga gtagttggaa 1080

ttacaggctc ctgccaccat gcccggctaa ttccctgatc tcaggatctg cccactttga 1140

cctccctaag tgctgggatt acaggtgtga gccactgcgc ctggccgaaa atttcgtttt 1200

taaagctagc aactttttaa gtgaggactg tgcagaaatc cacccagtta aacatgaagc 1260

tggcctttta aagttagccc aacccttttt tatggtagtt aacagcatca gtcatggctg 1320

aactacacct gctgaagaaa taatcttcat ggggctggcc cgtgaaaggc atggccccag 1380

cttctccctt tcacctcaga attgtcttcg gatggtagcc actcagccca ttcctaaagg 1440

ccatgagatt ttcaacactt atgggcaaat ggctaactgg caactgattc atatgtacgg 1500

ttttgttgaa ccatatcctg acaacacaga tgacacagct gacattcaga tggtgacagt 1560

tcgtgaggca gcattacagg gaacaaaaac tgaagctgaa aggcacctag tgacgaaagc 1620

tgaaaggcac ctagtgtacg agcgctggga tttcctatgc aaactggaga tggtagggga 1680

agagggagcc tttgtgatag ggagggagga ggtgctgact gaagaggagc tgaccaccac 1740

actaaaggta ctgtgcatgc ctgctgagga gttcagagag cttaaagatg atgccgctga 1800

ggagttcaga gagcttaaag accaggatgg agggggagat gataaaaggg aagagggcag 1860

cctgacgatc acaaatattc ccaagctcaa agcatcgtgg agacagctgc ttcaaaacag 1920

tgttctactg actttgcaga cctatgccac agacttaaaa actgaccaag gtttactcag 1980

taataaggaa gtctatgcga aactcagctg gagggaacag caagccttac aggttcgcta 2040

tggtcagaag atgatcttac atcagttgtt ggactgacaa gagttggaaa aaaatttatt 2100

ctaagctaat actcattgat gtttgaaaaa gggaaaattt ggatctttct tttgcttact 2160

aaacaccaag aggaaaagta gcaaagttgg tgtgctagga ttaactcagg taagggtgat 2220

gtgttttagg attgagaaca gcagacttgg gaatcactgc taattgttac ttaaagcatg 2280

ttacagtgtt ttgttctcag ttttaaccaa agccagtgga catacggtag taataagtaa 2340

gtcttgttgt gtttcagcat ttaataatag actttggagg tagacccctg gtttaaatct 2400

aagtctagtt tgaggaagtc acttaacctt tattgaaaag actctggatt taataagctg 2460

tgtaactgga ctcgatagtt acccaaagtt cagtctagat ggcacaaacc acctctcagg 2520

gaataaaccc taagacatca ctcaaggagt tcaattattt aattttgaac tgttttgtct 2580

ttggccataa aacttgacag tcatgagcaa attttaagtg ggttaagttt ttaaatacgt 2640

atctactcat ttttttaagg ggaaaggata ttaaaactgg gtattttaaa ctcagcttaa 2700

ccagataaac ctgctccagc atttgtccag tgttgaaata aataggaaga aaaatacaat 2760

ttccttaaac atgcctgtgg aatgacctat caatcaatca aatcattcaa tttgtcccgc 2820

cccaaggtct cagggctgga ttgtccactt ttcctcctca ccaccagcac tgctgctgct 2880

actgagaaag gcacagtgga ctcatgtgtg tgtcatgact ttaatggtta gctacagtat 2940

gcatacacat acaagaagta ggggagctga agcccaggaa aaggctacaa aatacaacac 3000

agggaccaat acatttgcaa aacattcaaa atccttataa aaggggctgt attggtcctt 3060

tcaactttgt cattattcag cttagcctgt atggccattc attcagtggg gtaatggggg 3120

agaacaatta attaatgaga tttggttccc tttcctatat tcccactgtt tttaagtttc 3180

aggagaggat tggggggtga gggtaggggg gttgcggttc ctttaaagag ctgaccccca 3240

tctcaggcgg ctgaggtgta taatccccaa aagaaataag atggtacaaa tgcagtcagc 3300

tttggaatac gtttgggtag agacaaaatg gaaactcatg ggattccaat agtgagccct 3360

tcccatgttc ctggacatat ccaattctag cacatccaca tttttaataa cttagtgatt 3420

ttcaaggtcc cctgtccaca ttaaaaaaat aagttacata atattcaaac tggctttttg 3480

ctattctttg ggacaccagg aatgtcagaa gacatggagc tatgccctca gacaagtgca 3540

tggcatcagc tgcctcttca ttacaaggta ccagtttatg tacttgcctt ggacacagct 3600

tgcacaaagc caagaagttc cagacaaagg ttttctcttt catgtattta cacaagttca 3660

aaatgatatt cacagcatct tctaaatttt ggccaagagt caaaaaaatg catttaaact 3720

ttggaacgtg cccacataag acaggaggct gatcccaaca gtagttgggg cagataccca 3780

caaaccaaag ggctgggaaa gtcaggaaga gctgaaagga ttcttcagtc agtttatgaa 3840

ctcggtgcag tgagacctct agactgacac gtacaacaga gatgcagttt cgtctaactg 3900

gcacctgtcc cttccattac ttgctgggcc tgcttctgtc ccatgcagca ctgtgcgacc 3960

gactggaata acttttcgat ttctggggca cagtgtacaa attcatggtt ccagaactta 4020

aactttcgat ttctggggca cagtgtacaa attcatggtt ccagaactta aacgctgggt 4080

ttttaatcag ctcaatgaag gtaataagaa gaccccaagg atgtggccta tttacaatca 4140

accgttccaa gagaactctt gtgatctgtt cttggatggc ttccgtattg gcctctgcaa 4200

aaaggcttgt gatctgttct tggatggctt ccgtattggc ctctgcaaaa aggtacagca 4260

tggtgcaact gaagtagtga gtgtggctat ttgggtaccg gagctgattt gcaattgcat 4320

tcaaaaagag atagcgaccc tcagtgtcca agtccacagc caaattctgg aagatacccc 4380

tcagtgtcca agtccacagc caaattctgg aagatatcca tgtgtgctga gtgagtgatg 4440

gtgctcattg aaggtgtgct gcccttgttg tggatgtgcg caatggcctg agtcccgaca 4500

tagagcacca gtgcattgat gagctggagg ttgtagcgat tcccaggttc attggatacc 4560

tgtaggttgc tgcgcagatc agacaggaaa gtgactggtg atcgaggtag gttgctgcgc 4620

agatcagaca ggaaagtgac tggtgatcga gttttaagat aggaatccaa atcctttttg 4680

aactgaggtg gcattactcc agtgaaattg gtgagaatcc ggggagcaat gttaatttca 4740

ctcaacatgt ccacctttag attaggagtg aatgggtcgg ggagcctcat gtttcttggt 4800

ttagattagg agtgaatggg tcggggagcc tcatgtttct tggaaaggca ctcaggatca 4860

aatttcttaa ctggatacaa ttaggtggga tcacatcaca gaacccataa tggtaatcac 4920

aaaggaactc tgggaaatca tgcaaaagaa ccagcagcac tcttaaagtg cccttgtaga 4980

ggatttgcat aggtttggtg agttccacat ttctaagact tgtagaggat ttgcataggt 5040
ttggtgagtt ccacatttct aaggaaaggc gctaaatatt tgaataaatc aatcagtagc 5100
tgtgcataca taggccaccc cttctgctgt ggcgtatgtg ccagcattct tgcaataaat 5160
atccgattct acaggagata aactgcagcc accttctgct gtggcgtatg tgccagcatt 5220
cttgcaataa atatccgatg ggaaatcagt tcaagccagg catatacaaa gccaggagct 5280
ttggtaggcc tcaagatgtg gaatgtattg cagaaagctg taagtgtctg gaaattaatg 5340
gtttccaaca catgcaagaa agctgtaagt gtctggaaat taatggtttc caacacatgc 5400
tcaggtgcat tgagttccaa gagaagcatg ataaaaattc gatggtaggg aagttgctga 5460
aattcactct gacgaacatc atgatcctga aggagaactc ccactactat accaaggacc 5520
ttgttcagca gattaatctt tgtgacagtg ttggtggcct cccccttgtt cagcagatta 5580
atctttgtga cagtgttggt ggcctcccct gagtgtttca cgagcagtgc aatgagtcga 5640
acaaaggcat ccaggttgtg atagcacttg gctcggatca tggtgggatt ggcagcagga 5700
ttgtgctgct gctcagcctg agcacggtaa ctgatttcaa cacacatttc agtacacaga 5760
cgaaagaacc ttgttatgag atcatcggtc ttcagtattc cttgctggtg catctgtcca 5820
acaaatgcag agaaagcttt ggtactgtcg cggcagcttc caaaagctct acctgtccaa 5880
caaatgcaga gaaagctttg gtactgtcgc ggccagctgc tgctgaatgg tagagattca 5940
cccattccct cagaagatac tctgccttct ccctcaggcc tggagggtca tcatactctg 6000
aggcttgaga gatcccagaa tgcatcataa agtttgggcc tccatgagca cgatcaatca 6060
ttgcttcata gttggatcgc actacttcca tcagctgggg caatccttct ggagcattgc 6120
ctctggaatg agcattaatc ctcatgagcc tggagcattg cctctggaat gagcattaat 6180
cctcatgagg gtttcaatgg tgtggaacag atctgcctca gtaacatgag caacactcct 6240
ttcatccacc agcaggattt ttactaactg catagcaaat gccacagcca tgtagtttaa 6300
gccattctcc attgactgcg ctaggtgaag atcatactgc tgcatattaa ccaaatcctg 6360
cgctaggtga agatcatact gctgcatatt aaccaaatga ttgcgaatta gcagctccac 6420
agcctccaca ttatatttat attcatctcg acattcaatt aggcaccttg tgatctgttt 6480
gttgcaccat ggagacccat atgcccggca tccttgtgat ctgtttgttg caccatggag 6540
acccatatgc ccggccatcc tgcagagctt ttaggaccaa gaggtggcat tccctgtagc 6600
gcagcagaag gtcagcatca gcaccacttg tggcatctag taagccctct acagcctttt 6660
ggagcaatcc aagagcagct atggcatccc gagagtttcg agctttggag caatccaaga 6720
gcagctatgg catcccgaga gtttcgagat aaaactacaa cctccaagag acttcgaaga 6780
gcctgagctt gagggttcat ggccaaagtt ggtgggatgg catgtagatg ttgctccagt 6840

tctgtaatac acttatcata aatctgagct acatcatctg ttgcccaagc ttgcttcatg 6900
ggctgggcta aaaatcccgt gggctgactt aagtcatttc cctgtatttg aaactacaac 6960
tcaaaacatt aatcaaaaac atttaaattg gctgggctaa aaatcccgtg ggctgactta 7020
agtcatttgt aggtaagaag ccaggaacat tgcgtgcaaa ctcttctaaa cagccaactg 7080
ctttgggtcc acaccaccaa ctttcagcct gatttgctct ggcatccgtt cagcttgata 7140
tgttaaatca gcctgatttg ctctggcatc cgttcagctt gatatgttaa aacaacagga 7200
tcacagtatc tgcgtccttc ttgcctagca tgttttctca gctcaaattc agttgctaat 7260
ctcttgtcca tctcagggcc tgctttttct actgcagtgc taatctcttg tccatctcag 7320
ggcctgcttt ttctactgca gtcttctgaa taaaacagca agccaactca caattgtcct 7380
gagctaattg agcagctgcc tgatccatca tttctctttg ttgtggggaa gcagtacgaa 7440
gggctgaggc aaaactgttt tttaagttgg tagatatgct ccataccaag gaaatagcca 7500
acttacacga agggctgagg caaaactgtt ttttaagttg gtagatatgc tcatgagcaa 7560
aggttccctg catgtaatca tagccattcc agctgtcaag ttacgcatca tgtgatgagc 7620
tgctattcgc attcgagatt cctccgaatc cagggcaaaa tccttcctga ctatttgctc 7680
acaagtagtc atggcaatct taattgatcg atccaccaca ggatggacca gctcctggac 7740
agcccgttca attgcctgac gcacacactg cttcaactgt ggatgggcct gaaacaaggg 7800
aattgttgga ttagagtaat gtgtggtgcc aagcccgcaa gggaatagtt ggattaaatg 7860
tgtggtgcca agcccgcaag ggaatagaca ttgatgtcgt ggtagctgta ctgtggctgt 7920
ggtggaaccg tggctgtaca agtggtgttg gtagctggtg tagtagaagt tgttgtggtt 7980
gtgatgggag ggagttcttc tggctgcttg acatcttttg tgatgggagg gagttcttct 8040
ggctgcttga catctttctt tggagcagag agttgctcat ctaaattctt caggcgatct 8100
ttatccttta ggaggtttcc aggttttagc tcattgatgt ctaatgcaag gttcttgcag 8160
agaacctcga tttcaaactt caagtttaac tttaagtcat gctcctgatg tagctcagct 8220
aatacattca taattgctta gtcatctcct gatgtagctc agctaataca ttcataattg 8280
ccattgtcca agggtttggt ggcctaaaaa ccacactacg aatgctagat tctaagactt 8340
tggcaacaaa gggcctacga atgctagatt ctaagacttt ggcaacaaag ggcactacat 8400
agagcaattc ttgttgtcct ttaacataag cctctagcag caatgatttc acatccaagt 8460
cagtgtgtaa gatgggtttg tttttagcta atgtgatcat tcctagaaga tgggtttgtt 8520
tttagctaat gtgatcattc ctagccaatg tcccaagttc ttcagcaaag aacgatctga 8580
gaaattggct gcagctttat cagaggtcag gagcacttta atgtttctgt aggtctcatt 8640
cagaaccatc ttgttaaatt caggtgtttc tgtaggtctc attcagaacc atcttgttaa 8700
attcaggatt cttcagcgtg tcaaggaagt ttgaatacag gctatgaaag tttggctcaa 8760

tactgactct cttcataacc agatactgtg aaacccaagg cataaattct tctttcaccg 8820

tttcctttag ctcttcaacc ttttgtgtca tatttgactg tgagagatta ttgaaaataa 8880

aagcttgtgt catatttgac tgtgagagat tattgaaaat aaaagcaatt ttctcctgga 8940

tattttctgg gggctccaca attctctcag tttgatctgt ggccacaagc aacgtatcta 9000

tatttgtagt attaatagaa ggtggcacat ctttcttaaa gctgactcca gttggcctgg 9060

tgaccgtaac tggcacatct ttcttaaagc tgactccagt tggcctggtg accgtaaccg 9120

ttttagcaac agtggtagtt gttgaggtgg ttaccatagt gctaacttga ccagcaagag 9180

gagcttttgc tggaacctgg gcctgagcct gggcctgagc cagtgcaata cttccagggg 9240

ttgtgataga gccttgcatt ttcacaggag gatctctaga ctgctgtcca tactcaatat 9300

actcctgtaa atgatgtgga aattgcataa agtgactgat agaagctctg taaatgatgt 9360

ggaaattgca taaagtgact gatagaagcc aaatgtgaca atactgggga tagtccttca 9420

atctgttttt aaatctatct agtgcagcaa tcccgaaata atacattttt tttttaaatc 9480

tatctagtgc agcaatcccg aaataataca ttttggatcc aaaaggcttg cgtaaggctt 9540

caagaacata tcgtagagcc agacctagtg ccatgtaagt gaccagtcct ttctcaatta 9600

taccaccaaa taggcaggct gttatatgta actctttatc aggatactgg ggaaaaaaac 9660

gatattcttc aaacaagttc cttagcatac agttaaatac ttctcgttcc ctctttatag 9720

tagagtcttt aaatctctgc agcatttcta atacctcatc aacagacatg gttggatgtg 9780

gtggatgatt atatattcga acagacatgg ttggatgtgg tggatgatta tatattcgct 9840

ggaaatagct gtttgcttca tcatctatct ctttactaaa gtgctggttt gcctctggcc 9900

acacctgaga caagtccggt atactgtata ggtcagtagt cttactctgc tggaatgtag 9960

gctgattcag tccagaggtg cccagtttcc tctgtacaaa agggtcgtta ttcactgcag 10020

ggagtccaag agcaccagtt cctataccag tcaggctgcc tgtgccaaga ccacctactg 10080

gaagctgtga tgaaagtcct ccaataccac tgaatgcagt gctggaagct gtgatgaaag 10140

tcctccaata ccactgaatg cagtggtctg attgggggtt gaaaggggtg gaaatgcttt 10200

tgctggtgac tgaggggtac tgaatgcaga acccaaattt ggaggaaaac cctgcatact 10260

ctgggtgtga ggggcagctg aaccacctat actaagagat gtcattccag caagagggtc 10320

aatctgaaca ggagaaatgg catctaagct gctagcacta ggaggacgtc aacaggagaa 10380

atggcatcta agctgctagc actaggagga cgtccttttg gcataactcc aggtggtggt 10440

tgtctggcct tattcataac attactgcaa ttggctacca tggtgaggat agtttctgat 10500

agctcctgag aaacactccc tgcacaagct tgcagacagg ccaacattgt cgccaaagtt 10560

tcaagcttgc agacaggcca acattgtcgc caaagtttct ggaggaagtt gagcactttt 10620

gggctggtct ttttctgggg caagtccgcc caaaatagaa ggacaccgtc tctttaaaaa 10680

acatacacgc ctggataaaa ggctccccat gctctcgaat tttatctgtg agccacttat 10740

caagtttgag ccatctctcg aattttatct gtgagccact tatcaagttt gaggtattca 10800

cgacgtgaag caagtgcagc aaggtcaata acaaaggcaa atggagtacc atttagcagc 10860

attgacaagg ccttcaagtc ctgggccaca tcaagtattc gagacaattt ggcctccaag 10920

tcctgggcca catcaagtat tcgagacaat ttggcctgat catactgctc ccctctcatg 10980

taccattctg ccattgcatg catgataagt tggcgaattg agggagactg tccctgccca 11040

tgccatgcat agtgcaaaat aatagctgag ttaggagtgc ccatgccatg catagtgcaa 11100

aataatagct gagttaggat ggtttccaag gaaaattggc atcagagtgg agataagttc 11160

atggcgcaag gtatgccaag aggtgttaat ttgtagtaag gccaatacca gcatgtctgg 11220

acagtgtttg atagggaagc tgaagagctg tttgacttgc tcatactgcc caacctctgc 11280

aagcctcagc agagattcaa tcaaatccaa gctcttccat gtggcaattt ctcgattgtc 11340

atcctctggt ggtgctttca gaggcaattt ctcgattgtc atcctctggt ggtgctttca 11400

gaatatcagt ggcaacagta tgacagggat agtcagcaaa acagaagatc tctggattta 11460

taagggaatg ttgaatgaag gagagctggc cttcagcatg tttccaaggt ctatatatga 11520

ggtctactgg gctggccttc agcatgtttc caaggtctat atatgaggtc tactgggaac 11580

acttccatac ccaaacccct ctgaatgcca taaaccacat tatgaagtcc tttactgtca 11640

cgaatttgaa atccaggatg gtccagttca taagttactt ccttgaaatt caaacttgga 11700

ttcagttctt taagaacgtc aatcaagact tctacattcc atgtgtggtt ctttaagaac 11760

gtcaatcaag acttctacat tccatgtgtg tgcctgtgct ccatcacttt tatctttccc 11820

atcactccag atcccactgc ccggagcaga aatactctgt aatggaatgc catctgttaa 11880

cctgaatgag ttcgagccat aatggaatgc catctgttaa cctgaatgag ttcgagccat 11940

cattcccaaa acccttgcaa cctgggcagc tgtgacctcc cgaacaccaa actgcacgat 12000

tatattgcga cattcttcaa tacttgcaca aaagccatag cctacttctt gcatgaaatc 12060

agccaaagaa agccatagcc tacttcttgc atgaaatcag ccaaagagct ctccatcatg 12120

gttttagcta cccctccgga atcaggcagg atcctgtcca ttagaatgtc ccgtttttca 12180

gggtataaaa gtggtgcgag caccacggga cagcgttctt ggggaaaatc tctgcgcagc 12240

gtcttaagaa aagcgtctat ctgttcttgt ccgccagcgt cttaagaaaa gcgtctatct 12300

gttcttgtcc aactccaaag gctcccttct gcccaaagag gagatgggag aggaggaggt 12360

gtaggacctc tattgctata tcttggaagc caccttcttg atttccactg acgtctgcgt 12420

caatgtaaga acgcagaaga tctggaagct tctgtttgat aaactgggca gcgaaacctc 12480

taagatctga gctggaagaa ttcaacaggg caaggccaag aacctaagat ctgagctgga 12540

agaattcaac agggcaaggc caaaaattac ctcttgtact ttgcttaatt tgagcacttt 12600

actcggaaac ttgtactttg cttaatttga gcactttact cagctgggca aataagtggg 12660

gtgcaggctt taaactcttc tgatagtgca atggattatc aatggcatag gacagcgtcg 12720

agttctgata gtgcaatgga ttatcaatgg cataggacag cgtcgagata aaatttggct 12780

ttgtaatcag caacgcacac tcctgaatca gaaactgagt ctgatggaaa tctttgccac 12840

tgcttttacc atcgccactg aaatccacgg aaatctttgc actgctttta ccatcgccac 12900

tgaaatccac atgcgaaaat aggcagcgta ataaatgcct gtctgcctca ggaccgtgcc 12960

gattcacaat atgctgtatt tcctgctggc tggctcggaa tttttcttgg ttaaatgctc 13020

ggtaattttt cttggttaaa ttgtccacca ggtagctgat ttgagacaag gccagcgaga 13080

gcgagtcaag attcattgct ggttggggcg gaagcaggcg gccgagcccg gcgcaaaatc 13140

accattattc ccctttagtc acctcagagg caggttaatg ctttctttgt aattaggcta 13200

tatctggtat ctgtataata tcttcagttc ttctttacca ggggtcttac tctgttctga 13260

aacatggcac ctatttcctg gctacaactt ctaagatcct tagataatcc cagcactcgg 13320

gaggcgaggg atacggggta gtgagccgag atcacaccac tgcttcaagc ctgggcaaca 13380

gagcaagact c 13391

<210> 31
<211> 11424
<212> DNA
<213> Homo sapiens

<400> 31
ggaggtgctg actgaagagg agctgaccac cacactaaag gtactgtgca tgcctgctga 60

ggagttcaga gtgtgcatgc ctgctgagga gttcagagag cttaaagacc aggatggagg 120

gggagatgat aaaagggaag agggcagcct gacgatcaca aatattccca agctcaaagc 180

atcgtggaga cagctgcttc aaaacagtgt tctactgact ttgcagacct atgccacaga 240

cttaaaaact gaccaaggtt tactcagtaa taaggaagtc tatgcgaaac tcagctggag 300

ggaacagcaa gccttacagg ttcgctatgg tcagaagatg atcttacatc agttgttgga 360

ctgacaagtt agcagtttcc ctgttccctg aaggaacagc aataagaact ttattctaag 420

ctaatactca ttgatgtttg aaaaagagga aaatttggat ctttcttttg cttactaaac 480

accaagagga aaagtagcaa agttggtgtg ctaggattaa ctcaggtaag ggtgatgtgt 540

tttaggattg agaacagcag acttgggaac actgctaatt gttacttaaa gcatgttaca 600

gtgttttgtt ctcagtttta accaaagcca gtggacatac ggtagtaata agtaagtctt 660

gttgtgtttc agcatttaat aatagacttt ggaggtagac ccctggttta aatctaagtc 720

tagtttgagg aagtcactta acctttattg aaaagactct ggatttaata agctgtgtaa 780

ctggactcga tagttaccca aagttcagtc tagatggcac aaaccacctc tcagggaata 840

aaccctaaga catcactcaa ggggactcaa ttatttaatt ttgaactgtt ttgtcctctc 900

tggcataaaa cttgacagtc atgaaaggaa ggcaaatttt aagtgggtta agtttttaaa 960

tacgtatcta ctcattttct ttaaaaaaag gtttaaactc agcttaacca gataaacctg 1020

ctccagcatt tgtccagtgt tgaaataaat aggaagaaaa atacaatttc cttaaacatg 1080

ccagcttagt agtaggaatg acctatcaat caatcaaaaa gatttgtggt ccctggcccc 1140

aaggtctgca gggctggatt gtccactttt cctcctcacc accagcactg gctgctgcta 1200

ctgagaaagg cacagtggac tcatgttttc atgactttaa tggttagcta cagtatgcat 1260

acacatacaa gaagtagggg agctgaagcc caggaaaagg ctacaaaata caacacaggg 1320

accaatacat ttgcaaaaca ttcaaaatcc ttataaaagg ggctgtattg gtcctttcaa 1380

ctttgtcatt attcagctta gcctgtatgg ccattcattc agtggggtaa tgggggagaa 1440

caattaatta atgagatttg gttccctttc ctatattccc actgttttta agtttcagga 1500

gaggattggg gggtgagggt aggggggttg cggttccttt taaagagctg acccccatct 1560

caggcggctg aggtgtataa tccccaaaag aaataagatg gtacaaatgc agtcagcttt 1620

ggaatacgtt tgggtagaga caaaatggaa actcatggga ttccaatagt gagcccttcc 1680

catgttcctg gacatatcca attctagcac atccacattt ttaataactt agtgattttc 1740

aaggtcccct gtccacatta aaaaaataag ttacataata ttcaaactgg ctttttgcta 1800

ttctttggga caccaggaat gtcagaagac atggagctat gccctcagac aagtgcatgg 1860

catcagctgc ctcttcatta caaggtacca gtttatgtac ttgccttgga cacagcttgc 1920

acaaagccaa gaagttccag acaaaggttt tctctttcat gtatttacac aagttcaaaa 1980

tgatattcac agcatcttct aaattttggc caagagtcaa aaaaatgcat ttaaactttg 2040

gaacgtgccc acataagaca ggaggctgat cccaacagta gttggggcag atacccacaa 2100

accaaagggc tgggaaagtc aggaagagct gaaaggattc ttcagtcagt ttatgaactc 2160

ggtgcagtga gacctctaga ctgacacgta caacagagat gcagtttcgt ctaactggca 2220

cctgtccctt ccattacttg ctgggcctgc ttctgtccca tgcagcactg tgcgaccgac 2280

tggaataact tttcgatttc tggggcacag tgtacaaatt catggttcca gaacttttcg 2340

atttctgggg cacagtgtac aaattcatgg ttccagaact taaacgctgg gtttttaatc 2400

agctcaatga aggtaataag aagaccccaa ggatgtggcc tatttacaat caaccgttcc 2460

aagagaactc ttgtgatctg ttcttggatg gcttccgtat tggcctctgc aaaaagcgct 2520

taccttgtga tctgttcttg gatggcttcc gtattggcct ctgcaaaaag gtacagcatg 2580

gtgcaactga atagtgagtg tggctatttg ggtaccggag ctgatttgca attgcattca 2640

aaaagagata gcgaccctca gtgtccaagt ccacagccaa attctggaga atccctcagt 2700

gtccaagtcc acagccaaat tctggaagat atccatgtgt gctgagtgag tgatggtgct 2760

cattgaaggt gtgctgccct tgttgtggat gtgcgcaatg gcctgagtcc cgacatagag 2820

caccagtgca ttgatgagct ggaggttgta gcgattccca ggttcattgg atacctgtag 2880

gttgctgcgc agatcagaca ggaaagtgac tggtgatcga ctgtaggttg ctgcgcagat 2940

cagacaggaa agtgactggt gatcgagttt taagatagga atccaaatcc tttttgaact 3000

gaggtggcat tactccagtg aaattggtga gaatccgggg agcaatgtta atttcactca 3060

acatgtccac ctttagatta ggagtgaatg ggtcggggag cctcatgttt cttgctttag 3120

attaggagtg aatgggtcgg ggagcctcat gtttcttgga aaggcactca ggatcaaatt 3180

tcttaactgg atacaattag gtgggatcac atcacagaac ccataatggt aatcacaaag 3240

gaactctggg aaatcatgca aaagaaccag cagcactctt aaagtgccct tgtagaggat 3300

ttgcataggt ttggtgagtt ccacatttct aactagagga tttgcatagg tttggtgagt 3360

tccacatttc taaggaaagg cgctaaatat ttgaataaat caatcagtag ctgtgcatac 3420

ataggccacc ccttctgctg tggcgtatgt gccagcattc ttgcaaaaat tctctgctgt 3480

gggtatgtgc cagcattctt gcaataaata tccgatggga aatcagttca agccaggcat 3540

atacaaagcc aggagctttg gtaggcctca agatgtggaa tgtattgcag aaagctgtaa 3600

gtgtctggaa attaatggtt tccaacacat gctcagaaag ctgtaagtgt ctggaaatta 3660

atggtttcca acacatgctc aggtgcattg agttccaaga gaagcatgat aaaaattcga 3720

tggtagggaa gttgctgaaa ttcactctga cgaacatcat gatcctgaag gagaactccc 3780

actactatac caaggacctt gttcagcaga ttaatctttg tgacagtgtt ggtggcctcc 3840

cccttgttca gcagattaat ctttgtgaca gtgttggtgg cctcccctga gtgtttcacg 3900

agcagtgcaa tgagtcgaac aaaggcatcc aggttgtgat agcacttggc tcggatcatg 3960

gtgggattgg cagcaggatt gtgctgctgc tcagcctgag cacggtaact gatttcaaca 4020

cacatttcag tacacagacg aaagaacctt gttatgagat catcggtctt cagtattcct 4080

tgctggtgca tcttccacaa agagaaagct ttggactgtc gcggagctct gtccaacaaa 4140

tgcagagaaa gctttggtac tgtcgcggcc agctgctgct gaatggtaga gattcaccca 4200

ttccctcaga agatactctg ccttctccct caggcctgga gggtcatcat actctgaggc 4260

ttgagagatc ccagaatgca tcataaagtt tgggcctcca tgagcacgat caatcattgc 4320

ttcatagttg gatcgcacta cttccatcag ctggggcaat ccttctggag cattgcctct 4380

ggaatgagca ttaatcctca tcttctggag cattgcctct ggaatgagca ttaatcctca 4440

tgagggtttc aatggtgtgg aacagatctg cctcagtaac atgagcaaca ctcctttcat 4500

ccaccagcag gatttttact aactgcatag caaatgccac agccatgtag tttaagccat 4560

tctccattga ctgcgctagg tgaagatcat actgctgcat attaaccaaa tgattgcgct 4620

gcgctaggtg aagatcatac tgctgcatat taaccaaatg attgcgaatt agcagctcca 4680

cagcctccac attatattta tattcatctc gacattcaat taggcacctt gtgatctgtt 4740

tgttgcacca tggagaccca tatgcccggc ttgtgatctg tttgttgcac catggagacc 4800

catatgcccg gccatcctgc agagctttta ggaccaagag gtggcattcc ctgtagcgca 4860

gcagaaggtc agcatcagca ccacttgtgg catctagtaa gccctctaca gccttttgga 4920

gcaatccaag agcagctatg gcatcccgag agtttcgagt ttggcaatcc aagagcagct 4980

atggcatccc gagagtttcg agataaaact acaacctcca agagacttcg aagagcctga 5040

gcttgagggt tcatggccaa agttggtggg atggcatgta gatgttgctc cagttctgta 5100

atacacttat cataaatctg agctacatca tctgttgccc aagcttgctt catgggctgg 5160

gctaaaaatc ccgtgggctg acttaagttt ttatgggctg ggctaaaaat cccgtgggct 5220

gacttaagtc atttgtaggt aagaagccag gaacattgcg tgcaaactct tcgtaaacag 5280

ccaactgctt tgggtccaca ccaccaactt tcagcctgat ttgctctggc atccgttcag 5340

cttgatatgt taatcagcct gatttgctct ggcatccgtt cagcttgata tgttaaaaca 5400

acaggatcac agtatctgcg tccttcttgc ctagcatgtt ttctcagctc aaattcagtt 5460

gctaatctct tgtccatctc agggcctgct ttttcttcta atctcttgtc catctcaggg 5520

cctgcttttt ctactgcagt cttctgaata aaacagcaag ccaactcaca attgtcctga 5580

gctaattgag cagctgcctg atccatcatt tctctttgtt gtggggaagc agtacgaagg 5640

gctgaggcaa aactgttttt taagttggta gatcagaaag ccaacttaca cgaagggctg 5700

aggcaaaact gttttttaag ttggtagata tgctcatgag caaaggttcc ctgcatgtaa 5760

tcatagccat tccagctgtc aagttacgca tcatgtgatg agctgctatt cgcattcgag 5820

attcctccga atccagggca aaatccttcc tgactatttg ctcacaagta gtcatggcaa 5880

tcttaattga tcgatccacc acaggatgga ccagctcctg gacagcccgt tcaattgcct 5940

gacgcacaca ctgcttcaac tgtggatggg cctgaaacaa gggaattgtt ggattagagt 6000

aatgtgtggt gccaagcccg caaggggttg gattagagta atgtgtggtg ccaagcccgc 6060

aagggaatag acattgatgt cgtggtagct gtactgtggc tgtggtggaa ccgtggctgt 6120

acaagtggtg ttggtagctg gtgtagtaga agttgttgtg gttgtgatgg gagggagttc 6180

ttctggctgc ttgacatctt tcgttgtgat gggagggagt tcttctggct gcttgacatc 6240

tttctttgga gcagagagtt gctcatctaa attcttcagg cgatctttat cctttaggag 6300

gtttccaggt tttagctcat tgatgtctaa tgcaaggttc ttgcagagaa cctcgatttc 6360

aaacttcaag tttaacttta agtcatgctc ctgatgtagc tcagctaata cattcataat 6420

tgcttagtca tgctcctgat gtagctcagc taatacattc ataattgcca ttgtccaagg 6480

gtttggtggc ctaaaaacca cactacgaat gctagattct aagactttgg caacaaaggg 6540

cctacgaatg ctagattcta agactttggc aacaaagggc actacataga gcaattcttg 6600

ttgtccttta acataagcct ctagcagcaa tgatttcaca tccaagtcag tgtgtaagat 6660

gggtttgttt ttagctaatg tgatcgtaag atgggtttgt ttttagctaa tgtgatcatt 6720

cctagccaat gtcccaagtt cttcagcaaa gaacgatctg agaaattggc tgcagcttta 6780

tcagaggtca ggagcacttt aatgtttctg taggtctcat tcagaaccat cttgttaaat 6840

tcaggtctgt aggtctcatt cagaaccatc ttgttaaatt caggattctt cagcgtgtca 6900

aggaagtttg aatacaggct atgaaagttt ggctcaatac tgactctctt cataaccaga 6960

tactgtgaaa cccaaggcat aaattcttct ttcaccgttt cctttagctc ttcaaccttt 7020

tgtgtcatat ttgactgtga gagattattg aaaataaaag ttttgtgtca tatttgactg 7080

tgagagatta ttgaaaataa aagcaatttt ctcctggata ttttctgggg gctccacaat 7140

tctctcagtt tgatctgtgg ccacaagcaa cgtatctata tttgtagtat taatagaagg 7200

tggcacatct ttcttaaagc tgactccagt tggcctggtg gcacatcttt cttaaagctg 7260

actccagttg gcctggtgac cgtaaccgtt ttagcaacag tggtagttgt tgaggtggtt 7320

accatagtgc taacttgacc agcaagagga gcttttgctg gaacctgggc ctgagcctgg 7380

gcctgagcca gtgcaatact tccaggggtt gtgatagagc cttgcatttt cacaggagga 7440

tctctagact gctgtccata ctcaatatac tcctgtaaat gatgtggaaa ttgcataaag 7500

tgactgatag aacctgtaaa tgatgtggaa attgcataaa gtgactgata gaagccaaat 7560

gctgacaata ctggggatag tccttcaatc tgtttttaaa tctatctagt gcagcaatcc 7620

cgaaataata cactttttaa atctatctag tgcagcaatc ccgaaataat acatttggga 7680

tccaaaaggc ttgcgtaagg cttcaagaac atatcgtaga gccagaccta gtgccatgta 7740

agtgaccagt cctttctcaa ttataccacc aaataggcag gctgttatat gtaactcttt 7800

atcaggatac tggggaaaaa aacgatattc ttcaaacaag ttccttagca tacagttaaa 7860

tacttctcgt tccctcttta tagtagagtc tttaaatctc tgcagcattt ctaatacctc 7920

atcaacagac atggttggat gtggtggatg attatatatt aacatggttg gatgtggtgg 7980

atgattatat attcgctgga aatagctgtt tgcttcatca tctatctctt tactaaagtg 8040

ctggtttgcc tctggccaca cctgagacaa gtccggtata ctgtatagca gttcttactc 8100

tgctggaatg taggctgatt cagtccagag gtgcccagtt tcctctgtac aaaagggtcg 8160

ttattcactg cagggagtcc aagagcacca gttcctatac cagtcaggct gcctgtgcca 8220

agaccaccta ctggaagctg tgatgaaagt cctccaatac cactgaatgc agtgtggaag 8280

ctgtgatgaa agtcctccaa taccactgaa tgcagtggtc tgattggggg ttgaaagggg 8340

tggaaatgct tttgctggtg actgaggggt actgaatgca gaacccaaat ttggaggaaa 8400

accctgcata ctctgggtgt gaggggcagc tgaaccacct atactaagag atgtcattcc 8460

agcaagaggg tcaatctgaa caggagaaat ggcatctaag ctgctagcac taggaggacg 8520

tccggagaaa tggcatctaa gctgctagca ctaggaggac gtccttttgg cataactcca 8580

ggtggtggtt gtctggcctt attcataaca ttactgcaat tggctaccat ggtgaggata 8640

gtttctgata gctcctgaga aacactccct gcacaagctt gcagacaggc caacattgtc 8700

gccaaagttt ccaggccaac attgtcgcca aagtttctgg aggaagttga gcacttttgg 8760

gctggtcttt ttctgggcca agtccgccca aaatagaagg acaccgtctc tttaaaaaag 8820

catacacgcc tggataaaag gctccccatg ctctcgaatt ttatctgtga gccacttatc 8880

aagtttgagg tcactctcga attttatctg tgagccactt atcaagtttg aggtattcac 8940

gacgtgaagc aagtgcagca aggtcaataa caaaggcaaa tggagtacca tttagcagca 9000

ttgacaaggc cttcaagtcc tgggccacat caagtattcg agacaatttg gcttcaagtc 9060

ctgggccaca tcaagtattc gagacaattt ggcctgatca tactgctccc ctctcatgta 9120

ccattctgcc attgcatgca tgataagttg gcgaattgag ggagactgtc cctgcccatg 9180

ccatgcatag tgcaaaataa tagctgagtt aggatggttc ccatgccatg catagtgcaa 9240

aataatagct gagttaggat ggtttccaag gaaaattggc atcagagtgg agataagttc 9300

atggcgcaag gtatgccaag aggtgttaat ttgtagtaag gccaatacca gcatgtctgg 9360

acagtgtttg atagggaagc tgaagagctg tttgacttgc tcatactgcc caacctctgc 9420

aagcctcagc agagattcaa tcaaatccaa gctcttccat gtggcaattt ctcgattgtc 9480

atcctctggt ggtgctttca gggcaatttc tcgattgtca tcctctggtg gtgctttcag 9540

aatatcagtg gcaacagtat gacaggatag gtcagcaaaa cagaagatct ctggatttat 9600

aagggaatgt tgaatgaagg agagctggcc ttcagcatgt ttccaaggtc tatatatgag 9660

gtctacgctg ccttcagcat gtttccaagg tctatatatg aggtctactg ggaacacttc 9720

catacccaaa cccctctgaa tgccataaac cacattatga agtcctttac tgtcacgaat 9780

ttgaaatcca ggatggtcca gttcataagt tacttccttg aaattcaaac ttggattcag 9840

ttctttaaga acgtcaatca agacttctac attccatgtg tggttcttta agaacgtcaa 9900

tcaagacttc tacattccat gtgtgtgcct gtgctccatc acttttatct ttcccatcac 9960

tccagatccc actgcccgga gcagaaatac tctgtaatgg aatgccatct gttaacctga 10020

atgagttcga gtgtaatgga atgccatctg ttaacctgaa tgagttcgag ccatcattcc 10080

caaaaccctt gcaacctggg cagctgtgac ctcccgaaca ccaaactgca cgattatatt 10140

gcgacattct tcaatacttg cacaaaagcc atagcctact tcttgcatga aatcaaaacc 10200

atagcctact tcttgcatga aatcagccaa agagctctcc atcatggttt tagctacccc 10260

tccggaatca ggcaggatcc tgtccattag aatgtcccgt ttttcagggt ataaaagtgg 10320

tgcgagcacc acgggacagc gttcttgggg aaaatctctg cgcagcgtct taagaaaagc 10380

gtctatctgt tcttgtccgc gcacttaaga aaagcgtcta tctgttcttg tccaactcca 10440

aaggctccct tctgcccaaa gaggagatgg gagaggagga ggtgtaggac ctctattgct 10500

atatcttgga agccaccttc ttgatttcca ctgacgtctg cgtcaatgta agaacgcaga 10560

agatctggaa gcttctgttt gataaactgg gcagcgaaac ctctaagatc tgagctggaa 10620

gaattcaaca gggcaaggcc aaaacctaag atctgagctg gaagaattca acagggcaag 10680

gccaaaaatt acctcttgta ctttgcttaa tttgagcact ttactcagct gggaatcttg 10740

tactttgctt aatttgagca ctttactcag ctgggcaaat aagtggggtg caggctttaa 10800

actcttctga tagtgcaatg gattatcaat ggcataggac agcgtcggtc tgatagtgca 10860

atggattatc aatggcatag gacagcgtcg agataaaatt tggctttgta atcagcaacg 10920

cacactcctg aatcagaaac tgagtctgat ggaaatcttt gccactgctt ttaccatcgc 10980

cactgaagaa attgccctgc ttttaccatc gccactgaaa tccacatgcg aaaataggca 11040

gcgtaataaa tgcctgtctg cctcaggacc gtgccgattc acaatatgct gtatttcctg 11100

ctggctggct cggtaatttt tcttggtttt cctgctggct ggctcggtaa tttttcttgg 11160

ttaaattgtc caccaggtag ctgatttgag acaaggccag cgagagcgag tcaagattca 11220

ttgctggttg gggcggaagc aggcggccga gcccggcgca aaatcaccat tattcccctt 11280

tagtcacctc agaggcaggt taatgctttc tttgtaatta ggctatatct ggtatctgta 11340

taatatcttc agttcttctt taccaggggt cttactctgt tctgaaacat ggcacctggc 11400

cagctgacac aggaaactgg gtcc 11424

<210> 32
<211> 9157
<212> DNA
<213> Homo sapiens

<400> 32
ggaggtgctg actgaagagg agctgaccac cacactaaag gtactgtgca tgcctgctga 60

ggagttcgtg catgcctgct gaggagttca gagagcttaa agaccaggat ggagggggag 120

atgataaaag ggaagagggc agcctgacga tcacaaatat tcccaagctc aaagcatcgt 180

ggagacagct gcttcaaaac agtgttctac tgactttgca gacctatgcc acagacttaa 240

aaactgacca aggtttactc agtaataagg aagtctatgc gaaactcagc tggagggaac 300

agcaagcctt acaggttcgc tatggtcaga agatgatctt acatcagttg ttggactgac 360

aagttagcag tttccctgtt ccctgaagga acagcaataa gaactttatt ctaagctaat 420

actcattgat gtttgaaaaa gaggaaaatt tggatctttc ttttgcttac taaacaccaa 480

gaggaaaagt agcaaagttg gtgtgctagg attaactcag gtaagggtga tgtgttttag 540

gattgagaac agcagacttg ggaatcactg ctaattgtta cttaaagcat gttacagtgt 600

tttgttctca gttttaacca aagccagtgg acatacggta gtaataagta agtcttgttg 660

tgtttcagca tttaataata gactttggag gtagacccct ggtttaaatc taagtctagt 720

ttgaggaagt cacttaacct ttattgaaaa gactctggat ttaataagct gtgtaactgg 780

actcgatagt tacccaaagt tcagtctaga tggcacaaac cacctctcag ggaataaacc 840

ctaagacatc actcaaggag gacttcaatt atttaatttt gaactgtttt gtcctctctg 900

gccataaaac ttgacagtca tgaaaggtaa ggcaaatttt aagtgggtaa gtttttaaat 960

acgtattttc tttaaaaaaa aaaaaaaagg ggaaaaggat attaaaacgg gtattttaaa 1020

ctcagcttaa ccagataaac ctgctccagc atttgtccag tgttgaaata aataggaaga 1080

aaaatacaat ttccttaaac atgccagctt agtagtagga atgacctatc aatcaatcaa 1140

aatcaagaag ttagttgtgg ttccccatgg ccccaaggtc tcagggctgg attgtccact 1200

tttcctcctc accaccagca ctggctgctg ctactgagaa aggcacagtg gactcatgtt 1260

ttatgacttt aatggttagc tacagtatgc atacacatac aagaagtagg ggagctgaag 1320

cccaggaaaa ggctacaaaa tacaacacag ggaccaatac atttgcaaaa cattcaaaat 1380

ccttataaaa ggggctgtat tggtcctttc aactttgtca ttattcagct tagcctgtat 1440

ggccattcat tcagtggggt aatgggggag aacaattaat taatgagatt tggttccctt 1500

tcctatattc ccactgtttt taagtttcag gagaggattg gggggtgagg gtaggggggt 1560

tgcggttcct ttataaagag ctgaccccca tctcaggcgg ctgaggtgta taatccccaa 1620

aagaaataag atggtacaaa tgcagtcagc tttggaatac gtttgggtag agacaaaatg 1680

gaaactcatg ggattccaat agtgagccct tcccatgttc ctggacatat ccaattctag 1740

cacatccaca tttttaataa cttagtgatt ttcaaggtcc cctgtccaca ttaaaaaaat 1800

aagttacata atattcaaac tggctttttg ctattctttg ggacaccagg aatgtcagaa 1860

gacatggagc tatgccctca gacaagtgca tggcatcagc tgcctcttca ttacaaggta 1920

ccagtttatg tacttgcctt ggacacagct tgcacaaagc caagaagttc cagacaaagg 1980

ttttctcttt catgtattta cacaagttca aaatgatatt cacagcatct tctaaatttt 2040

ggccaagagt caaaaaaatg catttaaact ttggaacgtg cccacataag acaggaggct 2100

gatcccaaca gtagttgggg cagataccca caaaccaaag ggctgggaaa gtcaggaaga 2160

gctgaaagga ttcttcagtc agtttatgaa ctcggtgcag tgagacctct agactgacac 2220

gtacaacaga gatgcagttt cgtctaactg gcacctgtcc cttccattac ttgctgggcc 2280

tgcttctgtc ccatgcagca ctgtgcgacc gactggaata acttttcgat ttctggggca 2340

cagtgtacaa attcatggtt ccagaacttt cgatttctgg ggcacagtgt acaaattcat 2400

ggttccagaa cttaaacgct gggtttttaa tcagctcaat gaaggtaata agaagacccc 2460

aaggatgtgg cctatttaca atcaaccgtt ccaagagaac tcttgtgatc tgttcttgga 2520

tggcttccgt attggcctct gcaaaaagct tgtgatctgt tcttggatgg cttccgtatt 2580

ggcctctgca aaaaggtaca gcatggtgca actgaagtag tgagtgtggc tatttgggta 2640

ccggagctga tttgcaattg cattcaaaaa gagatagcga ccctcagtgt ccaagtccac 2700

agccaaattc tggaagatat ccatgttgcc tcagtgtcca agtccacagc caaattctgg 2760

aagatatcca tgtgtgctga gtgagtgatg gtgctcattg aaggtgtgct gcccttgttg 2820

tggatgtgcg caatggcctg agtcccgaca tagagcacca gtgcattgat gagctggagg 2880

ttgtagcgat tcccaggttc attggatacc tgtaggttgc tgcgcagatc agacaggaaa 2940

gtgactggtg atcgagctgt aggttgctgc gcagatcaga caggaaagtg actggtgatc 3000

gagttttaag ataggaatcc aaatcctttt tgaactgagg tggcattact ccagtgaaat 3060

tggtgagaat ccggggagca atgttaattt cactcaacat gtccaccttt agattaggag 3120

tgaatgggtc ggggagcctc atgtttcttg gttagattag gagtgaatgg gtcggggagc 3180

ctcatgtttc ttggaaaggc actcaggatc aaatttctta actggataca attaggtggg 3240

atcacatcac agaacccata atggtaatca caaaggaact ctgggaaatc atgcaaaaga 3300

accagcagca ctcttaaagt gcccttgtag aggatttgca taggtttggt gagttccaca 3360

tttcagactt gtagaggatt tgcataggtt tggtgagttc cacatttcta aggaaaggcg 3420

ctaaatattt gaataaatca atcagtagct gtgcatacat aggccacccc ttctgctgtg 3480

gcgtatgtgc cagcattctt gcaataaata tccgatcttc tgctgtggcg tatgtgccag 3540

cattcttgca ataaatatcc gatgggaaat cagttcaagc caggcatata caaagccagg 3600

agctttggta ggcctcaaga tgtggaatgt attgcagaaa gctgtaagtg tctggaaatt 3660

aatggtttcc aacacatgct aagtaagtgt ctggaaatta atggtttcca acacatgctc 3720

aggtgcattg agttccaaga gaagcatgat aaaaattcga tggtagggaa gttgctgaaa 3780

ttcactctga cgaacatcat gatcctgaag gagaactccc actactatac caaggacctt 3840

gttcagcaga ttaatctttg tgacagtgtt ggtggcctcc cccgttcagc agattaatct 3900

ttgtgacagt gttggtggcc tcccctgagt gtttcacgag cagtgcaatg agtcgaacaa 3960

aggcatccag gttgtgatag cacttggctc ggatcatggt gggattggca gcaggattgt 4020

gctgctgctc agcctgagca cggtaactga tttcaacaca catttcagta cacagacgaa 4080

agaaccttgt tatgagatca tcggtcttca gtattccttg ctggtgcatc tgtccaacaa 4140

atgcagagaa agctttggta ctgtcgcgga gctcaaaagc tctacctgtc caacaaatgc 4200

agagaaagct ttggtactgt cgcggccagc tgctgctgaa tggtagagat tcacccattc 4260

cctcagaaga tactctgcct tctccctcag gcctggaggg tcatcatact ctgaggcttg 4320

agagatccca gaatgcatca taaagtttgg gcctccatga gcacgatcaa tcattgcttc 4380

atagttggat cgcactactt ccatcagctg gggcaatcct tctggagcat tgcctctgga 4440

atgagcatta atcctcatga gcttctggag cattgcctct ggaatgagca ttaatcctca 4500

tgagggtttc aatggtgtgg aacagatctg cctcagtaac atgagcaaca ctcctttcat 4560

ccaccagcag gatttttact aactgcatag caaatgccac agccatgtag tttaagccat 4620

tctccattga ctgcgctagg tgaagatcat actgctgcat attaaccaaa tgattgctgc 4680

gctaggtgaa gatcatactg ctgcatatta accaaatgat tgcgaattag cagctccaca 4740

gcctccacat tatatttata ttcatctcga cattcaatta ggcaccttgt gatctgtttg 4800

ttgcaccatg gagacccata tgcccggcac ttgtgatctg tttgttgcac catggagacc 4860

catatgcccg gccatcctgc agagctttta ggaccaagag gtggcattcc ctgtagcgca 4920

gcagaaggtc agcatcagca ccacttgtgg catctagtaa gccctctaca gccttttgga 4980

gcaatccaag agcagctatg gcatcccgag tttcgagttg gagcaatcca agagcagcta 5040

tggcatcccg agagtttcga gataaaacta caacctccaa gagacttcga agagcctgag 5100

cttgagggtt catggccaaa gttggtggga tggcatgtag atgttgctcc agttctgtaa 5160

tacacttatc ataaatctga gctacatcat ctgttgccca agcttgcttc atgggctggg 5220

ctaaaaatcc cgtgggctga cttaaggctg ggctaaaaat cccgtgggct gacttaagtc 5280

atttgtaggt aagaagccag gaacattgcg tgcaaactct tctaaacagc caactgcttt 5340

gggtccacac caccaacttt cagcctgatt tgctctggca tccgttcagc ttgatatgtt 5400

aaaaccagcc tgatttgctc tggcatccgt tcagcttgat atgttaaaac aacaggatca 5460

cagtatctgc gtccttcttg cctagcatgt tttctcagct caaattcagt tgctaatctc 5520

ttgtccatct cagggcctgc tttttctact gctaatctct tgtccatctc agggcctgct 5580

ttttctactg cagtcttctg aataaaacag caagccaact cacaattgtc ctgagctaat 5640

tgagcagctg cctgatccat catttctctt tgttgtgggg aagcagtacg aagggctgag 5700

gcaaaactgt tttttaagtt ggtagatatg ttctgagctg tgagaacatg cctaaacagt 5760

aaagcagacg cgcatcccag cactgggagg ccaaggcagg tagatcactt gaggccaggg 5820

tttagccagc ctcaccttct ctactaaaaa gggtagcatc cttgggaggt gaagaaagga 5880

tgcagtgagc caagactgca cacgtactct agcctaagct acagaggaga cctgtctcat 5940

gaaacattaa aaaaactact atctgtcaaa catcgtaaaa aaaaaaaaaa ttcctaaaat 6000

gatggaagac atcaaacaag tgattattaa gaataagtgg taagcccagg ccatatatag 6060

acaaagaaaa aaattattac agacagcagt aatactttcc agaaatagcc aactacaagg 6120

gctgaggcaa aactgttttt taagttggta gatatgctca tgagcaaagg ttccctgcat 6180

gtaatcatag ccattccagc tgtcaagtta cgcatcatgt gatgagctgc tattcgcatt 6240

cgagattcct ccgaatccag ggcaaaatcc ttcctgacta tttgctcaca agtagtcatg 6300

gcaatcttaa ttgatcgatc caccacagga tggaccagct cctggacagc ccgttcaatt 6360

gcctgacgca cacactgctt caactgtgga tgggcctgaa acaagggaat tgttggatta 6420

gagtaatgtg tggtgccaag cccgcaaggg aatagttgga ttagagtaat gtgtggtgcc 6480

aagcccgcaa gggaatagac attgatgtcg tggtagctgt actgtggctg tggtggaacc 6540

gtggctgtac aagtggtgtt ggtagctggt gtagtagaag ttgttgtggt tgtgatggga 6600

gggagttctt ctggctgctt gacatctttc tgttgtgatg ggagggagtt cttctggctg 6660

cttgacatct ttctttggag cagagagttg ctcatctaaa ttcttcaggc gatctttatc 6720

ctttaggagg tttccaggtt ttagctcatt gatgtctaat gcaaggttct tgcagagaac 6780

ctcgatttca aacttcaagt ttaactttaa gtcatgctcc tgatgtagct cagctaatac 6840

attcataatt gttacatgct cctgatgtag ctcagctaat acattcataa ttgccattgt 6900

ccaagggttt ggtggcctaa aaaccacact acgaatgcta gattctaaga ctttggcaac 6960

aaagggcacg aatgctagat tctaagactt tggcaacaaa gggcactaca tagagcaatt 7020

cttgttgtcc tttaacataa gcctctagca gcaatgattt cacatccaag tcagtgtgta 7080

agatgggttt gtttttagct aatgtgatca ttcctacgta agatgggttt gtttttagct 7140

aatgtgatca ttcctagcca atgtcccaag ttcttcagca aagaacgatc tgagaaattg 7200

gctgcagctt tatcagaggt caggagcact ttaatgtttc tgtaggtctc attcagaacc 7260

atcttgttaa attcaggttt tctgtaggtc tcattcagaa ccatcttgtt aaattcagga 7320

ttcttcagcg tgtcaaggaa gtttgaatac aggctatgaa agtttggctc aatactgact 7380

ctcttcataa ccagatactg tgaaacccaa ggcataaatt cttctttcac cgtttccttt 7440

agctcttcaa ccttttgtgt catatttgac tgtgagagat tattgaaaat aaaagttgtg 7500

tcatatttga ctgtgagaga ttattgaaaa taaaagcaat tttctcctgg atattttctg 7560

ggggctccac aattctctca gtttgatctg tggccacaag caacgtatct atatttgtag 7620

tattaataga aggtggcaca tctttcttaa agctgactcc agttggcctg gtgaccgtaa 7680

cgcacatctt tcttaaagct gactccagtt ggcctggtga ccgtaaccgt tttagcaaca 7740

gtggtagttg ttgaggtggt taccatagtg ctaacttgac cagcaagagg agcttttgct 7800

ggaacctggg cctgagcctg ggcctgagcc agtgcaatac ttccaggggt tgtgatagag 7860

ccttgcattt tcacaggagg atctctagac tgctgtccat actcaatata ctcctgtaaa 7920

tgatgtggaa attgcataaa gtgactgata gaagctgtaa atgatgtgga aattgcataa 7980

agtgactgat agaagccaaa tgctgacaat actggggata gtccttcaat ctgtttttaa 8040

atctatctag tgcagcaatc ccgaaataat acttttaaat ctatctagtg cagcaatccc 8100

gaaataatac attttggatc caaaaggctt gcgtaaggct tcaagaacat atcgtagagc 8160

cagacctagt gccatgtaag tgaccagtcc tttctcaatt ataccaccaa ataggcaggc 8220

tgttatatgt aactctttat caggatactg gggaaaaaaa cgatattctt caaacaagtt 8280

ccttagcata cagttaaata cttctcgttc cctctttata gtagagtctt taaatctctg 8340

cagcatttct aatacctcat caacagacat ggttggatgt ggtggatgat tatatattcg 8400

tcatcaacag acatggttgg atgtggtgga tgattatata ttcgctggaa atagctgttt 8460

gcttcatcat ctatctcttt actaaagtgc tggtttgcct ctggccacac ctgagacaag 8520

tccgagtata ctgtatagca gtgttcttac tcgctggaat gtaggctgat tcagtccaga 8580

ggtgcccagt ttcctctgta caaaagggtc gttattcact gcagggagtc caagagcacc 8640

agttcctata ccagtcaggc tgcctgtgcc aagaccacct actggaagct gtgatgaaag 8700

tcctccaata ccactgagca gggagctgtg atgaaagtcc tccaatacca ctgaatgcag 8760

tggtctgatt gggggttgaa aggggtggaa atgcttttgc tggtgactga ggggtactga 8820

atgcagaacc caaatttgga ggaaaaccct gcatactctg ggtgtgaggg gcagctgaac 8880

cacctatact aagagatgtc attccagcaa gagggtcaat ctgaacagga gaaatggcat 8940

ctaagctgct agcactagga ggacgaggag aaatggcatc taagctgcta gcactaggag 9000

gacgtccttt tggcataact ccaggtggtg gttgtctggc cttattcata acattactgc 9060

aattggctac catggtgagg atagtttctg atagctcctg agaaacactc cctgcacaag 9120

cttgcagaca ggccaacatt gtcgccaaag tttctgg 9157

<210> 33
<211> 14534
<212> DNA
<213> Homo sapiens

<400> 33
tctggccacg ccctacgaaa actaagccgc ccagttgcgt gctcaccacc ggccggcccc 60

gccaacgtat tttgtcttgc acccagacga agatcagccc tcggggacga ggccagggct 120

gtacctggcg ccagcggcac gggactctcc ttctgcactc gcagcgcgtg agtgcaatgg 180

gactcaagat gttttcatcc cacgaaacag gaccacgtgt ccggcgccgg ggcggggcag 240

agacgccgga agtgaccgcg cggcgccggc ccgcgaggaa acgcgctctt agaccatggc 300

gacccaggcg aagcgtccac gggtgggggc ttccccccct tctcgttaga acgcctgcgc 360

cgctccctcc cccctctccg ccgtcccccc tccacccaaa gcccgttctg cgccctcctg 420

tccagggcct gggggggtca ccgcacttat ctcagggggg gcggggctgc tctgagctgg 480

tgccggcggg ggggctggag ctgagtccca aggtgggggg gccgccggcg cgggctgccc 540
gaccagccct cctgctcagg tggcggtcag ccggcagggc acggtggccg gctacggcat 600
ggtggcccgg gagagcgtgc aggccggaga gctgtgttcg tggtgccgcg ggccgcgctc 660
ctgtcgcagc acacctgctc catcggcggc ctgctggagc gagagcgagt tgcgctgcag 720
agccagcggg cggggcgctg cagagccagt cgggctgggt gccactgctg ctggcgctgc 780
tccacgagct gcaggccccg gcctcacgct ggaggcccta ctttgcgctc tggcccgagc 840
tgggccgctt ggagcacccg atgttctgga gagggtgtgg gaacctgggt gtggggtccc 900
tgcagagggg gcgccggtgc ctgctccagg gcacaggcgt acctgaggcc gtggagaagg 960
atttggccaa catccgcagc gagtaccagt ccatcgtgct gcccttcatg gaagcccacc 1020
ccgatctctt cagcctcagg gttcgctccc tagaactcta ccaccagctg gtggcccttg 1080
tgatggccta tagtgggaga gagacctcgg tagcgttctg tcaatggcta acactggagg 1140
aagaagagga tgaaaaggag cccaactccc ccgtgatggt gcctgctgca gacatactaa 1200
accacttagc caatcacaac gccaatctag aatactctgc ggtctaattg tgtcttaggc 1260
tccattctct tactagtgct acaaaatgag taggtgaaat cagctcctcc tgtatgtcct 1320
tttagtatgc atattactgt agaatcattt gaatgcgaaa cattttaaag tcctcaaagt 1380
aagttgtggt taatcctctg ataaactata ctaccatctt tatttagtgc tagacattga 1440
gtggtctgca tagccatgtt tcattccttt cacatcagtg agcccagact gtgtgtggct 1500
ctgtggatgc agcctggaac agtgattctt tggagggatg aggacattca caggccaaag 1560
gtctaagacg atcctagaac tgatataagc ttttataaaa agtagcctga atggcacatt 1620
tataggatca gaaattagta agtatggtta tttcttaatc cttgcctcca ttttcctttc 1680
cctttgatct gcgaaagttc cattttttcc cccccgggcg gagtctcact gtgtcgccca 1740
ggctggagtg cagggcgctg ccgcctcctg ggttcaagca attctccctg cctcagcctc 1800
ccgagtagtt ggaattacag gctcctgccc atgcccggca atttttgtat tttttagtag 1860
ggatggggtt tctccgtgtt ggccaggctg gtcttgaact cctgatctca ggtattcttt 1920
gcctccctaa gtgctgggat tacaggtgtg agcactgcgc ctggccgaaa atttcgtttt 1980
taaagctagc aactttttaa gtgctgaaga ggactgtgca gaaatccacc cagttaaaca 2040
tgaagctggc cttttaaagt tagcccaacc cttttgaggt taacagcatc agtcatggct 2100
gaactacacc tgctgaaggc tcttcttaag tggaaataat cttcatgggg ctggcccgtg 2160
aaaggcatgg ccccagcttc tccctttcac ctcagaattg tcttcggatg gtagccactc 2220
agcccattcc taaaggccat gagattttca acacttatgg gcaaatggct aactggcaac 2280
tgattcatat gtacggtttt gttgaaccat atcctgacaa cacagatgac acagctgaca 2340
ttcagatggt gacagttcgt gaggcagcat tacagggaac aaaaactgag aggtaagaag 2400

aaaaactgaa gctgaaaggc acctagtgta cgagcgctgg gatttcctat gcaaactgga 2460

gatggtaggg gaagagggag cctttgtgat agggagggag gaggtgctga ctgaagagga 2520

gctgaccacc acactaaagg tactgtgcat gcctgctgag gagttcagag agcttaaagt 2580

gcatgcctgc tgaggagttc agagagctta aagaccagga tggaggggga gatgataaaa 2640

gggaagaggg cagcctgacg atcacaaata ttcccaagct caaagcatcg tggagacagc 2700

tgcttcaaaa cagtgttcta ctgactttgc agacctatgc cacagactta aaaactgacc 2760

aaggtttact cagtaataag gaagtctatg cgaaactcag ctggagggaa cagcaagcct 2820

tacaggttcg ctatggtcag aagatgatct tacatcagtt gttggactga caagttagca 2880

gtttccctgt tccctgaagg aacagcaata agaactttat tctaagctaa tactcattga 2940

tgtttgaaaa agaggaaaat ttggatcttt cttttgctta ctaaacacca agaggaaaag 3000

tagcaaagtt ggtgtgctag gattaactca ggtaagggtg atgtgtttta ggattgagaa 3060

cagcagactt gggaatcact gctaattgtt acttaaagca tgttacagtg ttttgttctc 3120

agttttaacc aaagccagtg gacatacggt agtaataagt aagtcttgtt gtgtttcagc 3180

atttaataat agactttgga ggtagacccc tggtttaaat ctaagtctag tttgaggaag 3240

tcacttaacc tttattgaaa agactctgga tttaataagc tgtgtaactg gactcgatag 3300

ttacccaaag ttcagtctag atggcacaaa ccacctctca gggaataaac cctaagacat 3360

cactcaagga ggacttcaat tatttaattt tgaactgttt tgtcctctct ggccataaaa 3420

cttgacagtc atgaaaggta aggcaaattt taagtgggtt aagtttttaa atacgtatct 3480

actatttttt aaaaaggata ttaaaactgg gtattttaaa ctcagcttaa ccagataaac 3540

ctgctccagc atttgtccag tgttgaaata aataggaaga aaaatacaat ttccttaaac 3600

atgccagctt agtagtagga atgacctatc aatcaatcaa aatcaaaagt tagttgtggt 3660

tccccatggc cccaaggtct cagggctgga ttgtccactt ttcctcctca ccaccagcac 3720

tgctgctgct actgagaaag gcacagtgga ctcatgtgtg tgtcatgact ttaatggtta 3780

gctacagtat gcatacacat acaagaagta ggggagctga agcccaggaa aaggctacaa 3840

aatacaacac agggaccaat acatttgcaa aacattcaaa atccttataa aaggggctgt 3900

attggtcctt tcaactttgt cattattcag cttagcctgt atggccattc attcagtggg 3960

gtaatggggg agaacaatta attaatgaga tttggttccc tttcctatat tcccactgtt 4020

tttaagtttc aggagaggat tggggggtga gggtaggggg gttgcggttc cttttaaaga 4080

gctgaccccc atctcaggcg gctgaggtgt ataatcccca aaagaaataa gatggtacaa 4140

atgcagtcag ctttggaata cgtttgggta gagacaaaat ggaaactcat gggattccaa 4200

tagtgagccc ttcccatgtt cctggacata tccaattcta gcacatccac atttttaata 4260

acttagtgat tttcaaggtc ccctgtccac attaaaaaaa taagttacat aatattcaaa 4320

ctggcttttt gctattcttt gggacaccag gaatgtcaga agacatggag ctatgccctc 4380

agacaagtgc atggcatcag ctgcctcttc attacaaggt accagtttat gtacttgcct 4440

tggacacagc ttgcacaaag ccaagaagtt ccagacaaag gttttctctt tcatgtattt 4500

acacaagttc aaaatgatat tcacagcatc ttctaaattt tggccaagag tcaaaaaaat 4560

gcatttaaac tttggaacgt gcccacataa gacaggaggc tgatcccaac agtagttggg 4620

gcagataccc acaaaccaaa gggctgggaa agtcaggaag agctgaaagg attcttcagt 4680

cagtttatga actcggtgca gtgagacctc tagactgaca cgtacaacag agatgcagtt 4740

tcgtctaact ggcacctgtc ccttccatta cttgctgggc ctgcttctgt cccatgcagc 4800

actgtgcgac cgactggaat aacttttcga tttctggggc acagtgtaca aattcatggt 4860

tccagaactt ttcgatttct ggggcacagt gtacaaattc atggttccag aacttaaacg 4920

ctgggttttt aatcagctca atgaaggtaa taagaagacc ccaaggatgt ggcctattta 4980

caatcaaccg ttccaagaga actcttgtga tctgttcttg gatggcttcc gtattggcct 5040

ctgcaaaaag cttgtgatct gttcttggat ggcttccgta ttggcctctg caaaaaggta 5100

cagcatggtg caactgaagt agtgagtgtg gctatttggg taccggagct gatttgcaat 5160

tgcattcaaa aagagatagc gaccctcagt gtccaagtcc acagccaaat tctggaagat 5220

atcacctcag tgtccaagtc cacagccaaa ttctggaaga tatccatgtg tgctgagtga 5280

gtgatggtgc tcattgaagg tgtgctgccc ttgttgtgga tgtgcgcaat ggcctgagtc 5340

ccgacataga gcaccagtgc attgatgagc tggaggttgt agcgattccc aggttcattg 5400

gatacctgta ggttgctgcg cagatcagac aggaaagtga ctggtgatgg taggttgctg 5460

cgcagatcag acaggaaagt gactggtgat cgagttttaa gataggaatc caaatccttt 5520

ttgaactgag gtggcattac tccagtgaaa ttggtgagaa tccggggagc aatgttaatt 5580

tcactcaaca tgtccacctt tagattagga gtgaatgggt cggggagcct catgtttctt 5640

ctttagatta ggagtgaatg ggtcggggag cctcatgttt cttggaaagg cactcaggat 5700

caaatttctt aactggatac aattaggtgg gatcacatca cagaacccat aatggtaatc 5760

acaaaggaac tctgggaaat catgcaaaag aaccagcagc actcttaaag tgcccttgta 5820

gaggatttgc ataggtttgg tgagttccac atttcacttg tagaggattt gcataggttt 5880

ggtgagttcc acatttctaa ggaaaggcgc taaatatttg aataaatcaa tcagtagctg 5940

tgcatacata ggccacccct tctgctgtgg cgtatgtgcc agcattcttg caataaatat 6000

ccgccttctg ctgtggcgta tgtgccagca ttcttgcaat aaatatccga tgggaaatca 6060

gttcaagcca ggcatataca aagccaggag ctttggtagg cctcaagatg tggaatgtat 6120

tgcagaaagc tgtaagtgtc tggaaattaa tggtttccaa cacatgctca agaaagctgt 6180

aagtgtctgg aaattaatgg tttccaacac atgctcaggt gcattgagtt ccaagagaag 6240

catgataaaa attcgatggt agggaagttg ctgaaattca ctctgacgaa catcatgatc 6300

ctgaaggaga actcccacta ctataccaag gaccttgttc agcagattaa tctttgtgac 6360

agtgttggtg gcctccccct tgttcagcag attaatcttt gtgacagtgt tggtggcctc 6420

ccctgagtgt ttcacgagca gtgcaatgag tcgaacaaag gcatccaggt tgtgatagca 6480

cttggctcgg atcatggtgg gattggcagc aggattgtgc tgctgctcag cctgagcacg 6540

gtaactgatt tcaacacaca tttcagtaca cagacgaaag aaccttgtta tgagatcatc 6600

ggtcttcagt attccttgct ggtgcatctg tccaacaaat gcagagaaag ctttggtact 6660

gtcgcggagc ttaagctcta cctgtccaac aaatgcagag aaagctttgg tactgtcgcg 6720

gccagctgct gctgaatggt agagattcac ccattccctc agaagatact ctgccttctc 6780

cctcaggcct ggagggtcat catactctga ggcttgagag atcccagaat gcatcataaa 6840

gtttgggcct ccatgagcac gatcaatcat tgcttcatag ttggatcgca ctacttccat 6900

cagctggggc aatccttctg gagcattgcc tctggaatga gcattaatcc tcatgagggc 6960

ttctggagca ttgcctctgg aatgagcatt aatcctcatg agggtttcaa tggtgtggaa 7020

cagatctgcc tcagtaacat gagcaacact cctttcatcc accagcagga tttttactaa 7080

ctgcatagca aatgccacag ccatgtagtt taagccattc tccattgact gcgctaggtg 7140

aagatcatac tgctgcatat taaccaaatg attgctgcgc taggtgaaga tcatactgct 7200

gcatattaac caaatgattg cgaattagca gctccacagc ctccacatta tatttatatt 7260

catctcgaca ttcaattagg caccttgtga tctgtttgtt gcaccatgga gacccatatg 7320

cccggccttg tgatctgttt gttgcaccat ggagacccat atgcccggcc atcctgcaga 7380

gcttttagga ccaagaggtg gcattccctg tagcgcagca gaaggtcagc atcagcacca 7440

cttgtggcat ctagtaagcc ctctacagcc ttttggagca atccaagagc agctatggca 7500

tcccgagagt ttcgagttgg agcaatccaa gagcagctat ggcatcccga gagtttcgag 7560

ataaaactac aacctccaag agacttcgaa gagcctgagc ttgagggttc atggccaaag 7620

ttggtgggat ggcatgtaga tgttgctcca gttctgtaat acacttatca taaatctgag 7680

ctacatcatc tgttgcccaa gcttgcttca tgggctgggc taaaaatccc gtgggtgact 7740

aagacaaaca aatcaagtca tttagaaaat aattaggaaa gatgaggaaa aactgagtta 7800

cctacaccag ctagaactga gggagacttt aaaaatatga caactgaaga cttttactat 7860

acattattgg gatacatggt gaaactcgag tgaggtctat tagaaggtta ttagtatttt 7920

ctgaccttaa tggttgtacc aggaaaagcg atgggggtat caggtcagca acttactatc 7980

gaccggttcg gggaaaaata tttttcccta tatttccaag ttttctgtaa ataggccgaa 8040

cattaaagtt aaatcaccca cacctgtcaa tttttggctg ggctaaaaat cccgtgggct 8100

gacttaagtc atttgtaggt aagaagccag gaacattgcg tgcaaactct tctaaacagc 8160

caactgcttt gggtccacac caccaacttt cagcctgatt tgctctggca tccgttcagc 8220

ttgatatgtt aaacctgatt tgctctggca tccgttcagc ttgatatgtt aaaacaacag 8280

gatcacagta tctgcgtcct tcttgcctag catgttttct cagctcaaat tcagttgcta 8340

atctcttgtc catctcaggg cctgcttttt ctttctaatc tcttgtccat ctcagggcct 8400

gctttttcta ctgcagtctt ctgaataaaa cagcaagcca actcacaatt gtcctgagct 8460

aattgagcag ctgcctgatc catcatttct ctttgttgtg gggaagcagt acgaagggct 8520

gaggcaaaac tgttttttaa gttggtagat atctcgtggt agcacatgcc tgtaatccca 8580

gctacttggg aggctgaagc acgagaatca gttgaacctc agaggtggag gatgcagtga 8640

gccaagactg cactactatc tgtcaaacat cgtgttgaga atataacaga aaaaaaaaaa 8700

aataaatcta aaatgatgga agacatcaaa caaatatggt gattattaag aataagtggt 8760

aaccagtgcc atatatagac aaagaaaaaa attattacag acaagcagta atacgttttg 8820

tacctatacc aaggaaatag ccaacttaca cgaagggctg aggcaaaact gttttttaag 8880

ttggtagata tgctcatgag caaaggttcc ctgcatgtaa tcatagccat tccagctgtc 8940

aagttacgca tcatgtgatg agctgctatt cgcattcgag attcctccga atccagggca 9000

aaatccttcc tgactatttg ctcacaagta gtcatggcaa tcttaattga tcgatccacc 9060

acaggatgga ccagctcctg gacagcccgt tcaattgcct gacgcacaca ctgcttcaac 9120

tgtggatggg cctgaaacaa gggaattgtt ggattagagt aatgtgtggt gccaagcccg 9180

caagggagtt ggattagagt aatgtgtggt gccaagcccg caagggaata gacattgatg 9240

tcgtggtagc tgtactgtgg ctgtggtgga accgtggctg tacaagtggt gttggtagct 9300

ggtgtagtag aagttgttgt ggttgtgatg ggagggagtt cttctggctg cttgacatct 9360

ttcgttgatg ggagggagtt cttctggctg cttgacatct ttctttggag cagagagttg 9420

ctcatctaaa ttcttcaggc gatctttatc ctttaggagg tttccaggtt ttagctcatt 9480

gatgtctaat gcaaggttct tgcagagaac ctcgatttca aacttcaagt ttaactttaa 9540

gtcatgctcc tgatgtagct cagctaatac attcataatt gcttagtcat gctcctgatg 9600

tagctcagct aatacattca taattgccat tgtccaaggg tttggtggcc taaaaaccac 9660

actacgaatg ctagattcta agactttggc aacaaagggc acgaatgcta gattctaaga 9720

ctttggcaac aaagggcact acatagagca attcttgttg tcctttaaca taagcctcta 9780

gcagcaatga tttcacatcc aagtcagtgt gtaagatggg tttgttttta gctaatgtga 9840

tcattcctaa gatggtttgt ttttagctaa tgtgatcatt cctagccaat gtcccaagtt 9900

cttcagcaaa gaacgatctg agaaattggc tgcagcttta tcagaggtca ggagcacttt 9960

aatgtttctg taggtctcat tcagaaccat cttgttaaat tcaggatgtt tctgtaggtc 10020

tcattcagaa ccatcttgtt aaattcagga ttcttcagcg tgtcaaggaa gtttgaatac 10080

aggctatgaa agtttggctc aatactgact ctcttcataa ccagatactg tgaaacccaa 10140

ggcataaatt cttctttcac cgtttccttt agctcttcaa ccttttgtgt catatttgac 10200

tgtgagagat tattgaaaat aaattgtgtc atatttgact gtgagagatt attgaaaata 10260

aaagcaattt tctcctggat attttctggg ggctccacaa ttctctcagt ttgatctgtg 10320

gccacaagca acgtatctat atttgtagta ttaatagaag gtggcacatc tttcttaaag 10380

ctgactccag ttggcctggt gaccgcatct ttcttaaagc tgactccagt tggcctggtg 10440

accgtaaccg ttttagcaac agtggtagtt gttgaggtgg ttaccatagt gctaacttga 10500

ccagcaagag gagcttttgc tggaacctgg gcctgagcct gggcctgagc cagtgcaata 10560

cttccagggg ttgtgataga gccttgcatt ttcacaggag gatctctaga ctgctgtcca 10620

tactcaatat actcctgtaa atgatgtgga aattgcataa agtgactgat agtctgtaaa 10680

tgatgtggaa attgcataaa gtgactgata gaagccaaat gtgacaatac tggggatagt 10740

ccttcaatct gtttttaaat ctatctagtg cagcaatccc gaaataatac attttctgtt 10800

tttaaatcta tctagtgcag caatcccgaa ataatacatt ttggatccaa aaggcttgcg 10860

taaggcttca agaacatatc gtagagccag acctagtgcc atgtaagtga ccagtccttt 10920

ctcaattata ccaccaaata ggcaggctgt tatatgtaac tctttatcag gatactgggg 10980

aaaaaaacga tattcttcaa acaagttcct tagcatacag ttaaatactt ctcgttccct 11040

ctttatagta gagtctttaa atctctgcag catttctaat acctcatcaa cagacatggt 11100

tggatgtggt ggatgattat atattcatca acagacatgg ttggatgtgg tggatgatta 11160

tatattcgct ggaaatagct gtttgcttca tcatctatct ctttactaaa gtgctggttt 11220

gcctctggcc acacctgaga caagtccggt atactgtata gcagttcttc tcgctggaat 11280

gtaggctgat tcagtccaga ggtgcccagt ttcctctgta caaaagggtc gttattcact 11340

gcagggagtc caagagcacc agttcctata ccagtcaggc tgcctgtgcc aagaccacct 11400

actgaagtgt gagatccaac ctgaagcagg aagctgtgat gaaagtcctc caataccact 11460

gaatgcagtg gtctgattgg gggttgaaag gggtggaaat gcttttgctg gtgactgagg 11520

ggtactgaat gcagaaccca aatttggagg aaaaccctgc atactctggg tgtgaggggc 11580

agctgaacca cctatactaa gagatgtcat tccagcaaga gggtcaatct gaacaggaga 11640

aatggcatct aagctgctag cactaggagg accaaaggag aaatggcatc taagctgcta 11700

gcactaggag gacgtccttt tggcataact ccaggtggtg gttgtctggc cttattcata 11760

acattactgc aattggctac catggtgagg atagtttctg atagctcctg agaaacactc 11820

cctgcacaag cttgcagaca ggccaacatt gtcgccaaag tttcttgcag acaggccaac 11880
attgtcgcca aagtttctgg aggaagttga gcacttttgg gctggtcttt ttctggggca 11940
agtccgccca aaatagaagg acaccgtctc tttaaaaaag tcatacacgc ctggataaaa 12000
ggctccccat gctctcgaat tttatctgtg agccacttat caagtttgag gccattttat 12060
ctgtgagcca cttatcaagt ttgaggtatt cacgacgtga agcaagtgca gcaaggtcaa 12120
taacaaaggc aaatggagta ccatttagca gcattgacaa ggccttcaag tcctgggcca 12180
catcaagtat tcgagacaat ttggcctcaa gtcctgggcc acatcaagta ttcgagacaa 12240
tttggcctga tcatactgct cccctctcat gtaccattct gccattgcat gcatgataag 12300
ttggcgaatt gagggagact gtccctgccc atgccatgca tagtgcaaaa taatagctga 12360
gttaggatgg ttcccagcca tgcatagtgc aaaataatag ctgagttagg atggtttcca 12420
aggaaaattg gcatcagagt ggagataagt tcatggcgca aggtatgcca agaggtgtta 12480
atttgtagta aggccaatac cagcatgtct ggacagtgtt tgatagggaa gctgaagagc 12540
tgtttgactt gctcatactg cccaacctct gcaagcctca gcagagattc aatcaaatcc 12600
aagctcttcc atgtggcaat ttctcgattg tcatcctctg gtggtgcttt cagaagcaat 12660
ttctcgattg tcatcctctg gtggtgcttt cagaatatca gtggcaacag tatgacaggg 12720
atagtcagca aaacagaaga tctctggatt tataaggaaa tgttgaatga aggagagctg 12780
gccttcagca tgtttccaag gtctatatat gaggtctacg cttcagcatg tttccaaggt 12840
ctatatatga ggtctactgg gaacacttcc atacccaaac ccctctgaat gccataaacc 12900
acattatgaa gtcctttact gtcacgaatt tgaaatccag gatggtccag ttcataagtt 12960
acttccttga aattcaaact tggattcagt tctttaagaa cgtcaatcaa gacttctaca 13020
tcggttcttt aagaacgtca atcaagactt ctacattcca tgtgtgtgcc tgtgctccat 13080
cacttttatc tttcccatca ctccagatcc cactgcccgg agcagaaata ctctgtaatg 13140
gaatgccatc tgttaacctg aatgagttcg agaatggaat gccatctgtt aacctgaatg 13200
agttcgagcc atcattccca aaacccttgc aacctgggca gctgtgacct cccgaacacc 13260
aaactgcacg attatattgc gacattcttc aatacttgca caaaagccat agcctacttc 13320
ttgcatgaaa tcagccaaag caaaagccat agcctacttc ttgcatgaaa tcagccaaag 13380
agctctccat catggtttta gctacccctc cggaatcagg caggatcctg tccattagaa 13440
tgtcccgttt ttcagggtat aaaagtggtg cgagcaccac gggacagcgt tcttggggaa 13500
aatctctgcg cagcgtctta agaaaagcgt ctatctgttc ttgtcgcgca gcgtcttaag 13560
aaaagcgtct atctgttctt gtccaactcc aaaggctccc ttctgcccaa agaggagatg 13620
ggagaggagg aggtgtagga cctctattgc tatatcttgg aagccacctt cttgatttcc 13680
actgacgtct gcgtcaatgt aagaacgcag aagatctgga agcttctgtt tgataaactg 13740

ggcagcgaaa cctctaagat ctgagctgga agaattcaac agggcaaggc caaacctcta 13800

agatctgagc tggaagaatt caacagggca aggccaaaaa ttacctcttg tactttgctt 13860

aatttgagca ctttactcag ctgggacttg tactttgctt aatttgagca ctttactcag 13920

ctgggcaaat aagtggggtg caggctttaa actcttctga tagtgcaatg gattatcaat 13980

ggcataggac agcgtcgtta gtgtatcaat ggcataggac agcgtcgaga taaaatttgg 14040

ctttgtaatc agcaacgcac actcctgaat cagaaactga gtctgatgga aatctttgcc 14100

actgctttta ccatcgccac tgaaatccac ggaaatcttt ccttttacca tcgccactga 14160

aatccacatg cgaaaatagg cagcgtaata aatgcctgtc tgcctcagga ccgtgccgat 14220

tcacaatatg ctgtatttcc tgctggctgg ctcggaattt ttcttgtttc tcggctggct 14280

cggtaatttt tcttggttaa attgtccacc aggtagctga tttgagacaa ggccagcgag 14340

agcgagtcaa gattcattgc tggttggggc ggaagcaggc ggccgagccc ggcgcaaaat 14400

caccattatt cccctttagt cacctcagag gcaggttaat gctttctttg taattaggct 14460

atatctggta tctgtataat atcttcagtt cttctttacc aggggtctta ctctgttctg 14520

aaacatggca cctc 14534

<210> 34
<211> 9547
<212> DNA
<213> Homo sapiens

<400> 34
ggaggaggtg ctgactgaag aggagctgac caccacacta aaggtactgt gcatgcctgc 60

tgaggagttc agagagctta aagaccaggg tactgtgcat gcctgctgag gagttcagag 120

agcttaaaga ccaggatgga gggggagatg ataaaaggga agagggcagc ctgacgatca 180

caaatattcc caagctcaaa gcatcgtgga gacagctgct tcaaaacagt gttctactga 240

ctttgcagac ctatgccaca gacttaaaaa ctgaccaagg tttactcagt aataaggaag 300

tctatgcgaa actcagctgg agggaacagc aagccttaca ggttcgctat ggtcagaaga 360

tgatcttaca tcagttgttg gactgacaag ttagcagttt ccctgttccc tgaaggaaca 420

gcaataagaa ctttattcta agctaatact cattgatgtt tgaaaaagag gaaaatttgg 480

atctttcttt tgcttactaa acaccaagag gaaaagtagc aaagttggtg tgctaggatt 540

aactcaggta agggtgatgt gttttaggat tgagaacagc agacttggga atcactgcta 600

attgttactt aaagcatgtt acagtgtttt gttctcagtt ttaaccaaag ccagtggaca 660

tacggagtaa taagtaagtc ttgttgtgtt tcagcattta ataatagact ttggaggtag 720

acccctggtt taaatctaag tctagtttga ggaagtcact taacctttat tgaaaagact 780

ctggatttaa taagctgtgt aactggactc gatagttacc caaagttcag tctagatggc 840

acaaaccacc tctcagggaa taaaccctaa gacatcactc aaggaggact tcaattattt 900

aattttgaac tgttttgtcc tctctggcca taaaacttga cagtcatgaa aggtaaggca 960

aattttaagt gggttaagtt tttaaatacg tatctactca ttttctttaa aaaaaaaaag 1020

ggaaggatat taaaatgggt attttaaact cagcttaacc agataaacct gctccagcat 1080

ttgtccagtg ttgaaataaa taggaagaaa aatacaattt ccttaaacat gccagcttag 1140

tagtaggaat gacctatcaa tcaatcaatc aatacaagaa gttagttgtg gttccccatg 1200

gccccaaggt ctcagggctg gattgtccac ttttcctcct caccaccagc actgctgctg 1260

ctactgagaa aggcacagtg gactcatgtt tcatgacttt aatggttagc tacagtatgc 1320

atacacatac aagaagtagg ggagctgaag cccaggaaaa ggctacaaaa tacaacacag 1380

ggaccaatac atttgcaaaa cattcaaaat ccttataaaa ggggctgtat tggtcctttc 1440

aactttgtca ttattcagct tagcctgtat ggccattcat tcagtggggt aatgggggag 1500

aacaattaat taatgagatt tggttccctt tcctatattc ccactgtttt taagtttcag 1560

gagaggattg gggggtgagg gtaggggggt tgcggttcct taacccccat ctcaggcggc 1620

tgaggtgtat aatccccaaa agaaataaga tggtacaaat gcagtcagct ttggaatacg 1680

tttgggtaga gacaaaatgg aaactcatgg gattccaata gtgagccctt cccatgttcc 1740

tggacatatc caattctagc acatccacat ttttaataac ttagtgattt tcaaggtccc 1800

ctgtccacat taaaaaaata agttacataa tattcaaact ggctttttgc tattctttgg 1860

gacaccagga atgtcagaag acatggagct atgccctcag acaagtgcat ggcatcagct 1920

gcctcttcat tacaaggtac cagtttatgt acttgccttg gacacagctt gcacaaagcc 1980

aagaagttcc agacaaaggt tttctctttc atgtatttac acaagttcaa aatgatattc 2040

acagcatctt ctaaattttg gccaagagtc aaaaaaatgc atttaaactt tggaacgtgc 2100

ccacataaga caggaggctg atcccaacag tagttggggc agatacccac aaaccaaagg 2160

gctgggaaag tcaggaagag ctgaaaggat tcttcagtca gtttatgaac tcggtgcagt 2220

gagacctcta gactgacacg tacaacagag atgcagtttc gtctaactgg cacctgtccc 2280

ttccattact tgctgggcct gcttctgtcc catgcagcac tgtgcgaccg actggaataa 2340

cttttcgatt tctggggcac agtgtacaaa ttcatggttc cagaactttt catttctggg 2400

gcacagtgta caaattcatg gttccagaac ttaaacgctg ggtttttaat cagctcaatg 2460

aaggtaataa gaagacccca aggatgtggc ctatttacaa tcaaccgttc caagagaact 2520

cttgtgatct gttcttggat ggcttccgta ttggcctctg caaaaaggca tggtggccac 2580

atgcctgtaa tcccagcagc ggacgctgag gagtgaagca tttaaaaaat aagctgctcg 2640

cttcttgtga tctgttcttg gatggcttcc gtattggcct ctgcaaaaag gtacagcatg 2700

gtgcaactga agtagtgagt gtggctattt gggtaccgga gctgatttgc aattgcattc 2760

aaaaagagat agcgaccctc agtgtccaag tccacagcca aattctggaa gatatccatg 2820

tggccctcag tgtccaagtc cacagccaaa ttctggaaga tatccatgtg tgctgagtga 2880

gtgatggtgc tcattgaagg tgtgctgccc ttgttgtgga tgtgcgcaat ggcctgagtc 2940

ccgacataga gcaccagtgc attgatgagc tggaggttgt agcgattccc aggttcattg 3000

gatacctgta ggttgctgcg cagatcagac aggaaagtga ctggtgatcg agggttttaa 3060

gccaaaccaa ttaactgtag gttgctgcgc agatcagaca ggaaagtgac tggtgatcga 3120

gttttaagat aggaatccaa atcctttttg aactgaggtg gcattactcc agtgaaattg 3180

gtgagaatcc ggggagcaat gttaatttca ctcaacatgt ccacctttag attaggagtg 3240

aatgggtcgg ggagcctcat gtttcttctt tagattagga gtgaatgggt cggggagcct 3300

catgtttctt ggaaaggcac tcaggatcaa atttcttaac tggatacaat taggtgggat 3360

cacatcacag aacccataat ggtaatcaca aaggaactct gggaaatcat gcaaaagaac 3420

cagcagcact cttaaagtgc ccttgtagag gatttgcata ggtttggtga gttccacatt 3480

tctaaggatt tagaggattt gcataggttt ggtgagttcc acatttctaa ggaaaggcgc 3540

taaatatttg aataaatcaa tcagtagctg tgcatacata ggccacccct tctgctgtgg 3600

cgtatgtgcc agcattcttg caataaatat ccgatgttat agtcttcatc tacaggagat 3660

aaactgcagc cacccttctg ctgtggcgta tgtgccagca ttcttgcaat aaatatccga 3720

tgggaaatca gttcaagcca ggcatataca aagccaggag ctttggtagg cctcaagatg 3780

tggaatgtat tgcagaaagc tgtaagtgtc tggaaattaa tggtttccaa cacatgctca 3840

cagaaagctg taagtgtctg gaaattaatg gtttccaaca catgctcagg tgcattgagt 3900

tccaagagaa gcatgataaa aattcgatgg tagggaagtt gctgaaattc actctgacga 3960

acatcatgat cctgaaggag aactcccact actataccaa ggaccttgtt cagcagatta 4020

atctttgtga cagtgttggt ggcctcccct cttgttcagc agattaatct ttgtgacagt 4080

gttggtggcc tcccctgagt gtttcacgag cagtgcaatg agtcgaacaa aggcatccag 4140

gttgtgatag cacttggctc ggatcatggt gggattggca gcaggattgt gctgctgctc 4200

agcctgagca cggtaactga tttcaacaca catttcagta cacagacgaa agaaccttgt 4260

tatgagatca tcggtcttca gtattccttg ctggtgcatc tgtccaacaa atgcagagaa 4320

agctttggta ctgtcgcggc cagctgttcc aaaagctcta cctgtccaac aaatgcagag 4380

aaagctttgg tactgtcgcg gccagctgct gctgaatggt agagattcac ccattccctc 4440

agaagatact ctgccttctc cctcaggcct ggagggtcat catactctga ggcttgagag 4500

atcccagaat gcatcataaa gtttgggcct ccatgagcac gatcaatcat tgcttcatag 4560

ttggatcgca ctacttccat cagctggggc aatccttctg gagcattgcc tctggaatga 4620

gcattaatcc tcatgaggtt ctggagcatt gcctctggaa tgagcattaa tcctcatgag 4680

ggtttcaatg gtgtggaaca gatctgcctc agtaacatga gcaacactcc tttcatccac 4740

cagcaggatt tttactaact gcatagcaaa tgccacagcc atgtagttta agccattctc 4800

cattgactgc gctaggtgaa gatcatactg ctgcatatta accaaatgat tgctgcgcta 4860

ggtgaagatc atactgctgc atattaacca aatgattgcg aattagcagc tccacagcct 4920

ccacattata tttatattca tctcgacatt caattaggca ccttgtgatc tgtttgttgc 4980

accatggaga cccatatgcc cggcttgtga tctgtttgtt gcaccatgga gacccatatg 5040

cccggccatc ctgcagagct tttaggacca agaggtggca ttccctgtag cgcagcagaa 5100

ggtcagcatc agcaccactt gtggcatcta gtaagccctc tacagccttt tggagcaatc 5160

caagagcagc tatggcatcc cgagagtttc gagctttgga gcaatccaag agcagctatg 5220

gcatcccgag agtttcgaga taaaactaca acctccaaga gacttcgaag agcctgagct 5280

tgagggttca tggccaaagt tggtgggatg gcatgtagat gttgctccag ttctgtaata 5340

cacttatcat aaatctgagc tacatcatct gttgcccaag cttgcttcat gggctgggct 5400

aaaaatcccg tgggctgact taagtcattt ttcatgggct gggctaaaaa tcccgtgggc 5460

tgacttaagt catttgtagg taagaagcca ggaacattgc gtgcaaactc ttctaaacag 5520

ccaactgctt tgggtccaca ccaccaactt tcagcctgat ttgctctggc atccgttcag 5580

cttgatatgt taaaacgcct gatttgctct ggcatccgtt cagcttgata tgttaaaaca 5640

acaggatcac agtatctgcg tccttcttgc ctagcatgtt ttctcagctc aaattcagtt 5700

gctaatctct tgtccatctc agggcctgct ttttctactg cagcttctaa tctcttgtcc 5760

atctcagggc ctgctttttc tactgcagtc ttctgaataa aacagcaagc caactcacaa 5820

ttgtcctgag ctaattgagc agctgcctga tccatcattt ctctttgttg tggggaagca 5880

gtacgaaggg ctgaggcaaa actgtttttt aagttggtag atatgctcac agcactttgg 5940

gaggccaagg caggtagatc acttgaggcc aggagtttga gaccagccta gccaacatgg 6000

tgaagccact tctctactaa aaaaatgggg cgtggtacag ctacttggga ggctgaagca 6060

cgagaatcag ttgaacctca gaggtggagg atgcagtgag ccaagactgt agctaagcta 6120

cagagtgaga ccctgtctca tgaaacatta aaaaaaaggc aaaaaaaaca aaaaaaataa 6180

aaacaaatat atatatgaat tattaaccat gaatctgaga aaatattgtc attttgtatc 6240

gcctcaaact tctcctcgtt acgaaaaatt tttatttaaa caaaatttaa gttgagaata 6300

taacagaaaa aaaaaaaatc taataatcta aaatgatgga agacatcaaa caaatatggt 6360

gattattaag aataagtggt aagcccagtg ccatatatag acaaagaaaa aaattattac 6420

agacaagcag taatacgttt tgtacctata ccaaggaaat agccaactta cacgaagggc 6480

tgaggcaaaa ctgtttttta agttggtaga tatgctcatg agcaaaggtt ccctgcatgt 6540

aatcatagcc attccagctg tcaagttacg catcatgtga tgagctgcta ttcgcattcg 6600

agattcctcc gaatccaggg caaaatcctt cctgactatt tgctcacaag tagtcatggc 6660

aatcttaatt gatcgatcca ccacaggatg gaccagctcc tggacagccc gttcaattgc 6720

ctgacgcaca cactgcttca actgtggatg ggcctgaaac aagggaattg ttggattaga 6780

gtaatgtgtg gtgccaagcc cgcaagggaa tagatgttgg attaagtaat gtgtggtgcc 6840

aagcccgcaa gggaatagac attgatgtcg tggtagctgt actgtggctg tggtggaacc 6900

gtggctgtac aagtggtgtt ggtagctggt gtagtagaag ttgttgtggt tgtgatggga 6960

gggagttctt ctggctgctt gacatctttc ttgtggttgt gatgggaggg agttcttctg 7020

gctgcttgac atctttcttt ggagcagaga gttgctcatc taaattcttc aggcgatctt 7080

tatcctttag gaggtttcca ggttttagct cattgatgtc taatgcaagg ttcttgcaga 7140

gaacctcgat ttcaaacttc aagtttaact ttaagtcatg ctcctgatgt agctcagcta 7200

atacattcat aattgccttt aagtcatgct cctgatgtag ctcagctaat acattcataa 7260

ttgccattgt ccaagggttt ggtggcctaa aaaccacact acgaatgcta gattctaaga 7320

ctttggcaac aaagggcaca atgctagatt ctaagacttt ggcaacaaag ggcactacat 7380

agagcaattc ttgttgtcct ttaacataag cctctagcag caatgatttc acatccaagt 7440

cagtgtgtaa gatgggtttg tttttagcta atgtgatcat tcctagcgta agatgggttt 7500

gtttttagct aatgtgatca ttcctagcca atgtcccaag ttcttcagca aagaacgatc 7560

tgagaaattg gctgcagctt tatcagaggt caggagcact ttaatgtttc tgtaggtctc 7620

attcagaacc atcttgttaa attcaggtta atgtttctgt aggtctcatt cagaaccatc 7680

ttgttaaatt caggattctt cagcgtgtca aggaagtttg aatacaggct atgaaagttt 7740

ggctcaatac tgactctctt cataaccaga tactgtgaaa cccaaggcat aaattcttct 7800

ttcaccgttt cctttagctc ttcaaccttt tgtgtcatat ttgactgtga gagattattg 7860

aaaataaaag attttgtgtc atatttgact gtgagagatt attgaaaata aaagcaattt 7920

tctcctggat attttctggg ggctccacaa ttctctcagt ttgatctgtg gccacaagca 7980

acgtatctat atttgtagta ttaatagaag gtggcacatc tttcttaaag ctgactccag 8040

ttggcctggt gaccgtaacg gcacatcttt cttaaagctg actccagttg gcctggtgac 8100

cgtaaccgtt ttagcaacag tggtagttgt tgaggtggtt accatagtgc taacttgacc 8160

agcaagagga gcttttgctg gaacctgggc ctgagcctgg gcctgagcca gtgcaatact 8220

tccaggggtt gtgatagagc cttgcatttt cacaggagga tctctagact gctgtccata 8280

ctcaatatac tcctgtaaat gatgtggaaa ttgcataaag tgactgatag aagcctgtaa 8340

atgatgtgga aattgcataa agtgactgat agaagccaaa tgtgacaata ctggggatag 8400

tccttcaatc tgtttttaaa tctatctagt gcagcaatcc cgaaataata cattttctgt 8460

ttttaaatct atctagtgca gcaatcccga aataatacat tttggatcca aaaggcttgc 8520

gtaaggcttc aagaacatat cgtagagcca gacctagtgc catgtaagtg accagtcctt 8580

tctcaattat accaccaaat aggcaggctg ttatatgtaa ctctttatca ggatactggg 8640

gaaaaaaacg atattcttca aacaagttcc ttagcataca gttaaatact tctcgttccc 8700

tctttatagt agagtcttta aatctctgca gcatttctaa tacctcatca acagacatgg 8760

ttggatgtgg tggatgatta tatattcgct acagacatgg ttggatgtgg tggatgatta 8820

tatattcgct ggaaatagct gtttgcttca tcatctatct ctttactaaa gtgctggttt 8880

gcctctggcc acacctgaga caagtccggt atactgtata gcagttttta ctctgctgga 8940

atgtaggctg attcagtcca gaggtgccca gtttcctctg tacaaaaggg tcgttattca 9000

ctgcagggag tccaagagca ccagttccta taccagtcag gctgcctgtg ccaagaccac 9060

ctactggaag ctgtgatgaa agtcctccaa tacccgaatg cagtggtctc tggaagctgt 9120

gatgaaagtc ctccaatacc actgaatgca gtggtctgat tgggggttga aaggggtgga 9180

aatgcttttg ctggtgactg aggggtactg aatgcagaac ccaaatttgg aggaaaaccc 9240

tgcatactct gggtgtgagg ggcagctgaa ccacctatac taagagatgt cattccagca 9300

agagggtcaa tctgaacagg agaaatggca tctaagctgc tagcactagg aggaccagga 9360

gaaatggcat ctaagctgct agcactagga ggacgtcctt ttggcataac tccaggtggt 9420

ggttgtctgg ccttattcat aacattactg caattggcta ccatggtgag gatagtttct 9480

gatagctcct gagaaacact ccctgcacaa gcttgcagac aggccaacat tgtcgccaaa 9540

gtttctg 9547

<210> 35
<211> 14008
<212> DNA
<213> Homo sapiens

<400> 35
cagcacacct gctccatcgg cggcctgctg gagcgagagc gagttgcgct gcagagccag 60

tcgggcgggg cgagccagtc gggctgggtg ccactgctgc tggcgctgct ccacgagctg 120

caggccccgg cctcacgctg gaggccctac tttgcgctct ggcccgagct gggccgcttg 180

gagcacccga tgttctggag agcgggggcg ccggtgcctg ctccagggca caggcgtacc 240

tgaggccgtg gagaaggatt tggccaacat ccgcagcgag taccagtcca tcgtgctgcc 300

cttcatggaa gcccaccccg atctcttcag cctcagggtt cgctccctag aactctacca 360

ccagctggtg gcccttgtga tggcctatag tgggagagca gacctcccca agacagggcc 420

ttagccaggt tctgtcaatg gcttcaggaa ccacgaggaa gaagaggatg aaaaggagcc 480

caactccccc gtgatggtgc ctgctgcaga catactaaac cacttagcca atcacaacgc 540

caatctagaa tactctgcgg tgccgctaag ttctaggctc cattctctta ctagtgctac 600

aaaatgagta ggtgaaatca gctcctcctg tatgtccttt tagtatgcat attactgtag 660

aatcatttga atgcgaaaca ttttaaagtc ctcaaagtaa gttgtggtta atcctctgat 720

aaactatact accatcattt atttagtgct agacattgag tggtctgcat agccagtttc 780

attctttgcg atcacatcag tgagcccaga ctgtgtgtgg ctctgtggat agcagcctgg 840

aacagtgata gtctccgtgt tttggaggga tgaggacatt cacaggccaa aggtctaaga 900

cgatcctaga actgatataa agcttgttta taccagctct attctatata aaaagtagcc 960

gaaaaagcac atttatagga tcagaaatta gtaagtatgg ttatttctta atccttgcct 1020

ccattttcct ttccctttga tctgcgaaag ttccattttt tcccccccga ggcggagtct 1080

cactgtgtcg cccaggctgg agtgcagtgg cgtgatctcg gctcactgca actgccgcct 1140

cctgggttca agcaattctc cctgcctcag cctcccgagt agttggaatt acaggctcct 1200

gccaccatgc ccggctaatt tttgtatttt ttagtaggga tggggtttct ccgtgttggc 1260

caggctggtc ttgaactcct gatctcaggt atctgcccac tttgacctcc ctaagtgctg 1320

ggattacagg tgtgagccac tgcgcctggc cgaaaatttc gtttttaaag ctagcaactt 1380

tttaagtgct gaagaggact gtgcagaaat ccacccagtt aaacatgaag ctggcctttt 1440

aaagttagcc caaccctttt ttttgtttgg tctaacagga actaaaggta gaactttggt 1500

agttaacagc atcagtcatg gctgaactac acctgctgaa ggctcttctt aagtggaaat 1560

aatcttcatg gggctggccc gtgaaaggca tggccccagc ttctcccttt cacctcagaa 1620

tgtcttcgga tggtagccac tcagcccatt cctaaaggcc atgagatttt caacacttat 1680

gggcaaatgg ctaactggca actgattcat atgtacggtt ttgttgaacc atatcctgac 1740

aacacagatg acacagctga cattcagatg gtgacagttc gtgaggcagc attacaggga 1800

acaaaaactg aagctgaaag gcaaaaactg aagctgaaag gcacctagtg tacgagcgct 1860

gggatttcct atgcaaactg gagatggtag gggaagaggg agcctttgtg atagggaggg 1920

aggaggtgct gactgaagag gagctgacca ccacactaaa ggtactgtgc atgcctgctg 1980

aggagttcag agtgcatgcc tgctgaggag ttcagagagc ttaaagacca ggatggaggg 2040

ggagatgata aaagggaaga gggcagcctg acgatcacaa atattcccaa gctcaaagca 2100

tcgtggagac agctgcttca aaacagtgtt ctactgactt tgcagaccta tgccacagac 2160

ttaaaaactg accaaggttt actcagtaat aaggaagtct atgcgaaact cagctggagg 2220

gaacagcaag ccttacaggt tcgctatggt cagaagatga tcttacatca gttgttggac 2280

tgacaagtta gcagtttccc tgttccctga aggaacagca ataagaactt tattctaagc 2340

taatactcat tgatgtttga aaaagaggaa aatttggatc tttcttttgc ttactaaaca 2400

ccaagaggaa aagtagcaaa gttggtgtgc taggattaac tcaggtaagg gtgatgtgtt 2460

ttaggattga gaacagcaga cttgggaatc actgctaatt gttacttaaa gcatgttaca 2520

gtgttttgtt ctcagtttta accaaagcca gtggacatac ggtagtaata agtaagtctt 2580

gttgtgtttc agcatttaat aatagacttt ggaggtagac ccctggttta aatctaagtc 2640

tagtttgagg aagtcactta acctttattg aaaagactct ggatttaata agctgtgtaa 2700

ctggactcga tagttaccca aagttcagtc tagatggcac aaaccacctc tcagggaata 2760

aaccctaaga catcactcaa ggaggacttc aattatttaa ttttgaactg ttttgtcctc 2820

tctggccata aaacttgaca gtcatgaaag gtaaggcaaa ttttaagtgg gttaagtttt 2880

taaatacgta tctactcatt ttctttaaaa aaaaaagaat taaaactggg tattttaaac 2940

tcagcttaac cagataaacc tgctccagca tttgtccagt gttgaaataa ataggaagaa 3000

aaatacaatt tccttaaaca tgccagctta gtagtaggaa tgacctatca atcaatcaat 3060

caatcattca gcaagaagtt agttgtggtt ccccatggcc ccaaggtctc agggctggat 3120

tgtccacttt tcctcctcac caccagcact gctgctgcta ctgagaaagg cacagtggac 3180

tcatgtgtgt gtcatgactt taatggttag ctacagtatg catacacata caagaagtag 3240

gggagctgaa gcccaggaaa aggctacaaa atacaacaca gggaccaata catttgcaaa 3300

acattcaaaa tccttataaa aggggctgta ttggtccttt caactttgtc attattcagc 3360

ttagcctgta tggccattca ttcagtgggg taatggggga gaacaattaa ttaatgagat 3420

ttggttccct ttcctatatt cccactgttt ttaagtttca ggagaggatt ggggggtgag 3480

ggtagggggg ttgcggttcc tttaaaagct gacccccatc tcaggcggct gaggtgtata 3540

atccccaaaa gaaataagat ggtacaaatg cagtcagctt tggaatacgt ttgggtagag 3600

acaaaatgga aactcatggg attccaatag tgagcccttc ccatgttcct ggacatatcc 3660

aattctagca catccacatt tttaataact tagtgatttt caaggtcccc tgtccacatt 3720

aaaaaaataa gttacataat attcaaactg gctttttgct attctttggg acaccaggaa 3780

tgtcagaaga catggagcta tgccctcaga caagtgcatg gcatcagctg cctcttcatt 3840

acaaggtacc agtttatgta cttgccttgg acacagcttg cacaaagcca agaagttcca 3900

gacaaaggtt ttctctttca tgtatttaca caagttcaaa atgatattca cagcatcttc 3960

taaattttgg ccaagagtca aaaaaatgca tttaaacttt ggaacgtgcc cacataagac 4020

aggaggctga tcccaacagt agttggggca gatacccaca aaccaaaggg ctgggaaagt 4080

caggaagagc tgaaaggatt cttcagtcag tttatgaact cggtgcagtg agacctctag 4140

actgacacgt acaacagaga tgcagtttcg tctaactggc acctgtccct tccattactt 4200

gctgggcctg cttctgtccc atgcagcact gtgcgaccga ctggaataac ttttcgattt 4260

ctggggcaca gtgtacaaat tcatggttcc agctaactgt tatcagcccc aaccagagac 4320

agatggtttt cagtctccag tctcattcct aaatttttcg atttctgggg cacagtgtac 4380

aaattcatgg ttccagaact taaacgctgg gtttttaatc agctcaatga aggtaataag 4440

aagaccccaa ggatgtggcc tatttacaat caaccgttcc aagagaactc ttgtgatctg 4500

ttcttggatg gcttccgtat tggcctctgc aaaaagttcc ttgtgatctg ttcttggatg 4560

gcttccgtat tggcctctgc aaaaaggtac agcatggtgc aactgaagta gtgagtgtgg 4620

ctatttgggt accggagctg atttgcaatt gcattcaaaa agagatagcg accctcagtg 4680

tccaagtcca cagccaaatt ctggaagata tccacctcag tgtccaagtc cacagccaaa 4740

ttctggaaga tatccatgtg tgctgagtga gtgatggtgc tcattgaagg tgtgctgccc 4800

ttgttgtgga tgtgcgcaat ggcctgagtc ccgacataga gcaccagtgc attgatgagc 4860

tggaggttgt agcgattccc aggttcattg gatacctgta ggttgctgcg cagatcagac 4920

aggaaagtga ctggtgatcg gtaggttgct gcgcagatca gacaggaaag tgactggtga 4980

tcgagtttta agataggaat ccaaatcctt tttgaactga ggtggcatta ctccagtgaa 5040

attggtgaga atccggggag caatgttaat ttcactcaac atgtccacct ttagattagg 5100

agtgaatggg tcggggagcc tcatgtttct tctttagatt aggagtgaat gggtcgggga 5160

gcctcatgtt tcttggaaag gcactcagga tcaaatttct taactggata caattaggtg 5220

ggatcacatc acagaaccca taatggtaat cacaaaggaa ctctgggaaa tcatgcaaaa 5280

gaaccagcag cactcttaaa gtgcccttgt agaggatttg cataggtttg gtgagttcca 5340

catttctaag gcttgtagag gatttgcata ggtttggtga gttccacatt tctaaggaaa 5400

ggcgctaaat atttgaataa atcaatcagt agctgtgcat acataggcca ccccttctgc 5460

tgtggcgtat gtgccagcat tcttgcaata aatatccgct agaatccccc caaacaaaac 5520

ttcatttaag cttttttagc agtcacattt cttctgattg agggcaaaaa ggcttctgct 5580

gtggcgtatg tgccagcatt cttgcaataa atatccgatg ggaaatcagt tcaagccagg 5640

catatacaaa gccaggagct ttggtaggcc tcaagatgtg gaatgtattg cagaaagctg 5700

taagtgtctg gaaattaatg gtttccaaca catgctcaca gaaagctgta agtgtctgga 5760

aattaatggt ttccaacaca tgctcaggtg cattgagttc caagagaagc atgataaaaa 5820

ttcgatggta gggaagttgc tgaaattcac tctgacgaac atcatgatcc tgaaggagaa 5880

ctcccactac tataccaagg accttgttca gcagattaat ctttgtgaca gtgttggtgg 5940

cctccccctt gttcagcaga ttaatctttg tgacagtgtt ggtggcctcc cctgagtgtt 6000

tcacgagcag tgcaatgagt cgaacaaagg catccaggtt gtgatagcac ttggctcgga 6060

tcatggtggg attggcagca ggattgtgct gctgctcagc ctgagcacgg taactgattt 6120

caacacacat ttcagtacac agacgaaaga accttgttat gagatcatcg gtcttcagta 6180
ttccttgctg gtgcatctgt ccaacaaatg cagagaaagc tttggtactg tcgcggagct 6240
agctctacct gtccaacaaa tgcagagaaa gctttggtac tgtcgcggcc agctgctgct 6300
gaatggtaga gattcaccca ttccctcaga agatactctg ccttctccct caggcctgga 6360
gggtcatcat actctgaggc ttgagagatc ccagaatgca tcataaagtt tgggcctcca 6420
tgagcacgat caatcattgc ttcatagttg gatcgcacta cttccatcag ctggggcaat 6480
ccttctggag cattgcctct ggaatgagca ttaatcctca tgagcttctg gagcattgcc 6540
tctggaatga gcattaatcc tcatgagggt ttcaatggtg tggaacagat ctgcctcagt 6600
aacatgagca acactccttt catccaccag caggattttt actaactgca tagcaaatgc 6660
cacagccatg tagtttaagc cattctccat tgactgcgct aggtgaagat catactgctg 6720
catattaacc aaatgattgg ctcgctaggt gaagatcata ctgctgcata ttaaccaaat 6780
gattgcgaat tagcagctcc acagcctcca cattatattt atattcatct cgacattcaa 6840
ttaggcacct tgtgatctgt ttgttgcacc atggagaccc atatgcccgg ttgtgatctg 6900
tttgttgcac catggagacc catatgcccg gccatcctgc agagctttta ggaccaagag 6960
gtggcattcc ctgtagcgca gcagaaggtc agcatcagca ccacttgtgg catctagtaa 7020
gccctctaca gccttttgga gcaatccaag agcagctatg gcatcccgag agtttcgagt 7080
ttggagcaat ccaagagcag ctatggcatc ccgagagttt cgagataaaa ctacaacctc 7140
caagagactt cgaagagcct gagcttgagg gttcatggcc aaagttggtg ggatggcatg 7200
tagatgttgc tccagttctg taatacactt atcataaatc tgagctacat catctgttgc 7260
ccaagcttgc ttcatgggct gggctaaaaa tcccgtgggc tgacttaagt aagtgttatg 7320
gtcagaaaga tgaggaaaaa ctgagttacc tttaaaaaag aaagtgatct aaaacttcat 7380
gggctgggct aaaaatcccg tgggctgact taagtcattt gtaggtaaga agccaggaac 7440
attgcgtgca aactcttcta aacagccaac tgctttgggt ccacaccacc aactttcagc 7500
ctgatttgct ctggcatccg ttcagcttga tatgttaaaa ctcgcctgat ttgctctggc 7560
atccgttcag cttgatatgt taaaacaaca ggatcacagt atctgcgtcc ttcttgccta 7620
gcatgttttc tcagctcaaa ttcagttgct aatctcttgt ccatctcagg gcctgctttt 7680
tctgtctaat ctcttgtcca tctcagggcc tgctttttct actgcagtct tctgaataaa 7740
acagcaagcc aactcacaat tgtcctgagc taattgagca gctgcctgat ccatcatttc 7800
tctttgttgt ggggaagcag tacgaagggc tgaggcaaaa ctgtttttta agttggtaga 7860
tgaggatgca gtgagccaag actgcaccac tgtactctag cctaagcaca gagggcccta 7920
aaaaaaaaaa aaatataata tacctgattt aacatgatat ctgagactca aaataaaatt 7980
ttgaaacata ttttcttcaa attcatcctt cctggttaac agaaatgaga ttgcagatat 8040

cactttttta taatctaaaa tgatggaaga catcaaacaa atatggtgat tattaagaat 8100

aagtggtaag cccagtgcca tatatagaca aagaaaaaaa ttattacaga caagcagtaa 8160

tacgttttgt acctatacca aggaaatagc caacttacac gaagggctga ggcaaaactg 8220

ttttttaagt tggtagatat gctcatgagc aaaggttccc tgcatgtaat catagccatt 8280

ccagctgtca agttacgcat catgtgatga gctgctattc gcattcgaga ttcctccgaa 8340

tccagggcaa aatccttcct gactatttgc tcacaagtag tcatggcaat cttaattgat 8400

cgatccacca caggatggac cagctcctgg acagcccgtt caattgcctg acgcacacac 8460

tgcttcaact gtggatgggc ctgaaacaag ggaattgttg gattagagta atgtgtggtg 8520

ccaagcccgc aagggaagtt ggattagagt aatgtgtggt gccaagcccg caagggaata 8580

gacattgatg tcgtggtagc tgtactgtgg ctgtggtgga accgtggctg tacaagtggt 8640

gttggtagct ggtgtagtag aagttgttgt ggttgtgatg ggagggagtt cttctggctg 8700

cttgacatct ttcgttgtga tgggagggag ttcttctggc tgcttgacat ctttctttgg 8760

agcagagagt tgctcatcta aattcttcag gcgatcttta tcctttagga ggtttccagg 8820

ttttagctca ttgatgtcta atgcaaggtt cttgcagaga acctcgattt caaacttcaa 8880

gtttaacttt aagtcatgct cctgatgtag ctcagctaat acattcataa ttgctttaag 8940

tcatgctcct gatgtagctc agctaataca ttcataattg ccattgtcca agggtttggt 9000

ggcctaaaaa ccacactacg aatgctagat tctaagactt tggcaacaaa gggcctacga 9060

atgctagatt ctaagacttt ggcaacaaag ggcactacat agagcaattc ttgttgtcct 9120

ttaacataag cctctagcag caatgatttc acatccaagt cagtgtgtaa gatgggtttg 9180

tttttagcta atgtgatcat tcctagtaag atgggtttgt ttttagctaa tgtgatcatt 9240

cctagccaat gtcccaagtt cttcagcaaa gaacgatctg agaaattggc tgcagcttta 9300

tcagaggtca ggagcacttt aatgtttctg taggtctcat tcagaaccat cttgttaaat 9360

tcaggtgttt ctgtaggtct cattcagaac catcttgtta aattcaggat tcttcagcgt 9420

gtcaaggaag tttgaataca ggctatgaaa gtttggctca atactgactc tcttcataac 9480

cagatactgt gaaacccaag gcataaattc ttctttcacc gtttccttta gctcttcaac 9540

cttttgtgtc atatttgact gtgagagatt attgaaaata aaagttgtgt catatttgac 9600

tgtgagagat tattgaaaat aaaagcaatt ttctcctgga tattttctgg gggctccaca 9660

attctctcag tttgatctgt ggccacaagc aacgtatcta tatttgtagt attaatagaa 9720

ggtggcacat ctttcttaaa gctgactcca gttggcctgg tgaccgcaca tctttcttaa 9780

agctgactcc agttggcctg gtgaccgtaa ccgttttagc aacagtggta gttgttgagg 9840

tggttaccat agtgctaact tgaccagcaa gaggagcttt tgctggaacc tgggcctgag 9900

cctgggcctg agccagtgca atacttccag gggttgtgat agagccttgc attttcacag 9960

gaggatctct agactgctgt ccatactcaa tatactcctg taaatgatgt ggaaattgca 10020

taaagtgact gatagaagct gtaaatgatg tggaaattgc ataaagtgac tgatagaagc 10080

caaatgtgac aatactgggg atagtccttc aatctgtttt taaatctatc tagtgcagca 10140

atcccgaaat aatacatttt gtttttaaat ctatctagtg cagcaatccc gaaataatac 10200

attttggatc caaaaggctt gcgtaaggct tcaagaacat atcgtagagc cagacctagt 10260

gccatgtaag tgaccagtcc tttctcaatt ataccaccaa ataggcaggc tgttatatgt 10320

aactctttat caggatactg gggaaaaaaa cgatattctt caaacaagtt ccttagcata 10380

cagttaaata cttctcgttc cctctttata gtagagtctt taaatctctg cagcatttct 10440

aatacctcat caacagacat ggttggatgt ggtggatgat tatatattcg atcaacagac 10500

atggttggat gtggtggatg attatatatt cgctggaaat agctgtttgc ttcatcatct 10560

atctctttac taaagtgctg gtttgcctct ggccacacct gagacaagtc cgtcttactc 10620

tgctggaatg taggctgatt cagtccagag gtgcccagtt tcctctgtac aaaagggtcg 10680

ttattcactg cagggagtcc aagagcacca gttcctatac cagtcaggct gcctgtgcca 10740

agaccaccta ctggaagctg tgatgaaagt cctccaatac cactgaagca gctggaagct 10800

gtgatgaaag tcctccaata ccactgaatg cagtggtctg attgggggtt gaaaggggtg 10860

gaaatgcttt tgctggtgac tgaggggtac tgaatgcaga acccaaattt ggaggaaaac 10920

cctgcatact ctgggtgtga ggggcagctg aaccacctat actaagagat gtcattccag 10980

caagagggtc aatctgaaca ggagaaatgg catctaagct gctagcacta ggaggacgtc 11040

aacaggagaa atggcatcta agctgctagc actaggagga cgtccttttg gcataactcc 11100

aggtggtggt tgtctggcct tattcataac attactgcaa ttggctacca tggtgaggat 11160

agtttctgat agctcctgag aaacactccc tgcacaagct tgcagacagg ccaacattgt 11220

cgccaaagtt tcttgtcgcc aaagtttctg gaggaagttg agcacttttg ggctggtctt 11280

tttctggggc aagtccgccc aaaatagaag gacaccgtct ctttaaaaaa gtcatacacg 11340

cctggataaa aggctcccca tgctctcgaa ttttatctgt gagccactta tcaagtttga 11400

ggccatctcg aattttatct gtgagccact tatcaagttt gaggtattca cgacgtgaag 11460

caagtgcagc aaggtcaata acaaaggcaa atggagtacc atttagcagc attgacaagg 11520

ccttcaagtc ctgggccaca tcaagtattc gagacaattt ggccttcttc aagtcctggg 11580

ccacatcaag tattcgagac aatttggcct gatcatactg ctcccctctc atgtaccatt 11640

ctgccattgc atgcatgata agttggcgaa ttgagggaga ctgtccctgc ccatgccatg 11700

catagtgcaa aataatagct gagttaggat ggtccagcca tgcatagtgc aaaataatag 11760

ctgagttagg atggtttcca aggaaaattg gcatcagagt ggagataagt tcatggcgca 11820

aggtatgcca agaggtgtta atttgtagta aggccaatac cagcatgtct ggacagtgtt 11880

tgatagggaa gctgaagagc tgtttgactt gctcatactg cccaacctct gcaagcctca 11940

gcagagattc aatcaaatcc aagctcttcc atgtggcaat ttctcgattg tcatcctctg 12000

gtggtgcttt cagaggcaat ttctcgattg tcatcctctg gtggtgcttt cagaatatca 12060

gtggcaacag tatgacaggg atagtcagca aaacagaaga tctctggatt tataagggaa 12120

tgttgaatga aggagagctg gccttcagca tgtttccaag gtctatatat gaggtctact 12180

ctggccttca gcatgtttcc aaggtctata tatgaggtct actgggaaca cttccatacc 12240

caaacccctc tgaatgccat aaaccacatt atgaagtcct ttactgtcac gaatttgaaa 12300

tccaggatgg tccagttcat aagttacttc cttgaaattc aaacttggat tcagttcttt 12360

aagaacgtca atcaagactt ctacattcca tgtgtggttc tttaagaacg tcaatcaaga 12420

cttctacatt ccatgtgtgt gcctgtgctc catcactttt atctttccca tcactccaga 12480

tcccactgcc cggagcagaa atactctgta atggaatgcc atctgttaac ctgaatgagt 12540

tcgagctaat ggaatgccat ctgttaacct gaatgagttc gagccatcat tcccaaaacc 12600

cttgcaacct gggcagctgt gacctcccga acaccaaact gcacgattat attgcgacat 12660

tcttcaatac ttgcacaaaa gccatagcct acttcttgca tgaaacacaa aagccatagc 12720

ctacttcttg catgaaatca gccaaagagc tctccatcat ggttttagct acccctccgg 12780

aatcaggcag gatcctgtcc attagaatgt cccgtttttc agggtataaa agtggtgcga 12840

gcaccacggg acagcgttct tggggaaaat ctctgcgcag cgtcttaaga aaagcgtcta 12900

tctgttcttg tcctgcgcag cgtcttaaga aaagcgtcta tctgttcttg tccaactcca 12960

aaggctccct tctgcccaaa gaggagatgg gagaggagga ggtgtaggac ctctattgct 13020

atatcttgga agccaccttc ttgatttcca ctgacgtctg cgtcaatgta agaacgcaga 13080

agatctggaa gcttctgttt gataaactgg gcagcgaaac ctctaagatc tgagctggaa 13140

gaattcaaca gggcaaggcc aaaacctaag atctgagctg gaagaattca acagggcaag 13200

gccaaaaatt acctcttgta ctttgcttaa tttgagcact ttactcagct gggatcttgt 13260

actttgctta atttgagcac tttactcagc tgggcaaata agtggggtgc aggctttaaa 13320

ctcttctgat agtgcaatgg attatcaatg gcataggaca gcgtcgagtt ctgatagtgc 13380

aatggattat caatggcata ggacagcgtc gagataaaat ttggctttgt aatcagcaac 13440

gcacactcct gaatcagaaa ctgagtctga tggaaatctt tgccactgct tttaccatcg 13500

ccactgaaat ccaggaaatc tttgccactg cttttaccat cgccactgaa atccacatgc 13560

gaaaataggc agcgtaataa atgcctgtct gcctcaggac cgtgccgatt cacaatatgc 13620

tgtatttcct gctggctggc tcggtaattt ttcttggtta aatttctgct ggctggctcg 13680

gtaatttttc ttggttaaat tgtccaccag gtagctgatt tgagacaagg ccagcgagag 13740

cgagtcaaga ttcattgctg gttggggcgg aagcaggcgg ccgagcccgg cgcaaaatca 13800

ccattattcc cctttagtca cctcagaggc aggttaatgc tttctttgta attaggctat 13860

atctggtatc tgtataatat cttcagttct tctttaccag gggtcttact ctgttctgaa 13920

acatggcacc tgggaggaga atcacttgaa cctgggaggc ggacgttgca gtgagccgag 13980

atcacaccac tgctctcaag cgggcaac 14008

<210> 36
<211> 1480
<212> DNA
<213> Homo sapiens

<400> 36
catttaatag taacatgctt acagtggctc aaagctgaat gtgacaaatg tactccagga 60

aaagtttatc attctaatcc taaacttact tttttggggg gggggttttg cccacgctgg 120

tcttgaactc ctgcaagtct ccccccctaa taaaaacatt tagtggttcc aatttaattt 180

aataaagcct acgacaaaag aaaaattttt aattttaaaa aaaatctaag aaaaaacttt 240

tttcaatgtt atttttaagc cggaagtctt aaacaccttt acttcgaaat actgttgatg 300

atctgatttt caatactgta ttgggtgcca ttaaagagac cattgttctt tgaatcagtc 360

tgtgttgctg tgggaaacac ggtaaggcag aagcactaga atcatggagg caaattactg 420

aaacactgtg atactgaaat actgctggct tctggaataa tcttctgaca tgtggaatta 480

gacatctgaa gtgtcgctcc aatcagtcac agttactaag ctgcttttta atgtgcttga 540

ttcattttct tgaagtcctt ctccctttgg ccccttagga ttttcctaaa tgcgccccag 600

gcatttagca catgagcaaa atgtggttca tttttcgcag gtggaggttc cttctgttct 660

ttcccagatt tttttccaaa gatatctctt cctctaggga tgatatgtcc cagtcttcat 720

cctccacatc cgcacacttt agttcttgat ttttttttaa tttttaggtg ttccaccgac 780

tggtttgttc ttttgcgatg tggaaacatc ttttcaactt tttcagtagg tgttttaacg 840

gcgactcctg cgggcttgga gaatcatcag tgtcctcgat ttcgccttcg cttccctcgg 900

tcccgtccgc gtcacttttc actgtgttct tcccgaagct gcccttgttt tgtggtggcg 960

gcacaggaag tgggcctggg gatgcgtatg cccggatgag gtcgtcgtcc tcccctcctc 1020

tgaaaaaagg aggggtctat tttcttaatt tttggaacag atgtcctatc aggaaacagc 1080

tttttgtcta atcagttgtc tgtttttgga aggaagttgt atcattttgg gtacttctcc 1140

agttgaaagg gtatccattt gagaaacact gcacgatgaa ttctcctcaa ttttcagctg 1200

acttgatgtt caaggaattc tcgaatttga tgaaagttag gtatttctct ttcttgctta 1260

tgtctttcga ttccacactt tttagtactc tatgcaactc taatgccacg tatatctgca 1320

ttaatcccca aggtttctgt ccaccattgt tcttagtccc ttagtgaggg aggagttact 1380

cttcaaagct tccttaaatg cattttgttg ttatttaatt tctgttcatt ttcccttcct 1440

cctctgaaag ttcttctatt ggttccagta tggacgagaa 1480

<210> 37
<211> 1637
<212> DNA
<213> Homo sapiens

<400> 37
ctttaatata ttaattctgt ttcaatttaa aatgcaaagc ccccaaatta aaacattcat 60

aaaacaagtt aacttaaaaa atacataaga aacttttttc aatcacaggt atttttttta 120

attctcagac tgtgctagag aatacgtacc atgaaataca tatatttcat aaggttcagt 180

tacaaaatgg attgtttcaa atggcaattt cttacactaa cctgattatg aaaaaaagaa 240

gtctgtatca tctgcttcca agtctgttat gtccaaatat attttaatta tgcatttatt 300

ttgctacttt tataaatatt agagatttca ccttaaatta tttttgtaac tagttctaga 360

acatgttttc caattattat ttttctaatg gagacatata attgacctat gtttatgcat 420

atatgttctc tacacagtga aacttttttt aaaaagaata gtaaagaaaa tgcggaagct 480

ctggctctcc aaggcaaagt caaaaaaaaa aaaaaaaggg ggggaaggag gaacatttta 540

ttacacctcc tgattttcac tccttgagtt ttattttctc cccttgttta tttgtttaat 600

gctagaaact gtattcctaa gagagcatac ctctttcagg tgagcatgat aattggaaga 660

ttttgatact tagagtatga acatataatt aagaagtgat tacctaataa agataacaat 720

gtgactattt taattatttt ggtggcaggg agttggtttt acatcaccca aaaaaaaaaa 780

aaaccgttaa ttattggtaa taaatatgct aactttctga atcaaaatgg agagcctctc 840

aagaaaaaga gctatgcagt cagcaatgac ttaaattagt caggatagca ggcatctggg 900

gttaaggctg tttccaccat tttggtctca ccaccatata cgagtgggac cacagctgtg 960

tagcacttgt ttctgtcata agtgtagcag gtctctgtag cactgtcttc atcacagata 1020

ttgctctggg tagcagtaac tatctgatta tccagctcca cttctgtagg atcacatttt 1080

ttacagaggt cagacaaatg gtacacaaat ctggttctca atggtgaggg tcagacaaat 1140

ggtacacaaa tctggttctc aatggtgagg tgggatcaga gatattctcc ctgttgttca 1200

gaggaacaat aattcggatg tttctctcca caatgtcctc attaggatct tcggaataat 1260

tcggatgttt ctctccacaa tgtcctcatt aggatcttcg gaagaacgga tgatcctgga 1320

agtaatccgg gcacacttac atttgttgtc aacaagaaca atcctttcat cttcttgggc 1380

tttcacatga acagccttaa taaaaaccgc caggactccc cagaaaactt tcacatgaac 1440

agccttaata aaaaccgcca ggactcccca gaaaagcaaa tggttcttca tcttgacttc 1500

acttcttctg aaaaactgga gcaaaaaaaa gggttaaggt gtgtgatcta taaggagtgt 1560

gcaaatgatg gtgcttctgc tttcttaaaa taacactcca ggcagctaac aaactaaaaa 1620

caatcacaac tgtggtt 1637

<210> 38
<211> 4795
<212> DNA
<213> Homo sapiens

<400> 38
gggagccgga ccgggaccag gaccgggccc cactttccaa tctgctgatt tgggtcctac 60

tttaatgaca agacctggac gaggggaatc caccattgct gaggcttgag tagcaagccc 120

cagggagagc agtggctcag catggtgttt gagctctgag aactgcatgg ctccagcaaa 180

ctccaacaga cctgcagctg agggacgtga tcagagcacc ccaaagcagc tccttgccag 240

caacggaaca aagctggaga atgactttga gaagacctta aagcctgatg gagctgaaaa 300

ccacttcgta tgcacgccaa gcttcaatag cagtaattga agatcaaatt gatgaaataa 360

atggagttgg gtcctacttt aatgacaaga cctggacaac cagcacttac cagagtgccc 420

ccacctattc ttccaaggcc atcacagcag acaggaagta gcagtgtgaa cacttttaga 480

cctgcttaca gttcattttc ttctggatat ggtgcctatg gaaattcatt ttatggaggc 540

tatagtcctt atagttatgg atataatggg ctgggctaca accgcctccg tgtagatgat 600

cttccaccca gtagatttgt tcagcaagct gaagaaagca gcaggggtgc atttcagtcc 660

attgaaagta ttgtgcatgc atttgcctct gtcagtatga tgatggatgc taccttttca 720

gctgtctata acagtttcag ggctgtattg gatgtagcaa atcacttttc ccgattgaaa 780

atacacttta caaaagtgtt ttcagctttt gcattggtta ggactatacg gtatctttac 840

agacggctac agcggatgtt aggtttaaga agaggctctg agaatgaaga cctctgggca 900

gagagtgaag gaactgtggc atgccttggt gctgaggacc gagcagctac ctcagcaaaa 960

tcttggccaa tattcttgtt ctttgctgtt atccttggtg gtccttacct catttggaaa 1020

ctattgtcta ctcacagtga tgaagtaaca gacagcatca actgggcaag tggtgaggat 1080

gaccatgtag ttgccagaga caactgggca agtggtgagg atgaccatgt agttgccaga 1140

gcagaatatg attttgctgc cgtatctgaa gaagaaattt ctttccgggc tggtgatatg 1200

ctgaacttag ctctcaaaga acaacaaccc aaagtgcgtg gttggcttct ggctagcctt 1260

gatggcaaca acaacccaaa gtgcgtggtt ggcttctggc tagccttgat ggccaaacaa 1320

caggacttat acctgcgaat tatgtcaaaa ttcttggcaa aagaaaaggt aggaaaacgg 1380

tggaatcaag taaagtttcc aagcagcaac aatcttttac caacccaaca ctaactaaag 1440

gagccacggt tgctgattct ttggatgaac aggaagctgc ctttgaatct gtttttgttg 1500

aaactaataa ggttccagtt gcacctgatt ccattgggaa agatggagaa aagcaagatc 1560
tttgatatct ttcatgtttg cctgcagttg aacaatactt tagagtactt tttaaaatta 1620
tttctcacaa agaaatgaat gtacaatcca atgaaaacat ttgttattgg ctatttcagg 1680
tgttttgctg ctagaaatta ttaaagttac acactagtat gttggtctgg tgacctggtt 1740
acattttatt atacacatta ttggaccata aggacatttg tttcacctag attttaagat 1800
tatggagact gctgtcattt ttatcttatt taaatctcta ggtttattgg aagagtaaga 1860
ttgatgaact atagcatgca cagtttggta cagtagagat cattaatact tttaaaagtt 1920
ctacattaat tgacttggaa tccttagaaa tggagtggtg acatgtcagt atgagaacag 1980
gcaaaaggta aatttttttt tttttttaaa aataaaattt tctgatctgt attatatcca 2040
gtgttgggtt tatattttca cccacaaaca actgacactg ctatctttta ctgtattttt 2100
aaaaatttaa tttgaaaagg tcacccagaa gcattctgaa ggaaatggtt ctagaattat 2160
agagtatgta gccctaatag ttttccctcc tagcaaaaag tctgaaattt atctttctta 2220
caaactgttc catttcttct aaggatccct taattattta tctctttaag ccaggcatgg 2280
tggctcacgc ctgtaatccc agcactttgg gaggctgagg cgggcagatc acctgaggtt 2340
gggagttcaa gaccaccctg accaacatgg agaaacccca tctttacgaa aaatacaaaa 2400
ttagctggtg tggtggcaca tgcctgtatc ccagctactc gggaggctga ggcaggagaa 2460
ttgcttgaac cccaggaggc ggaggttcgc tgagcggaga tctcgccatt gcacttcagc 2520
ctgggcaaca aaagcgaaac tccatctcaa aaaataaaat tatttatttc tttaaaatat 2580
gactagtttt catactgggt gaatcagaag tatatgaaag gtatactttc ttttcctaca 2640
acaaatactt gagttctttt gagtttgcac ttaatgatat aatcagttat aatagtaatt 2700
tttaatattt tcatagattg tttgcttcta ccttgttaat tttttaaatt ccatatttag 2760
gatgctctgt aaatattgaa aatgtgtcac attggataca tttttctttt aggtttagtt 2820
tttactactg agacttattt atagtcttag tgctctattg ccatttagaa tatgataatc 2880
ctcatgcctt taatctcagc actttgggag gccaaggcgg gcggatcacc tgcggtcagg 2940
agtttgagac cagcctggcc aacacggtga aaccctgtct ctactaaaaa tacgaaaatt 3000
agctgggtgc agtggcgtgc acctgtagtc ccagctactc aggaggctga ggcaggagaa 3060
tcacttgaac ccgggaggca gggtgggagc tgagatcatg ccactgcact ccagcctagg 3120
ggacagagca agactctgtc tcaaaacaaa caaacaaaaa ataataatac gataatgcta 3180
tttgacgtgt tttttggttt ataatgattt taaatgcagt taactttcag tacactgaat 3240
atttccccag aaaattggaa acttcatata cttggctacg aacatactac agagtaatac 3300
tatcaggaat acaggtgtat aagaatacat ttatagatat tgttgaaaac tttgaactgt 3360
ttgataaaaa ttgtgattta gtattttttc ttttgtcttt ttttgaaacg gagtctcact 3420

ctgtcgccca ggctggagtc cagtggcgcg atctcggctc actgcaagct ccgcctcccg 3480

ggttcacgcc tcagcctccc gagtgctggg actacaggcg cctgccacca ggcccgattt 3540

tttttttttg tagacggggt ttcaccgtgt tcccaggatg gtctccatct cctgacctcg 3600

tgatgagccc gcttcggtct cccaagtgtt gggattacag gcgtgagcca ccgcacccgc 3660

gatttagtat ttttttctaa tagactatgt tcaacaaata agtaattctc gaatagttca 3720

gattaaaaca tacaggaacc aagtacatac ccagcataga agaactttac taaaggcttc 3780

ttggaaagcc cttttttgaa acgacagtat cgtaagtaac atatcattta taatagaaat 3840

cttgacccag tgcagaaaaa taaatatagt aaaatttatt tatctttggc cagttttcca 3900

acacccaggt attagccttg aagttgaaga gataaggttt cttgtatatt atttttcatt 3960

tgtgttctac aattaaaggt tctgctttga tttgtcagat aatttataga aattttgttc 4020

tcaaaacaaa tgtttgataa aacagattat taaatttggg gttgagatgt ctaaattgaa 4080

tgctagaagt aaagtagaag tgaccactat taaagatgta tagggagaag agtacagcac 4140

aaagtgataa aatagtgaca cttttgtagg ggtgtttatt gtttggttaa gtctgctaaa 4200

ttacggtatg cattatctgg tgactatttg tgcctgaaaa ttcgttttgt attaaaattc 4260

tggagaagga attcaggact agaataaaga gaaattttgt aacctttttt atcatgacag 4320

ttttagaata atttttttag ctgagattaa atgttcaagg ctccaatatt atttttagga 4380

acttatttaa ggagtgctac tttacagaaa ttactaacac accaattaat ataaaatata 4440

agtttacaat aataaaacat gtttctttta atttttctga ttatatttta tgagttcaga 4500

aaggaaatgg taaaagaact atacattttc atgttttaac attttatgta cgtacttgat 4560

tctgtctgtg tcataattac acatttactt gaacacagct atcctttatc ttgtgctttc 4620

tttaatagaa aaatgaacag aaactgaatg cagttaaatt tttattttta gtaggttgtg 4680

aagttacttt tactggagaa tatgttaaac ttgagcctct caatgggatt cagccatacc 4740

ggccgaaagc ttttctcttt tatagctaat ttaaatctgc attagcgggg ccggt 4795

<210> 39
<211> 4400
<212> DNA
<213> Homo sapiens

<400> 39
agccggaccg ggaccaggac cgggccccac tttccaatct gctgatttgg gtcctacttt 60

aatgacaaga cctggcaact gggtcctact ttaatgacaa gacctggaca accagcactt 120

accagagtgc cccccaccta tcttccaagg ccatcacagc agacaggaag tagcagtgtg 180

aacactttta gacctgctta cagttcattt tcttctggat atggtgccta tggaaattca 240

ttttatggag gctatagtcc ttatagttat ggatataatg ggctgggcta caaccgcctc 300

cgtgtagatg atcttccacc cagtagattt gttcagcaag ctgaagaaag cagcaggggt 360
gcatttcagt ccattgaaag tattgtgcat gcatttgcct ctgtcagtat gatgatggat 420
gctacctttt cagctgtcta taacagtttc agggctgtat tggatgtagc aaatcacttt 480
tcccgattga aaatacactt tacaaaagtg ttttcagctt ttgcattggt taggactata 540
cggtatcttt acagacggct acagcggatg ttaggtttaa gaagaggctc tgagaatgaa 600
gacctctggg cagagagtga aggaactgtg gcatgccttg gtgctgagga ccgagcagct 660
acctcagcaa aatcttggcc aatattcttg ttctttgctg ttatccttgg tggtccttac 720
ctcatttgga aactattgtc tactcacagt gatgaagtaa cagacagcat caactgggca 780
agtggtgagg atgaccatgt agttgccaga gacaacggca agtggtgagg atgaccatgt 840
agttgccaga gcagaatatg attttgctgc cgtatctgaa gaagaaattt ctttccgggc 900
tggtgatatg ctgaacttag ctctcaaaga acaacaaccc aaagtgcgtg gttggcttct 960
ggctagcctt gatggccaac aacaacccaa agtgcgtggt tggcttctgg ctagccttga 1020
tggccaaaca acaggactta tacctgcgaa ttatgtcaaa attcttggca aagaaaaagg 1080
taggaaaacg gtggaatcaa gtaaagtttc caagcagcaa caatctttta ccaacccaac 1140
actaactaaa ggagccacgg ttgctgattc tttggatgaa caggaagctg cctttgaatc 1200
tgtttttgtt gaaactaata aggttccagt tgcacctgat tccattggga aagatggaga 1260
aaagcaagat ctttgatatc tttcatgttt gcctgcagtt gaacaatact ttagagtact 1320
ttttaaaatt atttctcaca aagaaatgaa tgtacaatcc aatgaaaaca tttgttattg 1380
gctatttcag gtgttttgct gctagaaatt attaaagtta cacactagta tgttggtctg 1440
gtgacctggt tacattttat tatacacatt attggaccat aaggacattt gtttcaccta 1500
gattttaaga ttatggagac tgctgtcatt tttatcttat ttaaatctct aggtttattg 1560
gaagagtaag attgatgaac tatagcatgc acagtttggt acagtagaga tcattaatac 1620
ttttaaaagt tctacattaa ttgacttgga atccttagaa atggagtggt gacatgtcag 1680
tatgagaaca ggcaaaaggt aaattttttt tttttttaaa taaaattttc tgatctgtat 1740
tatatccagt gttgggttta tattttcacc cacaaacaac tgacactgct atcttttact 1800
gtatttttaa aaatttaatt tgaaaaggtc acccagaagc attctgaagg aaatggttct 1860
agaattatag agtatgtagc cctaatagtt ttccctccta gcaaaaagtc tgaaatttat 1920
ctttcttaca aactgttcca tttcttctaa ggatccctta attatttatc tctttaagcc 1980
ggcatggtgg ctcacgcctg taatcccagc actttgggag gctgaggcgg gcagacacct 2040
gaggttggga gttcaagacc accctgacca acatggagaa accccatctt tacgaaaaat 2100
caaaattagc tggtgtggtg gcacatgcct gtatcccagc tactcgggag gctgaggcag 2160

gagaattgct tgaaccccag gaggcggagg ttcgctgagc ggagatctcg ccattgcact 2220
tcagcctggg caacaaaagc gaaactccat ctcaaaaaat aaaattattt atttctttaa 2280
aatatgacta gttttcatac tgggtgaatc agaagtatat gaaaggtata ctttcttttc 2340
ctacaacaaa tacttgagtt cttttgagtt tgcacttaat gatataatca gttataatag 2400
taatttttaa tattttcata gattgtttgc ttctaccttg ttaatttttt aaattccata 2460
tttaggatgc tctgtaaata ttgaaaatgt gtcacattgg atacattttt cttttaggtt 2520
tagtttttac tactgagact tatttatagt cttagtgctc tattgccatt tagaatatga 2580
taatcctcat gcctttaatc tcagcacttt gggaggccaa ggcgggcgga tcacctgcgg 2640
tcaggagttt gagaccagcc tggccaacac ggtgaaaccc tgtctctact aaaaatacga 2700
aaattagctg ggtgcagtgg cgtgcacctg tgtcccagct actcaggagg ctgaggcagg 2760
agaatcactt gaacccggga ggcagaggtt gcagggagct gagatcatgc cactgcactc 2820
cagcctaggg gacagagcaa gactctgtct caaaacaaac aaacaaaaaa taataatacg 2880
ataatgctat ttgacgtgtt ttttggttta taatgatttt aaatgcagtt aactttcagt 2940
acactgaata tttccccaga aaattggaaa cttcatatac ttggctacga acatactaca 3000
gagtaatact atcaggaata caggtgtata agaatacatt tatagatatt gttgaaaact 3060
ttgaactgtt tgataaaaat tgtgatttag tattttttct tttgtctttt tttgaacgga 3120
gtctcactct gtcgcccagg ctggagtcca gtggcgcgat ctcggctcac tgcaagctcc 3180
gcctcccggg ttcacgcctt ctgggactca ggcgcctgcc accaggcccg gcaatttttt 3240
ttgtattttt gtgaacgggg tttcaccgtg ttcccaggat ggtctccatc tcctgacctc 3300
gtgatgagcc cgcttcggtc tcccaagtgt tgggattaca ggcgtgagcc accgcacccc 3360
gtgtattttt ttctaataga ctatgttcaa caaataagta attctcgaat agttcagatt 3420
aaaacataca ggaaccaagt acatacccag catagaagaa ctttactaaa ggcttcttgg 3480
aaagcccttt tttgaaacga cagtatcgta agtaacatat catttataat agaaatcttg 3540
acccagtgca gaaaaataaa tatagtaaaa tttatttatc tttggccagt tttccaacac 3600
ccaggtatta gccttgaagt tgaagagata aggtttcttg tatattattt ttcatttgtg 3660
ttctacaatt aaaggttctg ctttgatttg tcagataatt tatagaaatt ttgttctcaa 3720
aacaaatgtt tgataaaaca gattattaaa tttggggttg agatgtctaa attgaatgct 3780
agaagtaaag tagaagtgac cactattaaa gatgtatagg gagaagagta cagcacaaag 3840
tgataaaata gtgacacttt tgtaggggtg tttattgttt ggttaagtct gctaaattac 3900
ggtatgcatt atctggtgac tatttgtgcc tgaaaattcg ttttgtatta aaattctgga 3960
gaaggaattc aggactagaa taaagagaaa ttttgtaacc ttttttatca tgacagtttt 4020
agaataattt ttttagctga gattaaatgt tcaaggctcc aatattattt ttaggaactt 4080

atttaaggag tgctacttta cagaaattac taacacacta aaattaaaaa tttcttttaa 4140

tttttctgat tatattttat gagttcagaa aggaaatggt aaaagaacta tacattttca 4200

tgttttaaca ttttatgtac gtacttgatt ctgtctgtgc ataaacacag tttatcttgt 4260

gctttcttta atagaaaaat gaacagaaac tgaatgcagt taaattttta tttttagtag 4320

gttgtgaagt tacttttact ggaggttaaa ctgatttaag ataaattgtt aacacttttg 4380

atttaaagaa aaatggccag 4400

<210> 40
<211> 3032
<212> DNA
<213> Homo sapiens

<400> 40
accttttggc acaccttttt aatgaatctt tggtggcaca tgcctgtatc ccagctactc 60

gggaggctga ggcaggagaa ttgcttgaac cccaggaggc ggaggttcgc tgagcggaga 120

tctcgccatt gcacttcagc ctgggcaaca aaagcgaaac tccatctcaa aaaataaaat 180

tatttatttc tttaaaatat gactagtttt catactgggt gaatcagaag tatatgaaag 240

gtatactttc ttttcctaca acaaatactt gagttctttt gagtttgcac ttaatgatat 300

aatcagttat aatagtaatt tttaatattt tcatagattg tttgcttcta ccttgttaat 360

tttttaaatt ccatatttag gatgctctgt aaatattgaa aatgtgtcac attggataca 420

tttttctttt aggtttagtt tttactactg agacttattt atagtcttag tgctctattg 480

ccatttagaa tatgataatc ctcatgcctt taatctcagc actttgggag gccaaggcgg 540

gcggatcacc tgcggtcagg agtttgagac cagcctggcc aacacggtga aaccctgtct 600

ctactaaaaa tacgaaaatt agctgggtgc agtggcgtgc acctgtagtc ccagctactc 660

aggaggctga ggcaggagaa tcacttgaac ccgggaggca gaggttgcag ggagctgaga 720

tcatgccact gcactccagc ctaggggaca gagcaagact ctgtctcaaa acaaacaaac 780

aaaaaataat aatacgataa tgctatttga cgtgtttttt ggtttataat gattttaaat 840

gcagttaact ttcagtacac tgaatatttc cccagaaaat tggaaacttc atatacttgg 900

ctacgaacat actacagagt aatactatca ggaatacagg tgtataagaa tacatttata 960

gatattgttg aaaactttga actgtttgat aaaaattgtg atttagtatt ttttcttttg 1020

tctttttttg aaacggagtc tcactctgtc gcccaggctg gagtccagtg gcgcgatctc 1080

ggctcactgc agctccgcct cccgggttca cgccttctcc gcctcagcct cccgagtgct 1140

gggctcaggc gcctgccacc aggcccggct aatttttttt gtatttttgt agagacgggg 1200

ttcaccgtgt tcccaggatg gtctccatct cctgacctcg tgatggcccg cttcggtctc 1260

ccaagtgttg ggattacagg cgtgagccac cgcacccgcg atttagtatt tttttctaat 1320

agactatgtt caacaaataa gtaattctcg aatagttcag attaaaacat acaggaacca 1380

agtacatacc cagcatagaa gaactttact aaaggcttct tggaaagccc ttttttgaaa 1440

cgacagtatc gtaagtaaca tatcatttat aatagaaatc ttgacccagt gcagaaaaat 1500

aaatatagta aaatttattt atctttggcc agttttccaa cacccaggta ttagccttga 1560

agttgaagag ataaggtttc ttgtatatta tttttcattt gtgttctaca attaaaggtt 1620

ctgctttgat ttgtcagata atttatagaa attttgttct caaaacaaat gtttgataaa 1680

acagattatt aaatttgggg ttgagatgtc taaattgaat gctagaagta aagtagaagt 1740

gaccactatt aaagatgtat agggagaaga gtacagcaca aagtgataaa atagtgacac 1800

ttttgtaggg gtgtttattg tttggttaag tctgctaaat tacggtatgc attatctggt 1860

gactatttgt gcctgaaaat tcgttttgta ttaaaattct ggagaaggaa ttcaggacta 1920

gaataaagag aaattttgta acctttttta tcatgacagt tttagaataa tttttttagc 1980

tgagattaaa tgttcaaggc tccaatatta tttttaggaa cttatttaag gagtgctact 2040

ttacagaaat tactaacaca ccaaaacatt attaattaaa taaaactgat tatattttat 2100

gagttcagaa aggaaatggt aaaagaacta tacattttca tgttttaaca ttttatgtac 2160

gtacttgatt ctgtctgtgt cataattaca catttacttg aacacagcta tcctttatct 2220

tgtgctttct ttaatagaaa aatgaacaga aactgaatgc agttaaattt ttatttttag 2280

taggttgtga agttatttta gagcgagact ccatctaaaa agaaaaaaac aaaaaaaaaa 2340

aaaaaatacg taaaaaaaca cttcatacct agattttggg ggatctagat ttgtttagac 2400

atcactgctt actgtaagat ttaagtccct tatttatttt tcagtttcct aaattataac 2460

aataggcata gctgctcacc tgcctcacat gaaagtgctt ttttttgatg gcgagatctt 2520

ggctcactac ccgcgattct cctgcttcgc ttactgtgct gggattcaga cccgcagtgg 2580

tgtgatctca cctcactgca acctctacct cctgagctca aaccatcctc ccacctcagc 2640

ctcccaagtt gctgggacta aaggcgcgta ccaccacgct tggctaattt ttgtgttttt 2700

ttgggttctc agacgtgagc tcaggagatc tgcctgcctc tctgccccca aagtggtggg 2760

attacaggtg tgagccactg ctcccagcca agaaattctt tatatgtaga tactattttc 2820

ttgtcaagtt cagatgttgg aaataacttg ccatttgttc attcttgtct ttgttgtttt 2880

tcatataata gaaatcccct tcttttatgt ctttattttg ttttgttttg tttttgagat 2940

ggagtttccc tcttgttgcc caggctggag tgcagtcaca gtcttggaac tcaggcattg 3000

ccaccacacc tagctaattt tttttgtatt tt 3032

<210> 41
<211> 8101
<212> DNA

<213>  Homo sapiens

<400>  41
tctcaagact tggctatgtg catttcagtt catctttaaa ataagttcag gtatacaaat      60

gttacatacc tcaagtacaa acagcacaga aaatgcatat ggtctcactg aatgtttttc     120

attcattcac ttcttaagtt gattacattt ttatttttaa ccaagacagc cttactttaa     180

aaaaatactc tattttcagc acaaagtcct catacagttt taaaattaga tcttggcgca     240

taatattgag aaattattaa aaaccaaact tggtaattta acaaaattgt cacatgccag     300

gatttctgaa tcaactcaaa ttatttcctt agctgtaatg tcaaatctgt gttcatacag     360

ataaataaag catggggaag acaggtggtg aaaacgcagt aacgggaaag gttctcagat     420

gtacaggtct tattagagtt tgtggctgag tccagacttt tctctaaaag cacaacagca     480

aatctcatgt tatcataatt gcaagaaaga tctgaaagaa gcgagtggtc tgagcctgcc     540

ctgcttgggt ctgttttgca aagaagagga gggtggggag gggagctgca gtaagagcct     600

taaaaagatg tctcagaaac tggcacatca tgatctagac gagggcccac tatgtttgtt     660

tgttttttgc ttaatttaat tctcgtgagg aaagtgcgaa ttagaccaag ggtccgagct     720

tcttcttcca atcgtggggc tccatatccg ccacgggttg taggtctgtc ccaagtcgtc     780

agctggactg gaggtggagg gagcgtgagg agcaggggag ttttctgtgc ccatgaggcc     840

gatgcttgcc agcgtgtttg ctggagtggt gaagggaagg gcgctgctaa ggttgctgga     900

ccaaatggag ctgctgaatg gagtggtgga ccacaggccg ctggtgttac cgaggaccga     960

tgtggctgtg tgggtcgggg agccggaact ggctggccga gcgtggctgt gtgggtcggg    1020

gagccggaac tggctggcca ggaaggactg ggatctgtcg ctggcgactc ccaaaggtat    1080

gaagggccac tattaaactc gttccagctc ctctgtgagg cctgattgca cgatcgagat    1140

aatccgagtt tgctgaaaac ttcaccagta gattaaaaga gtttccaagg ggaagagagg    1200

cactataaaa gagtttccaa aggcggagaa cgaattgagg ggagtgaagt cagggctgct     1260

tgggttgctg acgggactcc acaaacccga gctgtctgag ccatcgctgt caacaggagc    1320

gtgaaagtga aatcacaaac aggagcactg cttcaactgg gggtactcat ttcaaatcta    1380

gaactcattt cctgggctct attgttgctt atctggaaaa gtcaaccaag ggactgctgg    1440

ccacaccccg aaattttcca ttcccccttt tttacgtgtg tctgcctggc tgagacaggc    1500

atgggtgccc cctttctcta tctggacccc ctggggaagt ctgagccatc gctgtcaaca    1560

ggagcgtgtg aaatgccaag gctgctggag aaacctgttt tgtttgaaga aactttagca    1620

aagccattcc cacctgggct cttgtcgtag ccagccgtga ctgcagcaaa agtggggcct    1680

gggctcttgt cgtagccagc cgtgactgca gcaaaagtgg ggttgccgtt cttgcccggg    1740

agcgaggctg cctttgtcaa cttgtgtttg cttccatttg gctgttttat tttagggtca    1800

ctgctggagc tgctgttgac gatgctgctg tagctgcccc gggccacagc tggagctgct 1860

gttgacgatg ctgctgtagc tgccccgggc cacaaagggg cagggggcag ctgggggaga 1920

cggggaagca ggtgtcggtg aggtctggcg ttgttttaag aaaatgtctg cgttgagggt 1980

ttgcagagaa agtttataaa gactatcagt agagctgcca ggtttgtgaa ctggaacgga 2040

atcccactcc ggtggagcgc caggtttgtg aactggaacg gaatcccact ccggtggagg 2100

agagtctttt tcaccctctg agctactggt gttgcctaat tcttgactat tcgggaggaa 2160

ttttgctagg gcaggtggtc tgttatccac taacttactt gtaccttttg ttttctgggc 2220

atttcgtgat ttggatccac ttgtcattgc atttgttttc tgggcatttc gtgatttgga 2280

tccacttgtc attgcagttg gaagaggaat cttgcttggg agatttctac gttgcttact 2340

ttccaaaggg ggagtatatg gtagttctaa tgaactgggt ttcacatctg ttgggatttc 2400

tttcttaata tttaagaggt ttcacatctg ttgggatttc tttcttaata tttaagagtt 2460

ttttgctttt tttttttttc atgtttttca ggcatggctt gttttccctt ttgcttttct 2520

gtatcctcct tgaggagcgg ttctgtgtca gggttggagg tctctgtggg caggagacct 2580

ctctttctcc ctcgtgtttt gttgtgtgag atcccttgag gagcggttct gtgtcagggt 2640

tggaggtctc tgtggtggtg gaggagctat catcatcagc caaagagtcc ttcagctcat 2700

cttcctgtgg ctttcccttt cccttcttct ccttttcctc ttgcttctta ggtggtttca 2760

ccttgcgctg aagaggtttt cctttccctt tgctttttgg caatggcgat ggaggctgct 2820

ctgtaaacac ttttttgctt tttggcaatg gcgatggagg ctgctctgta aacacttcta 2880

gggctgggga gtcatggtgg tcgaagtctt tgtccatggc ttctatgaga ctggtgatgt 2940

ccgagtcctc ggaactgtgc ctcgcgctgc tggcacgttc tgggtgggaa gggtgtgcga 3000

ggggggcggg agacagcctt tcaggctgcg ctcctggggc tgaggcactg gcggtggcag 3060

aggaggctga gggtgctgct ccagcgggct gtgggcatgg tgctggctgc cgtgctggct 3120

ctgctttgcc cctttggact ttctgcccgc tgtatgacct tgagtcactg tgttcgaatc 3180

taagacaagg gggcttgttt tgttagcaga ctgtgaagaa gctgcttgag cagaagtcct 3240

actagaggta cttgaactgt ttttggctct gacgttttcc acgtcagctg agtttctatt 3300

gctgtgactg ctgtgtgggt ggaccgatgg gccacactgc ttatgactcc cggcactggg 3360

tcgggatgat gaaccgcctg ctccacagaa ccccctactg tgaccggggt cacagctgag 3420

tgtgttcaag ttccttcaga gaaaacaaca cttacatatc aaaaggaaaa aaaaaaaagg 3480

aaatttctac aactctctaa catctgttct tctgaacatt tataatttca gtatccagca 3540

tgagctctaa gtcactcaga gttccataca gggtgttatt aagtaatcag aattaagttt 3600

ttgcaaagat gaaaagaaac ttatttttta ttgtaatttt atcacaaatc tatgtacact 3660

agggaattta ttgccagcat gtgaacatac ttccttcaga tgaaatacca acgattctcc 3720

tgagatcaaa tggccttccc acatcgaagg gcgggttcga ggcctcaaag gatagccgcc 3780

ttcgaaatgg ctcccatatt ccttgagctt ccaaataggc tgttccaatg accaaaagaa 3840

acagtgcact cattattcct gagatgatga tatacagagc cagttcccat cattattcct 3900

gagatgatga tatacagagc cagttcccag ttaggtctgg gtagggcttc tgcacaggtt 3960

gctaacatat ggtaaggaag ggatgcattc aatataaata caaactcaga gccactggtt 4020

gttataaact tcagttcccg aataactcta gaagctgtaa aatcaggagt aaacaatatg 4080

attatatctc tagaagcatt ggcacttaga gtaaactcta tattatatct ctagaagcat 4140

tggcacttag agtaaactct tgacaattaa caactttaaa gccatatcct tcacatgagt 4200

atccactgat ttcaatggtt tctatgtgaa tttgaagttg tcctgtattc tctaccttaa 4260

atgttctttt caatgtgaaa tttggttctc ttagttttaa actatctgta caatctttta 4320

acaatgcttc cgtgatttta aagcgtaagt ctgtacaatc ttttaacaat gcttccgtga 4380

ttttaaagcg taaggagctt cctggacctg gaagcttgcc tgccaccctc aagttctcag 4440

ttgttccttg tccttggacc atcacagcat ccatcacagt caggttattt ctgactatga 4500

taagtgaaga aacagttctg ttgtgaactg gagtaaacga tatgaagaaa cagttctgtt 4560

gtgaactgga gtaaacttta ctttgacaga tttcttttct ccaggtttta aaattaggtt 4620

taaaattaaa tgtcgagaga ggccctccat aaatcctgtt gaactctgca gtggatgagc 4680

actgtttctg aagacttgaa attctagtgt tctcaaatct atctttgctg tttctgaaga 4740

cttgaaattc tagtgttctc aaatctatct ttgccacctt actcaagtta aaccttgata 4800

ctaacttatc tacaaacact gaagggttgg aatataaaat actaacttat ctacaaacac 4860

tgaagggttg gaatataaag ccagaggaat aaactgaaca tagacaggaa catctgcagg 4920

attttctaaa gtaatctctt cttctgagga gcagtttgta ttagtaagtg gaaatttcaa 4980

gtgccggggt gagagtttgt attagtaagt ggaaatttca agtgccgggg tgagctaagt 5040

atggaaggcc aggagagctc agcagtgatt tttgatatta tatttttttg aaggtctgta 5100

tttacttcaa atatagcact caatctatga cctgaatttt cctttattcc tgtccatccc 5160

ttgaacaggc ctgaattttc ctttattcct gtccatccct tgaacaggct ttgatgcaaa 5220

tcccagtcag catcccacat atcttcctgc atggctacac caggctgcac tttgggttca 5280

gatttggata gaaaaggcaa gccaacatag caatgatccc caccggataa aaggcaagcc 5340

aacatagcaa tgatccccac actgtagtcc aggatcaaaa taaatgtttg caatctttga 5400

tttttttctg gctccaattt ccttattgcc ccgtaatctg atttttctgt ccaagtcttc 5460

cttattgccc cgtaatcgtt tatagtaaaa tcgcacatct tctgacaaag atcgtatttg 5520

ctgtattttt accttctgtg agaaggaatt cataatattt aaactttgat gaactatttt 5580

ccctggaaag gaaggtggaa gaaccacgtg cttagggaag caggtcagtg aggaaaggaa 5640

ggtggaagaa ccacgtgctt agggaagcag gtcagtgagc ctactgcaat cacagccttc 5700

acagggattg tcaggatctc atagtctgtt gtgatctgga tggctccatc atgaatcccc 5760

tcctcatagt ctgttgtgat ctggatggct ccatcatgaa tcccctctaa tttttttgca 5820

gtaagtttga ctctgaagac tgcaaaatag cctgaagcta atgttaccga tgattgatct 5880

gataaagagg atttttcaaa ctctggcagg ccgatgattg atctgataaa gaggattttt 5940

caaactctgg caggcttgaa attattgtag ttctattgcc tctttccaca gctacaagtt 6000

ctattgataa accgtctcct atgatatgcc aactttttat agccaactca attggattgc 6060

tgtttataat tgcaaataaa atattacttg cttcaattgg attgctgttt ataattgcaa 6120

ataaaatatt acttgcttct gtagcactca gtactccaaa atctatgaaa cgttcctcta 6180

ttttgggggg caatacaaag taatctaaaa agcctgtgta tacccgcacg ggtaaatgaa 6240

atttagtaaa aacccgcacg gtaaatgaaa tttagaagca ttggtaataa gtaaaatgtt 6300

gttatcaatg tgcatggatg atgtggaagg cataaaaagc agggtaaaaa tgtatcctga 6360

ttcattagga agaattaaga ctggtttgct gaagttgtga actttaaaca ttgttttggc 6420

ttcttctggt agcaacacat cgtgaatgag aacattgttt tggcttcttc tggtagcaac 6480

acatcgtgaa tgaggatcgc aaaactgaaa gtgttagtaa ggtaaattgg cctttccaca 6540

ggatcagcag ggctgtctcg gatgtgaaat aatgttgcag catgatcaaa tcccaaataa 6600

ccatctaaaa cttctgcttg atatggtatt tcaagtttag aactaaaact tctgcttgat 6660

atggtatttc aagtttagaa taactctttt cctttgcttt aactgttatt ttcccagaaa 6720

actgagatgg cttttttgcc ttcgatgcat caacttgttt actttctgat gcttttaatg 6780

taattggttt aaagtgtacc gttatagcat cattttgtgg tgtaggtcga acacttgtta 6840

ttggtacatc ttttgttcct gaatttaata aatgaataca tctttttcct gaatttaata 6900

aatgaaggtt taaaactttt ggtagatctt gtgttcttag tgtaccaaaa tctaacattt 6960

cagttgagga ttagtgtcca aaatctaaca tttcagttga ggaataaatt ccaggagctg 7020

ttgtaacttc aacctcaaca ggaagaatga taaactctgt gttgtaactt caacctcaac 7080

aggaagaatg ataaactctg tgctgtctga agcattagtc tttattctta tgaaggctgt 7140

gtgattatct gcttctctag atgaaaaact ggctctcatc actcccttgg tttcataagg 7200

aggaatttcc cacagttttc tggtacctcc ttgttgaccc gttgggagtt ctaggcacag 7260

ttttctggta cctccttgtt gacccgttgg gagttctagg tgaaggtctc ctccactaga 7320

gtacatttct acaacctgta aaggctcact gtgaggattg tggatgttta ttatagggta 7380

aaggctcact gtgaggattg tggatgttta ttataggtga gaaactgcta ttcacaggga 7440

ctctggcccc aaggaacggc ctcaatcgat atggatttgg aactccaaca ccaaatacct 7500

ggtaagtaaa accctataag aaatacccca tgattagatg tattaataaa taaagtattt 7560

tctacatttc ctactactct tgcaagaaaa actacatcaa atgatgtatt tcctcctgga 7620

agaattttcc tattttgaaa aaatgatgca tgaaaatgtg atgttgtagc agatactatt 7680

tgaaaaaatg atgcatgaaa atgtgatgtt gtagcagata ttgatactaa agtaatcgtt 7740

tcttcagaac taggattatg taagtagact ttttccattt ttggcattcc aactggttgt 7800

tcatggaaat ccagcattgg tggctcaaat cgtatgggcc tgcatggaaa tccagcattg 7860

gtggctcaaa tcgtatgggc ctgcaattcc cccggtagag agatatactt ttctgctgat 7920

atgaactgag tccaagtgtt gtctcggttt ctgctgatat gaactgagtc caagtgttgt 7980

ctcggtctgt agtagccctc catcatcaaa acgcagtact tctattaagc ctccatctca 8040

aaacgcagta cttctattat gctctctgac tgaacgaatg cttccttctc ggcccgcgcc 8100

g 8101

<210> 42
<211> 2838
<212> DNA
<213> Homo sapiens

<400> 42
   atttttaaga aatacacatt caatcttgtc tcaagacttg gctatgtgca tttcagttca 60
   tctttaaaat aagttcaggt atacaaatgt tacatacctc aagtacaaac agcacagaaa 120
   atgcatatgg tctcactgaa tgtttttaca ttcattcacg ttcttaagtt gacttacatt 180
   tctgtaatct gcttttaaac caagacagcc ttactttaaa aaaatactct attttcagca 240
   caaagtcctc atacagtttt aaaattagat cttggcgcat aatattgaga aattattaaa 300
   aaccaaactt ggtaatttaa caaaattgtc acatgccagg atttctgaat caactcaaat 360
   tatttcctta gctgtaatgt caaatctgtg ttcatacaga taaataaagc atggggaaga 420
   caggtggtga aaacgcagta acgggaaagg ttctcagatg tacaggtctt attagagttt 480
   gtggctgagt ccagactttt ctctaaaagc acaacagcaa atctcatgtt atcataattg 540
   caagaaagat ctgaaagaag cgagtggtct gagcctgccc tgcttgggtc tgttttgcaa 600
   agaagaggag ggtggggagg ggagctgcag taagagcctt aaaaagatgt ctcagaaact 660
   ggcacatcat gatctagacg agggcccact atgtttgttt gtttttcttt aatttaattc 720
   tcgtgaggaa agtgcgaatt agaccaaggg tccgagcttc ttcttccaat cgtggggctc 780
   catatccgcc acgggttgta ggtctgtccc aagtcgtcag ctggactgga ggtggaggga 840
   gcgtgaggag caggggagtt ttctgtgccc atgaggccga tgcttgccag cgtgtttgct 900
   ggagtggtga agggaagggc gctgctaagg ttgctggacc aaatggagct gctgaatgga 960
   gtggtggacc acaggccgct ggtgttaccg aggaccgatg tggctgtgtg ggtcggggag 1020
   ccggaactgg ctggccagga aggtggctgt gtgggtcggg gagccggaac tggctggcca 1080
   ggaaggactg ggatctgtcg ctggcgactc ccaaaggtat gaagggccac tattaaactc 1140
   gttccagctc ctctgtgagg cctgattgca cgatcgagat aatccgagtt tgctgaaaac 1200
   ttcaccagtt agattaaaag caccgttaga ttaaaagagt ttccaaaggc ggagaacgaa 1260
   ttgaggggag tgaagtcagg gctgcttggg ttgctgacgg gactccacaa acccgagctg 1320
   tctgagccat cgctgtcaac aggagcgtgt gaaactctat tgttgcttat ctggaaaagt 1380
   caaccaaggt gatctgcccc tgcaaacaaa ggcttaatgt atttgctcca gtcccccccc 1440
   catcttttag gtgagaacag gtgtaacgtg tgtctgcctg gctgagacag gccttctgga 1500
   cccctgggga aggcaccgca gccggctttc tgctccctct gggaaaaatg ccctgaaacc 1560
   catttatgga taaaagacct agtctgagcc atcgctgtca acaggagcgt gtgaaatgcc 1620
   aaggctgctg gagaaacctg ttttgtttga agaaacttta gcaaagccat tcccacctgg 1680
   gctcttgtcg tagccagccg tggcaaaagg ctgggctctt gtcgtagcca gccgtgactg 1740
   cagcaaaagt ggggttgccg ttcttgcccg ggagcgaggc tgcctttgtc aacttgtgtt 1800
   tgcttccatt tggctgtttt attttagggt cactgctgga gctgctgttg acgatgctgc 1860
   tgtagccccc ggctggagct gctgttgacg atgctgctgt agctgccccg ggccacaaag 1920
   gggcaggggg cagctggggg agacggggaa gcaggtgtcg gtgaggtctg gcgttgtttt 1980
   aagaaaatgt ctgcgttgag ggtttgcaga gaaagtttat aaagactatc agtagagctg 2040
   ccaggtttgt gaactggaac ggaatcccac tgccaggttt gtgaactgga acggaatccc 2100
   actccggtgg aggagagtct ttttcaccct ctgagctact ggtgttgcct aattcttgac 2160
   tattcgggag gaattttgct agggcaggtg gtctgttatc cactaactta cttgtacctt 2220
   ttgttttctg ggcatttcgt gatttggatc cacttgtcat tttgttttct gggcatttcg 2280
   tgatttggat ccacttgtca ttgcagttgg aagaggaatc ttgcttggga gatttctacg 2340
   ttgcttactt tccaaagggg gagtatatgg tagttctaat gaactgggtt tcacatctgt 2400
   tgggatttct ttcttaatat ttaagctggg tttcacatct gttgggattt ctttcttaat 2460
   atttaagagt tttttgcttt tttttagaca tttcactttc atgtttttca ggcatggctt 2520
   gttttccctt ttgcttttct gtatcctcct tgaggagcgg ttctgtgtca gggttggagg 2580
   tctctccttg aggagcggtt ctgtgtcagg gttggaggtc tctgtggtgg tggaggagct 2640
   atcatcatca gccaaagagt ccttcagctc atcttcctgt ggctttccct ttcccttctt 2700
   ctccttttcc tcttgcttct taggtggttt caccttgcgc tgaagaggtt ttcctttccc 2760
   tttgcttttt ggcaatggcg atggaggctg ctctgtaaag cacggtgtag gtgaggagct 2820
   ctcattcaat gtgaagat 2838

<210>  43
<211>  8499
<212>  DNA
<213>  Homo sapiens

<400>  43
   tttttaagaa atacacattc aatcttgtat ctcaagactt ggctatgtgc atttcagttc      60
   atctttaaaa taagttcagg tatacaaatg ttacatacct caagtacaaa cagcacagaa     120
   aatgcatatg gtctcactga atgtttttac attcattcac gttcttaagt tgacttacat     180
   ttctgtaatc tgcttttaaa ccaagacagc cttactttaa aaaaatactc tattttcagc     240
   acaaagtcct catacagttt taaaattaga tcttggcgca taatattgag aaattattaa     300
   aaaccaaact tggtaattta acaaaattgt cacatgccag gatttctgaa tcaactcaaa     360
   ttatttcctt agctgtaatg tcaaatctgt gttcatacag ataaataaag catggggaag     420
   acaggtggtg aaaacgcagt aacgggaaag gttctcagat gtacaggtct tattagagtt     480
   tgtggctgag tccagacttt tctctaaaag cacaacagca aatctcatgt tatcataatt     540
   gcaagaaaga tctgaaagaa gcgagtggtc tgagcctgcc ctgcttgggt ctgttttgca     600
   aagaagagga gggtggggag gggagctgca gtaagagcct taaaaagatg tctcagaaac     660
   tggcacatca tgatctagac gagggcccac tatgtttgtt tgttttttgc ttaatttaat     720
   tctcgtgagg aaagtgcgaa ttagaccaag ggtccgagct tcttcttcca atcgtggggc     780
   tccatatccg ccacgggttg taggtctgtc ccaagtcgtc agctggactg gaggtggagg     840
   gagcgtgagg agcaggggag ttttctgtgc ccatgaggcc gatgcttgcc agcgtgtttg     900
   ctggagtggt gaagggaagg gcgctgctaa ggttgctgga ccaaatggag ctgctgaatg     960
   gagtggtgga ccacaggccg ctggtgttac cgaggaccga tgtggctgtg tgggtcgggg    1020
   agccggaact ggctggccag gaaggatgtg tgggtcgggg agccggaact ggctggccag    1080
   gaaggactgg gatctgtcgc tggcgactcc caaaggtatg aagggccact attaaactcg    1140
   ttccagctcc tctgtgaggc ctgattgcac gatcgagata atccgagttt gctgaaaact    1200
   tcaccagtta gattaaaagt tccaaaggcg gaggggcccc agaacaacat aggtgctgct    1260
   gtgctgtgtt ctcccaccag ttagattaaa agagtttcca aaggcggaga acgaattgag    1320
   gggagtgaag tcagggctgc ttgggttgct gacggactcc cacaaacccg agctgtctga    1380
   gccatcgctg tcaacaggag cgtgtgaaat gtgaattagc ttcacaaaca ggagacactg    1440
   cttcaacggg gataaaaaca aaagaggaaa atggttccac aaggttgagt cggagggagc    1500
   cccagagctc cggatccctc tcacagccag gtcaaagcaa gtgttttgaa gaaacagaat    1560
   tttttctttt gaattattta aaaactattt caaatctaga actcatttcc tattagtgca    1620
   aagtaggtaa attggggctc tattgttgct tatctggaaa agtcaaccaa ggtgactgct    1680
   gcaggccacc acaccccctg catgaacaag agaatggcca gtgatttact ataaattggc 1740
   tagtatttgc tccagatttc ccccctccca agtcactttc tcttcttttt attaaacact 1800
   tccacagacc aggagatgga ggagaggaga acaggtgtaa cgtgtgtctg cctggctgag 1860
   acaggcatgg gtgccccctt tctctatctg gccctgggag ccgccgtttt tccccaattc 1920
   ttgtaatggg aaaaacaaac ccatttatga cctactcttg ttggggtctc cgcctgtctg 1980
   agccatcgct gtcaacagga gcgtgtgaaa tgccaaggct gctggagaaa cctgttttgt 2040
   ttgaagaaac tttagcaaag ccattcccac ctgggctctt gtcgtagcca gccgtgactg 2100
   cgcaaagggt cggctgggct cttgtcgtag ccagccgtga ctgcagcaaa agtggggttg 2160
   ccgttcttgc ccgggagcga ggctgccttt gtcaacttgt gtttgcttcc atttggctgt 2220
   tttattttag ggtcactgct ggagctgctg ttgacgatgc tgctgtagct gccccgggcc 2280
   acctggagct gctgttgacg atgctgctgt agctgccccg ggccacaaag gggcaggggg 2340
   cagctggggg agacggggaa gcaggtgtcg gtgaggtctg gcgttgtttt aagaaaatgt 2400
   ctgcgttgag ggtttgcaga gaaagtttat aaagactatc agtagagctg ccaggtttgt 2460
   gaactggaac ggaatcccac tccgggccag gtttgtgaac tggaacggaa tcccactccg 2520
   gtggaggaga gtctttttca ccctctgagc tactggtgtt gcctaattct tgactattcg 2580
   ggaggaattt tgctagggca ggtggtctgt tatccactaa cttacttgta ccttttgttt 2640
   tctgggcatt tcgtgatttg gatccacttg tcattttgtt ttctgggcat ttcgtgattt 2700
   ggatccactt gtcattgcag ttggaagagg aatcttgctt gggagatttc tacgttgctt 2760
   actttccaaa gggggagtat atggtagttc taatgaactg ggtttcacat ctgttgggat 2820
   ttctttctta atatttaaga gttctgggtt tcacatctgt tgggatttct ttcttaatat 2880
   ttaagagttt tttgcttttt tttttccttt catgtttttc aggcatggct tgttttccct 2940
   tttgcttttc tgtatcctcc ttgaggagcg gttctgtgtc agggttggag gtctctggtg 3000
   agacgcccta cctccttgag gagcggttct gtgtcagggt tggaggtctc tgtggtggtg 3060
   gaggagctat catcatcagc caaagagtcc ttcagctcat cttcctgtgg ctttcccttt 3120
   cccttcttct ccttttcctc ttgcttctta ggtggtttca ccttgcgctg aagaggtttt 3180
   cctttccctt tgctttttgg caatggcgat ggaggctgct ctgtaaacac ttcttgcttt 3240
   ttggcaatgg cgatggaggc tgctctgtaa acacttctag ggctggggag tcatggtggt 3300
   cgaagtcttt gtccatggct tctatgagac tggtgatgtc cgagtcctcg gaactgtgcc 3360
   tcgcgctgct ggcacgttct gggtgggaag ggtgtgcgag gggggcggga gacagccttt 3420
   caggctgcgc tcctggggct gaggcactgg cggtggcaga ggaggctgag ggtgctgctc 3480
   cagcgggctg tgggcatggt gctggctgcc gtgctggctc tgctttgccc ctttggactt 3540
   tctgcccgct gtatgacctt gagtcactgt gttcgaatct aagacaaggg ggcttgtttt 3600
   gttagcagac tgtgaagaag ctgcttgagc agaagtccta ctagaggtac ttgaactgtt 3660
   tttggctctg acgttttcca cgtcagctga gtttctattg ctgtgactgc tgtgtgggtg 3720
   gaccgatggg ccacactgct tatgactccc ggcactgggt cgggatgatg aaccgcctgc 3780
   tccacagaac cccctactgt gaccggggtc acagctgagt gtgttcaagt ttcctagaac 3840
   aatacattaa aaaaggaaaa aacaagaaat agggaaattt ctacaactct ctaacatctg 3900
   ttctatctga acatttataa tttaccatca gtgatctaac taaaagtatt ttaaccctta 3960
   ccaatataat aaaaacacag tatccagcat gagctctaag tcactcagag ttccatacag 4020
   ggtgttatta agtaatcaga attaagattt tttttcgcaa agatgaaaag aaacttattt 4080
   tttattgtaa ttttatcagc caaatctatg tacactaggg aatttattgc cagcatgtga 4140
   aaaccttcag atgaaatacc aacgattctc ctgagatcaa atggccttcc cacatcgaag 4200
   ggcgggttcg aggcctcaaa ggatagccgc cttcgaaatg gctcccatat tccttgagct 4260
   tccaaatagg ctgttccaat gaccaaaaga aacagtgcac tcattattcc tgagatgatg 4320
   atatacagag ccagttccca ttattcctga gatgatgata tacagagcca gttcccagtt 4380
   aggtctgggt agggcttctg cacaggttgc taacatatgg taaggaaggg atgcattcaa 4440
   tataaataca aactcagagc cactggttgt tataaacttc agttcccgaa taactctaga 4500
   agctgtaaaa tcaggagtaa acaatatgat tatatctcta gaagcattgg cacttagagt 4560
   aaactcttgt atctctagaa gcattggcac ttagagtaaa ctcttgacaa ttaacaactt 4620
   taaagccata tccttcacat gagtatccac tgatttcaat ggtttctatg tgaatttgaa 4680
   gttgtcctgt attctctacc ttaaatgttc ttttcaatgt gaaatttggt tctcttagtt 4740
   ttaaactatc tgtacaatct tttaacaatg cttccgtgat tttaaagcgt tacaatcttt 4800
   taacaatgct tccgtgattt taaagcgtaa ggagcttcct ggacctggaa gcttgcctgc 4860
   caccctcaag ttctcagttg ttccttgtcc ttggaccatc acagcatcca tcacagtcag 4920
   gttatttctg actatgataa gtgaagaaac agttctgttg tgaactggag taaactaagt 4980
   gaagaaacag ttctgttgtg aactggagta aactttactt tgacagattt cttttctcca 5040
   ggttttaaaa ttaggtttaa aattaaatgt cgagagaggc cctccataaa tcctgttgaa 5100
   ctctgcagtg gatgagcact gtttctgaag acttgaaatt ctagtgttct caaatctatc 5160
   tttgcgtttc tgaagacttg aaattctagt gttctcaaat ctatctttgc caccttactc 5220
   aagttaaacc ttgatactaa cttatctaca aacactgaag ggttggaata taaatgatac 5280
   taacttatct acaaacactg aagggttgga atataaagcc agaggaataa actgaacata 5340
   gacaggaaca tctgcaggat tttctaaagt aatctcttct tctgaggagc agtttgtatt 5400
   agtaagtgga aatttcaagt gccggggtga gcagtttgta ttagtaagtg gaaatttcaa 5460
   gtgccggggt gagctaagta tggaaggcca ggagagctca gcagtgattt ttgatattat 5520
   atttttttga aggtctgtat ttacttcaaa tatagcactc aatctatgac ctgaattttc 5580
   ctttattcct gtccatccct tgaacaggat gacctgaatt ttcctttatt cctgtccatc 5640
   ccttgaacag gctttgatgc aaatcccagt cagcatccca catatcttcc tgcatggcta 5700
   caccaggctg cactttgggt tcagatttgg atagaaaagg caagccaaca tagcaatgat 5760
   ccccacacta tagaaaaggc aagccaacat agcaatgatc cccacactgt agtccaggat 5820
   caaaataaat gtttgcaatc tttgattttt ttcctggctc caagtcttcc ttattgcccc 5880
   gtaatttttc ctggctccaa gtcttcctta ttgccccgta atcgtttata gtaaaatcgc 5940
   acatcttctg acaaagatcg tatttgctgt atttttacct tctgtgagaa ggaattcata 6000
   atatttaaac tttgatgaac tattttccct ggaaaggaag gtggaagaac cacgtgctta 6060
   gggaagcagg tcagtgagga aaggaaggtg gaagaaccac gtgcttaggg aagcaggtca 6120
   gtgagcctac tgcaatcaca gccttcacag ggattgtcag gatctcatag tctgttgtga 6180
   tctggatggc tccatcatga atccccatct gttgtgatct ggatggctcc atcatgaatc 6240
   ccctctaatt tttttgcagt aagtttgact ctgaagactg caaaatagcc tgaagctaat 6300
   gttaccgatg attgatctga taaagaggat tttcaaactc ctgggcgatg attgatctga 6360
   taaagaggat tttcaaactc ctggcaggct tgaaattatt gtagttctat tgcctctttc 6420
   cacagctaca agttctattg ataaaccgtc tcctatgata tgccaacttt ttatagccaa 6480
   ctcaattgga ttgctgttta taattgcaaa taaaatattc ttcagattgc tgtttataat 6540
   tgcaaataaa atattacttg cttctgtagc actcagtact ccaaaatcta tgaaacgttc 6600
   ctctattttg gggggcaata caaagtaatc taaaaagcct gtgtataccc gcacgggtaa 6660
   atgaaattta gaaacccgca cgggtaaatg aaatttagaa gcattggtaa taagtaaaat 6720
   gttgttatca atgtgcatgg atgatgtgga aggcataaaa agcagggtaa aaatgtatcc 6780
   tgattcatta ggaagaatta agactggttt gctgaagttg tgaactttaa acattgtttt 6840
   ggcttcttct ggtagcaaca catcgtgaat gacattgttt tggcttcttc tggtagcaac 6900
   acatcgtgaa tgaggatcgc aaaactgaaa gtgttagtaa ggtaaattgg cctttccaca 6960
   ggatcagcag ggctgtctcg gatgtgaaat aatgttgcag catgatcaaa tcccaaataa 7020
   ccatctaaaa cttctgcttg atatggtatt tcaagtttag aataacctaa aacttctgct 7080
   tgatatggta tttcaagttt agaataactc ttttcctttg ctttaactgt tattttccca 7140
   gaaaactgag atggcttttt tgctttactt tctgatgctt ttaatgtaat tggtttaaag 7200
   tgtaccgtta tagcatcatt ttgtggtgta ggtcgaacac ttgttattgg tacatctttt 7260
   gttcctgaat ttaataaatg aatggtcatc ttttgttcct gaatttaata aatgaaggtt 7320
   taaaactttt ggtagatctt gtgttcttag tgtaccaaaa tctaacattt cagttgagga 7380
   tgttcttagt gtaccaaaat ctaacatttc agttgaggaa taaattccag gagctgttgt 7440
   aacttcaacc tcaacaggaa gaatgataaa ctgtaacttc aacctcaaca ggaagaatga 7500
   taaactctgt gctgtctgaa gcattagtct ttattcttat gaaggctgtg tgattatctg 7560
   cttctctaga tgaaaaactg gctctcatca ctcccttggt ttcataagga ggaatttccc 7620
   acagttttct ggtacctcct tgttgacccg ttgggagttc taggcagttt tctggtacct 7680
   ccttgttgac ccgttgggag ttctaggtga aggtctcctc cactagagta catttctaca 7740
   acctgtaaag gctcactgtg aggattgtgg atgtttatta tagtaaaggc tcactgtgag 7800
   gattgtggat gtttattata ggtgagaaac tgctattcac agggactctg gccccaagga 7860
   acggcctcaa tcgatatgga tttggaactc caacaccaaa tacctggtaa gtaaataccc 7920
   catgattaga tgtattaagt aagtaaatac cccatgatta gatgtattaa taaataaagt 7980
   attttctaca tttcctacta ctcttgcaag aaaaactaca tcaaatgatg tatttcctcc 8040
   tggaagaatt ttcctatttt gaaaaaatga tgcatgaaaa tgtgatgttg tagttttaaa 8100
   aatgatgcat gaaaatgtga tgttgtagca gatattgata ctaaagtaat cgtttcttca 8160
   gaactaggat tatgtaagta gactttttcc atttttggca ttccaactgg ttgttcatgg 8220
   aaatccagca ttggtggctc aaatcgtatg ggcctgccag cattggtggc tcaaatcgta 8280
   tgggcctgca attcccccgg tagagagata tacttttctg ctgatatgaa ctgagtccaa 8340
   gtgttgtctc ggtttctgct gatatgaact gagtccaagt gttgtctcgg tctgtagtag 8400
   ccctccatca tcaaaacgca gtactcctcc atcatcaaaa cgcagtactt ctattatgct 8460
   ctctgactga acgaatgctt ccttctcggc ccgcgccgc 8499

<210> 44
<211> 6219
<212> DNA
<213> Homo sapiens

<400> 44
ctgggcccgg ggcgcgcagc tgcccgccgg ggcggttcag catcaacccc ctggagaacc 60

tgaaggtgta catcagcagt cggcctcccc tggtggtctt catgatcagc gtaagcgcca 120

tggccatagc tttcctgacc ctgggctact tcttcaaaat caaggagatt aaatccccag 180

aaatggcaga ggattggaat acttttctgc tacggttcaa tgatttggag attggaatac 240

ttttctgcta cggttcaatg atttggactt gtgtgtatca gagaatgaaa ccctcaagca 300

tctcacaaac gacaccacaa ctccggaaag tacaatgacc agcgggcagg cccgagcttc 360

cacccagtcc ccccaggccc tggaggactc gggcccggtg aatatctcag tctcaatcac 420

cctaaccctg gacccactga aacccttcgg agggtattcc cgcaacgtca cccatctgta 480

ctcaaccatc ttagggcatc agattggact ttcaggcagg gaagcccacg aggagataaa 540

catcaccttc accctgccag ggaagcccac gaggagataa acatcacctt caccctgcct 600

acagcgtgga gctcagatga ctgcgccctc cacggtcact gtgagcaggt ggtattcaca 660

gcctgcatga ccctcacggc cagccctggg gtgttccccg tcactgtaca gccaccgcac 720

tgtgttcctg acacgtacag caacgccacg ccaccgcact gtgttcctga cacgtacagc 780

aacgccacgc tctggtacaa gatcttcaca actgccagag atgccaacac aaaatacgcc 840

caagattaca atcctttctg gtgttataag ggggccattg gaaaagtcta tcatgcttta 900

aatcccaagc ttacagtgat tgttccagat gatgaccgtt cattaataaa tttgcatctc 960

atgcacacca tctttgggtg tcaaccgttg gtgtaggccc tgctgacttc cctgggtaag 1020

cgtatcctct gccactttag atttccaggt gagttaggac actaggcgga gtggtggatg 1080

cttggaaaaa catgttaccc attggttaag aaaaatctgg tttttctctt cattttctct 1140

gttgttcagc agcagttatt tcttgagcaa ctgcctaatc cctggggggg gttacaaggg 1200

atccttctat taatattatc ttagagggat gttcacagac gttcatagaa gactatctag 1260

ttagtgtccg tgtgaacaag aaatagtgct gcttagagtt ctgaaagata gaagggagta 1320

gtaggaggag atgaatcttc ggctagagag tgaggcctga ataggattta acttaaccaa 1380

gaggcaaggg gaacaaaata cttacctatc tcatctagta gctgtgtgct gctagctcag 1440

cttcctactt agacttgagc ccacttactt ttttagagac aggatcttgc tctgtcgccc 1500

agattggggt ctagtggcgc aatcatagct cactgcagct tcagcctcct gggctcaagc 1560

ttcccaagtg gctgggacta caggtgcatg tcaccatgcc gggataattt ttaaactttt 1620

tgtagagacg ggggtcttgc tgtgttgccc aggctggtct tgaacccctg gtctcaagcg 1680

atcctcctgc cttggcctcc aaagtttctg ggatgacagg tatgagccgc tgcacctggc 1740

ccaagcccac tttttaacac tgctttgata ttttataaat atgttccttt ctggcagtgt 1800

gtgttagctg gatctctctt cttttggcct ggccctttgc cctttctttt ttttctttat 1860

tttaattttt tttttttttt tttttgagat tgagtctttc tctgtcaccc aggctggagt 1920

gcagtggcac aatctcggtt cgttgcaacc tctgcctcct ggattgaagt gattctaagt 1980

agctgggatt acaggcacct gccaacacgc ccagctaatt tttgtatttt tggtagagat 2040

aaggtttcga catgttggcc aggttggtgt tgaactgctg acttcaggtg atcctcccac 2100

ctcagcctcc caaagtgctg ggattatagg catgagccac tatgcccgac ctttgccctt 2160

ttctggggct catttgtagg ccagggtcct gcaggagcca gctatggagg tcaggctgga 2220

tggtgggctg aggcccttag tgcctggcca agggcagtcc ctgcctgtgt ttggggcgtg 2280

ggtgttggta aagcagtgtg ttgagaagac tgagctgccc cgctatgtta taaatgacct 2340

gggggagccc agagctgtcc tgaggcctgg agatactaga gggtaaagga atggaaccat 2400

gtctggggtc cctttagtaa cttgcagggt ctgggaattg actgcatgtg ggaggtcaaa 2460

gggtagtgaa tcaggatgat ctcagcattt tcatccaagg acttagggaa atagagaaaa 2520

gcaggaggaa gggcgtgacc cctggggagg ggtgaggtgc cgaggccttc ctggtggggg 2580

catagtcagg agttaggaat gcagcctcgg ctgtcagcca tgcaacctga gggcagtctt 2640

gtgacgagga tgtgtccaga aaggagtggg gcttaaagat ggagtgtctg cagcatttgc 2700

aggggaggaa gcctgctgga ggggcagggt tcaggcagtg tgggggtgca gaaggcaggg 2760

cagaggacag cgctaggaag gcgtggtgct ctccaacaga ggctcggggc accatgtctt 2820

cacttaatga gacccagctt tggtcattta ggaggcaaaa ggagaaacct cctcttgcag 2880

cttaagaaga aaagttgtgg caagtctgga ggctgtcaat cctgtctctc aaaacccagc 2940

attaaaacct ttcagtcact cagagcagat gtttctgatt ttttttcttt ctttatttag 3000

gatgaccgtt cattaataaa tttgcatctc atgcacacca gttacttcct ctttgtgatg 3060

gtgataacaa tgttttgcta tgctgttatc aagggcagac ctagcaaatt gcgtcagagc 3120

aatcctgaat tttgtcccga gaaggtggct ttggctgaag cctaattcca cagctccttg 3180

ttttttggtg gctttggctg aagcctaatt ccacagctcc ttgttttttg agagagactg 3240

agagaaccat aatccttgcc tgctgaaccc agcctgggcc tggatgctct gtgaatacat 3300

tatcttgcga tgttgggtta ttccagccaa agacatttca agtgcctgta actgatttgt 3360

acatatttat aaaaatctat tcagaaattg gtccaataat gcacgtgctt tgccctgggt 3420

acagccagag cccttcaacc ccaccttgga cttgaggacc tacctgatgg gacgtttcca 3480

cgtgtctcta gagaaggatt cctggatcta gctggtcacg acgatgtttt caccaaggtc 3540

acaggagcat tgcgtcgctg atggggttga agtttggttt ggttcttgtt tcagcccaat 3600

atgtagagaa catttgaaac agtctgcacc tttgatacgg tattgcattt ccaaagccac 3660

caatccattt tgtggatttt atgtgtctgt ggcttaataa tcatagtaac aacaataata 3720

cctttttctc catttgcttt gcaggaaaca taccttaagt tttttttgtt ttgtttttgt 3780

ttttttgttt tttgttttcc tttatgaaga aaaaataaaa tagtcacatt ttaatactac 3840

tctagttagg acagacttgt gcttttatcc tgactaaaaa gttaaatatt taagtcacat 3900

acatggtaaa gatctaagtt tcattttcca catggatgac caggaacctg gtcttgttgg 3960

agctggcata ggaggttcta ctgtcttgtc gtccactctt gtccctccct tctgttatca 4020

aagttaaagc gcctcttcac tcaaaagggc ttcaaaatgt aaatggctct agagttagag 4080

tgactgtttg ctcttctgct ttctggaatg cagatgacag tatggaaaga aagaataatg 4140

attcatctaa taggaaagga tttcttcttt cttttttaaa gagtgggtgg attttctccc 4200

ttttgttttg cttttatttt catgctacta acatatcttc agaaggaaat ggcaactagt 4260

agatcagtaa aattacgaaa acgatgcctg caaaatttga aggaatgcaa ttctgatttg 4320

ccatttatca gggaaaactg aaagcatttt tcttataact tttttctttt ttcttttttt 4380

ttggaggggg gaggcagggt cttgctctgt ccctcaggct ggagcttagc tcagtgcagc 4440

ctccaactcc tgggctcaag caatcctccc actttagcct cctgagtagc tgggactaca 4500

ggcgtgtgcc agcgcgcctg gctaatttta aaattttttt gtagagacag agtctccctc 4560

tgttgcccag gctggtctgg aactcctagc ttcaaatgat cctctcacct cggcctccca 4620

aaatcctggg attccaggcg tcagccactg ctctcggccc tcttacacca ttttgtttga 4680

ttgtctagtc cctgtttctt tttctttcta atccttattc atttaagcaa aaccatacat 4740

tatcttttcc agtcctttct tgtattctta ctgttttttt aaaataactt tttgccttaa 4800

tagctcattg tcactaataa cttctgtgtt ctcccaggca gtgttataac agaagggcaa 4860

tgaaaacatt ggacatgacc atgagtgctt cctttcctta actcttgtag tctgttttaa 4920

attaggatta gttcctgcat tctgaaatac attggcctag aacattttat cccaggcatt 4980

tttgaataga aaacagttgc gtgcttttat tgaagattgt taaaataatc accatagtaa 5040

cttgatatgt tagtattttt ctttttcctt ttagtatgtt tcaagtgcag taatcgtact 5100

tgtctatcca gtttctatgt atgatacgag ttgcttacac gatgcatggc taacttgtta 5160

atttagtttt ctccttagag actttgagac ccttaagaga caagattgag aaagagccat 5220

gtggcttggc tctagaaacg tcattatcat taggaccatc agattttaga ttaagctgct 5280

attgaattaa taaaatccca atgaagcaga gttataggga tagatttata gctggcagag 5340

tggtatcaaa ggagaaaaac agtgaaaaag ccaatttcac tggttcgttc aatccagctt 5400

gttgctaata ttagttaccc ttgttttaat gacagagagt ggctggaatc tgtagctagg 5460

ggaggggcaa cactgttaga tgtgaggaaa ggaagtgcca aaaatgcctg gacagatggc 5520

ttgtcccaag gccaggacac acactttaaa atccaacatt cacctaagca agtaattctt 5580

aaagatctta cagaaacgca gagtcaatca ggtttataaa ggaaggctga ggggagagag 5640

gaggcctggg ggcctggacg aagaggccta ggacctgaag agactccagc gagtctcggg 5700

aagcccaggg gacctgtcac catctgggca cttcccaggc tcaatgctca gccagagttt 5760

gctggtggac ctcaggattc gaagtgtgca acagacggag tgcgcttgct gttcagggcg 5820

gccccgtgca ggagcaccag ctcaccctca ggcatctcgg actggagggc cagcagaagg 5880

tcagcaactt actcttaaaa cgattgtttc ggatcatatt gatctctttc cttttccttt 5940

ttgtcctttc actgtatgac ttgaatcagt tttgattcta tttgtaagtg tttcttgtcg 6000

tgaggcacct gccatgtctg ttgcgttttc ctcggcagcg tgtagggctt cagcttcctg 6060

ggttttcacc cttcctgcag gcactgtggg gtcctgaggg ccagtgggtc catttcacct 6120

cttgctgcat gatggtcagt agctgatctg ttatggcatt cacttccaga ttaattttcc 6180

gtgtttgaag tatgtgcata tgtgctttac agaataaaa                    6219

<210>  45
<211>  6286
<212>  DNA
<213>  Homo sapiens

<400>  45
ggcccggctg ggcccggggc gcgcagctgc ccgccggggc ggggtggagc tgatcagaat      60

aatgttcagc atcaaccccc tggagaaaat aatgttcagc atcaaccccc tggagaacct     120

gaaggtgtac atcagcagtc ggcctcccct ggtggtcttc atgatcagcg taagcgccat     180

ggccatagct ttcctgaccc tgggctactt cttcaaaatc aaggagatta aatccccaga     240

aatggcagag gattggaata cttttctgct acggttcaat gatttgggat tggaatactt     300

ttctgctacg gttcaatgat ttggacttgt gtgtatcaga gaatgaaacc ctcaagcatc     360

tcacaaacga caccacaact ccggaaagta caatgaccag cgggcaggcc cgagcttcca     420

cccagtcccc ccaggccctg gaggactcgg gcccggtgaa tatctcagtc tcaatcaccc     480

taaccctgga cccactgaaa cccttcggag ggtattcccg caacgtcacc catctgtact     540

caaccatctt agggcatcag attggacttt caggcaggga agcccacgag gagataaaca     600

tcaccttcac cctgcccagg gaagcccacg aggagataaa catcaccttc accctgccta     660

cagcgtggag ctcagatgac tgcgccctcc acggtcactg tgagcaggtg gtattcacag     720

cctgcatgac cctcacggcc agccctgggg tgttccccgt cactgtacag ccaccgcact     780

gtgttcctga cacgtacagc aacgccacgc tcagccaccg cactgtgttc ctgacacgta     840

cagcaacgcc acgctctggt acaagatctt cacaactgcc agagatgcca acacaaaata     900

cgcccaagat tacaatcctt tctggtgtta taagggggcc attggaaaag tctatcatgc     960

tttaaatccc aagcttacag tgattgttcc agatgatgac cgttcattaa taaatttgca    1020

tctcatgcac accatctttg ggtgtagttg gtgtaggccc tgctgacttc cctgggtaag    1080

cgtatcctct gccactttag atttccaggt gagttaggac actaggcgga gtggtggatg    1140

cttggaaaaa catgttaccc attggttaag aaaaatctgg tttttctctt cattttctct    1200

gttgttcagc agcagttatt tcttgagcaa ctgcctaatc cctggggggg gttacaaggg    1260

atccttctat taatattatc ttagagggat gttcacagac gttcatagaa gactatctag    1320

ttagtgtccg tgtgaacaag aaatagtgct gcttagagtt ctgaaagata gaagggagta     1380

gtaggaggag atgaatcttc ggctagagag tgaggcctga ataggattta acttaaccaa    1440

gaggcaaggg gaacaaaata cttacctatc tcatctagta gctgtgtgct gctagctcag    1500

cttcctactt agacttgagc ccacttactt ttttagagac aggatcttgc tctgtcgccc    1560

agattggggt ctagtggcgc aatcatagct cactgcagct tcagcctcct gggctcaagc    1620

ttcccaagtg gctgggacta caggtgcatg tcaccatgcc gggataattt ttaaactttt 1680

tgtagagacg ggggtcttgc tgtgttgccc aggctggtct tgaacccctg gtctcaagcg 1740

atcctcctgc cttggcctcc aaagtttctg ggatgacagg tatgagccgc tgcacctggc 1800

ccaagcccac tttttaacac tgctttgata ttttataaat atgttccttt ctggcagtgt 1860

gtgttagctg gatctctctt cttttggcct ggccctttgc ccttttcttt ttttctttat 1920

tttaattttt ttttattttt ttttttgaga ttgagtcttt ctctgtcacc caggctggag 1980

tgcagtggca caatctcggt tcgttgcaac ctctgcctcc tggattgaag tgattctaag 2040

tagctgggat tacaggcacc tgccaacacg cccagctaat tttgtatttt ttggtagaga 2100

taaggtttcg acatgttggc caggttggtg ttgaactgct gacttcaggt gatcctccca 2160

cctcagcctc ccaaagtgct gggattatag gcatgagcca ctatgcccga cctttgccct 2220

tttctggggc tcatttgtag gccagggtcc tgcaggagcc agctatggag gtcaggctgg 2280

atggtgggct gaggccctta gtgcctggcc aagggcagtc cctgcctgtg tttggggcgt 2340

gggtgttggt aaagcagtgt gttgagaaga ctgagctgcc ccgctatgtt ataaatgacc 2400

tgggggagcc cagagctgtc ctgaggcctg gagatactag agggtaaagg aatggaacca 2460

tgtctggggt ccctttagta acttgcaggg tctgggaatt gactgcatgt gggaggtcaa 2520

agggtagtga atcaggatga tctcagcatt ttcatccaag gacttaggga aatagagaaa 2580

agcaggagga agggcgtgac ccctggggag gggtgaggtg ccgaggcctt cctggtgggg 2640

gcatagtcag gagttaggaa tgcagcctcg gctgtcagcc atgcaacctg agggcagtct 2700

tgtgacgagg atgtgtccag aaaggagtgg ggcttaaaga tggagtgtct gcagcatttg 2760

caggggagga agcctgctgg aggggcaggg ttcaggcagt gtgggggtgc agaaggcagg 2820

gcagaggaca gcgctaggaa ggcgtggtgc tctccaacag aggctcggga gcaccatgtc 2880

ttcacttaat gagacccagc tttggtcatt taggaggcaa aaggagaaac ctcctcttgc 2940

agcttaagaa gaaaagttgt ggcaagtctg gaggctgtca atcctgtctc tcaaaaccca 3000

gcattaaaac ctttcagtca ctcagagcag atgtttctga ttttttttct cttctttatt 3060

taggatgacc gttcattaat aaatttgcat ctcatgcaca ccagttactt cctctttgtg 3120

atggtgataa caatgttttg ctatgctgtt atcaagggca gacctagcaa attgcgtcag 3180

agcaatcctg aattttgtcc cgagaaggtg gctttggctg aagcctaatt ccacagctcc 3240

ttgttttttg gtggctttgg ctgaagccta attccacagc tccttgtttt ttgagagaga 3300

ctgagagaac cataatcctt gcctgctgaa cccagcctgg gcctggatgc tctgtgaata 3360

cattatcttg cgatgttggg ttattccagc caaagacatt tcaagtgcct gtaactgatt 3420

tgtacatatt tataaaaatc tattcagaaa ttggtccaat aatgcacgtg ctttgccctg 3480

ggtacagcca gagcccttca accccacctt ggacttgagg acctacctga tgggacgttt 3540

ccacgtgtct ctagagaagg attcctggat ctagctggtc acgacgatgt tttcaccaag 3600

gtcacaggag cattgcgtcg ctgatggggt tgaagtttgg tttggttctt gtttcagccc 3660

aatatgtaga gaacatttga aacagtctgc acctttgata cggtattgca tttccaaagc 3720

caccaatcca ttttgtggat tttatgtgtc tgtggcttaa taatcatagt aacaacaata 3780

ataccttttt ctccattttg cttgcaggaa acatacctta agtttttttt gttttgtttt 3840

tgtttttttg ttttttgttt tcctttatga agaaaaaata aaatagtcac attttaatac 3900

tacttagtta ggacagactt gtgcttttat cctgactaaa aagttaaata tttaagtcac 3960

atacatggta aagatctaag tttcattttc cacatggatg accaggaacc tggtcttgtt 4020

ggagctggca taggaggttc tactgtcttg tcgtccactc ttgtccctcc cttctgttat 4080

caaagttaaa gcgcctcttc actcaaaagg gcttcaaaat gtaaatggct ctagagttag 4140

agtgactgtt tgctcttctg ctttctggaa tgcagatgac agtatggaaa gaaagaataa 4200

tgattcatct aataggaaag gatttcttct ttctttttta aagagtgggt ggattttctc 4260

ccttttgttt tgcttttatt ttcatgctac taacatatct tcagaaggaa atggcaacta 4320

gtagatcagt aaaattacga aaacgatgcc tgcaaaattt gaaggaatgc aattctgatt 4380

tgccatttat cagggaaaac tgaaagcatt tttcttataa ctttttttct ttttcttttt 4440

ttttggaggg gggaggcagg gtcttgctct gtccctcagg ctggagctta gctcagtgca 4500

gcctccaact cctgggctca agcaatcctc ccactttagc ctcctgagta gctgggacta 4560

caggcgtgtg ccagcgcgcc tggctaattt taaaattttt ttgtagagac agagtctccc 4620

tctgttgccc aggctggtct ggaactccta gcttcaaatg atcctctcac ctcggcctcc 4680

caaaatcctg ggattccagg cgtcagccac tgctctcggc cctcttacac cattttgttt 4740

gattgtctag tccctgtttc tttttctttc taatccttat tcatttaagc aaaaccatac 4800

attatctttt ccagtccttt cttgtattct tactgttttt ttaaaataac tttttgcctt 4860

aatagctcat tgtcactaat aacttctgtg ttctcccagg cagtgttata acagaagggc 4920

aatgaaaaca ttggacatga ccatgagtgc ttcctttcct taactcttta gtctgtttta 4980

aattaggatt agttcctgca ttctgaaata cattggccta gaacatttta tcccaggcat 5040

ttttgaatag aaaacagttg cgtgctttta ttgaagattg ttaaaataat caccatagta 5100

acttgatatg ttagtatttt tctttttcct tttagtatgt ttcaagtgca gtaatcgtac 5160

ttgtctatcc agtttctatg tatgatacga gttgcttaca cgatgcatgg ctaacttgtt 5220

aatttagttt tctccttaga gactttgaga cccttaagag acaagattga gaaagagcca 5280

tgtggcttgg ctctagaaac gtcattatca ttaggaccat cagattttag attaagctgc 5340

tattgaatta ataaaatccc aatgaagcag agttataggg atagatttat agctggcaga 5400

gtggtatcaa aggagaaaaa cagtgaaaaa gccaatttca ctggttcgtt caatccagct 5460

tgttgctaat attagttacc cttgttttaa tgacagagag tggctggaat ctgtagctag 5520

gggaggggca acactgttag atgtgaggaa aggaagtgcc aaaaatgcct ggacagatgg 5580

cttgtcccaa ggccaggaca cacactttaa aatccaacat tcacctaagc aagtaattct 5640

taaagatctt acagaaacgc agagtcaatc aggtttataa aggaaggctg aggggagaga 5700

ggaggcctgg gggcctggac gaagaggcct aggacctgaa gagactccag cgagtctcgg 5760

gaagcccagg ggacctgtca ccatctgggc acttcccagg ctcaatgctc agccagagtt 5820

tgctggtgga cctcaggatt cgaagtgtgc aacagacgga gtgcgcttgc tgttcagggc 5880

ggccccgtgc aggagcacca gctcaccctc aggcatctcg gactggaggg ccagcagaag 5940

gtcagcaact tactcttaaa acgattgttt cggatcatat tgatctcttt cctttttcct 6000

tttgtccttt cactgtatga cttgaatcag ttttgattct atttgtaagt gtttcttgtc 6060

gtgaggcacc tgccatgtct gttgcgtttt cctcggcagc gtgtagggct tcagcttcct 6120

gggttttcac ccttcctgca ggcactgtgg ggtcctgagg gccagtgggt ccatttcacc 6180

tcttgctgca tgatggtcag tagctgatct gttatggcat tcacttccag attaattttc 6240

cgtgtttgaa gtatgtgcat atgtgcttta cagaataaaa catctg 6286

<210> 46
<211> 21
<212> DNA
<213> Unknown

<220>
<223> artificial sequence

<400> 46
cacaggtgat tatgatttaa a 21

<210> 47
<211> 19
<212> DNA
<213> Unknown

<220>
<223> artificial sequence

<400> 47
cacattcagt caacattta 19